(19) Europäisches Patentamt / European Patent Office / Office européen des brevets

(11) EP 3 009 513 A1

(12) EUROPEAN PATENT APPLICATION

(43) Date of publication:
**20.04.2016 Bulletin 2016/16**

(21) Application number: **15180871.4**

(22) Date of filing: **02.02.2009**

(51) Int Cl.:
*C12N 15/85* (2006.01) *C12N 15/67* (2006.01)
*C07K 14/435* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **01.02.2008 US 63219 P**

(62) Document number(s) of the earlier application(s) in accordance with Art. 76 EPC:
**09709529.3 / 2 245 171**

(71) Applicant: **Chromocell Corporation**
**North Brunswick, NJ 08902 (US)**

(72) Inventor: **SHEKDAR, Kambiz**
**New York, NY 10010 (US)**

(74) Representative: **Vossius & Partner**
**Patentanwälte Rechtsanwälte mbB**
**Siebertstrasse 3**
**81675 München (DE)**

Remarks:
•Claims filed after the date of filing of the application (Rule 68(4) EPC).
•This application was filed on 13-08-2015 as a divisional application to the application mentioned under INID code 62.

# (54) NOVEL CELL LINES AND METHODS

(57) The invention relates to novel cells and cell lines, and methods for making and using them.

EP 3 009 513 A1

SEQUENCE LISTING

<110> CHROMOCELL CORPORATION

<120> NOVEL CELL LINES AND METHODS

<130> S2285 EP/1 S3

<140>
<141> 2009-02-02

<150> US 61/063,219
<151> 2008-02-01

<160> 27

<170> PatentIn version 3.5

<210> 1
<211> 1371
<212> DNA
<213> Homo sapiens

<400> 1
atgaggaaaa gtccaggtct gtctgactgt ctttgggcct ggatcctcct tctgagcaca 60

ctgactggaa gaagctatgg acagccgtca ttacaagatg aacttaaaga caataccact 120

gtcttcacca ggattttgga cagactccta gatggttatg acaatcgcct gagaccagga 180

ttgggagagc gtgtaaccga agtgaagact gatatcttcg tcaccagttt cggacccgtt 240

tcagaccatg atatggaata tacaatagat gtatttttcc gtcaaagctg gaaggatgaa 300

aggttaaaat ttaaaggacc tatgacagtc ctccggttaa ataacctaat ggcaagtaaa 360

atctggactc cggacacatt tttccacaat ggaaagaagt cagtggccca caacatgacc 420

atgcccaaca aactcctgcg gatcacagag gatggcacct tgctgtacac catgaggctg 480

acagtgagag ctgaatgtcc gatgcatttg gaggacttcc ctatggatgc ccatgcttgc 540

ccactaaaat ttggaagtta tgcttataca agagcagaag ttgtttatga atggaccaga 600

gagccagcac gctcagtggt tgtagcagaa gatggatcac gtctaaacca gtatgacctt 660

cttggacaaa cagtagactc tggaattgtc cagtcaagta caggagaata tgttgttatg 720

accactcatt tccacttgaa gagaaagatt ggctactttg ttattcaaac atacctgcca 780

tgcataatga cagtgattct ctcacaagtc tccttctggc tcaacagaga gtctgtacca 840

gcaagaactg tctttggagt aacaactgtg ctcaccatga caacattgag catcagtgcc 900

agaaactccc tccctaaggt ggcttatgca acagctatgg attggtttat tgccgtgtgc 960

tatgcctttg tgttctcagc tctgattgag tttgccacag taaactattt cactaagaga 1020

ggttatgcat gggatggcaa aagtgtggtt ccagaaaagc caaagaaagt aaaggatcct 1080

cttattaaga aaaacaacac ttacgctcca acagcaacca gctacacccc taatttggcc 1140

aggggcgacc cgggcttagc caccattgct aaaagtgcaa ccatagaacc taaagaggtc 1200

aagcccgaaa caaaaccacc agaacccaag aaaaccttta acagtgtcag caaaattgac 1260

cgactgtcaa gaatagcctt cccgctgcta tttggaatct ttaacttagt ctactgggct 1320

acgtatttaa acagagagcc tcagctaaaa gcccccacac cacatcaata g 1371

<210> 2
<211> 1356
<212> DNA
<213> Homo sapiens

<400> 2
atgaagacaa aattgaacat ctacaacatg cagttcctgc tttttgtttt cttggtgtgg 60

gaccctgcca ggttggtgct ggctaacatc caagaagatg aggctaaaaa taacattacc 120

atctttacga gaattcttga cagacttctg gatggttacg ataatcggct tagaccagga 180

ctgggagaca gtattactga agtcttcact aacatctacg tgaccagttt tggccctgtc 240

tcagatacag atatggaata tacaattgat gttttctttc gacaaaaatg gaaagatgaa 300

cgtttaaaat ttaaaggtcc tatgaatatc cttcgactaa acaatttaat ggctagcaaa 360

atctggactc cagatacctt ttttcacaat gggaaaaaat cagtagctca taatatgaca 420

atgccaaata agttgcttcg aattcaggat gatgggactc tgctgtatac catgaggctt 480

acagttcaag ctgaatgccc aatgcacttg gaggatttcc caatggatgc tcattcatgt 540

cctctgaaat ttggcagcta tgcatataca acttcagagg tcacttatat ttggacttac 600

aatgcatctg attcagtaca ggttgctcct gatggctcta ggttaaatca atatgacctg 660

ctgggccaat caatcggaaa ggagacaatt aaatccagta caggtgaata tactgtaatg 720

acagctcatt tccacctgaa aagaaaaatt gggtattttg tgattcaaac ctatctgcct 780

tgcatcatga ctgtcattct ctcccaagtt tcattctggc ttaacagaga atctgtgcct 840

gcaagaactg tgtttggagt aacaactgtc ctaacaatga caactctaag catcagtgct 900

cggaattctc tccccaaagt ggcttatgca actgccatgg actggtttat tgctgtttgt 960

tatgcatttg tgttctctgc cctaattgaa tttgcaactg ttaattactt caccaaaaga 1020

ggatggactt gggatgggaa gagtgtagta aatgacaaga aaaaagaaaa ggcttccgtt 1080

atgatacaga acaacgctta tgcagtggct gttgccaatt atgccccgaa tctttcaaaa 1140

gatccagttc tctccaccat ctccaagagt gcaaccacgc cagaacccaa caagaagcca 1200

gaaaacaagc cagctgaagc aaagaaaact ttcaacagtg ttagcaaaat tgacagaatg 1260

tccagaatag tttttccagt tttgtttggt acctttaatt tagtttactg ggctacatat 1320

ttaaacagag aacctgtatt aggggtcagt ccttga 1356

<210> 3
<211> 1479

<212> DNA
<213> Homo sapiens

<400> 3
atgataatca cacaaacaag tcactgttac atgaccagcc ttgggattct tttcctgatt 60

aatattctcc ctggaaccac tggtcaaggg gaatcaagac gacaagaacc cggggacttt 120

gtgaagcagg acattggcgg gctgtctcct aagcatgccc cagatattcc tgatgacagc 180

actgacaaca tcactatctt caccagaatc ttggatcgtc ttctggacgg ctatgacaac 240

cggctgcgac ctgggcttgg agatgcagtg actgaagtga agactgacat ctacgtgacc 300

agttttggcc ctgtgtcaga cactgacatg gagtacacta ttgatgtatt ttttcggcag 360

acatggcatg atgaaagact gaaatttgat ggccccatga agatccttcc actgaacaat 420

ctcctggcta gtaagatctg gacaccggac accttcttcc acaatggcaa gaaatcagtg 480

gctcataaca tgaccacgcc caacaagctg ctcagattgg tggacaacgg aaccctcctc 540

tatacaatga ggttaacaat tcatgctgag tgtcccatgc atttggaaga ttttcccatg 600

gatgtgcatg cctgcccact gaagtttgga agctatgcct atacaacagc tgaagtggtt 660

tattcttgga ctctcggaaa gaacaaatcc gtggaagtgg cacaggatgg ttctcgcttg 720

aaccagtatg accttttggg ccatgttgtt gggacagaga taatccggtc tagtacagga 780

gaatatgtcg tcatgacaac ccacttccat ctcaagcgaa aaattggcta ctttgtgatc 840

cagacctact tgccatgtat catgactgtc attctgtcac aagtgtcgtt ctggctcaac 900

agagagtctg ttcctgcccg tacagtcttt ggtgtcacca ctgtgcttac catgaccacc 960

ttgagtatca gtgccagaaa ttccttacct aaagtggcat atgcgacggc catggactgg 1020

ttcatagccg tctgttatgc ctttgtattt tctgcactga ttgaatttgc cactgtcaac 1080

tatttcacca agcggagttg ggcttgggaa ggcaagaagg tgccagaggc cctggagatg 1140

aagaagaaaa caccagcagc cccagcaaag aaaaccagca ctaccttcaa catcgtgggg 1200

accacctatc ccatcaacct ggccaaggac actgaatttt ccaccatctc caagggcgct 1260

gctcccagtg cctcctcaac cccaacaatc attgcttcac ccaaggccac ctacgtgcag 1320

gacagcccga ctgagaccaa gacctacaac agtgtcagca aggttgacaa aatttcccgc 1380

atcatctttc ctgtgctctt tgccatattc aatctggtct attgggccac atatgtcaac 1440

cgggagtcag ctatcaaggg catgatccgc aaacagtag 1479

<210> 4
<211> 1389
<212> DNA
<213> Homo sapiens

<400> 4
atggacaatg gaatgttctc tggttttatc atgatcaaaa acctccttct cttttgtatt 60

tccatgaact tatccagtca ctttggcttt tcacagatgc caaccagttc agtgaaagat 120

gagaccaatg acaacatcac gatatttacc aggatcttgg atgggctctt ggatggctac 180

gacaacagac ttcggcccgg gctgggagag cgcatcactc aggtgaggac cgacatctac 240

gtcaccagct tcggcccggt gtccgacacg gaaatggagt acaccataga cgtgtttttc 300

cgacaaagct ggaaagatga aaggcttcgg tttaaggggc ccatgcagcg cctccctctc 360

aacaacctcc ttgccagcaa gatctggacc ccagacacgt tcttccacaa cgggaagaag 420

tccatcgctc acaacatgac cacgcccaac aagctgctgc ggctggagga cgacggcacc 480

ctgctctaca ccatgcgctt gaccatctct gcagagtgcc ccatgcagct tgaggacttc 540

ccgatggatg cgcacgcttg ccctctgaaa tttggcagct atgcgtaccc taattctgaa 600

gtcgtctacg tctggaccaa cggctccacc aagtcggtgg tggtggcgga agatggctcc 660

agactgaacc agtaccacct gatggggcag acggtgggca ctgagaacat cagcaccagc 720

acaggcgaat acacaatcat gacagctcac ttccacctga aaaggaagat tggctacttt 780

gtcatccaga cctaccttcc ctgcataatg accgtgatct tatcacaggt gtccttttgg 840

ctgaaccggg aatcagtccc agccaggaca gtttttgggg tcaccacggt gctgaccatg 900

acgaccctca gcatcagcgc caggaactct ctgcccaaag tggcctacgc caccgccatg 960

gactggttca tagccgtgtg ctatgccttc gtcttctcgg cgctgataga gtttgccacg 1020

gtcaattact ttaccaagag aggctgggcc tgggatggca aaaaagcctt ggaagcagcc 1080

aagatcaaga aaaagcgtga agtcatacta aataagtcaa caaacgcttt tacaactggg 1140

aagatgtctc acccccccaaa cattccgaag gaacagaccc cagcagggac gtcgaataca 1200

acctcagtct cagtaaaacc ctctgaagag aagacttctg aaagcaaaaa gacttacaac 1260

agtatcagca aaattgacaa aatgtcccga atcgtattcc cagtcttgtt cggcactttc 1320

aacttagttt actgggcaac gtatttgaat agggagccgg tgataaaagg agccgcctct 1380

ccaaaataa 1389

<210> 5
<211> 1422
<212> DNA
<213> Homo sapiens

<400> 5
atgtggggcc ttgcgggagg aaggcttttc ggcatcttct cggccccggt gctggtggct 60

gtggtgtgct gcgcccagag tgtgaacgat cccgggaaca tgtcctttgt gaaggagacg 120

gtggacaagc tgttgaaagg ctacgacatt cgcctaagac ccgacttcgg gggtcccccg 180

gtctgcgtgg ggatgaacat cgacatcgcc agcatcgaca tggtttccga agtcaacatg 240

gattatacct taaccatgta ttttcaacaa tattggagag ataaaaggct cgcctattct 300

gggatccctc tcaacctcac gcttgacaat cgagtggctg accagctatg ggtgcccgac 360

acatatttct taaatgacaa aaagtcattt gtgcatggag tgacagtgaa aaaccgcatg 420

atccgtcttc accctgatgg gacagtgctg tatgggctca gaatcaccac gacagcagca 480

tgcatgatgg acctcaggag ataccccctg gacgagcaga actgcactct ggaaattgaa 540

agctatggct acaccacgga tgacattgag ttttactggc gaggcgggga caaggctgtt 600

accggagtgg aaaggattga gctcccgcag ttctccatcg tggagcaccg tctggtctcg 660

aggaatgttg tcttcgccac aggtgcctat cctcgactgt cactgagctt tcggttgaag 720

aggaacattg gatacttcat tcttcagact tatatgccct ctatactgat aacgattctg 780

tcgtgggtgt ccttctggat caattatgat gcatctgctg ctagagttgc cctcgggatc 840

acaactgtgc tgacaatgac aaccatcaac acccaccttc gggagacctt gcccaaaatc 900

ccctatgtca aagccattga catgtacctt atgggctgct tcgtctttgt gttcctggcc 960

cttctggagt atgcctttgt caactacatt ttctttggaa gaggccctca aaggcagaag 1020

aagcttgcag aaaagacagc caaggcaaag aatgaccgtt caaagagcga aagcaaccgg 1080

gtggatgctc atggaaatat tctgttgaca tcgctggaag ttcacaatga aatgaatgag 1140

gtctcaggcg gcattggcga taccaggaat tcagcaatat cctttgacaa ctcaggaatc 1200

cagtacagga aacagagcat gcctcgagaa gggcatgggc gattcctggg ggacagaagc 1260

ctcccgcaca agaagaccca tctacggagg aggtcttcac agctcaaaat taaaatacct 1320

gatctaaccg atgtgaatgc catagacaga tggtccagga tcgtgtttcc attcactttt 1380

tctcttttca acttagttta ctggctgtac tatgttaact ga 1422

<210> 6
<211> 1404
<212> DNA
<213> Homo sapiens

<400> 6
atgagttcgc caaatatatg gagcacagga agctcagtct actcgactcc tgtattttca 60

cagaaaatga cggtgtggat tctgctcctg ctgtcgctct accctggctt cactagccag 120

aaatctgatg atgactatga agattatgct tctaacaaaa catgggtctt gactccaaaa 180

gttcctgagg gtgatgtcac tgtcatctta aacaacctgc tggaaggata tgacaataaa 240

cttcggcctg atataggagt gaagccaacg ttaattcaca cagacatgta tgtgaatagc 300

attggtccag tgaacgctat caatatggaa tacactattg atatattttt tgcgcaaacg 360

tggtatgaca gacgtttgaa atttaacagc accattaaag tcctccgatt gaacagcaac 420

atggtgggga aaatctggat tccagacact ttcttcagaa attccaaaaa agctgatgca 480

cactggatca ccaccccaa caggatgctg agaatttgga atgatggtcg agtgctctac 540

accctaaggt tgacaattga tgctgagtgc caattacaat tgcacaactt tccaatggat 600

gaacactcct gccccttgga gttctcaagt tatggctatc cacgtgaaga aattgtttat 660

caatggaagc gaagttctgt tgaagtgggc gacacaagat cctggaggct ttatcaattc 720

tcatttgttg gtctaagaaa taccaccgaa gtagtgaaga caacttccgg agattatgtg 780

gtcatgtctg tctactttga tctgagcaga agaatgggat actttaccat ccagacctat 840

atcccctgca cactcattgt cgtcctatcc tgggtgtctt tctggatcaa taaggatgct 900

gttccagcca gaacatcttt aggtatcacc actgtcctga caatgaccac cctcagcacc 960

attgcccgga aatcgctccc caaggtctcc tatgtcacag cgatggatct ctttgtatct 1020

gtttgtttca tctttgtctt ctctgctctg gtggagtatg gcaccttgca ttattttgtc 1080

agcaaccgga aaccaagcaa ggacaaagat aaaaagaaga aaaaccctgc ccctaccatt 1140

gatatccgcc caagatcagc aaccattcaa atgaataatg ctacacacct tcaagagaga 1200

gatgaagagt acggctatga gtgtctggac ggcaaggact gtgccagttt tttctgctgt 1260

tttgaagatt gtcgaacagg agcttggaga catgggagga tacatatccg cattgccaaa 1320

atggactcct atgctcggat cttcttcccc actgccttct gcctgtttaa tctggtctat 1380

tgggtctcct acctctacct gtga 1404

<210> 7
<211> 25
<212> DNA
<213> Artificial Sequence

<220>
<221> source
<223> /note="Description of Artificial Sequence: Synthetic oligonucleotide"

<400> 7
gttcttaagg cacaggaact gggac 25

<210> 8
<211> 25
<212> DNA
<213> Artificial Sequence

<220>
<221> source
<223> /note="Description of Artificial Sequence: Synthetic oligonucleotide"

<400> 8
gaagttaacc ctgtcgttct gcgac 25

<210> 9
<211> 25
<212> DNA
<213> Artificial Sequence

<220>
<221> source
<223> /note="Description of Artificial Sequence: Synthetic oligonucleotide"

<400> 9
gttctatagg gtctgcttgt cgctc 25

<210> 10
<211> 34
<212> DNA
<213> Artificial Sequence

<220>
<221> source
<223> /note="Description of Artificial Sequence: Synthetic probe"

<400> 10
gccagtccca gttcctgtgc cttaagaacc tcgc 34

<210> 11
<211> 34
<212> DNA
<213> Artificial Sequence

<220>
<221> source
<223> /note="Description of Artificial Sequence: Synthetic probe"

<400> 11
gcgagtcgca gaacgacagg gttaacttcc tcgc 34

<210> 12
<211> 34
<212> DNA
<213> Artificial Sequence

<220>
<221> source
<223> /note="Description of Artificial Sequence: Synthetic probe"

<400> 12
gcgagagcga caagcagacc ctatagaacc tcgc 34

<210> 13
<211> 25
<212> DNA
<213> Artificial Sequence

<220>
<221> source
<223> /note="Description of Artificial Sequence: Synthetic oligonucleotide"

<400> 13
gttcttaagg cacaggaact gggac 25

<210> 14
<211> 34
<212> DNA
<213> Artificial Sequence

<220>
<221> source
<223> /note="Description of Artificial Sequence: Synthetic probe"

<400> 14
gccagtccca gttcctgtgc cttaagaacc tcgc 34

<210> 15
<211> 3222
<212> DNA
<213> Homo sapiens

<400> 15
atgaagacgt tgctgttgga cttggctttg tggtcactgc tcttccagcc cgggtggctg 60

tcctttagtt cccaggtgag tcagaactgc cacaatggca gctatgaaat cagcgtcctg 120

atgatgggca actcagcctt tgcagagccc ctgaaaaact tggaagatgc ggtgaatgag 180

gggctggaaa tagtgagagg acgtctgcaa aatgctggcc taaatgtgac tgtgaacgct 240

actttcatgt attcggatgg tctgattcat aactcaggcg actgccggag tagcacctgt 300

gaaggcctcg acctactcag gaaaatttca aatgcacaac ggatgggctg tgtcctcata 360

gggccctcat gtacatactc caccttccag atgtaccttg acacagaatt gagctacccc 420

atgatctcag ctggaagttt tggattgtca tgtgactata aagaaacctt aaccaggctg 480

atgtctccag ctagaaagtt gatgtacttc ttggttaact tttggaaaac caacgatctg 540

cccttcaaaa cttattcctg gagcacttcg tatgtttaca agaatggtac agaaactgag 600

gactgtttct ggtaccttaa tgctctggag gctagcgttt cctatttctc ccacgaactc 660

ggctttaagg tggtgttaag acaagataag gagtttcagg atatcttaat ggaccacaac 720

aggaaaagca atgtgattat tatgtgtggt ggtccagagt tcctctacaa gctgaagggt 780

gaccgagcag tggctgaaga cattgtcatt attctagtgg atcttttcaa tgaccagtac 840

ttggaggaca atgtcacagc ccctgactat atgaaaaatg tccttgttct gacgctgtct 900

cctgggaatt cccttctaaa tagctctttc tccaggaatc tatcaccaac aaaacgagac 960

tttgctcttg cctatttgaa tggaatcctg ctctttggac atatgctgaa gatatttctt 1020

gaaaatggag aaaatattac cacccccaaa tttgctcatg ctttcaggaa tctcactttt 1080

gaagggtatg acggtccagt gaccttggat gactgggggg atgttgacag taccatggtg 1140

cttctgtata cctctgtgga caccaagaaa tacaaggttc ttttgaccta tgatacccac 1200

gtaaataaga cctatcctgt ggatatgagc ccccacattca cttggaagaa ctctaaactt 1260

cctaatgata ttacaggccg ggccctcag atcctgatga ttgcagtctt caccctcact 1320

ggagctgtgg tgctgctcct gctcgtcgct ctcctgatgc tcagaaaata tagaaaagat 1380
tatgaacttc gtcagaaaaa atggtcccac attcctcctg aaaatatctt tcctctggag 1440
accaatgaga ccaatcatgt tagcctcaag atcgatgatg acaaaagacg agatacaatc 1500
cagagactac gacagtgcaa atacgacaaa aagcgagtga ttctcaaaga tctcaagcac 1560
aatgatggta atttcactga aaaacagaag atagaattga acaagttgct tcagattgac 1620
tattacaacc tgaccaagtt ctacggcaca gtgaaacttg ataccatgat cttcggggtg 1680
atagaatact gtgagagagg atccctccgg gaagttttaa atgacacaat ttcctaccct 1740
gatggcacat tcatggattg ggagtttaag atctctgtct tgtatgacat tgctaaggga 1800
atgtcatatc tgcactccag taagacagaa gtccatggtc gtctgaaatc taccaactgc 1860
gtagtggaca gtagaatggt ggtgaagatc actgattttg gctgcaattc cattttacct 1920
ccaaaaaagg acctgtggac agctccagag cacctccgcc aagccaacat ctctcagaaa 1980
ggagatgtgt acagctatgg gatcatcgca caggagatca ttctgcggaa agaaaccttc 2040
tacactttga gctgtcggga ccggaatgag aagattttca gagtggaaaa ttccaatgga 2100
atgaaaccct tccgcccaga tttattcttg gaaacagcag aggaaaaaga gctagaagtg 2160
tacctacttg taaaaaactg ttgggaggaa gatccagaaa agagaccaga tttcaaaaaa 2220
attgagacta cacttgccaa gatatttgga ctttttcatg accaaaaaaa tgaaagctat 2280
atggatacct tgatccgacg tctacagcta tattctcgaa acctggaaca tctggtagag 2340
gaaaggacac agctgtacaa ggcagagagg gacagggctg acagacttaa ctttatgttg 2400
cttccaaggc tagtggtaaa gtctctgaag gagaaaggct ttgtggagcc ggaactatat 2460
gaggaagtta caatctactt cagtgacatt gtaggtttca ctactatctg caaatacagc 2520
acccccatgg aagtggtgga catgcttaat gacatctata agagttttga ccacattgtt 2580
gatcatcatg atgtctacaa ggtggaaacc atcggtgatg cgtacatggt ggctagtggt 2640
ttgcctaaga gaaatggcaa tcggcatgca atagacattg ccaagatggc cttggaaatc 2700
ctcagcttca tggggacctt tgagctggag catcttcctg gcctcccaat atggattcgc 2760
attggagttc actctggtcc ctgtgctgct ggagttgtgg gaatcaagat gcctcgttat 2820
tgtctatttg gagatacggt caacacagcc tctaggatgg aatccactgg cctccctttg 2880
agaattcacg tgagtggctc caccatagcc atcctgaaga gaactgagtg ccagttcctt 2940
tatgaagtga gaggagaaac atacttaaag ggaagaggaa atgagactac ctactggctg 3000
actgggatga aggaccagaa attcaacctg ccaacccctc ctactgtgga gaatcaacag 3060
cgtttgcaag cagaattttc agacatgatt gccaactctt tacagaaaag acaggcagca 3120
gggataagaa gccaaaaacc cagacgggta gccagctata aaaaaggcac tctggaatac 3180
ttgcagctga ataccacaga caaggagagc acctattttt aa 3222

<210> 16
<211> 4443
<212> DNA
<213> Homo sapiens

<400> 16
atgcagaggt cgcctctgga aaaggccagc gttgtctcca aacttttttt cagctggacc 60

agaccaattt tgaggaaagg atacagacag cgcctggaat tgtcagacat ataccaaatc 120

ccttctgttg attctgctga caatctatct gaaaaattgg aaagagaatg ggatagagag 180

ctggcttcaa agaaaaatcc taaactcatt aatgcccttc ggcgatgttt tttctggaga 240

tttatgttct atggaatctt tttatattta ggggaagtca ccaaagcagt acagcctctc 300

ttactgggaa gaatcatagc ttcctatgac ccggataaca aggaggaacg ctctatcgcg 360

atttatctag gcataggctt atgccttctc tttattgtga ggacactgct cctacaccca 420

gccatttttg gccttcatca cattggaatg cagatgagaa tagctatgtt tagtttgatt 480

tataagaaga ctttaaagct gtcaagccgt gttctagata aaataagtat tggacaactt 540

gttagtctcc tttccaacaa cctgaacaaa tttgatgaag gacttgcatt ggcacatttc 600

gtgtggatcg ctcctttgca agtggcactc ctcatggggc taatctggga gttgttacag 660

gcgtctgcct tctgtggact tggtttcctg atagtccttg ccctttttca ggctgggcta 720

gggagaatga tgatgaagta cagagatcag agagctggga agatcagtga aagacttgtg 780

attacctcag aaatgattga aaatatccaa tctgttaagg catactgctg ggaagaagca 840

atggaaaaaa tgattgaaaa cttaagacaa acagaactga aactgactcg gaaggcagcc 900

tatgtgagat acttcaatag ctcagccttc ttcttctcag ggttctttgt ggtgttttta 960

tctgtgcttc cctatgcact aatcaaagga atcatcctcc ggaaaatatt caccaccatc 1020

tcattctgca ttgttctgcg catggcggtc actcggcaat ttccctgggc tgtacaaaca 1080

tggtatgact ctcttggagc aataaacaaa atacaggatt tcttacaaaa gcaagaatat 1140

aagacattgg aatataactt aacgactaca gaagtagtga tggagaatgt aacagccttc 1200

tgggaggagg gatttgggga attatttgag aaagcaaaac aaaacaataa caatagaaaa 1260

acttctaatg gtgatgacag cctcttcttc agtaatttct cacttcttgg tactcctgtc 1320

ctgaaagata ttaatttcaa gatagaaaga ggacagttgt tggcggttgc tggatccact 1380

ggagcaggca agacttcact tctaatggtg attatgggag aactggagcc ttcagagggt 1440

aaaattaagc acagtggaag aatttcattc tgttctcagt tttcctggat tatgcctggc 1500

accattaaag aaaatatcat ctttggtgtt tcctatgatg aatatagata cagaagcgtc 1560

atcaaagcat gccaactaga agaggacatc tccaagtttg cagagaaaga caatatagtt 1620

cttggagaag gtggaatcac actgagtgga ggtcaacgag caagaatttc tttagcaaga 1680

gcagtataca aagatgctga tttgtattta ttagactctc cttttggata cctagatgtt 1740

ttaacagaaa aagaaatatt tgaaagctgt gtctgtaaac tgatggctaa caaaactagg 1800

attttggtca cttctaaaat ggaacattta aagaaagctg acaaaatatt aattttgcat 1860

gaaggtagca gctattttta tgggacattt tcagaactcc aaaatctaca gccagacttt 1920

agctcaaaac tcatgggatg tgattctttc gaccaattta gtgcagaaag aagaaattca 1980

atcctaactg agaccttaca ccgtttctca ttagaaggag atgctcctgt ctcctggaca 2040

gaaacaaaaa aacaatcttt taaacagact ggagagtttg gggaaaaaag gaagaattct 2100

attctcaatc caatcaactc tatacgaaaa ttttccattg tgcaaaagac tcccttacaa 2160

atgaatggca tcgaagagga ttctgatgag cctttagaga gaaggctgtc cttagtacca 2220

gattctgagc agggagaggc gatactgcct cgcatcagcg tgatcagcac tggccccacg 2280

cttcaggcac gaaggaggca gtctgtcctg aacctgatga cacactcagt taaccaaggt 2340

cagaacattc accgaaagac aacagcatcc acacgaaaag tgtcactggc ccctcaggca 2400

aacttgactg aactggatat atattcaaga aggttatctc aagaaactgg cttggaaata 2460

agtgaagaaa ttaacgaaga agacttaaag gagtgctttt ttgatgatat ggagagcata 2520

ccagcagtga ctacatggaa cacatacctt cgatatatta ctgtccacaa gagcttaatt 2580

tttgtgctaa tttggtgctt agtaattttt ctggcagagg tggctgcttc tttggttgtg 2640

ctgtggctcc ttggaaacac tcctcttcaa gacaaaggga atagtactca tagtagaaat 2700

aacagctatg cagtgattat caccagcacc agttcgtatt atgtgtttta catttacgtg 2760

ggagtagccg acactttgct tgctatggga ttcttcagag gtctaccact ggtgcatact 2820

ctaatcacag tgtcgaaaat tttacaccac aaaatgttac attctgttct tcaagcacct 2880

atgtcaaccc tcaacacgtt gaaagcaggt gggattctta atagattctc caaagatata 2940

gcaattttgg atgaccttct gcctcttacc atatttgact tcatccagtt gttattaatt 3000

gtgattggag ctatagcagt tgtcgcagtt ttacaaccct acatctttgt tgcaacagtg 3060

ccagtgatag tggcttttat tatgttgaga gcatatttcc tccaaacctc acagcaactc 3120

aaacaactgg aatctgaagg caggagtcca attttcactc atcttgttac aagcttaaaa 3180

ggactatgga cacttcgtgc cttcggacgg cagccttact ttgaaactct gttccacaaa 3240

gctctgaatt tacatactgc caactggttc ttgtacctgt caacactgcg ctggttccaa 3300

atgagaatag aaatgatttt tgtcatcttc ttcattgctg ttaccttcat ttccatttta 3360

acaacaggag aaggagaagg aagagttggt attatcctga ctttagccat gaatatcatg 3420

agtacattgc agtgggctgt aaactccagc atagatgtgg atagcttgat gcgatctgtg 3480

agccgagtct ttaagttcat tgacatgcca acagaaggta aacctaccaa gtcaaccaaa 3540

ccatacaaga atggccaact ctcgaaagtt atgattattg agaattcaca cgtgaagaaa 3600

gatgacatct ggccctcagg gggccaaatg actgtcaaag atctcacagc aaaatacaca 3660

gaaggtggaa atgccatatt agagaacatt tccttctcaa taagtcctgg ccagagggtg 3720

ggcctcttgg gaagaactgg atcagggaag agtactttgt tatcagcttt tttgagacta 3780

ctgaacactg aaggagaaat ccagatcgat ggtgtgtctt gggattcaat aactttgcaa 3840

cagtggagga aagcctttgg agtgatacca cagaaagtat ttattttttc tggaacattt 3900

agaaaaaact tggatcccta tgaacagtgg agtgatcaag aaatatggaa agttgcagat 3960

gaggttgggc tcagatctgt gatagaacag tttcctggga agcttgactt tgtccttgtg 4020

gatgggggct gtgtcctaag ccatggccac aagcagttga tgtgcttggc tagatctgtt 4080

ctcagtaagg cgaagatctt gctgcttgat gaacccagtg ctcatttgga tccagtaaca 4140

taccaaataa ttagaagaac tctaaaacaa gcatttgctg attgcacagt aattctctgt 4200

gaacacagga tagaagcaat gctggaatgc caacaatttt tggtcataga agagaacaaa 4260

gtgcggcagt acgattccat ccagaaactg ctgaacgaga ggagcctctt ccggcaagcc 4320

atcagcccct ccgacagggt gaagctcttt ccccaccgga actcaagcaa gtgcaagtct 4380

aagccccaga ttgctgctct gaaagaggag acagaagaag aggtgcaaga tacaaggctt 4440

tga 4443

<210> 17
<211> 25
<212> DNA
<213> Artificial Sequence

<220>
<221> source
<223> /note="Description of Artificial Sequence: Synthetic oligonucleotide"

<400> 17
gttcttaagg cacaggaact gggac 25

<210> 18
<211> 34
<212> DNA
<213> Artificial Sequence

<220>
<221> source
<223> /note="Description of Artificial Sequence: Synthetic probe"

<400> 18
gccagtccca gttcctgtgc cttaagaacc tcgc 34

<210> 19
<211> 5934
<212> DNA

<213> Homo sapiens

<400> 19
atggcaatgt tgcctccccc aggacctcag agctttgtcc atttcacaaa acagtctctt 60
gccctcattg aacaacgcat tgctgaaaga aaatcaaagg aacccaaaga agaaaagaaa 120
gatgatgatg aagaagcccc aaagccaagc agtgacttgg aagctggcaa acaactgccc 180
ttcatctatg gggacattcc tcccggcatg gtgtcagagc ccctggagga cttggacccc 240
tactatgcag acaaaaagac tttcatagta ttgaacaaag ggaaaacaat cttccgtttc 300
aatgccacac ctgctttata tatgctttct cctttcagtc ctctaagaag aatatctatt 360
aagattttag tacactcctt attcagcatg ctcatcatgt gcactattct gacaaactgc 420
atatttatga ccatgaataa cccgccggac tggaccaaaa atgtcgagta cacttttact 480
ggaatatata cttttgaatc acttgtaaaa atccttgcaa gaggcttctg tgtaggagaa 540
ttcacttttc ttcgtgaccc gtggaactgg ctggattttg tcgtcattgt ttttgcgtat 600
ttaacagaat ttgtaaacct aggcaatgtt tcagctcttc gaactttcag agtattgaga 660
gctttgaaaa ctatttctgt aatcccaggc ctgaagacaa ttgtaggggc tttgatccag 720
tcagtgaaga agctttctga tgtcatgatc ctgactgtgt tctgtctgag tgtgtttgca 780
ctaattggac tacagctgtt catgggaaac ctgaagcata aatgttttcg aaattcactt 840
gaaaataatg aaacattaga aagcataatg aataccctag agagtgaaga agactttaga 900
aaatattttt attacttgga aggatccaaa gatgctctcc tttgtggttt cagcacagat 960
tcaggtcagt gtccagaggg gtacacctgt gtgaaaattg gcagaaaccc tgattatggc 1020
tacacgagct ttgacacttt cagctgggcc ttcttagcct tgtttaggct aatgacccaa 1080
gattactggg aaaaccttta ccaacagacg ctgcgtgctg ctggcaaaac ctacatgatc 1140
ttctttgtcg tagtgatttt cctgggctcc ttttatctaa taaacttgat cctggctgtg 1200
gttgccatgg catatgaaga acagaaccag gcaaacattg aagaagctaa acagaaagaa 1260
ttagaatttc aacagatgtt agaccgtctt aaaaaagagc aagaagaagc tgaggcaatt 1320
gcagcggcag cggctgaata tacaagtatt aggagaagca gaattatggg cctctcagag 1380
agttcttctg aaacatccaa actgagctct aaaagtgcta aagaaagaag aaacagaaga 1440
aagaaaaaga atcaaaagaa gctctccagt ggagaggaaa agggagatgc tgagaaattg 1500
tcgaaatcag aatcagagga cagcatcaga agaaaaagtt tccaccttgg tgtcgaaggg 1560
cataggcgag cacatgaaaa gaggttgtct acccccaatc agtcaccact cagcattcgt 1620
ggctccttgt tttctgcaag gcgaagcagc agaacaagtc tttttagttt caaaggcaga 1680
ggaagagata taggatctga gactgaattt gccgatgatg agcacagcat tttggagac 1740
aatgagagca gaaggggctc actgtttgtg ccccacagac cccaggagcg acgcagcagt 1800

aacatcagcc aagccagtag gtccccacca atgctgccgg tgaacgggaa aatgcacagt 1860

gctgtggact gcaacggtgt ggtctccctg gttgatggac gctcagccct catgctcccc 1920

aatggacagc ttctgccaga gggcacgacc aatcaaatac acaagaaaag gcgttgtagt 1980

tcctatctcc tttcagagga tatgctgaat gatcccaacc tcagacagag agcaatgagt 2040

agagcaagca tattaacaaa cactgtggaa gaacttgaag agtccagaca aaaatgtcca 2100

ccttggtggt acagatttgc acacaaattc ttgatctgga attgctctcc atattggata 2160

aaattcaaaa agtgtatcta ttttattgta atggatcctt ttgtagatct tgcaattacc 2220

atttgcatag ttttaaacac attatttatg gctatggaac accacccaat gactgaggaa 2280

ttcaaaaatg tacttgctat aggaaatttg gtctttactg gaatctttgc agctgaaatg 2340

gtattaaaac tgattgccat ggatccatat gagtatttcc aagtaggctg gaatattttt 2400

gacagcctta ttgtgacttt aagtttagtg gagctctttc tagcagatgt ggaaggattg 2460

tcagttctgc gatcattcag actgctccga gtcttcaagt tggcaaaatc ctggccaaca 2520

ttgaacatgc tgattaagat cattggtaac tcagtagggg ctctaggtaa cctcacctta 2580

gtgttggcca tcatcgtctt catttttgct gtggtcggca tgcagctctt tggtaagagc 2640

tacaaagaat gtgtctgcaa gatcaatgat gactgtacgc tcccacggtg gcacatgaac 2700

gacttcttcc actccttcct gattgtgttc cgcgtgctgt gtggagagtg gatagagacc 2760

atgtgggact gtatggaggt cgctggtcaa gctatgtgcc ttattgttta catgatggtc 2820

atggtcattg gaaacctggt ggtcctaaac ctatttctgg ccttattatt gagctcattt 2880

agttcagaca atcttacagc aattgaagaa gaccctgatg caaacaacct ccagattgca 2940

gtgactagaa ttaaaaaggg aataaattat gtgaaacaaa ccttacgtga atttattcta 3000

aaagcatttt ccaaaaagcc aaagatttcc agggagataa gacaagcaga agatctgaat 3060

actaagaagg aaaactatat ttctaaccat acacttgctg aaatgagcaa aggtcacaat 3120

ttcctcaagg aaaaagataa aatcagtggt tttggaagca gcgtggacaa acacttgatg 3180

gaagacagtg atggtcaatc atttattcac aatcccagcc tcacagtgac agtgccaatt 3240

gcacctgggg aatccgattt ggaaaatatg aatgctgagg aacttagcag tgattcggat 3300

agtgaataca gcaaagtgag attaaaccgg tcaagctcct cagagtgcag cacagttgat 3360

aacccctttgc ctggagaagg agaagaagca gaggctgaac ctatgaattc cgatgagcca 3420

gaggcctgtt tcacagatgg ttgtgtacgg aggttctcat gctgccaagt taacatagag 3480

tcagggaaag gaaaaatctg gtggaacatc aggaaaacct gctacaagat tgttgaacac 3540

agttggtttg aaagcttcat tgtcctcatg atcctgctca gcagtggtgc cctggctttt 3600

gaagatattt atattgaaag gaaaaagacc attaagatta tcctggagta tgcagacaag 3660

atcttcactt acatcttcat tctggaaatg cttctaaaat ggatagcata tggttataaa 3720

acatatttca ccaatgcctg gtgttggctg gatttcctaa ttgttgatgt ttctttggtt 3780
actttagtgg caaacactct tggctactca gatcttggcc ccattaaatc ccttcggaca 3840
ctgagagctt taagacctct aagagcctta tctagatttg aaggaatgag ggtcgttgtg 3900
aatgcactca taggagcaat tccttccatc atgaatgtgc tacttgtgtg tcttatattc 3960
tggctgatat tcagcatcat gggagtaaat ttgtttgctg gcaagttcta tgagtgtatt 4020
aacaccacag atgggtcacg gtttcctgca agtcaagttc caaatcgttc cgaatgtttt 4080
gcccttatga atgttagtca aaatgtgcga tggaaaaacc tgaaagtgaa ctttgataat 4140
gtcggacttg gttacctatc tctgcttcaa gttgcaactt ttaagggatg gacgattatt 4200
atgtatgcag cagtggattc tgttaatgta gacaagcagc ccaaatatga atatagcctc 4260
tacatgtata tttattttgt cgtctttatc atctttgggt cattcttcac tttgaacttg 4320
ttcattggtg tcatcataga taatttcaac caacagaaaa agaagcttgg aggtcaagac 4380
atctttatga cagaagaaca gaagaaatac tataatgcaa tgaaaaagct ggggtccaag 4440
aagccacaaa agccaattcc tcgaccaggg aacaaaatcc aaggatgtat atttgaccta 4500
gtgacaaatc aagcctttga tattagtatc atggttctta tctgtctcaa catggtaacc 4560
atgatggtag aaaaggaggg tcaaagtcaa catatgactg aagttttata ttggataaat 4620
gtggttttta taatcctttt cactggagaa tgtgtgctaa aactgatctc cctcagacac 4680
tactacttca ctgtaggatg gaatattttt gattttgtgg ttgtgattat ctccattgta 4740
ggtatgtttc tagctgattt gattgaaacg tattttgtgt cccctaccct gttccgagtg 4800
atccgtcttg ccaggattgg ccgaatccta cgtctagtca aaggagcaaa ggggatccgc 4860
acgctgctct ttgctttgat gatgtccctt cctgcgttgt ttaacatcgg cctcctgctc 4920
ttcctggtca tgttcatcta cgccatcttt ggaatgtcca actttgccta tgttaaaaag 4980
gaagatggaa ttaatgacat gttcaatttt gagacctttg gcaacagtat gatttgcctg 5040
ttccaaatta caacctctgc tggctgggat ggattgctag cacctattct taacagtaag 5100
ccacccgact gtgacccaaa aaaagttcat cctggaagtt cagttgaagg agactgtggt 5160
aacccatctg ttggaatatt ctactttgtt agttatatca tcatatcctt cctggttgtg 5220
gtgaacatgt acattgcagt catactggag aattttagtg ttgccactga agaaagtact 5280
gaacctctga gtgaggatga ctttgagatg ttctatgagg tttgggagaa gtttgatccc 5340
gatgcgaccc agtttataga gttctctaaa ctctctgatt ttgcagctgc cctggatcct 5400
cctcttctca tagcaaaacc caacaaagtc cagctcattg ccatggatct gcccatggtt 5460
agtggtgacc ggatccattg tcttgacatc ttatttgctt ttacaaagcg tgttttgggt 5520
gagagtgggg agatggattc tcttcgttca cagatggaag aaaggttcat gtctgcaaat 5580

ccttccaaag tgtcctatga acccatcaca accacactaa aacggaaaca agaggatgtg 5640

tctgctactg tcattcagcg tgcttataga cgttaccgct taaggcaaaa tgtcaaaaat 5700

atatcaagta tatacataaa agatggagac agagatgatg atttactcaa taaaaaagat 5760

atggcttttg ataatgttaa tgagaactca agtccagaaa aaacagatgc cacttcatcc 5820

accacctctc caccttcata tgatagtgta acaaagccag acaaagagaa atatgaacaa 5880

gacagaacag aaaaggaaga caaagggaaa gacagcaagg aaagcaaaaa atag 5934

<210> 20
<211> 657
<212> DNA
<213> Homo sapiens

<400> 20
atggggaggc tgctggcctt agtggtcggc gcggcactgg tgtcctcagc ctgcgggggc 60

tgcgtggagg tggactcgga gaccgaggcc gtgtatggga tgaccttcaa aattctttgc 120

atctcctgca agcgccgcag cgagaccaac gctgagacct tcaccgagtg gaccttccgc 180

cagaagggca ctgaggagtt tgtcaagatc ctgcgctatg agaatgaggt gttgcagctg 240

gaggaggatg agcgcttcga gggccgcgtg gtgtggaatg gcagccgggg caccaaagac 300

ctgcaggatc tgtctatctt catcaccaat gtcacctaca accactcggg cgactacgag 360

tgccacgtct accgcctgct cttcttcgaa aactacgagc acaacaccag cgtcgtcaag 420

aagatccaca ttgaggtagt ggacaaagcc aacagagaca tggcatccat cgtgtctgag 480

atcatgatgt atgtgctcat tgtggtgttg accatatggc tcgtggcaga gatgatttac 540

tgctacaaga agatcgctgc cgccacggag actgctgcac aggagaatgc ctcggaatac 600

ctggccatca cctctgaaag caaagagaac tgcacgggcg tccaggtggc cgaatag 657

<210> 21
<211> 648
<212> DNA
<213> Homo sapiens

<400> 21
atgcacagag atgcctggct acctcgccct gccttcagcc tcacggggct cagtctcttt 60

ttctctttgg tgccaccagg acggagcatg gaggtcacag tacctgccac cctcaacgtc 120

ctcaatggct ctgacgcccg cctgccctgc accttcaact cctgctacac agtgaaccac 180

aaacagttct ccctgaactg gacttaccag gagtgcaaca actgctctga ggagatgttc 240

ctccagttcc gcatgaagat cattaacctg aagctggagc ggtttcaaga ccgcgtggag 300

ttctcaggga accccagcaa gtacgatgtg tcggtgatgc tgagaaacgt gcagccggag 360

gatgagggga tttacaactg ctacatcatg aacccccctg accgccaccg tggccatggc 420

aagatccatc tgcaggtcct catggaagag ccccctgagc gggactccac ggtggccgtg 480

attgtgggtg cctccgtcgg gggcttcctg gctgtggtca tcttggtgct gatggtggtc 540

aagtgtgtga ggagaaaaaa agagcagaag ctgagcacag atgacctgaa gaccgaggag 600

gagggcaaga cggacggtga aggcaacccg gatgatggcg ccaagtag 648

<210> 22
<211> 25
<212> DNA
<213> Artificial Sequence

<220>
<221> source
<223> /note="Description of Artificial Sequence: Synthetic oligonucleotide"

<400> 22
gttcttaagg cacaggaact gggac 25

<210> 23
<211> 25
<212> DNA
<213> Artificial Sequence

<220>
<221> source
<223> /note="Description of Artificial Sequence: Synthetic oligonucleotide"

<400> 23
gaagttaacc ctgtcgttct gcgac 25

<210> 24
<211> 25
<212> DNA
<213> Artificial Sequence

<220>
<221> source
<223> /note="Description of Artificial Sequence: Synthetic oligonucleotide"

<400> 24
gttctatagg gtctgcttgt cgctc 25

<210> 25
<211> 34
<212> DNA
<213> Artificial Sequence

<220>
<221> source
<223> /note="Description of Artificial Sequence: Synthetic probe"

<400> 25
gccagtccca gttcctgtgc cttaagaacc tcgc 34

```
<210> 26
<211> 34
<212> DNA
<213> Artificial Sequence

<220>
<221> source
<223> /note="Description of Artificial Sequence: Synthetic probe"

<400> 26
gcgagtcgca gaacgacagg gttaacttcc tcgc                          34


<210> 27
<211> 34
<212> DNA
<213> Artificial Sequence

<220>
<221> source
<223> /note="Description of Artificial Sequence: Synthetic probe"

<400> 27
gcgagagcga caagcagacc ctatagaacc tcgc                          34
```

## Description

Field of the Invention

The invention relates to novel cells and cell lines, and methods for making and using them.

Background of the Invention

Currently, the industry average failure rate for drug discovery programs in pharmaceutical companies is reported to be approximately 98%. Although this includes failures at all stages of the process, the high failure rate points to a dire need for any improvements in the efficiency of the process.

One factor contributing to the high failure rate is the lack of cell lines expressing therapeutic targets for used in cell-based functional assays during drug discovery. Indisputably, research using cell-based assays, especially drug discovery research, would benefit from cells and cell lines for use in cell-based assays.

Consequently, there is a great need for rapid and effective establishment of cell based assays for more rapid discovery of new and improved drugs. Preferably, for more effective drug discovery, the assay system should provide a more physiologically relevant predictor of the effect of a modulator *in vivo.*

Beyond the need for cell-based assays is a need for improved cells for protein production, cell-based therapy and a variety of other uses.

Accordingly, there is an urgent need for cells and cell lines that express a function protein or RNA of interest.

**Summary of the Invention**

In some embodiments, the invention provides a cell that expresses a heterodimeric protein of interest from an introduced nucleic acid encoding at least one of the subunits of the heterodimeric protein of interest, said cell being characterized in that it produces the heterodimeric protein of interest in a form suitable for use in a functional assay, wherein said protein of interest does not comprise a protein tag, or said protein is produced in that form consistently and reproducibly such that the cell has a Z' factor of at least 0.4 in the functional assay, or said cell is cultured in the absence of selective pressure, or any combinations thereof

In some embodiments, the invention provides a cell that expresses a heterodimeric protein of interest, wherein the cell is engineered to activate transcription of an endogenous nucleic acid encoding at least one of the subunits of the heterodimeric protein of interest, said cell being characterized in that it produces the heterodimeric protein of interest in a form suitable for use in a functional assay, wherein said protein of interest does not comprise a protein tag, or said protein is produced in that form consistently and reproducibly such that the cell has a Z' factor of at least 0.4 in the functional assay, or said cell is cultured in the absence of selective pressure, or any combinations thereof.

In some embodiments, the invention provides a cell that expresses a heterodimeric protein of interest from an introduced nucleic acid encoding at least one of the subunits of the heterodimeric protein of interest, said cell being characterized in that it produces the protein of interest in a form that is or is capable of becoming biologically active, wherein the cell is cultured in the absence of selective pressure.

In some embodiments, the invention provides a cell that expresses a heterodimeric protein of interest wherein the cell is engineered to activate transcription of an endogenous nucleic acid encoding at least one of the subunits of the heterodimeric protein of interest, said cell being characterized in that it produces the protein of interest in a form that is or is capable of becoming biologically active, wherein the cell is cultured in the absence of selective pressure.

In some embodiments, the nucleic acid encoding the second subunit of the heterodimeric protein of interest is endogenous. In other embodiments, the nucleic acid encoding the second subunit of the heterodimeric protein of interest is introduced. In yet other embodiments, the protein of interest does not comprise a protein tag.

In some embodiments, the heterodimeric protein of interest is selected from the group consisting of: an ion channel, a G protein coupled receptor (GPCR), tyrosine receptor kinase, cytokine receptor, nuclear steroid hormone receptor and immunological receptor. In some embodiments, the heterodimeric protein of interest is selected from the group consisting of: a sweet taste receptor and an umami taste receptor. In other embodiments, the heterodimeric protein of interest has no known ligand.

In some embodiments, the heterodimeric protein of interest is not expressed in a cell of the same type. In some embodiments the cell is a mammalian cell.

In some embodiments, the cell is further characterized in that it has an additional desired property selected from the group consisting of: a signal to noise ratio greater than 1, being stable over time, growth without selective pressure without losing expression, physiological EC50 values, and physiological IC50 values. In some embodiments, the heterodimeric protein of interest is produced in a form consistently and reproducibly for a period of time selected from: at least one week, at least two weeks, at least three weeks, at least one month, at least two months, at least three months at least four months, at least five months, at least six months, at least seven months, at least eight months, and at least nine months. In some embodiments, the functional assay is selected from the group consisting of: a cell-based assay, a fluorescent cell-based assay, a high throughput screening assay, a reporter cell-based assay, a G protein mediated cell-based assay, and a calcium flux cell-based assay. In other embodiments, the cell is suitable for utilization in a cell based high throughput screening.

In some embodiments, the selective pressure is an antibiotic. In other embodiments, the cell expresses the heterodimeric protein in the absence of selective pressure for at least 15 days, 30 days, 45 days, 60 days, 75 days, 100 days, 120 days, or 150 days.

In some embodiments, the invention provides a cell that expresses a heteromultimeric protein of interest wherein said heteromultimeric protein comprises at least 3 subunits, wherein at least one subunit of the heteromultimeric protein interest is encoded by an introduced nucleic acid, said cell being characterized in that it produces the heteromultimeric protein of interest in a form suitable for use in a functional assay, wherein said protein of interest does not comprise a protein tag, or said protein produced in that form consistently and reproducibly such that the ceii has a Z' factor of at least 0.4 in the functional assay, or said cell is cultured in the absence of selective pressure, or any combinations thereof.

In some embodiments, the invention provides a cell that expresses a heteromultimeric protein of interest wherein said heteromultimeric protein comprises at least 3 subunits, wherein the cell is engineered to activate transcription of an endogenous nucleic acid encoding at least one of the subunits of the heteromultimeric protein of interest, said cell being characterized in that it produces the heteromultimeric protein of interest in a form suitable for use in a functional assay, wherein said protein of interest does not comprise a protein tag, or said protein produced in that form consistently and reproducibly such that the cell has a Z' factor of at least 0.4 in the functional assay, or said cell is cultured in the absence of selective pressure, or any combinations thereof.

In some embodiments, the invention provides a cell that expresses a heteromultimeric protein of interest wherein said heteromultimeric protein comprises at least 3 subunits, wherein at least one subunit of the heteromultimeric protein interest is encoded by an introduced nucleic acid, said cell being characterized in that it produces the protein of interest in a form that is or is capable of becoming biologically active.

In some embodiments, the invention provides a cell that expresses a heteromultimeric protein of interest wherein said heteromultimeric protein comprises at least 3 subunits, wherein the cell is engineered to activate transcription of an endogenous nucleic acid encoding at least one of the subunits of the heteromultimeric protein of interest, said cell being characterized in that it produces the protein of interest in a form that is or is capable of becoming biologically active.

In some embodiments, the nucleic acid encoding at least one of the subunits of the heteromultimeric protein of interest is endogenous.

In some embodiments, the nucleic acid encoding at least one of the subunits of the heteromultimeric protein of interest is introduced.

In some embodiments, the protein of interest does not comprise a protein tag.

In some embodiments, the heteromultimeric protein of interest is selected from the group consisting of: an ion channel, a G protein coupled receptor (GPCR), tyrosine receptor kinase, cytokine receptor, nuclear steroid hormone receptor and immunological receptor. In other embodiments, the heteromultimeric protein of interest is selected from the group consisting of: GABA, ENaC and NaV. In some embodiments, the heteromultimeric protein of interest has no known ligand.

In some embodiments, the heteromultimeric protein of interest is not expressed in a cell of the same type. In other embodiments, the cell is a mammalian cell.

In some embodiments, the cell is further characterized in that it has an additional desired property selected from the group consisting of: a signal to noise ratio greater than 1, being stable over time, growth without selective pressure without losing expression, physiological EC50 values, and physiological IC50 values. In other embodiments, the heteromultimeric protein of interest is produced in a form consistently and reproducibly for a period of time selected from: at least one week, at least two weeks, at least three weeks, at least one month, at least two months, at least three months at least four months, at least five months, at least six months, at least seven months, at least eight months, and at least nine months.

In some embodiments, the functional assay is selected from the group consisting of: a cell-based assay, a fluorescent cell-based assay, a high throughput screening assay, a reporter cell-based assay, a G protein mediated cell-based assay, and a calcium flux cell-based assay. In other embodiments, the cell expressing the heteromultimeric protein is suitable for utilization in a cell based high throughput screening.

In some embodiments, the cells expressing the heteromultimeric protein are cultured in the absence of selective pressure. In some embodiments, the selective pressure is an antibiotic. In other embodiments, The cell according to claim 35 or 36, wherein the cell expresses the heteromultimeric protein in the absence of selective pressure for at least 15 days, 30 days, 45 days, 60 days, 75 days, 100 days, 120 days, or 150 days.

In some embodiments, the invention provides a cell that expresses two or more proteins of interest from an introduced nucleic acid encoding at least one of the proteins of interest, said cell being characterized in that it produces the proteins of interest in a form suitable for use in a functional assay, wherein said proteins of interest do not comprise a protein tag, or said proteins are produced in that form consistently and reproducibly such that the cell has a Z' factor of at least 0.4 in the functional assay, or said cell is cultured in the absence of selective pressure, or any combinations thereof.

In some embodiments, the invention provides a cell that expresses two or more proteins of interest, wherein the cell is engineered to activate transcription of an endogenous nucleic acid encoding at least one of the proteins of interest, said cell being characterized in that it produces the proteins of interest in a form suitable for use in a functional assay, wherein said proteins of interest do not comprise a protein tag, or said proteins are produced in that form consistently and reproducibly such that the cell has a Z' factor of at least 0.4 in the functional assay, or said cell is cultured in the absence of selective pressure, or any combinations thereof.

In some embodiments, the invention provides a cell that expresses two or more proteins of interest from an introduced nucleic acid encoding at least one of the proteins of interest, said cell being characterized in that it produces the proteins of interest in a form that is or is capable of becoming biologically active.

In some embodiments, the invention provides a cell that expresses two or more proteins of interest, wherein the cell is engineered to activate transcription of an endogenous nucieic acid encoding at least one of the proteins of interest, said cell being characterized in that it produces the proteins of interest in a form that is or is capable of becoming biologically active.

In some embodiments, at least one of the two or more proteins of interest is a dimeric protein. In other embodiments, the dimeric protein of interest is a homodimeric protein. In other embodiments, the dimeric protein of interest is a heterodimeric protein. In some embodiments, at least one of the two or more proteins of interest is a multimeric protein. In other embodiments, the multimeric protein of interest is a homomultimeric protein. In other embodiments, the multimeric protein of interest is a heteromultimeric protein.

In some of the embodiments, one of the two or more proteins of interest is encoded by an endogenous nucleic acid. In other embodiments, one of the two or more proteins of interest is encoded by an introduced nucleic acid. In other embodiments, the proteins of interest do not comprise a protein tag.

In some embodiments, one of the two or more proteins of interest is selected from the group consisting of: an ion channel, a G protein coupled receptor (GPCR), tyrosine receptor kinase, cytokine receptor, nuclear steroid hormone receptor and immunological receptor. In other embodiments one of the proteins of interest has no known ligand.

In some embodiments, one of the two or more proteins of interest is not expressed in a cell of the same type. In some embodiments, the cell expressing the two or more proteins is a mammalian cell.

In some embodiments, the cell expressing the two or more proteins is further characterized in that it has an additional desired property selected from the group consisting of: a signal to noise ratio greater than 1, being stable over time, growth without selective pressure without losing expression, physiological EC50 values, and physiological IC50 values.

In some embodiments, the two or more proteins of interest are produced in a form consistently and reproducibly for a period of time selected from: at least one week, at least two weeks, at least three weeks, at least one month, at least two months, at least three months at least four months, at least five months, at least six months, at least seven months, at least eight months, and at least nine months.

In some embodiments, the functional assay is selected from the group consisting of: a cell-based assay, a fluorescent cell-based assay, a high throughput screening assay, a reporter cell-based assay, a G protein mediated cell-based assay, and a calcium flux cell-based assay. In some embodiments, the cell expressing the two or more proteins is suitable for utilization in a cell based high throughput screening.

In some embodiments, the cell expressing the two or more proteins is cultured in the absence of selective pressure. In some embodiments, the selective pressure is an antibiotic. In some embodiments, the cell expresses the two or more proteins in the absence of selective pressure for at least 15 days, 30 days, 45 days, 60 days, 75 days, 100 days, 120 days, or 150 days.

In some embodiments, the invention provides a cell that expresses at least one RNA of interest, wherein said RNA of interest is encoded by an introduced nucleic acid, said cell being characterized in that it produces the at least one RNA of interest in a form suitable for use in a functional assay, wherein said RNA of interest do not comprise a tag, or said RNA is produced in that form consistently and reproducibly such that the cell has a Z' factor of at least 0.4 in the functional assay, or said cell is cultured in the absence of selective pressure, or any combinations thereof.

In some embodiments, the invention provides a cell that expresses at least one RNA of interest, wherein the cell is engineered to activate transcription of an endogenous nucleic acid encoding the at least one RNA of interest, said cell being characterized in that it produces the at least one RNA of interest in a form suitable for use in a functional assay, wherein said RNA of interest do not comprise a tag, or said RNA is produced in that form consistently and reproducibly such that the cell has a Z' factor of at least 0.4 in the functional assay or said cell is cultured in the absence of selective pressure, or any combinations thereof.

In some embodiments, the cell expresses at least two RNAs of interest. In other embodiments, the cell expresses at least three RNAs of interest. In some embodiments, the cell further expresses a RNA encoded by an introduced nucleic acid. In some embodiments, the the RNA of interest is selected from the group consisting of: a RNA encoding an ion channel, a RNA encoding a G protein coupled receptor (GPCR), a RNA encoding a tyrosine receptor kinase, a RNA encodinga cytokine receptor, a RNA encoding a nuclear steroid hormone receptor and a RNA encoding an immunological receptor.

In some embodiments, the RNA of interest is not expressed in a cell of the same type. In some embodiments, the cell expressing the RNA of interest is a mammalian cell.

In some embodiments, the cell expressing the RNA of interest is further characterized in that it has an additional desired property selected from the group consisting of: a signal to noise ratio greater than 1, being stable over time, growth without selective pressure without losing expression, physiological EC50 values, and physiological IC50 values. In some embodiments, the RNA of interest is produced in a form consistently and reproducible for a period of time selected from: at least one week, at least two weeks, at least three weeks, at least one month, at least two months, at least three months at least four months, at least five months, at least six months, at least seven months, at least eight months, and at least nine months.

In some embodiments, the functional assay is selected from the group consisting of: a cell-based assay, a fluorescent cell-based assay, a high throughput screening assay, a reporter cell-based assay, a G protein mediated cell-based assay, and a calcium flux cell-based assay.

In some embodiments, the cell expressing the RNA of interest is suitable for utilization in a cell based high throughput screening.

in some embodiments, the invention provides a cell line produced from a cell described herein.

In some embodiments, the invention provides a method for producing a cell that expresses a protein of interest, wherein the cell has at least one desired property that is consistent over time, comprising the steps of:

a) providing a plurality of cells that express mRNA encoding the protein of interest;
b) dispersing cells individually into individual culture vessels, thereby providing a plurality of separate cell cultures
c) culturing the cells under a set of desired culture conditions using automated cell culture methods characterized in that the conditions are substantially identical for each of the separate cell cultures, during which culturing the number of cells per separate cell culture is normalized, and wherein the separate cultures are passaged on the same schedule;
d) assaying the separate cell cultures for at least one desired characteristic of the protein of interest at least twice; and
e) identifying a separate cell culture that has the desired characteristic in both assay.

In some embodiments, the plurality of cells in step a) of the methods described herein are cultured for some period of time prior to the dispersing in step b).

In some embodiments, the individual culture vessels used in the methods of this invention are selected from the group consisting of: individual wells of a multiwell plate and vials.

In some embodiments, the method further comprises the step of determining the growth rate of a plurality of the separate cell cultures and grouping the separate cell cultures by their growth rates into groups such that the difference between the fastest and slowest growth rates in any group is no more than 1, 2, 3, 4 or 5 hours between steps b) and c).

In some embodiments, the method further comprises the step of preparing a stored stock of one or more of the separate cultures. In some embodiments, the method further comprises the step of one or more replicate sets of the separate cell cultures and culturing the one or more replicate sets separately from the source separate cell cultures.

In some embodiments, the assaying in step d) of the method of this invention is a functional assay for the protein.

In some embodiments, the at least one characteristic that has remained constant in step e) is protein function.

in some embodiments, the culturing in step c) of the methods of this invention is in a robotic cell culture apparatus. In some embodiments, the robotic cell culture apparatus comprises a multi-channel robotic pipettor. In some embodiments, the multi-channel robotic pipettor comprises at least 96 channels. In some embodiments, the robotic cell culture apparatus further comprises a cherry-picking arm.

In some embodiments, the automated methods include one or more of: media removal, media replacement, cell washing, reagent addition, removal of cells, cell dispersal, and cell passaging.

In some embodiments, the plurality of separate cell cultures used in the methods of this invention is at least 50 culture. In other embodiments, the plurality of separate cell cultures is at least 100 cultures. In other embodiments, the plurality of separate cell cultures is at least 500 cultures. In yet other embodiments, the plurality of separate cell cultures is at least 1000 cultures.

In some embodiments, the growth rate is determined by a method selected from the group consisting of: measuring ATP, measuring cell confluency, light scattering, optical density measurement. In some embodiments, the difference between the fastest and slowest growth rates in a group is no more than 1, 2, 3, 4, or 5 hours.

In some embodiments, the culturing in step c) of the methods of this invention is for at least 2 days.

In some embodiments, the growth rates of the plurality of separate cell cultures are determined by dispersing the cells and measuring ceii confluency. In some embodiments, the cells in each separate cell culture of the methods of this invention are dispersed prior to measuring cell confluency. In some embodiments, the dispersing step comprises adding trypsin to the well and to eliminate clumps. In some embodiments, the cell confluency of the plurality of separate cell cultures is measured using an automated microplate reader.

In some embodiments, at least two confluency measurements are made before growth rate is calculated. In some embodiments, the cell confluency is measured by an automated plate reader and the confluency values are used with a software program that calculates growth rate.

In some embodiments, the separate cell cultures in step d) are characterization for a desired trait selected from one or more of: fragility, morphology, adherence to a solid surface; lack of adherence to a solid surface and protein function.

In some embodiments, the cells used in the methods of this invention are eukaryotic cells. In some embodiments, the eukaryotic cells used in the methods of this invention are mammalian cells. In some embodiments, the mammalian cell line is selected from the group consisting of: NS0 cells, CHO cells, COS cells, HEK-293 cells, HUVECs, 3T3 cells and HeLa cells.

In some embodiments, the protein of interest expressed in the methods of this invention is a human protein. In some embodiments, the protein of interest is a heteromultimer. In some embodiments, the protein of interest is a G protein coupled receptor. In other embodiments, the protein has no known ligand.

In some embodiments, the method of this invention, further comprises after the identifying step, the steps of:

a) expanding a stored aliquot of the cell culture identified in step e) under desired culture conditions;
b) determining if the expanded cell culture of a) has the desired characteristic.

In some embodiments, the invention provides a matched panel of clonal cell lines, wherein the clonal cell lines are of the same cell type, and wherein each cell line in the panel expresses a protein of interest, and wherein the clonal cell lines in the panel are matched to share the same physiological property to allow parallel processing. In some embodiments, the physiological property is growth rate. In other embodiments, the physiological property is adherence to a tissue culture surface. In other embodiments, the physiological property is Z' factor. In other embodiments, the physiological property is expression level of RNA encoding the protein of interest. In yet other embodiments, the physiological property is expression level of the protein of interest.

In some embodiments, the growth rates of the clonal cell lines in the panel are within 1, 2, 3, 4, or 5 hours of each other. In other embodiments, the culture conditions used for the matched panel are the same for aii clonal cell lines in the panel.

In some embodiments, the clonal cell line used in the matched panels is a eukaryotic cell line. In some embodiments, the eukaryotic cell line is a mammalian cell line. In some embodiments, the cell line cells used in the matched panels are selected from the group consisting of: primary cells and immortalized cells.

In some embodiments, the cell line cells used in the matched panels are prokaryotic or eukaryotic. In some embodiments, the cell line cells used in the matched panels are eukaryotic and are selected from the group consisting of: fungal cells, insect cells, mammalian cells, yeast cells, algae, crustacean cells, arthropod cells , avian cells, reptilian cells, amphibian cells and plant cells. In some embodiments, the cell line cells used in the matched panels are mammalian and are selected from the group consisting of: human, non-human primate, bovine, porcine, feline, rat, marsupial, murine, canine, ovine, caprine, rabbit, guinea pig hamster.

In some embodiments, the cells in the cell line of the matched panels are engineered to express the protein of interest. In some embodiments, the cells in the cell line of the matched panels express the protein of interest from an introduced nucleic acid encoding the protein or, in the case of a multimeric protein, encoding a subunit of the protein. In some embodiments, the cells express the protein of interest from an endogenous nucleic acid and wherein the cell is engineered to activate transcription of the endogenous protein or, in the case of a multimeric protein, activates transcription of a subunit of the protein.

In some embodiments, the panel comprises at least four clonal cell lines. In other embodiments, the panel comprises at least six clonal cell lines. In yet other embodiments, the panel comprises at least twenty five clonal cell lines.

in some embodiments, two or more of the clonal ceii lines in the panel express the same protein of interest. In other embodiments, two or more of the clonal cell lines in the panel express a different protein of interest.

In some embodiments, the cell lines in the panel express different forms of a protein of interest, wherein the forms are selected from the group consisting of: isoforms, amino acid sequence variants, splice variants, truncated forms, fusion proteins, chimeras, or combinations thereof.

In some embodiments, the cell lines in the panel express different proteins in a group of proteins of interest, wherein the groups of proteins of interest are selected from the group consisting of: proteins in the same signaling pathway, expression library of similar proteins, monoclonal antibody heavy chain library, monoclonal antibody light chain library and SNPs.

In some embodiments, the protein of interest expressed in the panel is a single chain protein. In some embodiments, the single chain protein is a G protein coupled receptor. In some embodiments, the G protein coupled receptor is a taste receptor. In some embodiments, the taste receptor is selected from the group consisting of: a bitter taste receptor, a sweet taste receptor, a salt taste receptor and a umami taste receptor.
In other embodiments, the protein of interest expressed in the panel is a multimeric protein. In some embodiments, the protein is a heterodimer or a heteromultimer.
In some embodiments, the protein of interest expressed in the panel is selected from the group consisting of: an ion channel, an ion channel, a G protein coupled receptor (GPCR), tyrosine receptor kinase, cytokine receptor, nuclear steroid hormone receptor and immunological receptor. In some embodiments, the protein expressed in the matched panel is Epithelial sodium Channel (ENaC). In some embodiments, the ENaC comprises an alpha subunit, a beta subunit and a gamma subunit. In other embodiments, the cell lines in the panel express different ENaC isoforms. In other embodiments, the cell lines in the panel comprise different proteolyzed isoforms of ENaC. In some embodiments, the ENaC is human ENaC. In some embodiments the protein expressed in the matched panel is voltage gated sodium channel (NaV). In some embodiments, the NaV comprises an alpha subunit and two beta subunits. In some embodiments, the NaV is human NaV.
In some embodiments, the protein expressed in the matched panel is selected from the group consisting of: gamma-aminobutyric acid A receptor ($GABA_A$ receptor), gamma-aminobutyric acid B receptor ($GABA_B$ receptor) and gamma-aminobutyric acid C receptor ($GABA_C$ receptor). In some embodiments, the protein is $GABA_A$ receptor. In some embodiments, the $GABA_A$ receptor comprises two alpha subunits, two beta subunits and a gamma or delta subunit.
In some embodiments, the clonal cell lines in the panel are produced simultaneously, or within no more than 4 weeks of each other.
in some embodiments, the invention provides a cell that expresses a monomeric protein of interest from an introduced nucleic acid encoding said monomeric protein of interest, characterized in that it produces the protein of interest in a form that is or is capable of becoming biologically active, wherein the cell is cultured in the absence of selective pressure and wherein the expression of the protein does not vary by more than 5% over 3 months. In some embodiments the expression of the protein does not vary by more than 5% over 6 months. In some embodiments, the monomeric protein of interest has no known ligand.
In some embodiments, the invention provides A method for identifying a modulator of a protein of interest comprising the steps of:

a) contacting a cell according to any one of the above-described cell embodiments with a test compound; and
b) detecting a change in the activity of the protein of interest in the cell contacted with the test compound compared to the activity of the protein in a cell not contacted by the test compound;
wherein a compound that produces a difference in the activity in the presence compared to in the absence is a modulator of the protein of interest.

In another embodiment, the invention provides a modulator identified by the method of the preceding paragraph.

Detailed Description

Unless otherwise defined, all technical and scientific terms used herein have the same meaning as commonly understood by one of ordinary skill in the art to which this invention belongs. Exemplary methods and materials are described below, although methods and materials similar or equivalent to those described herein can also be used in the practice or testing of the present invention. In case of conflict, the present specification, including definitions, will control.
All publications and other references mentioned herein are incorporated by reference in their entirety. Although a number of documents are cited herein, this citation does not constitute an admission that any of these documents forms part of the common general knowledge in the art.
Throughout this specification and claims, the word "comprise," or variations such as "comprises" or "comprising" will be understood to imply the inclusion of a stated integer or group of integers but not the exclusion of any other integer or group of integers. Unless otherwise required by context, singular terms shall include pluralities and plural terms shall include the singular. The materials, methods, and examples are illustrative only and not intended to be limiting.
In order that the present invention may be more readily understood, certain terms are first defined. Additional definitions are set forth throughout the detailed description.
The term "stable" or "stably expressing" is meant to distinguish the cells and cell lines of the invention from cells that transiently express proteins as the terms "stable expression" and "transient expression" would be understood by a person of skill in the art.
As used herein, a "functional" RNA or protein of interest is one that has a signal to noise ratio greater than 1:1 in a cell based assay. In some embodiments, a functional protein or RNA of interest has one or more of the biological activities of the naturally occurring or endogenously expressed protein or RNA.

The term "cell line" or "clonal cell line" refers to a population of cells that is progeny of a single original cell. As used herein, cell lines are maintained *in vitro* in cell culture and may be frozen in aliquots to establish banks of clonal cells.

The term "stringent conditions" or "stringent hybridization conditions" describe temperature and salt conditions for hybridizing one or more nucleic acid probes to a nucleic acid sample and washing off probes that have not bound specifically to target nucleic acids in the sample. Stringent conditions are known to those skilled in the art and can be found in, for example, Current Protocols in Molecular Biology, John Wiley & Sons, N.Y. (1989), 6.3.1-6.3.6. Aqueous and nonaqueous methods are described in the Protocols and either can be used. One example of stringent hybridization conditions is hybridization in 6X SSC at about 45°C, followed by at least one wash in 0.2X SSC, 0.1% SDS at 60°C. Another example of stringent hybridization conditions is hybridization in 6X SSC at about 45°C, followed by at least one wash in 0.2X SSC, 0.1% SDS at 65°C. Stringent hybridization conditions also include hybridization in 0.5M sodium phosphate, 7% SDS at 65°C, followed by at least one wash at 0.2X SSC, 1% SDS at 65°C.

The phrase "percent identical" or "percent identity" in connection with amino acid and/or nucleic acid sequences refers to the similarity between at least two different sequences. The percent identity can be determined by standard alignment algorithms, for example, the Basic Local Alignment Tool (BLAST) described by Altshul et al. ((1990) J. Mol. Biol., 215: 403-410); the algorithm of Needleman et al. ((1970) J. Mol. Biol., 48: 444-453); or the algorithm of Meyers et al. ((1988) Comput. Appl. Biosci., 4: 11-17). A set of parameters may be the Blosum 62 scoring matrix with a gap penalty of 12, a gap extend penalty of 4, and a frameshift gap penalty of 5. The percent identity between two amino acid or nucleotide sequences can also be determined using the algorithm of E. Meyers and W. Miller ((1989) CABIOS, 4:11-17) that has been incorporated into the ALIGN program (version 2.0), using a PAM120 weight residue table, a gap length penalty of 12 and a gap penalty of 4. The percent identity is usually calculated by comparing sequences of similar length.

Protein analysis software matches similar amino acid sequences using measures of similarity assigned to various substitutions, deletions and other modifications, including conservative amino acid substitutions. For instance, the GCG Wisconsin Package (Accelrys, Inc.) contains programs such as "Gap" and "Bestfit" that can be used with default parameters to determine sequence identity between closely related polypeptides, such as homologous polypeptides from different species or between a wild type protein and a mutein thereof. See, e.g., GCG Version 6.1. Polypeptide sequences also can be compared using FASTA using default or recommended parameters. A program in GCG Version 6.1. FASTA (e.g., FASTA2 and FASTA3) provides alignments and percent sequence identity of the regions of the best overlap between the query and search sequences (Pearson, Methods Enzymol. 183:63-98 (1990); Pearson, Methods Mol. Biol. 132:185-219 (2000)).

The length of polypeptide sequences compared for identity will generally be at least about 16 amino acid residues, usually at least about 20 residues, more usually at least about 24 residues, typically at least about 28 residues, and preferably more than about 35 residues. The length of a DNA sequence compared for identity will generally be at least about 48 nucleic acid residues, usually at least about 60 nucleic acid residues, more usually at least about 72 nucleic acid residues, typically at least about 84 nucleic acid residues, and preferably more than about 105 nucleic acid residues.

The phrase "substantially as set out," "substantially identical" or "substantially homologous" in connection with an amino acid or nucleotide sequence means that the relevant amino acid or nucleotide sequence will be identical to or have insubstantial differences (e.g., conserved amino acid substitutions or nucleic acids encoding such substitutions) in comparison to the comparator sequences. Insubstantial differences include minor amino acid changes, such as 1 or 2 substitutions in a 50 amino acid sequence of a specified region and the nucleic acids that encode those sequences.

Modulators include any substance or compound that alters an activity of a protein of interest. The modulator can be an agonist (potentiator or activator) or antagonist (inhibitor or blocker), including partial agonists or antagonists, selective agonists or antagonists and inverse agonists, and can also be an allosteric modulator. A substance or compound is a modulator even if its modulating activity changes under different conditions or concentrations or with respect to different forms of a protein of interest. In other aspects, a modulator may change the ability of another modulator to affect the function of a protein of interest.

The terms "potentiator", "agonist" or "activator" refer to a compound or substance that increases one or more activities of a protein of interest.

The terms "inhibitor", "antagonist" or "blocker" refer to a compound or substance that decreases or blocks one or more activities of a protein of interest.

The invention provides for the first time novel cells and cell lines produced from the cells that meet the urgent need for cells that stably express a functional RNA of interest or a functional protein of interest, including complex proteins such as heteromultimeric proteins and proteins for which no ligand is known. The cells and cell lines of the invention are suitable for any use in which consistent, functional expression of an RNA or protein of interest are desirable. Applicants have produced cell lines meeting this description for a variety of proteins, both single subunit and heteromultimeric (including heterodimeric and proteins with more than two different subunits), including membrane proteins, cytosolic proteins and secreted proteins, as well as various combinations of these.

In one aspect, the cells and cell lines of the invention are suitable for use in a cell-based assay. Such cells and cell lines provide consistent and reproducible expression of the protein of interest over time and, thus, are particularly advantageous in such assays.

In another aspect, the invention provides cells and cell lines that are suitable for the production of biological molecules. The cells and cell lines for such use are characterized, for example, by consistent expression of a protein or polypeptide that is functional or that is capable of becoming functional.

The invention further provides a method for producing cells and cell lines that stably express an RNA or a protein of interest. Using the method of the invention, one can produce cells and cell lines that express any desired protein in functional form, including complex proteins such as multimeric proteins, (e.g., heteromultimeric proteins) and proteins that are cytotoxic. The method disclosed herein makes possible the production of engineered cells and cell lines stably expressing functional proteins that prior to this invention have not previously been produced. Without being bound by theory, it is believed that because the method permits investigation of very large numbers of cells or cell lines under any desired set of conditions, it makes possible the identification of rare cells that would not have been produced in smaller populations or could not otherwise be found and that are optimally suited to express a desired protein in a functional form under desired conditions.

In a further aspect, the invention provides a matched panel of cell lines, i.e., a collection of clonal cell lines that are matched for one or more physiological properties. Because the method of the invention permits maintenance and characterization of large numbers of cell lines under identical conditions, it is possible to identify any number of cell lines with similar physiological properties. Using the method of the invention, it is possible to make matched panels comprising any desired number of cell lines or make up Such matched panels may be maintained under identical conditions, including cell density and, thus, are useful for high throughput screening and other uses where it is desired to compare and identify differences between cell lines. Also within the invention are matched panels of cell lines that are matched for growth rate.

In another aspect, the invention provides a method for producing cells or ceii lines that express a protein of previously unknown function and/or for which no ligand had previously been identified. Such a protein may be a known naturally occurring protein, a previously unknown naturally occurring protein, a previously unknown form of a known naturally occurring protein or a modified form of any of the foregoing.

Any desired cell type may be used for the cells of the invention. The cells may be prokaryotic or eukaryotic. The cells may express the protein of interest in their native state or not. Eukaryotic ceiis that may be used include but are not limited to fungi cells such as yeast cells, plant cells and animal cells. Animal cells that can be used include but are not limited to mammalian cells and insect cells, Primary or immortalized cells may be derived from mesoderm, ectoderm or endoderm layers of eukaryotic organisms. The cells may be endothelial, epidermal, mesenchymal, neural, renal, hepatic, hematopoietic, or immune cells. For example, the cells may be intestinal crypt or villi cells, clara cells, colon cells, intestinal cells, goblet cells, enterochromafin cells, enteroendocrine cells. Mammalian cells that are useful in the method include but are not limited to human, non-human primate, cow, horse, goat, sheep, pig, rodent (including rat, mouse, hamster, guinea pig), marsupial, rabbit, dog and cat. The cells can be differentiated cells or stem cells, including embryonic stem cells.

Cells of the invention can be primary, transformed, oncogenically transformed, virally transformed, immortalized, conditionally transformed, explants, cells of tissue sections, animals, plants, fungi, protists, archaebacteria and eubacteria, mammals, birds, fish, reptiles, amphibians, and arthropods, avian, chicken, reptile, amphibian, frog, lizard, snake, fish, worms, squid, lobster, sea urchin, sea slug, sea squirt, fly, squid, hydra, arthropods, beetles, chicken, lamprey, ricefish, zebra finch, pufferfish, and Zebrafish,

Additionally, cells such as blood/immune cells, endocrine (thyroid, parathyroid, adrenal), GI (mouth, stomach, intestine), liver, pancreas, gallbladder, respiratory (lung, trachea, pharynx), Cartilage, bone, muscle, skin, hair, urinary (kidney, bladder), reproductive (sperm, ovum, testis, uterus, ovary, penis, vagina), sensory (eye, ear, nose, mouth, tongue, sensory neurons), Blood/immune cells such as_B cell, T cell (Cytotoxic T cell, Natural Killer T cell, Regulatory T cell, T helper cell,

Tcell, Natural killer cell; granulocytes (basophil granulocyte, eosinophil granulocyte, neutrophil granulocyte/hypersegmented neutrophil), monocyte/macrophage, red blood cell (reticulocyte), mast cell, thrombocyte/Megakaryocyte, dendritic cell; endocrine cells such as: thyroid (thyroid epithelial cell, parafollicular cell), parathyroid (parathyroid chief cell, oxyphil cell), adrenal (chromaffin cell), nervous system cells such as: glial cells (astrocyte, microglia), magnocellular neurosecretory cell, stellate cell, nuclear chain cell, boettcher cell, pituitary, (gonadotrope, corticotrope, thyrotrope, somatotrope, lactotroph), respiratory system cells such as pneumocyte (type I pneumocyte, type II pneumocyte), ciara cell, goblet cell; circulatory system cells such as myocardiocyte · pericyte; digestive system cells such as stomach (gastric chief cell, parietal cell), goblet cell, paneth cell, G cells, D cells, ECL cells, I cells, K cells, ,enteroendocrine cells, enterochromaffin cell, APUD cell, liver (hepatocyte, kupffer cell), pancreas (beta cells, alpha cells), gallbladder; cartilage/bone/muscle/integumentary system cells such as osteoblast, osteocyte, steoclast, tooth cells (cementoblast, ameloblast), cartilage cells: chondroblast, chondrocyte, skin/hair cells: trichocyte, keratinocyte, melanocyte, muscle cells:

myocyte, adipocyte, fibroblast, urinary system cells such as podocyte, juxtaglomerular cell, intraglomerular mesangial cell/extraglomerular mesangial cell, kidney proximal tubule brush border cell, macula densa cell; reproductive system cells such as spermatozoon, sertoli cell, leydig cell, ovum, ovarian follicle cell; sensory cells such as organ of corti cells, olfactory epithelium, temperature sensitive sensory neurons, merckel cells, olfactory receptor neuron, pain sensitive neurons, photoreceptor cells, taste bud cells, hair cells of the vestibular apparatus, carotid body cells are useful to make cells or cell lines of the invention.

Plant cells that are useful include roots, stems and leaves and plant tissues include meristematic tissues, parenchyma collenchyma, sclerenchyma, secretory tissues, xylem, phloem, epidermis, periderm (bark).

Cells that are useful for the cells and cell lines of the invention also include but are not limited to: Chinese hamster ovary (CHO) cells, established neuronal cell lines, pheochromocytomas, neuroblastomas fibroblasts, rhabdomyosarcomas, dorsal root ganglion cells, NS0 cells, CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), L-cells, HEK-293 (ATCC CRL1573) and PC12 (ATCC CRL-1721), HEK293T (ATCC CRL-11268), RBL (ATCC CRL-1378), SH-SY5Y (ATCC CRL-2266), MDCK (ATCC CCL-34), SJ-RH30 (ATCC CRL-2061), HepG2 (ATCC HB-8065), ND7/23 (ECACC 92090903), CHO (ECACC 85050302), Vero (ATCC CCL 81), Caco-2 (ATCC HTB 37), K562 (ATCC CCL 243), Jurkat (ATCC TIB-152), Per.C6 (Crucell, Leiden, The Netherlands), Huvec (ATCC Human Primary PCS 100-010, Mouse CRL 2514, CRL 2515, CRL 2516), HuH-7D12 (ECACC 01042712), 293 (ATCC CRL 10852), A549 (ATCC CCL 185), IMR-90 (ATCC CCL 186), MCF-7 (ATC HTB-22), U-2 OS (ATCC HTB-96), T84 (ATCC CCL 248), or any established cell line (polarized or nonpolarized) or any cell line available from repositories such as American Type Culture Collection (ATCC, 10801 University Blvd. Manassas, Va. 20110-2209 USA) or European Collection of Cell Cultures (ECACC, Salisbury Wiltshire SP4 0JG England).

Further, cells that are useful in the method of the invention are mammalian cells amenable to growth in serum containing media, serum free media, fully defined media without any animal-derived products, and cells that can be converted from one of these conditions to another.

Cells of the invention include cells into which a nucleic acid that encodes the protein of interest (or in the case of a heteromultimeric protein, a nucleic acid that encodes one or more of the subunits of the protein) has been introduced. Engineered cells also include cells into which nucleic acids for transcriptional activation of an endogenous sequence encoding a protein of interest (or for transcriptional activation of endogenous sequence encoding one or more subunits of a heteromultimeric protein) have been introduced. Engineered cells also include cells comprising a nucleic acid encoding a protein of interest that is activated by contact with an activating compound. Engineered cells further include combinations of the foregoing, that is, cells that express one or more subunits of a heteromultimeric protein from an introduced nucleic acid encoding it and that express one or more subunits of the protein by gene activation.

Any of the nucleic acids may be introduced into the cells using known means. Techniques for introducing nucleic acids into cells are well-known and readily appreciated by the skilled worker. The methods include but are not limited to transfection, viral delivery, protein or peptide mediated insertion, coprecipitation methods, lipid based delivery reagents (lipofection), cytofection, lipopolyamine delivery, dendrimer delivery reagents, electroporation or mechanical delivery. Examples of transfection reagents are GENEPORTER, GENEPORTER2, LIPOFECTAMINE, LIPOFECTAMINE 2000, FUGENE 6, FUGENE HD, TFX-10, TFX-20, TFX-50, OLIGOFECTAMINE, TRANSFAST, TRANSFECTAM, GENESHUTTLE, TROJENE, GENESILENCER, X-TREMEGENE, PERFECTIN, CYTOFECTIN, SIPORT, UNIFECTOR, SIFECTOR, TRANSIT-LT1, TRANSIT-LT2, TRANSIT-EXPRESS, IFECT, RNAI SHUTTLE, METAFECTENE, LYOVEC, LIPOTAXI, GENEERASER, GENEJUICE, CYTOPURE, JETSI, JETPEI, MEGAFECTIN, POLYFECT, TRANSMESSANGER, RNAiFECT, SUPERFECT, EFFECTENE, TF-PEI-KIT, CLONFECTIN, and METAFECTINE.

Where two or more nucleotide sequences are introduced, such as sequences encoding two or more subunits of a heteromultimeric protein or sequences encoding two or more different proteins of interest, the sequences may be introduced on the same vector or, preferably, on separate vectors. The DNA can be genomic DNA, cDNA, synthetic DNA or mixtures of them. In some embodiments, nucleic acids encoding a protein of interest or a partial protein of interest do not include additional sequences such that the protein of interest is expressed with additional amino acids that may alter the function of the cells compared to the physiological function of the protein.

In some embodiments, the nucleic acid encoding the protein of interest comprises one or more substitutions, insertions, mutations or deletions, as compared to a nucleic acid sequence encoding the wild-type protein. In embodiments comprising a nucleic acid comprising a mutation, the mutation may be a random mutation or a site-specific mutation. These nucleic acid changes may or may not result in an amino acid substitution. In some embodiments, the nucleic acid is a fragment of the nucleic acid that encodes the protein of interest. Nucleic acids that are fragments or have such modifications encode polypeptides that retain at least one biological property of the protein of interest.

The invention also encompasses cells and cell lines stably expressing a nucleic acid, whose sequence is at least about 85% identical to the "wild type" sequence encoding the protein of interest, or a counterpart nucleic acid derived from a species other than human or a nucleic acid that encodes the same amino acid sequence as any of those nucleic acids. In some embodiments, the sequence identity is at least 85%, 90%, 95%, 96%, 97%, 98%, 99% or higher compared to those sequences. The invention also encompasses cells and cell lines wherein the nucleic acid encoding a protein of interest hybridizes under stringent conditions to the wild type sequence or a counterpart nucleic acid derived from a species other than human, or a nucleic acid that encodes the same amino acid sequence as any of those nucleic acids.

In some embodiments, the cell or cell line comprises a protein-encoding nucleic acid sequence comprising at least one substitution as compared to the wild-type sequence or a counterpart nucleic acid derived from a species other than human or a nucleic acid that encodes the same amino acid sequence as any of those nucleic acids. The substitution may comprise less than 10, 20, 30, or 40 nucleotides or, up to or equal to 1%, 5%, 10% or 20% of the nucleotide sequence. In some embodiments, the substituted sequence may be substantially identical to the wild-type sequence or a counterpart nucieic acid derived from a species other than human a nucleic acid that encodes the same amino acid sequence as any of those nucleic acids (*e.g.*, a sequence at least 85%, 90%, 95%, 96%, 97%, 98%, 99% or higher identical thereto), or be a sequence that is capable of hybridizing under stringent conditions to the wild type sequence or a counterpart nucleic acid derived from a species other than human or a nucleic acid that encodes the same amino acid sequence as any one of those nucleic acids.

In some embodiments, the cell or cell line comprises protein-encoding nucleic acid sequence comprising an insertion into or deletion from the wild type sequence or a counterpart nucleic acid derived from a species other than human or a nucleic acid that encodes the same amino acid sequence as any of those nucleic acids. The insertion or deletion may be less than 10, 20, 30, or 40 nucleotides or up to or equal to 1%, 5%, 10% or 20% of the nucleotide sequence. In some embodiments, the sequences of the insertion or deletion may be substantially identical to the wild type sequence or a counterpart nucleic acid derived from a species other than human or a nucleic acid that encodes the same amino acid sequence as any of those nucleic acids (e.g., a sequence at least 85%, 90%, 95%, 96%, 97%, 98%, 99% or higher identical thereto), or be a sequence that is capable of hybridizing under stringent conditions to the wild-type sequence or a counterpart nucleic acid derived from a species other than human, or a nucleic acid that encodes the same amino acid sequence as any of those nucleic acids.

In some embodiments, the nucleic acid substitution or modification results in an amino acid change, such as an amino acid substitution. For example, an amino acid residue of the wild type protein of interest or a counterpart amino acid derived from a species other than human may be replaced by a conservative or a non-conservative substitution. In some embodiments, the sequence identity between the original and modified amino acid sequence can differ by about 1%, 5%, 10% or 20% or from a sequence substantially identical thereto (*e.g.*, a sequence at least 85%, 90%, 95%, 96%, 97%, 98%, 99% or higher identical thereto).

A "conservative amino acid substitution" is one in which an amino acid residue is substituted by another amino acid residue having a side chain R group with similar chemical properties to the parent amino acid residue (*e.g.*, charge or hydrophobicity). In cases where two or more amino acid sequences differ from each other by conservative substitutions, the percent sequence identity or degree of similarity may be adjusted upwards to correct for the conservative nature of the substitution. Means for making this adjustment are well-known to those of skill in the art. *See, e.g.*, Pearson, Methods Mo!. Biol. 243:307-31 (1994).

Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Preferred conservative amino acids substitution groups are: valine-leucine-isoleucine, phenylalanine-tyrosine, lysine-arginine, alanine-valine, glutamate-aspartate, and asparagine-glutamine. Alternatively, a conservative amino acid substitution is any change having a positive value in the PAM250 log-likelihood matrix disclosed in Gonnet et al., Science 256:1443-45 (1992). A "moderately conservative" replacement is any change having a nonnegative value in the PAM250 log-likelihood matrix.

Conservative modifications in the protein of interest will produce proteins having functional and chemical characteristics similar (*i.e.* at least 50%, 60%, 70%, 80%, 90% or 95% the same) to those of the unmodified protein.

In one embodiment, the host cell is an embryonic stem cell that is then used as the basis for the generation of transgenic animals that produce the protein of interest. Embryonic stem cells stably expressing a functional protein of interest, may be implanted into organisms directly, or their nuclei may be transferred into other recipient cells and these may then be implanted, or they may be used to create transgenic animals. In some embodiments the protein may be expressed in the animal with desired temporal and/or tissue specific expression.

As will be appreciated by those of skill in the art, any vector that is suitable for use with a chosen host cell may be used to introduce a nucleic acid encoding a protein of interest into a host cell. Where more than one vector is used, for example, to introduce two or more different subunits or two or more proteins of interest, the vectors may be the same type or may be of different types.

Examples of vectors that may be used to introduce the nucleic acids into host cells include but are not limited to plasmids, viruses, including retroviruses and lentiviruses, cosmids, artificial chromosomes and may include, for example, pCMVScript, pcDNA3.1 Hygro, pcDNA3.1 neo, pcDNA3.1puro, pSV2neo, pIRES puro, pSV2 zeo. Exemplary mammalian expression vectors that are useful to make the cells and cell lines of the invention include: pFN11A (BIND) Flexi®, pGL4.31, pFC14A (HaloTag® 7) CMV Flexi®, pFC14K (HaloTag® 7) CMV Flexi®, pFN24A (HaloTag® 7) CMVd3 Flexi®, pFN24K (HaloTag® 7) CMVd3 Flexi®,HaloTag™ pHT2,pACT , pAdVAntage™, pALTER®-MAX,pBIND, pCAT®3-Basic, pCAT®3-Control, pCAT®3-Enhancer, pCAT®3-Promoter, pCI, pCMVTNT™ , pG5luc, pSI, pTARGET™, pTNT™, pF12A RM Flexi®, pF12K RM Flexi®, pReg neo,pYES2/GS, pAd/CMV/V5-DEST Gateway® Vector, pAd/PL-DEST™ Gateway® Vector,Gateway® pDEST™27 Vector, Gateway® pEF-DEST51 Vector, Gateway® pcDNAT™-DEST47 vector, pCMV/Bsd Vector, pEF6/His A, B, & c,pcDNAT™6.2-DEST, pLenti6/TR, pLP-AcGFP1-C, pLPS-AcGFP1-N,pLP-IRESneo, pLP-TRE2, pLP-RevTRE, pLP-LNCX, pLP-CMV-HA, pLP-CMV-Myc, pLP-RetroQ and pLP-CMVneo.

In some embodiments, the vectors comprise expression control sequences such as constitutive or conditional promoters, preferably, constitutive promoters are used. One of ordinary skill in the art will be able to select such sequences. For example, suitable promoters include but are not limited to CMV, TK, SV40 and EF-1$\alpha$. In some embodiments, the promoters are inducible, temperature regulated, tissue specific, repressible, heat-shock, developmental, cell lineage specific, eukaryotic, prokaryotic or temporal promoters or a combination or recombination of unmodified or mutagenized, randomized, shuffled sequences of any one or more of the above. In other embodiments, the protein of interest is expressed by gene activation or episomally.

In some embodiments, the vector lacks a selectable marker or drug resistance gene. In other embodiments, the vector optionally comprises a nucleic acid encoding a selectable marker, such as a protein that confers drug or antibiotic resistance or more generally any product that exerts selective pressure on the cell. Where more than one vector is used, each vector may have the same or a different drug resistance or other selective pressure marker. If more than one of the drug resistance or selective pressure markers are the same, simultaneous selection may be achieved by increasing the level of the drug. Suitable markers are well-known to those of skill in the art and include but are not limited to polypeptides products conferring resistance to any one of the following: Neomycin/G418, Puromycin, hygromycin, Zeocin, methotrexate and blasticidin. Although drug selection (or selection using any other suitable selection marker) is not a required step in producing the cells and cell lines of this invention, it may be used to enrich the transfected cell population for stably transfected cells, provided that the transfected constructs are designed to confer drug resistance. If subsequent selection of cells expressing the protein of interest is accomplished using signaling probes, selection too soon following transfection can result in some positive cells that may only be transiently and not stably transfected. However, this effect can be minimized by allowing sufficient cell passage to allow for dilution of transient expression in transfected cells.

In some embodiments, the protein-encoding nucleic acid sequence further comprises a tag. Such tags may encode, for example, a HIS tag, a myc tag, a hemagglutinin (HA) tag, protein C, VSV-G, FLU, yellow fluorescent protein (YFP), green fluorescent protein, FLAG, BCCP, maltose binding protein tag, Nus-tag, Softag-1, Softag-2, Strep-tag, S-tag, thioredoxin, GST, V5, TAP or CBP. A tag may be used as a marker to determine protein expression levels, intracellular localization, protein-protein interactions, regulation of the protein of interest, or the protein's function. Tags may also be used to purify or fractionate proteins.

In the case of cells and cell lines expressing an RNA of interest, the RNA can be of any type including antisense RNA, short interfering RNA (siRNA), transfer RNA (tRNA), structural RNA, ribosomal RNA, heterogeneous nuclear RNA (hnRNA) and small nuclear RNA (snRNA).

In embodiments in which the cells and cell lines of the invention express a functional protein of interest, the protein can be any protein including but not limited to single chain proteins, multi-chain proteins, hetero-multimeric proteins. In the case of multimeric proteins, in some embodiments the cells express all of the subunits that make up the native protein. The protein can have a "wild type" sequence or may be a variant. In some embodiments, the cells express a protein that comprises a variant of one or more of the subunits including allelic variants, splice variants, truncated forms, isoforms, chimeric subunits and mutated forms that comprise amino acid substitutions (conservative or non-conservative), modified amino acids including chemically modified amino acids, and non-naturally occurring amino acids. A heteromultimeric protein expressed by cells or cell lines of the invention may comprise subunits from two or more species, such as from species homoiogs of the protein of interest.

In some embodiments, the cells of the invention express two or more functional proteins of interest. According to the invention, such expression can be from the introduction of a nucleic acid encoding all or part of a protein of interest, from the introduction of a nucleic acid that activates the transcription of all or part of a protein of interest from an endogenous sequence or from any combination thereof. The cells may express any desired number of proteins of interest. In various embodiments, the cells express three, four, five, six, or more proteins of interest. For example, the invention contemplates cells and cell lines that stably express functional proteins in a pathway of interest, proteins from intersecting pathways induding enzymatic pathways, signaling pathways regulatory pathways and the like.

In particular, the protein expressed by the cells or cell lines used in the method are proteins for which stable functional cell lines have not previously been available. Without being bound by theory, it is believed that some reasons why such cell lines have not heretofore been possible include that the protein is highly complex and without preparing a large number of cells expressing the protein, it has not been possible to identify one in which the protein is properly assembled; or because no ligand or modulator of the protein is known for use in identifying a cell or cell line that expresses the protein in functional form; or because the protein is cytotoxic when expressed outside its natural context, such as in a content that does not naturally express it.

Cells and cell lines of the invention can be made that consistently express any protein of interest either intracellular, surface or secreted. Such proteins include heteromultimeric ion channels, ligand gated (such as GABA A receptor), ion channels (such as CFTR), heteromultimeric ion channels, voltage gated (such as NaV), heteromultimeric ion channel, non-ligand gated (Epithelial sodium channel, ENaC), heterodimeric GPCRs (such as opioid receptors, taste receptors including sweet, umami and bitter), other GPCRs, Orphan GPCRs, GCC, opioid receptors, growth hormone receptors, estrogen/hgh, nuclear or membrane bound, TGF receptors, PPAR nuclear hormone receptor, nicotinics/Ach and immune receptors such as B-cell/T-cell receptors.

Cells and cell lines of the invention can express functional proteins including any protein or combination of proteins listed in Tables 2-13 (Mammalian G proteins, Human orphan GPCRs, Human opioid receptors, Human olfactory receptors, Canine olfactory receptors, Mosquito olfactory receptors, Other heteromultimeric receptors and GABA receptors.

The cells and cell lines of the invention have a number of attributes that make them particularly advantageous for any use where it is desired that cells provide consistent expression of a functional protein of interest over time. The terms "stable" or "consistent" as applied to the expression of the protein and the function of the protein is meant to distinguish the cells and cell lines of the invention from cells with transient expression or variable function, as the terms "stable expression" and "transient expression" would be understood by a person of skill in the art. A cell or cell line of the invention has stable or consistent expression of functional protein that has less than 10% variation for at least 2-4 days.

In various embodiments, the cells or cell lines of the invention express the functional RNA or protein of interest, i.e., the cells are consistently functional after growth for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, 200 days or over 200 days, where consistent expression or consistently functional refers to a level of expression that does not vary by more than: 1%, 2%, 3%, 4%, 5%, 6%, 7%, 8% 9% or 10% over 2 to 4 days of continuous cell culture; 1%, 2%, 4%, 6%, 8%, 10% or 12% over 5 to 15 days of continuous cell culture; 1%, 2%, 4%, 6%, 8%, 10%, 12%, 14%, 16%, 18% or 20% over 16 to 20 days of continuous cell culture; 1%, 2%, 4%, 6%, 8%, 10%, 12%, 14%, 16%, 18%, 20%, 22%, 24% over 21 to 30 days of continuous cell culture; 1%, 2%, 4%, 6%, 8%, 10%, 12%, 14%, 16%, 18%, 20%, 22%, 24%, 26%, 28% or 30% over 30 to 40 days of continuous cell culture; 1%, 2%, 4%, 6%, 8%, 10%, 12%, 14%, 16%, 18%, 20%, 22%, 24%, 26%, 28% or 30% over 41 to 45 days of continuous cell culture; 1%, 2%, 4%, 6%, 8%, 10%, 12%, 14%, 16%, 18%, 20%, 22%, 24%, 26%, 28% or 30% over 45 to 50 days of continuous cell culture; 1%, 2%, 4%, 6%, 8%, 10%, 12%, 14%, 16%, 18%, 20%, 22%, 24%, 26%, 28%, 30% or 35% over 45 to 50 days of continuous cell culture; 1%, 2%, 4%, 6%, 8%, 10%, 12%, 14%, 16%, 18%, 20%, 22%, 24%, 26%, 28% or 30% or 35% over 50 to 55 days of continuous cell culture; 1%, 2%, 4%, 6%, 8%, 10%, 12%, 14%, 16%, 18%, 20%, 22%, 24%, 26%, 28%, 30% or 35% over 50 to 55 days of continuous cell culture; 1%, 2%, 3%, 4%, 5%, 10%, 15%, 20%, 25%, 30%, 35% or 40% over 55 to 75 days of continuous cell culture; 1%, 2%, 3%, 4%, 5%, 5%, 10%, 15%, 20%, 25%, 30%, 35%, 40% or 45% over 75 to 100 days of continuous cell culture; 1%, 2%, 3%, 4%, 5%, 5%, 10%, 15%, 20%, 25%, 30%, 35%, 40% or 45% over 101 to 125 days of continuous cell culture; 1%, 2%, 3%, 4%, 5%, 5%, 10%, 15%, 20%, 25%, 30%, 35%, 40% or 45% over 126 to 150 days of continuous cell culture; 1%, 2%, 3%, 4%, 5%, 5%, 10%, 15%, 20%, 25%, 30%, 35%, 40% or 45% over 151 to 175 days of continuous cell culture; 1%, 2%, 3%, 4%, 5%, 5%, 10%, 15%, 20%, 25%, 30%, 35%, 40% or 45% over 176 to 200 days of continuous cell culture; 1%, 2%, 3%, 4%, 5%, 5%, 10%, 15%, 20%, 25%, 30%, 35%, 40% or 45% over more than 200 days of continuous cell culture.

Cells may be selected that have desirable properties in addition to the stable expression of functional protein. Any desired property that can be detected may be selected for. Those of skill in the art will aware of such characteristics. By way of non-limiting example, such properties include:

fragility, morphology and adherence to a solid surface, monodispersion by trypsin or cell dissociation reagent, adaptability to the automated culture conditions,
performance under serum-containing conditions, performance in serum-free conditions, convertability to serum-free suspension conditions, propensity to form clumps, propensity to form monodisperse cell layers following passaging, resilience, propensity to remain attached to growth chamber surfaces under fluid addition steps of different force, non-fragmented nucleus, lack of intracellular vacuoles, lack of microbial contamination, lack of mycoplasma, lack of viral contamination, clonality, consistency of gross physical properties of cells within wells, propensity for growth below/at/above room temperature, propensity for tolerance of various temperatures for various time periods, propensity of cells to evenly uptake

plasmid/oligonucleotides/fluorogenic probes/peptides/proteins/compounds, propensity of cells to withstand incubation with DMSO/EtOH/MeOH, organic solvent/detergent, propensity of cells to withstand maintained UPR induction, propensity of cells to withstand exposure to DTT, propensity of cells to be infected with viral/lentiviral/cosmid vectors, endogenous expression of desired RNA(s)/protein(s) or lack thereof, chromosomal number, chromosomal aberrations, amenable to growth at 5/6/7/8/9pH, tolerance to UV/mutagen/radiation, ability to maintain the above characteristics under altered/manual/scaled-up growth conditions (i.e., including reactors).

Cells and cell lines of the invention have enhanced properties as compared to cells and cell lines made by conventional methods. For example, the cells and cell lines of this invention have enhanced stability of expression and/or levels of expression (even when maintained in cultures without selective pressure, including, for example, antibiotics and other drugs). In other embodiments, the cells and cell lines of the invention have high Z' values in various assays. In still other embodiments, the cells and cell lines of this invention are improved in context of their expression of a physiologically relevant protein activity as compared to more conventionally engineered cells. These properties enhance and improve the ability of the cells and cell lines of this invention to be used for any use, whether in assays to identify modulators, for cell therapy, for protein production or any other use and improve the functional attributes of the identified modulators.

A further advantageous property of the cells and cell lines of the invention is that they stably express the protein of interest in the absence of drug or other selective pressure. Thus, in preferred embodiments, the cells and cell lines of the invention are maintained in culture without any selective pressure. In further embodiments, cells and cell lines are maintained without any drug or antibiotics. As used herein, cell maintenance refers to culturing cells after they have been selected as described for protein expression. Maintenance does not refer to the optional step of growing cells under selective pressure (*e.g.*, an antibiotic) prior to cell sorting where marker(s) introduced into the cells allow enrichment of stable transfectants in a mixed population.

Drug-free and selective pressure-free cell maintenance of the cells and cell lines of this invention provides a number of advantages. For example, drug-resistant cells may not express the co-transfected transgene of interest at adequate levels, because the selection relies on survival of the cells that have taken up the drug resistant gene, with or without the transgene. Further, selective drugs and other selective pressure factors are often mutagenic or otherwise interfere with the physiology of the cells, leading to skewed results in cell-based assays. For example, selective drugs may decrease susceptibility to apoptosis (Robinson et al., Biochemistry, 36(37):11169-11178 (1997)), increase DNA repair and drug metabolism (Deffie et al., Cancer Res. 48(13):3595-3602 (1988)), increase cellular pH (Thiebaut et al., J Histochem Cytochem. 38(5):685-690 (1990); Roepe et al., Biochemistry. 32(41):11042-11056 (1993); Simon et al., Proc Natl Acad Sci USA. 91(3):1128-1132 (1994)), decrease lysosomal and endosomal pH (Schindler et al., Biochemistry. 35(9):2811-2817 (1996); Altan et al., J Exp Med. 187(10):1583-1598 (1998)), decrease plasma membrane potential (Roepe et al., Biochemistry. 32(41):11042-11056 (1993)), increase plasma membrane conductance to chloride (Gill et al., Cell. 71(1):23-32 (1992)) and ATP (Abraham et al., Proc Natl Acad Sci U S A. 90(1):312-316 (1993)), and increase rates of vesicle transport (Altan et al., Proc Natl Acad Sci USA. 96(8):4432-4437 (1999)). Thus, the cells and cell lines of this invention allow screening assays that are free from the artifacts caused by selective pressure. In some preferred embodiments, the cells and cell lines of this invention are not cultured with selective pressure factors, such as antibiotics, before or after cell sorting, so that cells and cell lines with desired properties are isolated by sorting, even when not beginning with an enriched cell population.

The cells and cell lines of the invention have enhanced stability as compared to cells and cell lines produced by conventional methods in the context of expression and expression levels (RNA or protein). To identify cells and cell lines characterized by such stable expression, a cell or cell line's expression of a protein of interest is measured over a timecourse and the expression levels are compared. Stable cell lines will continue expressing (RNA or protein) throughout the timecourse. In some aspects of the invention, the timecourse may be for at least one week, two weeks, three weeks, etc., or at least one month, or at least two, three, four, five, six, seven, eight or nine months, or any length of time in between.

Isolated cells and cell lines may be further characterized, such as by PCR, RT-PCR, qRT-PCR and single end-point RT-PCR to determine the absolute amounts and relative amounts (in the case of multisubunit proteins or multiple proteins of interest) being expressed (RNA). Preferably, the expansion levels of the subunits of a multi-subunit protein are substantially the same in the cells and cell lines of this invention.

In other embodiments, the expression of a functional protein of interest is assayed over time. In these embodiments, stable expression is measured by comparing the results of functional assays over a timecourse. The assay of cell and cell line stability based on a functional assay provides the benefit of identifying cells and cell lines that not only stably express the protein (RNA or protein), but also stably produce and properly process (*e.g.*, post-translational modification, subunit assembly, and localization within the cell) the protein to produce a functional protein.

Cells and cell lines of the invention have the further advantageous property of providing assays with high reproducibility as evidenced by their Z' factor. See Zhang JH, Chung TD, Oldenburg KR, "A Simple Statistical Parameter for Use in Evaluation and Validation of High Throughput Screening Assays." J. Biomol. Screen. 1999;4(2):67-73, which is incorporated herein by reference in its entirety. Z' values relate to the quality of a cell or cell line because it reflects the degree to which a cell or cell line will respond consistently to modulators. Z' is a statistical calculation that takes into account the signal-to-noise range and signal variability (*i.e.,* from well to well) of the functional response to a reference compound across a multiwell plate is Z' calculated using Z' data obtained from multiple wells with a positive control and multiple wells with a negative control. The ratio of their combined standard deviations multiplied by three to the difference factor, in their mean values is subtracted from one to give the Z' according the equation below:

$$Z' \text{ factor} = 1 - ((3\sigma_{\text{positive control}} + 3\sigma_{\text{negative control}}) / (\mu_{\text{positive control}} - \mu_{\text{negative control}}))$$

If the factor is 1.0, which would indicate an ideal assay with theoretical maximum Z' no variability and limitless dynamic range. As used herein, a "high Z'" refers to a Z' factor of Z' of at least 0.6, at least 0.7, at least 0.75 or at least 0.8, or any decimal in between 0.6 and 1.0. In the case of a complex target, a high Z' means a Z' of at least 0.4 or greater. A score of close to 0 is undesirable because it indicates that there is overlap between positive and negative controls. In the industry, for simple cell-based assays, Z' scores up to 0.3 are considered marginal scores, Z' scores between 0.3 and 0.5 are considered acceptable, and Z' scores above 0.5 are considered excellent. Cell-free or biochemical assays may approach scores for cell-based systems tend to be lower because higher Z' scores, but Z' cell-based systems are complex.

As those of ordinary skill in the art will recognize cell-based assays using conventional cells expressing even a single chain protein do not typically achieve a Z' higher than 0.5 to 0.6. Cells with engineered expression (either from introduced coding sequences or gene activation) of multi-subunit proteins, if even reported in the art, would be lower due to their added complexity. Such cells would not be reliable for use in assays because the results would not be reproducible. Cells and cell lines of this invention, on the other hand, have higher Z' values and advantageously produce consistent results in assays. Indeed, the cells and cell lines of the invention provide the basis for high throughput screening (HTS) compatible assays because they generally have values than conventionally produced cells. In some aspects of the invention, the cells and cell lines result in Z' of at least 0.3, at least 0.4, at least 0.5, at least 0.6, at least 0.7, or at least 0.8, Even Z' values of at least 0.3 - 0.4 for the cells and cell lines of the invention are advantageous because the proteins of interest are multigene targets. In other aspects of the invention, the cells and cell lines of the invention result in a Z' of at least 0.7, at least 0.75 or at least 0.8 even after the cells are maintained for multiple passages, *e.g.*, between 5-20 passages, including any integer in between 5 and 20. In some aspects of the invention, the cells and cell lines result in a Z' of at least 0.7, at least 0.75 or at least 0.8 in cells and cell lines maintained for 1, 2, 3, 4 or 5 weeks or 2, 3, 4, 5, 6, 7, 8 or 9 months, including any period of time in between.

In a further aspect, the invention provides a method for producing the cells and cell lines of the invention. In one embodiment, the method comprises the steps of:

a) providing a plurality of cells that express mRNA encoding the protein of interest;
b) dispersing cells individually into individual culture vessels, thereby providing a plurality of separate cell cultures
c) culturing the cells under a set of desired culture conditions using automated cell culture methods characterized in that the conditions are substantially identical for each of the separate cell cultures, during which culturing the number of cells in each separate cell culture is normalized, and wherein the separate cultures are passaged on the same schedule;
d) assaying the separate cell cultures for at least one desired characteristic of the protein of interest at least twice; and
e) identifying a separate cell culture that has the desired characteristic in both assays.

According to the method, the cells are cultured under a desired set of culture conditions. The conditions can be any desired conditions. Those of skill in the art will understand what parameters are comprised within a set of culture conditions. For example, culture conditions include but are not limited to: the media (Base media (DMEM, MEM, RPMI, serum-free, with serum, fully chemically defined, without animal-derived components), mono and divalent ion (sodium, potassium, calcium, magnesium) concentration, additional components added (amino acids, antibiotics, glutamine, glucose or other carbon source, HEPES, channel blockers, modulators of other targets, vitamins, trace elements, heavy metals, co-factors, growth factors, anti-apoptosis reagents), fresh or conditioned media, with HEPES, pH, depleted of certain nutrients or limiting (amino acid, carbon source)), level of confluency at which cells are allowed to attain before split/passage, feeder layers of cells, or gamma-irradiated cells, $CO_2$, a three gas system (oxygen, nitrogen, carbon dioxide), humidity, temperature, still or on a shaker, and the like, which will be well known to those of skill in the art.

The cell culture conditions may be chosen for convenience or for a particular desired use of the cells. Advantageously, the invention provides cells and cell lines that are optimally suited for a particular desired use. That is, in embodiments of the invention in which cells are cultured under conditions for a particular desired use, cells are selected that have desired characteristics under the condition for the desired use. By way of illustration, if cells will be used in assays in plates where it is desired that the cells are adherent, cells that display adherence under the conditions of the assay may be selected. Similarly, if the cells will be used for protein production, cells may be cultured under conditions appropriate for protein production and selected for advantageous properties for this use.

In some embodiments, the method comprises the additional step of measuring the growth rates of the separate cell cultures. Growth rates may be determined using any of a variety of techniques means that will be well known to the skilled worker. Such techniques include but are not limited to measuring ATP, cell confluence, light scattering, optical density (e.g., OD 260 for DNA). Preferably growth rates are determined using means that minimize the amount of time that the cultures spend outside the selected culture conditions.

In some embodiments, cell confluency is measured and growth rates are calculated from the confiuency values. In some embodiments, cells are dispersed and clumps removed prior to measuring cell confluency for improved accuracy. Means for monodispersing cells are well-known and can be achieved, for example, by addition of a dispersing reagent to a culture to be measured. Dispersing agents are well-known and readily available, and include but are not limited to enzymatic dispering agents, such as trypsin, and EDTA-based dispersing agents. Growth rates can be calculated from confluency date using commercially available software for that purpose such as HAMILTON VECTOR. Automated confluency measurement, such as using an automated microscopic plate reader is particularly useful. Plate readers that measure confluency are commercially available and include but are not limited to the CLONE SELECT IMAGER (Genetix). Typically, at least 2 measurements of cell confluency are made before calculating a growth rate. The number of confluency values used to determine growth rate can be any number that is convenient or suitable for the culture. For example, confluency can be measured multiple times over e.g., a week, 2 weeks, 3 weeks or any length of time and at any frequency desired.

When the growth rates are known, according to the method, the plurality of separate cell cultures are divided into groups by similarity of growth rates. By grouping cultures into growth rate bins, one can manipulate the cultures in the group together, thereby providing another level of standardization that reduces variation between cultures. For example, the cultures in a bin can be passaged at the same time, treated with a desired reagent at the same time, etc. Further, functional assay results are typically dependent on cell density in an assay well. A true comparison of individual clones is only accomplished by having them plated and assayed at the same density. Grouping into specific growth rate cohorts enables the plating of clones at a specific density that allows them to be functionally characterized in a high throughput format

The range of growth rates in each group can be any convenient range. It is particularly advantageous to select a range of growth rates that permits the cells to be passaged at the same time and avoid frequent renormalization of cell numbers. Growth rate groups can include a very narrow range for a tight grouping, for example, average doubling times within an hour of each other. But according to the method, the range can be up to 2 hours, up to 3 hours, up to 4 hours, up to 5 hours or up to 10 hours of each other or even broader ranges. The need for renormalization arises when the growth rates in a bin are not the same so that the number of cells in some cultures increases faster than others. To maintain substantially identical conditions for all cultures in a bin, it is necessary to periodically remove cells to renormalize the numbers across the bin. The more disparate the growth rates, the more frequently renormalization is needed.

In step d) the cells and cell lines may be tested for and selected for any physiological property including but not limited to: a change in a cellular process encoded by the genome ;a change in a cellular process regulated by the genome; a change in a pattern of chromosomal activity; a change in a pattern of chromosomal silencing; a change in a pattern of gene silencing; a change in a pattern or in the efficiency of gene activation; a change in a pattern or in the efficiency of gene expression; a change in a pattern or in the efficiency of RNA expression; a change in a pattern or in the efficiency of RNAi expression; a change in a pattern or in the efficiency of RNA processing; a change in a pattern or in the efficiency of RNA transport; a change in a pattern or in the efficiency of protein translation; a change in a pattern or in the efficiency of protein folding; a change in a pattern or in the efficiency of protein assembly; a change in a pattern or in the efficiency of protein modification; a change in a pattern or in the efficiency of protein transport; a change in a pattern or in the efficiency of transporting a membrane protein to a cell surface change in growth rate; a change in cell size; a change in cell shape; a change in cell morphology; a change in % RNA content; a change in % protein content; a change in % water content; a change in % lipid content; a change in ribosome content; a change in mitochondrial content; a change in ER mass; a change in plasma membrane surface area; a change in cell volume; a change in lipid composition of plasma membrane; a change in lipid composition of nuclear envelope; a change in protein composition of plasma membrane; a change in protein; composition of nuclear envelope; a change in number of secretory vesicles; a change in number of lysosomes; a change in number of vacuoles; a change in the capacity or potential of a cell for: protein production, protein secretion, protein folding, protein assembly, protein modification, enzymatic modification of protein, protein glycosylation, protein phosphorylation, protein dephosphorylation, metabolite biosynthesis, lipid biosynthesis, DNA synthesis, RNA synthesis, protein synthesis, nutrient absorption, cell growth, mitosis, meiosis, cell division, to dedifferentiate, to transform into a stem cell, to transform into a pluripotent cell, to transform into a omnipotent cell, to transform into a stem cell type of any organ (i.e. liver, lung, skin, muscle, pancreas, brain, testis, ovary, blood, immune system, nervous system, bone, cardiovascular system, central nervous system, gastro-intestinal tract, stomach, thyroid, tongue, gall bladder, kidney, nose, eye, nail, hair, taste bud), to transform into a differentiated any cell type (i.e. muscle, heart muscle, neuron, skin, pancreatic, blood, immune, red blood cell, white blood cell, killer T-cell, enteroendocrine cell, taste, secretory cell, kidney, epithelial cell, endothelial cell, also including any of the animal or human cell types already listed that can be used for introduction of nucleic acid sequences), to uptake DNA, to uptake small molecules, to uptake fluorogenic probes, to uptake RNA, to adhere to solid surface, to adapt to serum-free conditions, to adapt to serum-free suspension conditions, to adapt to scaled-up cell culture, for use for large scale cell culture, for use in drug discovery, for use in high throughput screening, for use in a functional cell based assay, for use in membrane potential assays, for use in calcium flux assays, for use in G-protein reporter assays, for use in reporter cell based assays, for use in ELISA studies, for use in in vitro assays, for use in vivo applications, for use in secondary testing, for use in compound testing, for use in a binding assay, for use in panning assay, for use in an antibody panning assay, for use in imaging assays, for use in microscopic imaging assays, for use in multiwell plates, for adaptation to automated cell culture, for adaptation to miniaturized automated cell culture, for adaptation to large-scale automated cell culture, for adaptation to cell culture in multiwell plates (6, 12, 24, 48, 96, 384, 1536 or higher density), for use in cell chips, for use on slides, for use on glass slides, for microarray on slides or glass slides, for immunofluorescence studies, for use in protein purification, for use in biologics production, for use in the production of industrial enzymes, for use in the production of reagents for research, for use in vaccine development, for use in cell therapy, for use in implantation into animals or humans, for use in isolation of factors secreted by the cell, for preparation of cDNA libraries, for purification of RNA, for purification of DNA, for infection by pathogens, viruses or other agent, for resistance to infection by pathogens, viruses or other agents, for resistance to drugs, for suitability to be maintained under automated miniaturized cell culture conditions, for use in the production of protein for characterization, including: protein crystallography, vaccine development, stimulation of the immune system, antibody production or generation or testing of antibodies. Those of skill in the art will readily recognize suitable tests for any of the above-listed properties.

Tests that may be used to characterize cells and cell lines of the invention and/or matched panels of the invention include but are not limited to: Amino acid analysis, DNA sequencing, Protein sequencing, NMR, A test for protein transport, A test for nucelocytoplasmic transport, A test for subcellular localization of proteins, A test for subcellular localization of nucleic acids, Microscopic analysis, Submicroscopic analysis, Fluorescence microscopy, Electron microscopy, Confocal microscopy, Laser ablation technology, Cell counting and Dialysis. The skilled worker would understand how to use any of the above-listed tests.

When collections or panels of cells or cell lines are produced, e.g., for drug screening, the cells or cell lines in the collection or panel may be matched such that they are the same (including substantially the same) with regard to one or more selective physiological properties. The "same physiological property" in this context means that the selected physiological property is similar enough amongst the members in the collection or panel such that the cell collection or panel can produce reliable results in drug screening assays; for example, variations in readouts in a drug screening assay will be due to, e.g., the different biological activities of test compounds on cells expressing different forms of a protein, rather than due to inherent variations in the cells. For example, the cells or cell lines may be matched to have the same growth rate, i.e., growth rates with no more than one, two, three, four, or five hour difference amongst the members of the cell collection or panel. This may be achieved by, for example, binning cells by their growth rate into five, six, seven, eight, nine, or ten groups, and creating a panel using cells from the same binned group. Methods of determining cell growth rate are well known in the art. The cells or cell lines in a panel also can be matched to have the same Z' factor (e.g., Z' factors that do not differ by more than 0.1), protein expression level (e.g., CFTR expression levels that do not differ by more than 5%, 10%, 15%, 20%, 25%, or 30%), RNA expression level, adherence to tissue culture surfaces, and the like. Matched cells and cell lines can be grown under identical conditions, achieved by, e.g., automated parallel processing, to maintain the selected physiological property.

In one embodiment, the panel is matched for growth rate under the same set of conditions. Such a panel, also referred to herein as a matched panel, are highly desirable for use in a wide range of cell-based studies in which it is desirable to compare the effect of an experimental variable across two or more cell lines. Cell lines that are matched for growth rate maintain roughly the same number of cells per well over time thereby reducing variation in growth conditions, such as nutrient content between cell lines in the panel

According to the invention, matched panels may have growth rates within any desired range, depending on a number of factors including the characteristics of the cells, the intended use of the panel, the size of the panel, the culture conditions, and the like. Such factors will be readily appreciated by the skilled worker.

Growth rates may be determined by any suitable and convenient means, the only requirement being that the growth rates for all of the cell lines for a matched panel are determined by the same means. Numerous means for determining growth rate are known as described herein.

A matched panel of the invention can comprise any number of clonal cell lines. The maximum number of clonal cell lines in the panel will differ for each use and user and can be as many as can be maintained. In various embodiments, the panel may comprise 2, 3, 4, 5, 6, 7, 8, 9, 10 or more clonal cell lines, for example, at least 12, at least 15, at least 20, at least 24, at least 25, at least 30, at least 35, at least 40, at least 45, at least 48, at least 50, at least 75, at least 96, at least 100, at least 200, at least 300, at least 384, at least 400 or more clonal cell lines.

According to the invention, the panel comprises a plurality of clonal cell lines, that is, a plurality of cell lines generated from a different single parent cell. Any desired cell type may be used in the production of a matched panel. The panel can comprise cell lines of all the same cell type or cell lines of different cell types.

The clonal cell lines in the panel stably express one or more proteins of interest. The stable expression can be for any length of time that is suitable for the desired use of the panel but at a minimum, is sufficiently long to permit selection and use in a matched panel.

The clonal cell lines in the matched panel may all express the same one or more proteins of interest or some clonal cell lines in the panel may express different proteins of interest.

in some embodiments, the matched panel comprises one or more clonal cell lines that express different proteins of interest. That is, a first clonal cell line in the panel may express a first protein of interest, a second clonal cell line in the panel may express a second protein of interest, a third cell line may express a third protein of interest, etc. for as many different proteins of interest as are desired. The different proteins of interest may be different isoforms, allelic variants, splice variants, or mutated (including but not limited to sequence mutated or truncated), chimeric or chemically including enzymatically modified forms of a protein of interest. In some embodiments the different proteins can be members of a functionally defined group of proteins, such as a panel of bitter taste receptors or a panel of kinases. In some embodiments the different proteins may be part of the same or interrelated signaling pathways. In still other panels involving heteromultimeric proteins (including heterodimers), the panel may comprise two or more different combinations of subunits up to all possible combinations of subunits. The combinations may comprise subunit sequence variants, subunit isoform combinations, interspecies combinations of subunits and combinations of subunit types.

By way of example, Gamma-aminobutyric acid $(GABA)_A$ receptors typically comprise two alpha subunits, two beta subunits and a gamma subunit. There are 6 alpha isoforms, 5 beta isoforms, 4 gamma isoforms, and a delta, a pi, a theta and an epsilon subunit. The present invention contemplates panels comprising two or more combinations of any of these subunits including panels comprising every possible combination of alpha, beta, gamma, delta, pi, epsilon and theta subunit. Further, the GABA receptor family also includes $GABA_B$ and $GABA_C$ receptors. The invention also contemplates panels that comprise any combination of $GABA_{A,}$, $GABA_B$ and $GABA_C$ subunits. In some embodiments, such panels comprise human GABA subunits. mammalian GABA receptor panel such as a non-human primate (eg, cynomolgus) GABA receptor, mouse, rat or human GABA receptor panels or mixtures thereof

In a further example, the invention contemplates one or more epithelial sodium channel (ENaC) panels, including any mammalian ENaC panel such as a non-human primate (eg, cynomolgus) ENaC, mouse, rat or human ENaC panels or mixtures thereof . Like GABA receptors, intact ENaC comprise multiple subunits: alpha or delta, beta and gamma. The invention contemplates panels with at least two different combinations of ENaC subunits and also contemplates all possible combinations of ENaC subunits, including combinations of subunits from different species, combinations of isoforms, allelic variants, SNPs, chimeric subunits, forms comprising modified and/or non-natural amino acids and chemically modified such as enzymatically modified subunits. The present invention also contemplates panels comprising any ENaC form set forth in International Application PCT/US09/31936, the contents of which are incorporated by reference in its entirety.

In a further particular embodiment, a matched panel of 25 bitter taste receptors comprising cell lines that express native (no tag) functional bitter receptors listed in Table 10. In some embodiments, the panel is matched for growth rate. In some embodiments the panel is matched for growth rate and an additional physiological property of interest. In some embodiments the cell lines in the panel were generated in parallel and/or screened in parallel.

Further exemplary but non-limited examples of panels and their uses are the following: a panel of odorant receptors (insect, canine, human, bed bug), for example to profile of fragrances or to discovery of modulators; panels of cells expressing a gene fused to a test peptide, i.e., to find a peptide that works to internalize a cargo such as a protein, including a monoclonal antibody or a non-protein drug into cells (the cargo could be a reporter such as GFP or AP). Related to this embodiment, supernatants from cells of this panel could be added to other cells for assessment of internalization. In such an embodiment, the panel may comprise different cell types to assess cell-type specific delivery. A panel of cell lines expressing different monoclonal antibody heavy chain/light chain combinations to identify active mAbs. An antibody panel also could provide a series of derivatized versions of a monoclonal antibody to identify one with improved characteristics, such as stability in serum, binding affinity and the like. Yet another panel could be used to express a target protein in the presence of various signaling molecules, such as different G-proteins. Still another type of panel could be used to test variants of a target proteins for improved activity/stability. A panels could comprise single nucleotide polymorphs (SNPs) or other mutated forms of a target protein to select modulators that act on a subset, many or all forms. Other panels could be used to define the patterns of activity of test compounds on a family of proteins or isoforms of a protein (such as $GABA_A$ or other CNS ion channels). Differentially acting compounds could then be used in further study to determine the function/role/localization of corresponding subunit combinations *in vivo.* The test compounds could be known modulators that failed in the clinic or ones that have adverse off-target effects, to determine subunit combinations that may correlate with such effects. Still other panels could be used in HTS for parallel screening for reliable assessment of compounds' activity at multiple target subtypes to assist in finding compounds active at desired targets and that have minimal off target effects.

The panels can include any desired group of proteins and all such panels are contemplated by the invention.

A matched panel of the invention may be produced by generating the different cell lines for the panel sequentially, in parallel or a combination of both. For example, one can make each cell line individually and then match them. More preferably, to minimize difference between the cell lines, sequentially generated cell lines can be frozen at the same stage or passage number and thawed in parallel. Even more preferably, the cell lines are made in parallel.

In a preferred embodiments, the cell lines in a panel are screened or assayed in parallel.

According to the invention, the cell lines of the matched panel are maintained under the same cell culture conditions including but not limited to the same culture media, temperature, and the like. All of the cell lines in the panel are passaged at the same frequency which may be any desired frequency depending on a number of factors including cell type, growth rate, As will be appreciated, to maintain roughly equal numbers of cells from cell line to cell line of the panel, the number of cells should be normalized periodically.

According to the method, cells may be cultured in any cell culture format so long as the cells or cell lines are dispersed in individual cultures prior to the step of measuring growth rates. For example, for convenience, cells may be initially pooled for culture under the desired conditions and then individual cells separated one cell per well or vessel. Cells may be cultured in multi-well tissue culture plates with any convenient number of wells. Such plates are readily commercially available and will be well knows to a person of skill in the art. In some cases, cells may preferably be cultured in vials or in any other convenient format, the various formats will be known to the skilled worker and are readily commericially available.

In embodiments comprising the step of measuring growth rate, prior to measuring growth rates, the cells are cultured for a sufficient length of time for them to acclimate to the culture conditions. As will be appreciated by the skilled worker, the length of time wiii vary depending on a number of factors such as the cell type, the chosen conditions, the culture format and may be any amount of time from one day to a few days, a week or more.

Preferably, each individual culture in the plurality of separate cell cultures is maintained under substantially identical conditions a discussed below, including a standardized maintenance schedule. Another advantageous feature of the method is that large numbers of individual cultures can be maintained simultaneously, so that a cell with a desired set of traits may be identified even if extremely rare. For those and other reasons, according to the invention, the plurality of separate cell cultures are cultured using automated cell culture methods so that the conditions are substantially identical for each well. Automated cell culture prevents the unavoidable variability inherent to manual cell culture.

Any automated cell culture system may be used in the method of the invention. A number of automated cell culture systems are commercially available and will be well-known to the skilled worker. In some embodiments, the automated system is a robotic system. Preferably, the system includes independently moving channels, a multichannel head (for instance a 96-tip head) and a gripper or cherry-picking arm and a HEPA filtration device to maintain sterility during the procedure. The number of channels in the pipettor should be suitable for the format of the culture. Convenient pipettors have, e.g., 96 or 384 channels. Such systems are known and are commercially available. For example, a MICROLAB STAR™ instrument (Hamilton) may be used in the method of the invention. The automated system should be able to perform a variety of desired cell culture tasks. Such tasks will be known by a person of skill in the art. They include but are not limited to: removing media, replacing media, adding reagents, cell washing, removing wash solution, adding a dispersing agent, removing cells from a culture vessel, adding cells to a culture vessel an the like.

The production of a cell or cell line of the invention may include any number of separate cell cultures. However, the advantages provided by the method increase as the number of cells increases. There is no theoretical upper limit to the number of cells or separate cell cultures that can be utilized in the method. According to the invention, the number of separate cell cultures can be two or more but more advantageously is at least 3, 4, 5, 6, 7, 8, 9, 10 or more separate cell cultures, for example, at least 12, at least 15, at least 20, at least 24, at least 25, at least 30, at least 35, at least 40, at least 45, at least 48, at least 50, at least 75, at least 96, at least 100, at least 200, at least 300, at least 384, at least 400, at least 500, at least 1000, at least 10,000, at least 100,000, at least 500,000 or more.

In some embodiments, the cells and cell lines of the invention that are cultured as described are cells that have previously been selected as positive for a nucleic acid of interest, which can be an introduced nucleic acid encoding all or part of a protein of interest or an introduced nucleic acid that activates transcription of a sequence encoding all or part of a protein of interest. In some embodiments, the cells that are cultured as described herein are cells that have been selected as positive for mRNA encoding the protein of interest.

To make cells and cell lines of the invention, one can use, for example, the technology described in U.S. Patent 6,692,965 and WO/2005/079462. Both of these documents are incorporated herein by reference in their entirety. This technology provides real-time assessment of millions of cells such that any desired number of clones (from hundreds to thousands of clones). Using cell sorting techniques, such as flow cytometric cell sorting (*e.g.,* with a FACS machine) or magnetic cell sorting (e.g., with a MACS machine), one cell per well is automatically deposited with high statistical confidence in a culture vessel (such as a 96 well culture plate). The speed and automation of the technology allows multigene recombinant cell lines to be readily isolated.

Using the technology, the RNA sequence for a protein of interest may be detected using a signaling probe, also referred to as a molecular beacon or fluorogenic probe. In some embodiments, the vector containing the coding sequence has an additional sequence coding for an RNA tag sequence. "Tag sequence" refers to a nucleic acid sequence that is an expressed RNA or portion of an RNA that is to be detected by a signaling probe. Signaling probes may detect a variety of RNA sequences, any of which may be used as tags, including those encoding peptide and protein tags described above. Signaling probes may be directed against the tag by designing the probes to include a portion that is complementary to the sequence of the tag. The tag sequence may be a 3' untranslated region of the plasmid that is cotranscribed with the transcript of the protein of interest and comprises a target sequence for signaling probe binding. The tag sequence can be in frame with the protein-coding portion of the message of the gene or out of frame with it, depending on whether one wishes to tag the protein produced. Thus, the tag sequence does not have to be translated for detection by the signaling probe. The tag sequences may comprise multiple target sequences that are the same or different, wherein one signaling probe hybridizes to each target sequence. The tag sequence may be located within the RNA encoding the gene of interest, or the tag sequence may be located within a 5'- or 3'-untranslated region. The tag sequences may be an RNA having secondary structure. The structure may be a three-arm junction structure. In some embodiments, the signaling probe detects a sequence within the coding sequence for the protein of interest.

Following transfection of the DNA constructs into cells and subsequent drug selection (if used), or following gene activation, molecular beacons (e.g., fluorogenic probes), each of which is targeted to a different tag sequence and differentially labeled, may be introduced into the cells, and a flow cytometric cell sorter is used to isolate cells positive for their signals (multiple rounds of sorting may be carried out). In one embodiment, the flow cytometric cell sorter is a FACS machine. MACS (magnetic cell sorting) or laser ablation of negative cells using laser-enabled analysis and processing can also be used. Other fluorescence plate readers, including those that are compatible with high-throughput screening can also be used. Signal-positive cells take up and may integrate into their genomes at least one copy of the introduced sequence(s). Cells introduced with message for the protein of interest are then identified. By way of example, the coding sequences may be integrated at different locations of the genome in the cell. The expression level of the introduced sequence may vary based upon copy number or integration site. Further, cells comprising a protein of interest may be obtained wherein one or more of the introduced nucleic acids is episomal or results from gene activation.

Signaling probes useful in this invention are known in the art and generally are oligonucleotides comprising a sequence complementary to a target sequence and a signal emitting system so arranged that no signal is emitted when the probe is not bound to the target sequence and a signal is emitted when the probe binds to the target sequence. By way of non-limiting illustration, the signaling probe may comprise a fluorophore and a quencher positioned in the probe so that the quencher and fluorophore are brought together in the unbound probe. Upon binding between the probe and the target sequence, the quencher and fluorophore separate, resulting in emission of signal. International publication WO/2005/079462, for example, describes a number of signaling probes that may be used in the production of the present cells and cell lines. The methods described above for introducing nucleic acids into cells may be used to introduce signaling probes.

Where tag sequences are used, each vector (where multiple vectors are used) can comprise the same or a different tag sequence. Whether the tag sequences are the same or different, the signaling probes may comprise different signal emitters, such as different colored fluorophores and the like so that expression of each subunit may be separately detected. By way of illustration, the signaling probe that specifically detects a first mRNA of interest can comprise a red fluorophore, the probe that detects a second mRNA of interest can comprise a green fluorophore, and the probe that detects a third mRNA of interest can comprise a blue fluorophore. Those of skill in the art will be aware of other means for differentially detecting the expression of the three subunits with a signaling probe in a triply transfected cell.

In one embodiment, the signaling probes are designed to be complementary to either a portion of the RNA encoding the protein of interest or to portions of the 5' or 3' untranslated regions. Even if the signaling probe designed to recognize a messenger RNA of interest is able to detect spuriously endogenously expressed target sequences, the proportion of these in comparison to the proportion of the sequence of interest produced by transfected cells is such that the sorter is able to discriminate the two cell types.

The expression level of a protein of interest may vary from cell to cell or cell line to cell line. The expression level in a cell or cell line may also decrease over time due to epigenetic events such as DNA methylation and gene silencing and loss of transgene copies. These variations can be attributed to a variety of factors, for example, the copy number of the transgene taken up by the cell, the site of genomic integration of the transgene, and the integrity of the transgene following genomic integration. One may use FACS or other cell sorting methods (i.e., MACS) to evaluate expression levels. Additional rounds of introducing signaling probes may be used, for example, to determine if and to what extent the cells remain positive over time for any one or more of the RNAs for which they were originally isolated.

Optionally, one or more replicate sets of cultures for one or more of the growth rate groups may be prepared. In some cases, it may be advantageous to freeze a replicate set of one or more growth bins, for example, to serve as a frozen stock. However, according to the method, frozen cell stocks can be made as often as desired and at any point and at as many points during their production. Methods for freezing cell cultures are well-known to those of skill in the art. By way of example, the replicate set can be frozen at any temperature, for example, at -70° to-80° C. In one embodiment, cells were incubated until 70-100% confluency was reached. Next, media was aspirated and a solution of 90% FBS and 10% media was added to the plates, insulated and frozen.

The invention contemplates performing the method with any number of replicate sets using different culture conditions. That is, the method can be formed with a first plurality (set) of separate cell cultures under a first set of culture conditions and with a second set of separate cell cultures that are cultured under a second set of conditions that are different from the first conditions, and so on for any desired number of sets of conditions. The methods using different sets of conditions can be performed simultaneously or sequentially or a combination of both (such as two sets simultaneously followed by two more sets, and so on).

One advantage of the method described herein for selecting a cell with consistent functional expression of a protein of interest is that cells are selected by function, not by the presence of a particular nucleic acid in the cell. Cells that comprise a nucleic acid encoding a protein of interest may not express it, or even if the protein is produced, for many reasons the protein may not be functional or have altered function compared to "native" function, i.e., function in a cell in its normal contextthat naturally expresses the protein. By selecting cells based on function, the methods described herein make it possible to identify novel functional forms. For example, it is possible to identify multiple cells that have various degrees of function in the same assay, such as with the same test compound or with a series of compounds. The differential function provides a series of functional "profiles". Such profiles are useful, for example, to identify compounds that differentially affect different functional forms of a protein. Such compounds are useful to identify the functional form of a protein in a particular tissue or disease state, an the like.

A further advantage of the method for making cells and cell lines of the invention including cells that express complex proteins or multiple proteins of interest is that the cells can be produced in significantly less time that by conventional methods. For example, depending on a number of factors including the number of cells required for the functional assay, whether growth rate binning is done and other factors, cells expressing a demonstrably functional protein may be produced in as little as 2 day, or a week but even production time of 2 weeks, 3 weeks, 1 month, 2 months, 3 months or even 6 months are significantly faster than was possible by conventional methods, even for complex or multiple proteins.

in another aspect, the invention provides methods of using the cells and cell lines of the invention. The cells and cell lines of the invention may be used in any application for which the functional protein of interest are needed. The cells and cell lines may be used, for example, in an *in vitro* cell-based assay or an *in vivo* assay where the cells are implanted in an animal (e.g., a non-human mammal) to, e.g., screen for modulators; produce protein for crystallography and binding studies; and investigate compound selectivity and dosing, receptor/compound binding kinetic and stability, and effects of receptor expression on cellular physiology (*e.g.*, electrophysiology, protein trafficking, protein folding, and protein regulation). The cells and cell lines of the invention also can be used in knock down studies to examine the roles of the protein of interest.

Cells and cell lines of the invention also may be used to identify soluble biologic competitors, for functional assays, bio-panning (*e.g.*, using phage display libraries), gene chip studies to assess resulting changes in gene expression, two-hybrid studies to identify protein-protein interactions, knock down of specific subunits in cell lines to assess its role, electrophysiology, study of protein trafficking, study of protein folding, study of protein regulation, production of antibodies to the protein, isolation of probes to the protein, isolation of fluorescent probes to the protein, study of the effect of the protein's expression on overall gene expression/processing, study of the effect of the protein's expression on overall protein expression and processing, and study of the effect of protein's expression on cellular structure, properties, characteristics.

The cells and cell lines of the invention further are useful to characterize the protein of interest (DNA, RNA or protein) including DNA, RNA or protein stoichiometry, protein folding, assembly, membrane integration or surface presentation, conformation, activity state, activation potential, response, function, and the cell based assay function, where the protein of interest comprises a multigene system, complex or pathway whether ail components of these are provided by one or more target genes introduced into cells or by any combination of introduced and endogenously expressed sequences.

The invention makes possible the producition of multiple cell lines expressing a protein of interest. Clonal cell lines of the invention will have different absolute and relative levels of such expression. A large panel of such clones can be screened for activity with a number of known reference compounds. In this way, each isolated cell line will have a "fingerprint" of responses to test compounds which represent the activities of differential functional expression of the protein. The cell lines can then be grouped based on the similarity of such responses to the compounds. At least one cell line representing each functionally distinct expression profile can be chosen for further study. A collection of these cell lines can then be used to screen a large number of compounds. In this way, compounds which selectively modulate one or more of the corresponding distinct functional forms of the protein may be identified. These modulators can then be tested in secondary assays or *in vivo* models to determine which demonstrate activity in these assays or models. In this connection, the modulators would be used as reference compounds to identify which corresponding functional forms of the protein may be present or play a role in the secondary assay or model system employed. Such testing may be used to determine the functional forms of a protein that may exist *in vivo* as well as those that may be physiologically relevant. These modulators could be used to discern which of the functionally distinct forms are involved in a particular phenotype or physiological function such as disease.

This method is also useful when creating cell lines for proteins that have not been well characterized. For such proteins, there is often little information regarding the nature of their functional response to known compounds. Such a lack of established functional benchmarks to assess the activity of clones may be one challenge in producing physiologically relevant cell lines. The method described above provides a way to obtain physiologically relevant cell lines even for proteins that are not well characterized where there is a lack of such information. Cell lines comprising the physiologically relevant form of a protein may be obtained by pursuing clones representing a number or all of the functional forms that may result from the expression of genes comprising a protein.

The cells and cell lines of the invention may be used to identify the roles of different forms of the protein of interest in different pathologies by correlating the identity of *in vivo* forms of the protein with the identity of known forms of the protein based on their response to various modulators. This allows selection of disease- or tissue-specific modulators for highly targeted treatment of pathologies associated with the protein.

To identify a modulator, one exposes a cell or cell line of the invention to a test compound under conditions in which the protein would be expected to be functional and then detects a statistically significant change (*e.g.*, $p<0.05$) in protein activity compared to a suitable control, e.g., cells that are not exposed to the test compound. Positive and/or negative controls using known agonists or antagonists and/or cells expressing the protein of interest may also be used. One of ordinary skill in the art would understand that various assay parameters may be optimized, e.g., signal to noise ratio.

In some embodiments, one or more cells or cell lines of the invention are exposed to a plurality of test compounds, for example, a library of test compounds. Such libraries of test compounds can be screened using the cell lines of the invention to identify one or more modulators of the protein of interest. The test compounds can be chemical moieties including small molecules, polypeptides, peptides, peptide mimetics, antibodies or antigen-binding portions thereof, natural compounds, synthetic compounds , extracts, lipids, detergents, and the like. In the case of antibodies, they may be non-human antibodies, chimeric antibodies, humanized antibodies, or fully human antibodies. The antibodies may be intact antibodies comprising a full complement of heavy and light chains or antigen-binding portions of any antibody, including antibody fragments (such as Fab and Fab, Fab', $F(ab')_2$, Fd, Fv, dAb and the like), single chain antibodies (scFv), single domain antibodies, all or an antigen-binding portion of a heavy chain or light chain variable region.

In some embodiments, prior to exposure to a test compound, the cells or cell lines of the invention may be modified by pretreatment with, for example, enzymes, including mammalian or other animal enzymes, plant enzymes, bacterial enzymes, protein modifying enzymes and lipid modifying enzymes. Such enzymes can include, for example, kinases, proteases, phosphatases, glycosidases, oxidoreductases, transferases, hydrolases, lyases, isomerases, ligases bacterial proteases, proteases from the gut, proteases from the GI tract, proteases in saliva, in the oral cavity, proteases from lysol cells/bacteria, and the like. Alternatively, the cells and cell lines may be exposed to the test compound first followed by enzyme treatment to identify compounds that alter the modification of the protein by the treatment.

In some embodiments, large compound collections are tested for protein modulating activity in a cell-based, functional, high-throughput screen (HTS), *e.g.*, using 96-well, 384-well, 1536-well or higher density formats. In some embodiments, a test compound or multiple test compounds, including a library of test compounds, may be screened using more than one cell or cell line of the invention.

In some embodiments, the cells and cell lines of the invention have increased sensitivity to modulators of the protein of interest. Cells and cell lines of the invention also respond to modulators with a physiological range $EC_{50}$ or $IC_{50}$ values for the protein. As used herein, $EC_{50}$ refers to the concentration of a compound or substance required to induce a half-maximal activating response in the cell or cell line. As used herein, $IC_{50}$ refers to the concentration of a compound or substance required to induce a half-maximal inhibitory response in the cell or cell line. $EC_{50}$ and $IC_{50}$ values may be determined using techniques that are well-known in the art, for example, a dose-response curve that correlates the concentration of a compound or substance to the response of the protein -expressing cell line.

A further advantageous property of the cells and cell lines of the invention is that modulators identified in initial screening using those cells and cell lines are functional in secondary functional assays. As those of ordinary skill in the art will recognize, compounds identified in initial screening assays typically must be modified, such as by combinatorial chemistry, medicinal chemistry or synthetic chemistry, for their derivatives or analogs to be functional in secondary functional assays. However, due to the high physiological relevance of the cells and cell lines of this invention, many compounds identified using those cells and cell lines are functional without further modification. In some embodiments, at least 25%, 30%, 40%, 50% or more of the modulators identified in an initial assay are functional in a secondary assay. Further, cell lines of the invention perform in functional assays on a par with the "gold standard" assays. For example, cell lines of the invention expressing GABA A receptors perform substantially the same in membrane potential assays and in electrophysiology.

These and other embodiments of the invention may be further illustrated in the following non-limiting Examples.

## EXAMPLES

### Example 1 Generating a Stable $GABA_A$-Expressing Cell Line

*Generating Expression Vectors*

Plasmid expression vectors that allowed streamlined cloning were generated based on pCMV-SCRIPT (Stratagene) and contained various necessary components for transcription and translation of a gene of interest, including: CMV and SV40 eukaryotic promoters; SV40 and HSV-TK polyadenylation sequences; multiple cloning sites; Kozak sequences; and neomycin/kanamycin resistance cassettes.

**Step 1--Transfection**

We transfected both 293T and CHO cells. The example focuses on CHO cells, where the CHO cells were cotransfected with three separate plasmids, one encoding a human GABA alpha subunit (SEQ ID NO: GABA1-GABA4), one encoding the human GABA beta 3 subunit (SEQ ID NO: GABA5) and the other encoding the human GABA gamma 2 subunit (SEQ ID NO: GABA6) in the following combinations: $\alpha1\beta3\gamma2s$ ($\alpha1$), $\alpha2\beta3\gamma2s$ ($\alpha2$), $\alpha3\beta3\gamma2s$ ($\alpha3$) and $\alpha5\beta3\gamma2s$ ($\alpha5$). As will be appreciated by those of skill in the art, any reagent that is suitable for use with a chosen host cell may be used to introduce a nucleic acid, e.g. plasmid, oligonucleotide, labeled oligonucleotide, into a host cell with proper optimization. Examples of reagents that may be used to introduce nucleic acids into host cells include but are not limited to Lipofectamine, Lipofectamine 2000, Oligofectamine, TFX reagents, Fugene 6, DOTAP/DOPE, Metafectine, or Fecturin.

Although drug selection is optional in the methods of this invention, we included one drug resistance marker per plasmid. The sequences were under the control of the CMV promoter. An untranslated sequence encoding a tag for detection by a signaling probe was also present along with a sequence encoding a drug resistance marker. The target sequences utilized were GABA Target Sequence 1 (SEQ ID NO: GABA7), GABA Target Sequence 2 (SEQ ID NO: GABA8) and GABA Target Sequence 3 (SEQ ID NO: GABA9). In these examples, the GABA alpha subunit gene-containing vector contained GABA Target Sequence 1, the GABA beta subunit gene-containing vector contained GABA Target Sequence 2 and the GABA gamma subunit gene-containing vector contained the GABA Target Sequence 3.

**Step 2 - Selection step**

Transfected cells were grown for 2 days in HAMF12-FBS, followed by 14 days in antibiotic-containing HAMF12-FBS. The antibiotic containing period had antibiotics added to the media as follows: Puromycin (3.5 ug/ml), Hygromycin (150 ug/ml), and G418/Neomycin (300 ug/ml)

**Step 3 - Cell passaging**

Following antibiotic selection, and prior to introduction of fluorogenic probes, cells were passaged 6 to 18 times in the absence of antibiotics to allow time for expression that is not stable over the selected period of time to subside.

**Step 4 - Exposure of cells to fluorogenic probes**

Cells were harvested and transfected with GABA signaling probes (SEQ ID NO: GABA10-GABA12). As will be appreciated by those of skill in the art, any reagent that is suitable for use with a chosen host cell may be used to introduce a nucleic acid, e.g. plasmid, oligonucleotide, labeled oligonucleotide, into a host cell with proper optimization. Examples of reagents that may be used to introduce nucleic acids into host cells include but are not limited to Lipofectamine, Lipofectamine 2000, Oligofectamine, TFX reagents, Fugene 6, DOTAP/DOPE, Metafectine, or Fecturin.

GABA Signaling Probe 1 binds GABA Target Sequence 1, GABA Signaling Probe 2 binds GABA Target Sequence 2 and GABA Signaling Probe 3 binds GABA Target Sequence 3. The cells were then collected for analysis and sorted using a fluorescence activated cell sorter (below).

**Target Sequences detected by signaling probes**

**GABA Target 1**
5'-GTTCTTAAGGCACAGGAACTGGGAC-3' (SEQ ID NO:GABA7) (alpha subunit)
**GABA Target 2**
5'-GAAGTTAACCCTGTCGTTCTGCGAC-3' (SEQ ID NO: GABA8) (beta subunit)
**GABA Target 3**
5'-GTTCTATAGGGTCTGCTTGTCGCTC-3' (SEQ ID NO: GABA9) (gamma subunit)

**Signaling probes**

Supplied as 100μM stocks

A similar probe using a Quasar Dye (BioSearch) with spectral properties similar to Cy5 was used in certain experiments. Note also that 5-MedC and 2-aminodA mixmer probes rather than DNA probes were used in some instances.

**GABA Signaling probe 1 -** binds (GABA Target 1)

**5' -** Cy5 GCCAGTCCCAGTTCCTGTGCCTTAAGAACCTCGC **BHQ3 quench -3'** (SEQ ID NO:GABA10)

**GABA Signaling probe 2** -binds (GABA Target 2)

**5'- Cy5**.**5** GCGAGTCGCAGAACGACAGGGTTAACTTCCTCGC **BHQ3 quench -3'** (SEQ ID NO: GABA11)
Note that BHQ3 could be substituted with BHQ2 or a gold particle in Probe 1 or Probe 2.

**GABA Signaling probe 3** -binds (GABA Target 3)

**5'- Fam** GCGAGAGCGACAAGCAGACCCTATAGAACCTCGC **BHQ1 quench -3"** (SEQ ID NO: GABA12)
Note that BHQ1 could be substituted with BHQ2 or Dabcyl in Probe 3.

**Step 5 - Isolation of positive cells**

The cells were dissociated and collected for analysis and sorting using a fluorescence activated cell sorter. Standard analytical methods were used to gate cells fluorescing above background and to isolate individual cells falling within the gate into barcodcd 96-well plates. The gating hierarchy was as follows: Gating hierarchy: coincidence gate> singlets gate> live gate > Sort gate. With this gating strategy, the top 0.04-0.4% of triple positive cells were marked for sorting into barcoded 96-well plates.

**Step 6 - Additional cycles of steps 1-5 and/or 3-5**

Steps 1 to 5 and/or 3-5 were repeated to obtain a greater number of cells. Two independent rounds of steps 1-5 were completed, and for each of these cycles, at least three internal cycles of steps 3-5 were performed for the sum of independent rounds.

**Step 7 - Estimation of growth rates for the populations of cells**

The plates were transferred to a Hamilton Microlabstar automated liquid handler. Cells were incubated for 5-7 days in a 1:1 mix of 2-3 day conditioned growth medium:fresh growth medium (growth medium is Ham's F12/10% FBS) supplemented with 100 units penicillin/ml plus 0.1mg/ml streptomycin and then dispersed by trypsinization with 0.25% trypsin to minimize clumps and transferred to new 96-well plates. After the clones were dispersed, plates were imaged to determine confluency of wells (Genetix). Each plate was focused for reliable image acquisition across the plate. Reported confluencies of greater than 70% were not relied upon. Confluency measurements were obtained at days every 3 times over 9 days (between days 1 and 10 post-dispersal) and used to calculate growth rates.

**Step 8 - Binning populations of cells according to growth rate estimates**

Cells were binned (independently grouped and plated as a cohort) according to growth rate between 10-11 days following the dispersal step in step 7. Bins were independently collected and plated on individual 96 well plates for downstream handling, and there could be more than one target plate per specific bin. Bins were calculated by considering the spread of growth rates and bracketing a range covering a high percentage of the total number of populations of cells.

Depending on the sort iteration (see Step 5), between 5 and 6 growth bins were used with a partition of 1-4 days. Therefore each bin corresponded to a growth rate or population doubling time between 12 and 14.4 hours depending on the iteration.

**Step 9 - Replica plating to speed parallel processing and provide stringent QC**

The plates were incubated under standard and fixed conditions (humidified 37°C, 5% $CO_2$/95% air) in Ham's F12 media/10%FBS without antibiotics. The plates of cells were split to produce 4 sets (the set consists of all plates with all growth bins - these steps ensure there are 4 replicates of the initial set) of target plates. Up to 2 target plate sets were committed for cryopreservation (see below), and the remaining set was scaled and further replica plated for passage and for functional assay experiments. Distinct and independent tissue culture reagents, incubators, personnel and carbon dioxide sources were used for each independently carried set of plates. Quality control steps were taken to ensure the proper production and quality of all tissue culture reagents: each component added to each bottle of media prepared for use was added by one designated person in one designated hood with only that reagent in the hood while a second designated person monitored to avoid mistakes. Conditions for liquid handling were set to eliminate cross contamination across wells. Fresh tips were used for all steps or stringent tip washing protocols were used. Liquid handling conditions were set for accurate volume transfer, efficient cell manipulation, washing cycles, pipetting speeds and locations, number of pipetting cycles for cell dispersal, and relative position of tip to plate.

**Step 10 - Freezing early passage stocks of populations of cells**

At least two sets of plates were frozen at -70 to -80C. Plates in each set were first allowed to attain confluencies of 70 to 100%. Media was aspirated and 90%FBS and 10% DMSO was added. The plates were sealed with Parafilm and then individually surrounded by 1 to 5cm of foam and placed into a -80C freezer.

**Step 11- Methods and conditions for initial transformative steps to produce VSF**

The remaining set of plates were maintained as described in step 9 (above). All cell splitting was performed using automated liquid handling steps, including media removal, cell washing, trypsin addition and incubation, quenching and cell dispersal steps.

**Step 12 - Normalization methods to correct any remaining variability of growth rates**

The consistency and standardization of cell and culture conditions for all populations of cells was controlled. Any differences across plates due to slight differences in growth rates could be controlled by periodic normalization of cell numbers across plates.

**Step 13** - **Characterization of population of cells**

The cells were maintained for 6 to 8 weeks of cell culture to allow for their in vitro evolution under these conditions. During this time, we observed size, morphology, fragility, response to trypsinization or dissociation, roundness/average circularity post-dissociation, percentage viability, tendency towards microconfluency, or other aspects of cell maintenance such as adherence to culture plate surfaces.

**Step 14 - Assessment of potential functionality of populations of cells under VSF conditions**

Populations of cells were tested using functional criteria. Membrane potential assay kits (Molecular Devices/MDS) were used according to manufacturer's instructions. Cells were tested at multiple different densities in 96 or 384-well plates and responses were analyzed. A variety of time points post plating were used, for instance 12-48 hours post plating. Different densities of plating were also tested for assay response differences.

**Step 15**

The functional responses from experiments performed at low and higher passage numbers were compared to identify cells with the most consistent responses over defined periods of time, ranging from 3 to 9 weeks. Other characteristics of the cells that changed over time are also noted, including morphology, tendency toward microconfluency, and time to attach to culture matrices post-plating.

**Step 16**

Populations of cells meeting functional and other criteria were further evaluated to determine those most amenable to production of viable, stable and functional cell lines. Selected populations of cells were expanded in larger tissue culture vessels and the characterization steps described above were continued or repeated under these conditions. At this point, additional standardization steps were introduced for consistent and reliable passages. These included different plating cell densities, time of passage, culture dish size/format and coating, fluidics optimization, cell dissociation optimization (type, volume used, and length of time), as well as washing steps. Assay Z' scores were stable when tested every few days over the course of four weeks in culture.

Also, viability of cells at each passage were determined. Manual intervention was increased and cells were more closely observed and monitored. This information was used to help identify and select final cell lines that retained the desired properties Final cell lines and back-up cell lines were selected that showed consistent growth, appropriate adherence, as well as functional response,

**Step 17 - Establishment of cell banks**

The low passage frozen plates (see above) corresponding to the final cell line and back-up cell lines were thawed at 37°C, washed two times with Ham's F12/10% FBS and incubated in humidified 37°C/5% CO2 conditions. The cells were then expanded for a period of 2-3 weeks. Cell banks for each final and back-up cell line consisting of 25 vials each with 10 million cells were established.

**Step 18**

At least one vial from the cell bank was thawed and expanded in culture. The resulting cells were tested to confirm that they met the same characteristics for which they were originally selected.

**Example 2 Verification of $GABA_A$ Cell Lines Response to GABA Ligand.**

The response of CHO cell lines expressing $GABA_A$ (subunit combinations of α1β3γ2s (α1), α2β3γ2s (α2), α3β3γ2s (α3) and α5β3γ2s (α5)) GABA, the endogenous $GABA_A$ ligand, was evaluated. Interaction of cell lines with GABA was evaluated by measuring the membrane potential of $GABA_A$, in response to GABA using the following protocol.

Cells were plated 24 hours prior to assay at 10-25,000 cells per well in 384 well plates in growth media (Ham's F-12 media plus FBS and glutamine). Media removal was followed by the addition of membrane potential dye diluted in load buffer (137mM NaCl, 5mMKCl, 1.25mM CaCl, 25mM HEPES, 10mM Glucose). Incubation was for 1 hour, followed by plate loading onto the high throughput fluorescent plate reader (Hamamastu FDSS). GABA ligand was diluted in MP assay buffer (137mM NaCl, 5mM KGluconate, 1.25mM CaCl, 25mM HEPES, 10mM Glucose) to the desired concentration (when needed, serial dilutions of GABA were generated, concentrations used: 3nM, 10nM, 30nM, 100nM, 300nM, 1 uM, 3uM, 10uM) and added to each well. The plates were read for 90 seconds.

**Table GABA1** (below) demonstrates that each of the cell lines generated responds to GABA ligand. These results indicate that the $GABA_A$ cell lines produced, which respond as expected to the endogenous ligand, are physiologically relevant for use in high-throughput screening assays. Further, the replicate wells produced precise $EC_{50}$ values from well to well indicating high reproducibility of the $GABA_A$ cell lines. Z' values generated using the membrane potential assay were α1β3γ2s 0.58, α2β3γ2s 0.67, α3β3γ2s 0.69 and α5β3γ2s 0.62.

**Example 3 Additional Verification of $GABA_A$ Cell Lines Using A Known $GABA_A$ Modulator.**

The $GABA_A$ cell lines and membrane potential assay were verified by the methods described in Example 2 using serial dilutions in assay buffer of bicuculline (a known antagonist) at 30uM, 10uM, 3uM, 1uM, 300nM, 100nM and 30nM.

Bicuculline was found to interact with all four $GABA_A$ cell lines in the presence of $EC_{50}$ concentrations of GABA. These results indicate that the $GABA_A$ cell lines produced, which respond as expected to this known modulator of $GABA_A$, are physiologically and pharmacologically relevant for use in high-throughput screening assays.

**Example 4 Characterization of Cell Line Expressing $GABA_A$ for Native $GABA_A$ Function Using Membrane Potential Assay**

The interaction of CHO cell lines expressing $GABA_A$ (subunit combinations of α1β3γ2s (α1), α2β3γ2s (α2), α3β3γ2s (α3) and α5β3γ2s (α5)) with 1280 compounds from the LOPAC 1280 (Library of Pharmacologically Active Compounds) was evaluated (Sigma-RBI Prod. No. LO1280). The LOPAC 1280 library contains high purity, small organic ligands with well documented pharmacological activities. Interaction of cell lines with test compounds was evaluated by measuring the membrane potential of $GABA_A$, in response to test compounds using the following protocol.

Cells were plated 24 hours prior to assay at 10-25,000 cells per well in 384 well plates in growth media (Ham's F-12 media plus FBS and glutamine). Media removal was followed by the addition of membrane potential dye diluted in load buffer (137mM NaCl, 5mMKCl, 1.25mM CaCl, 25mM HEPES, 10mM Glucose). Incubation was for 1 hour, followed by plate loading onto the high throughput fluorescent plate reader (Hamamastu FDSS). Test compounds were diluted in MP assay buffer (137mM NaCl, 5mM KGluconate, 1.25mM CaCl, 25mM HEPES, 10mM Glucose) to the desired concentration (when needed, serial dilutions of each test compound were generated, concentrations used: 3nM, 10nM, 30nM, 100nM, 300nM, 1uM, 3uM, 10uM) and added to each well. The plates were read for 90 seconds.

Results

The activity of each compound towards the $GABA_A$ ceii lines produced was measured and compounds which exhibited similar or greater activity as GABA (the endogenous ligand) were scored as positive hits. Of the 1280 compounds screened, 34 activated at least one cell line (i.e., either α1, α2, α3 and α5) as well as, if not better, than GABA. The interaction of 17 of these compounds with the produced $GABA_A$ cell lines was confirmed in the following dose response studies. Modulators which require GABA to be present, partial agonists and low potency compounds were not included in the list.

The screening assay identified each of the $GABA_A$ agonists in the LOPAC library: GABA (endogenous ligand), propofol, isoguvacine hydrochloride, muscimol hydrobromide, piperidine-4-sulphonic acid, 3-alpha,21-dihydroxy-5-alpha-pregnan-20-one (a neurosteroid), 5-alpha-pregnan-3alpha-ol-11,20-dione (a neurosteroid), 5-alpha-pegnan-3alpha-ol-20-one (a neurosteroid), and tracazolate. The results indicate that the produced $GABA_A$ cell lines respond in a physiologically relevant manner (e.g., they respond to agonists of the endogenous receptor). $EC_{50}$ values for these eight agonists were determined and are included in **Table GABA1** (below).

The screening assay also identified four compounds in the LOPAC library not described as GABA agonist but known to have other activities associated with $GABA_A$ which we noted: etazolate (a phospodiesterase inhibitor), androsterone (a steroid hormone), chlormezanone (a muscle relaxant), and ivermectin (an anti-parasitic known to effect chlorine channels). $EC_{50}$ values for these four compounds were determined and are summarized in **Table GABA1** (below).

The screening assay further identified four compounds in the LOPAC library which, until now, were not known to interact with $GABA_A$. These novel compounds include: dipyrimidole (an adenosine deaminase inhibitor), niclosamide (an anti-parasitic), tyrphosin A9 (a PDGFR inhibitor), and I-Ome-Tyrphosin AG 538 (an IGF RTK inhibitor). EC50 values for these four compounds were determined and are summarized in **Table GABA1** (below).

The results of the screening assays summarized in **Table GABA1:**

| Compound | Description | Chromocell Target | $EC_{50}$ Values |
|---|---|---|---|
| GABA | endogenous ligand | α1, α2, α3, α5 | α1 3.29μM<br>α2 374nM<br>α3 131nM<br>α5 144nM |
| Muscimol | agonist | α1, α2, α3, α5 | α1 4μM<br>α2 675nM<br>α3 367nM<br>α5 80nM |

(continued)

| Compound | Description | Chromocell Target | $EC_{50}$ Values |
|---|---|---|---|
| Propofol | agonist | α1, α2, α3, α5 | α1 33.4μM α2 42.8μM α3 12.9μM α5 2.0μM |
| Isoguvacine hydrochloride | agonist | α1, α2, α3, α5 | α1 3.57μM α2 3.42μM α3 6.78μM α5 1.13μM |
| Piperidine-4-sulphonic acid | agonist | α1, α2, α3, α5 | α1 13μM α2 20μM α3 8.33μM α5 14.2μM |
| 3-alpha, 21-dihydroxy-5-alpha-pregnan-20-one | neurosteroid (agonist) | α1, α2, α3, α5 | α1 382nM α2 123nM α3 80.2nM α5 17.3nM |
| 5-alpha-Pregnan-3alpha-ol-11,20-dione | neurosteroid (agonist) | α1, α2, α3, α5 | α1 762nM α2 338nM α3 168nM α5 122nM |
| 5-alpha-Pregnan-3alpha-ol-20-one | neurosteroid (agonist) | α1, α2, α3, α5 | α1 692nM α2 140nM α3 80.0nM α5 33.6nM |

(continued)

| Compound | Description | Chromocell Target | $EC_{50}$ Values |
|---|---|---|---|
| Tracazolate | agonist | α1, α2, α3, α5 | α1 10.6μM α2 8.9μM α3 4.3μM α5 762nM |
| Androsterone | Steroid with $GABA_A$ receptor activity | α1, α2, α3, α5 | α1 1.48μM α2 1.52μM α3 1.12μM α5 337nM |
| Ivermectin | Phospho-diesterase inhibitor: Known GABAergic | α1, α2, α3, α5 | α1 4.26μM α2 767nM α3 798nM α5 687nM |
| Chlormezanone | Muscle relaxant: known GABA ligand | α1, α2, α3, α5 | α1 1.74nM α2 5.42nM α3 7.0nM α5 14.1nM |
| Etazolate | Anti-parasitic: known effector of chlorine channels | α1, α2, α3, α5 | α1 2.54μM α2 790nM α3 569nM α5 281nM |
| Dipyridamole | Adenosine inhibitor known to effect GABA release in neurons (not known to bind to $GABA_A$) | α1, α2, α3, α5 | α1 7.16μM α2 3.68μM α3 3.69μM α5 1.37μM |

(continued)

| Compound | Description | Chromocell Target | $EC_{50}$ Values |
|---|---|---|---|
| Niclosamide | Anti parasitic (side effects include drowsiness and dizziness) | α1, α2, α3, α5 | α1 1.2μM α2 1.26μM α3 0.55μM α5 0.69μM |
| Tyrphostin A9 | PDGFR inhibitor | α1, α2, α3, α5 | α1 1.8μM α2 0.88μM α3 5.0μM α5 54.0 μM |
| I-OMe Tyrphostin 538 | IGF RTK inhibitor | α1, α2, α3, α5 | α1 3.5μM α2 1.5μM α3 2.2μM α5 Not active |

**<u>Example 5</u> Characterization $GABA_A$-CHO cells for native $GABA_A$ function using Electrophysiological Assay**

The following voltage-clamp protocol was used: the membrane potential was clamped to a holding potential of -60 mV. Currents were evoked by 2-sec applications of increasing concentrations of GABA (0.10-100 μM) with intermediate wash with buffer.

Whole cell receptor current traces for the α2, α3, and α5 $GABA_A$ cell lines in response to 100uM GABA, and the α1 $GABA_A$ cell line in response to increasing concentrations of GABA (0.10-100μM in log increments), confirm that the $GABA_A$ cell lines can be used in traditional electrophysiology assays in addition to the High-Throughput Screening assays described above. These electrophysiology assay results, along with the membrane potential assay of Example 2, confirm the physiological and pharmacological relevance of the $GABA_A$ cell lines produced herein. Electrophysiology is accepted as a reliable method of detecting modulators of $GABA_A$ receptors. Our data indicate that the cell lines of the invention can produce similarly reliable results using a membrane potential assay. Cell lines of the prior art are not reliable or sensitive enough to effectively utilize this membrane potential assay, which is cheaper and faster than electrophysiology. Thus, the cell lines of the invention allow screening on a much larger scale than is available using electrophysiology (10,000's of assays per day using the membrane potential assay compared to less than 100 per day using electrophysiology).

**<u>Example 6</u> Characterization of an in-cell readout assay for native $GABA_A$ function using halide-sensitive meYFP**

The response of $GABA_A$ (subunit combinations of α1β3y2s (A1), α2β3y2s (A2), α3β3y2s (A3) and α5β3y2s (A5)) expressing CHO cells of the invention to test compounds was evaluated using the following protocol for an in-cell readout assay.

Cells were plated 24 hours prior to assay at 10-25,000 cells per well in 384 well plates in growth media (Ham's F-12 media plus FBS and glutamine). Media removal was followed by the addition of loading buffer (135mM NaCl, 5mM KCl, 2mM $CaCl_2$, 1 mM $MgCl_2$, 10mM HEPES, 10mM glucose) and incubation for 1 hour. The assay plates were then loaded on the FDSS (Hamamatsu Corporation). Test compounds (e.g. GABA ligand) were diluted in assay buffer (150mM

NaI, 5mMKCl, 1.25mM $CaCl_2$, 1 mM $MgCl_2$, 25mM HEPES, 10mM glucose) to the desired concentration (when needed, serial dilutions of each test compound were generated, effective concentrations used: 3nM, 10nM, 30nM, 100nM, 300nM, 1uM, 3uM, 10uM) and added to each well. The plates were read for 90 seconds.

In response to increasing concentrations of GABA ligand, $GABA_A$-meYFP-CHO cells show increasing quench of meYFP signal. This quench can be used to calculate dose response curves for GABA activation. The GABA dose response curves generated by the in-cell readout assay are similar to the curves generated by the Membrane Potential Blue assay described in Example 3. These data demonstrate that the cells of the invention can be used in an in-cell readout assay to determine modulators of $GABA_A$.

## Example 8 Generating a stable GC-C-expressinq cell line

293T cells were transfected with a plasmid encoding the human GC-C gene (SEQ ID NO: GCC 3) using standard techniques. (Examples of reagents that may be used to introduce nucleic acids into host cells include, but are not limited to, LIPOFECTAMINE™, LIPOFECTAMINE™ 2000, OLIGOFECTAMINE™, TFX™ reagents, FUGENE®6, DOTAP/DOPE, Metafectine or FECTURIN™.)

Although drug selection is optional in the methods of this invention, we included one drug resistance marker in the plasmid encoding the human GC-C gene. The GC-C sequence was under the control of the CMV promoter. An untranslated sequence encoding a tag for detection by a signaling probe was also present along with a sequence encoding a drug resistance marker. The target sequence utilized was GC-C Target Sequence 1 (SEQ ID NO: GCC 1). In this example, the GC-C gene-containing vector contained GC-C Target Sequence 1.

Transfected cells were grown for 2 days in DMEM-FBS, followed by 10 days in 500μg/ml hygromycin-containing DMEM-FBS, then in DMEM-FBS for the remainder of the time, totaling between 4 and 5 weeks (depending on which independent isolation) in DMEM/10% FBS, prior to the addition of the signaling probe.

Following enrichment on antibiotic, cells were passaged 8-10 times in the absence of antibiotic selection to allow time for expression that is not stable over the selected period of time to subside.

Cells were harvested and transfected with GC-C Signaling Probe 1 (SECT ID NO: GCC 2) using standard techniques. (Examples of reagents that may be used to introduce nucleic acids into host cells include, but are not limited to, LIPOFECTAMINE™, LIPOFECTAMINE™ 2000, OLIGOFECTAMINE™, TFXT™ reagents, FUGENE®6, DOTAP/DOPE, Metafectine or FECTURIN™.) The cells were then dissociated and collected for analysis and sorted using a fluorescence activated cell sorter.

### GC-C Target Sequence 1 detected by GC-C Signaling probe 1

5'-GTTCTTAAGGCACAGGAACTGGGAC-3' (SEQ ID NO: GCC 1)

### GC-C Signaling probe 1

(Supplied as 100μM stock)

5' - Cy5 GCCAGTCCCAGTTCCTGTGCCTTAAGAACCTCGC BHQ2 -3' (SEQ ID NO: GCC 2)

In addition, a similar probe using a QUASAR® Dye (BioSearch) with spectral properties similar to Cy5 was used in certain experiments. In some experiments, 5-MedC and 2-amino dA mixmers were used rather than DNA probes.

The cells were dissociated and collected for analysis and sorting using a fluorescence activated cell sorter. Standard analytical methods were used to gate cells fluorescing above background and to isolate individual cells falling within the gate into bar-coded 96-well plates. The following gating hierarchy was used: coincidence gate → singlets gate → live gate → Sort gate in plot FAM vs. Cy5: 0.3% of live cells

The above steps were repeated to obtain a greater number of cells. Two rounds of all the above steps were performed. In addition, the cell passaging, exposure to the signaling probe and isolation of positive cells by the fluorescence activated cell sorter sequence of steps was performed a total of two times for one of the independent transfection rounds.

The plates were transferred to a MICROLAB STAR™ (Hamilton Robotics). Cells were incubated for 9 days in 100μl of 1: 1 mix of fresh complete growth medium and 2-day-conditioned growth medium, supplemented with 100U penicillin and 0.1mg/ml streptomycin, dispersed by trypsinization twice to minimize clumps and transferred to new 96-well plates. Plates were imaged to determine confluency of wells (Genetix). Each plate was focused for reliable image acquisition across the plate. Reported confluencies of greater than 70% were not relied upon. Confluency measurements were obtained on 3 consecutive days and used to calculate growth rates.

Cells were binned (independently grouped and pirated as a cohort) according to growth rate 3 days following the dispersal step. Each of the 4 growth bins was separated into individual 96-well plates; some growth bins resulted in more than one 96-well plate. Bins were calculated by considering the spread of growth rates and bracketing a range covering a high percentage of the total number of populations of cells. Bins were calculated to capture 12-hour differences in growth rate.

Cells can have doubling times from less than 1 day to more than 2 weeks. In order to process the most diverse clones that at the same time can be reasonably binned according to growth rate, it is preferable to use 3-9 bins with a 0.25 to 0.7 day doubling time per bin. One skilled in the art will appreciate that the tightness of the bins and number of bins can be adjusted for the particular situation and that the tightness and number of bins can be further adjusted if cells were synchronized for their cell cycle.

The plates were incubated under standardized and fixed conditions (DMEM/FBS, 37°C, 5% $CO_2$) without antibiotics. The plates of cells were split to produce 5 sets of 96-well plates (3 sets for freezing, 1 for assay and 1 for passage). Distinct and independent tissue culture reagents, incubators, personnel and carbon dioxide sources were used downstream in the workflow for each of the sets of plates. Quality control steps were taken to ensure the proper production and quality of all tissue culture reagents: each component added to each bottle of media prepared for use was added by one designated person in one designated hood with only that reagent in the hood while a second designated person monitors to avoid mistakes. Conditions for liquid handling were set to eliminate cross contamination across wells. Fresh tips were used for all steps, or stringent tip washing protocols were used. Liquid handling conditions were set for accurate volume transfer, efficient cell manipulation, washing cycles, pipetting speeds and locations, number of pipetting cycles for cell dispersal, and relative position of tip to plate.

One set of plates was frozen at -70 to -80°C. Plates in the set were first allowed to attain confluencies of 70 to 100%. Medium was aspirated and 90% FBS and 10% DMSO was added. The plates were individually sealed with Parafilm, surrounded by 1 to 5cm of foam and placed into a freezer.

The remaining two sets of plates were maintained under standardized and fixed conditions as described above. All cell splitting was performed using automated liquid handling steps, including media removal, cell washing, trypsin addition and incubation, quenching and cell dispersal steps.

The consistency and standardization of cell and culture conditions for all populations of cells was controlled. Differences across plates due to slight differences in growth rates were controlled by normalization of cell numbers across plates and occurred 3 passages after the rearray. Populations of cells that are outliers were detected and eliminated.

The cells were maintained for 3 to 6 weeks to allow for their *in vitro* evolution under these conditions. During this time, we observed size, morphology, tendency towards microconfluency, fragility, response to trypsinization and average circularity post-trypsinization, or other aspects of cell maintenance such as adherence to culture plate surfaces and resistance to blow-off upon fluid addition.

Populations of cells were tested using functional criteria. The Direct Cyclic GMP Enzyme Immunoassay Kit (Cat. 900-014; AssayDesigns, Inc.) was used according to manufacturer's instructions:

(http://www.assaydesigns.com/objects/catalog//product/extras/900-014.pdf). Cells were tested at 4 different densities in 96- or 384-well plates and responses were analyzed. The following conditions were used for the GC-C-expressing cell lines of the invention:

- Clone screening: 1:2 and 1:3 splits of confluent 96-well plates 48 hour prior to assay, 30 minutes guanylin treatment.
- Dose-response studies: densities of 20,000, 40,000, 60,000, 80,000, 120,000 and 160,000 per well, 30 minutes guanylin treatment (see Example 9).
- Z' studies: densities of 160,000 and 200,000 per well were used, 30 minutes guanylin treatment (see Example 10).

The functional responses from experiments performed at low and higher passage numbers were compared to identify cells with the most consistent responses over defined periods of time, ranging from 4 to 10 weeks. Other characteristics of the cells that changed over time were also noted.

Populations of cells meeting functional and other criteria were further evaluated to determine those most amenable to production of viable, stable and functional cell lines. Selected populations of cells were expanded in larger tissue culture vessels, and the characterization steps described above were continued or repeated under these conditions. At this point, additional standardization steps, such as different cell densities; time of plating, length of cell culture passage; cell culture dishes format and coating; fluidics optimization, including speed and shear force; time of passage; and washing steps, were introduced for consistent and reliable passages. Also, viability of cells at each passage was determined. Manual intervention was increased, and cells were more closely observed and monitored. This information was used to help identify and select final cell lines that retain the desired properties. Final cell lines and back-up cell lines (20 clones total) were selected that showed appropriate adherence/stickiness and growth rate and even plating (lack of microconfluency) when produced following this process and under these conditions.

The initial frozen stock of 3 vials per each of the selected 20 clones was generated by expanding the non-frozen populations from the re-arrayed 96-well plates via 24-well, 6-well and 10cm dishes in DMEM/10%FBS/HEPES/L-Glu. The low passage frozen stocks corresponding to the final cell line and back-up cell lines were thawed at 37°C, washed two times with DMEM containing FBS and incubated in the same manner. The cells were then expanded for a period of 2 to 4 weeks. Two final clones were selected.

One vial from one clone of the initial freeze was thawed and expanded in culture. The resulting cells were tested to confirm that they met the same characteristics for which they were originally selected. Cell banks for each cell line consisting of 20 to over 100 vials may be established.

The following step can also be conducted to confirm that the cell lines are viable, stable and functional: At least one vial from the cell bank is thawed and expanded in culture; the resulting cells are tested to determine if they meet the same characteristics for which they were originally selected.

**Example 9 Characterizing the cell lines for native GC-C function**

A competitive ELISA for detection of cGMP was used to characterize native GC-C function in the produced GC-C-expressing cell line. Cells expressing GC-C were maintained under standard cell culture conditions in Duibecco's Modified Eagles medium (DMEM) supplemented with 10% fetal bovine serum, glutamine and HEPES and grown in T175cm flasks. For the ELISA, the cells were plated into coated 96-well plates (poly-D-lysine).

*Cell treatment and cell lysis protocol*

Cells were washed twice with serum-free medium and incubated with 1 mM IBMX for 30 minutes. Desired activators (*i.e.,* guanylin, 0.001-40 μM) were then added to the cells and incubated for 30-40 minutes. Supernatant was removed, and the cells were washed with TBS buffer. The cells were lysed with 0.1 N HCl. This was followed by lysis with 0.1 N HCl and a freeze/thaw cycle at -20°C/room temperature. Defrosted lysates (samples were spun in Eppendorf tubes at 10,000rpm) were centrifuged to pellet cell debris. The cleared supernatant lysate was then transferred to ELISA plates.

*ELISA protocol*

All of the following steps were performed at room temperature, unless otherwise indicated. ELISA plates were coated with anti-IgG antibodies in coating buffer (Na-carbonate/bi-carbonate buffer, 0.1M final, pH 9.6) overnight at 4°C. Plates were then washed with wash buffer (TBS-Tween 20, 0.05%), followed by blocking reagent addition. Incubation for 1 hour with blocking reagent at 37°C was followed by a wash of the plates with wash buffer. A rabbit anti-cGMP polyclonal antibody (Chemicon) was then added, followed by incubation for 1 hour and a subsequent wash with wash buffer. Cell lysate was then added, and incubated for 1 hour before the subsequent addition of a cGMP-biotin conjugate (1 and 10nM of 8-Biotin-AET-cGMP (Biolog)). Plates were incubated for 2 hours and then washed with wash buffer. Streptavidin-alkaline phosphate was then added and incubated for 1 hour, then washed with wash buffer. Plates were incubated for at least 1 hour (preferably 2-5 hours) with PNPP substrate (Sigma). The absorbance was then read at 405nm on a SAFIRE$^{2}$™ plate reader (Tecan).

Maximum absorbance was seen when no cell lysate was used in the ELISA (Control). Reduction in absorbance (corresponding to increased cGMP levels) was observed with cell lysate from the produced GC-C-expressing cell line treated with 100 nM guanylin (Clone).

The cGMP level in the produced GC-C-expressing cell line treated with 100 nM guanylin was also compared to that of parental cell line control samples not expressing GC-C (not shown) using the Direct Cyclic GMP Enzyme Immunoassay Kit (Cat. 900-014; AssayDesigns, Inc.). The GC-C-expressing cell line showed a greater reduction in absorbance (corresponding to increased cGMP levels) than parental cells treated and untreated with guanylin.

For guanylin dose-response experiments, cells of the produced GC-C-expressing cell line, plated at densities of 20,000, 40,000, 60,000, 80,000, 120,000 and 160,000 cells/well in a 96-well plate, were challenged with increasing concentration of guanylin for 30 minutes. The cellular response (i.e., absorbance) as a function of changes in cGMP levels (as measured using the Direct Cyclic GMP Enzyme Immunoassay Kit (Cat. 900-014; AssayDesigns, Inc.) was detected using a SAFIRE$^{2}$™ plate reader (Tecan). Data were then plotted as a function of guanylin concentration and analyzed using non-linear regression analysis using GraphPad Prism 5.0 software, resulting in an $EC_{50}$ value of 1.1 nM. The produced GC-C-expressing cell line shows a higher level of cGMP (6 pmol/ml) when treated with low concentrations of guanylin in comparison to that previously reported in other cell lines (3.5 pmol/ml) (Forte et al., Endocr. 140(4):1800-1806 (1999)), indicating the potency of the clone.

**Example 10 Generation of GC-C-expressing cell line Z' value**

Z' for the produced GC-C-expressing cell line was calculated using a direct competitive ELISA assay. The ELISA was performed using the Direct Cyclic GMP Enzyme Immunoassay Kit (Cat. 900-014; AssayDesigns, Inc.). Specifically, for the Z' assay, 24 positive control wells in a 96-well assay plate (plated at a density of 160,000 or 200,000 cells/well) were challenged with a GC-C activating cocktail of 40μM guanylin and IBMX in DMEM media for 30 minutes. Considering the volume and surface area of the 96-well assay plate, this amount of guanylin created a concentration comparable to the 10μM used by Forte et al. (1999) Endocr. 140(4), 1800-1806. An equal number of wells containing clonal cells in DMEM/IMBX were challenged with vehicle alone (in the absence of activator). Absorbance (corresponding to cGMP levels) in the two conditions was monitored using a SAFIRE$^{2}$™ plate reader (Tecan). Mean and standard deviations in the two conditions were calculated and Z' was computed using the method of Zhang et al., J Biomol Screen, 4(2):67-73 (1999)). The Z' value of the produced GC-C-expressing cell line was determined to be 0.72.

**Example 11 Short-circuit current measurements**

Ussing chamber experiments are performed 7-14 days after plating GC-C-expressing cells (primary or immortalized epithelial cells, for example, lung, intestinal, mammary, uterine, or renal) on culture inserts (Snapwell, Corning Life Sciences). Cells on culture inserts are rinsed, mounted in an Ussing type apparatus (EasyMount Chamber System, Physiologic Instruments) and bathed with continuously gassed Ringer solution (5% $CO_2$ in $O_2$, pH 7.4) maintained at 37°C containing (in mM) 120 NaCl, 25 $NaHCO_3$, 3.3 $KH_2PO_4$, 0.8 $K_2HPO_4$, 1.2 $CaCl_2$, 1.2 $MgCl_2$, and 10 glucose. The hemichambers are connected to a multichannel voltage and current clamp (VCC-MC8, Physiologic Instruments). Electrodes [agar bridged (4% in 1 M KCl) Ag-AgCl] are used, and the inserts are voltage clamped to 0 mV. Transepithelial current, voltage and resistance are measured every 10 seconds for the duration of the experiment. Membranes with a resistance of <200mOhms are discarded. This secondary assay can provide confirmation that in the appropriate ceii type *(i.e.,* cell that form tight junctions) the introduced GC-C is altering CFTR activity and modulating a transepithelial current.

**Example 12 Generating a Stable CFTR-Expressing Cell Line**

***Generating Expression Constructs***

Plasmid expression vectors that allowed streamlined cloning were generated based on pCMV-SCRIPT (Stratagene) and contained various necessary components for transcription and translation of a gene of interest, including: CMV and SV40 eukaryotic promoters; SV40 and HSV-TK polyadenylation sequences; multiple cloning sites; Kozak sequences; and drug resistance cassettes.

***Generating Cell Lines***

*Step 1: Transfection*

CHO cells were transfected with a plasmid encoding a human CFTR (SEQ ID NO: CFTR1) using standard techniques. (Example of reagents that may be used to introduce nucleic acids into host cells include, but are not limited to, LIPOFECTAMINE™, LIPOFECTAMINE™ 2000, OLIGOFECTAMINE™, TFX™ reagents, FUGENE®6, DOTAP/DOPE, Metafectine or FECTURIN™.)

Although drug selection is optional to produce the cells or cell lines of this invention, we included one drug resistance marker in the plasmid (i.e., puromycin). The CFTR sequence was under the control of the CMV promoter. An untranslated sequence encoding a CFTR Target Sequence for detection by a signaling probe was also present along with the sequence encoding the drug resistance marker. The target sequence utilized was CFTR Target Sequence 1 (SEQ ID NO: CFTR2), and in this example, the CFTR gene-containing vector comprised CFTR Target Sequence 1 (SEQ ID NO: CFTR2).

*Step 2*: *Selection*

Transfected cells were grown for 2 days in Ham's F12-FBS media without antibiotics, followed by 10 days in 12.5 μg/ml puromycin-containing Ham's F12-FBS media. The cells were then transferred to Ham's F12-FBS media without antibiotics for the remainder of the time, prior to the addition of the signaling probe.

*Step 3: Cell passaging*

Following enrichment on antibiotic, cells were passaged 5 - 14 times in the absence of antibiotic selection to allow time for expression that was not stable over the selected period of time to subside.

*Step 4: Exposure of cells to fluorogenic probes*

Ceiis were harvested and transfected with CFTR Signaling Probe 1 (SEQ ID NO: CFTR3) using standard techniques. (examples of reagents that may be used to introduce nucleic acids into host cells include, but are not limited to, LIPOFECTAMINE™, LIPOFECTAMINE™ 2000, OLIGOFECTAMINE™, TFX™ reagents, FUGENE™6, DOTAP/DOPE, Metafectine or FECTURIN™.) CFTR Signaling Probe 1 (SEQ iD NO: CFTR3) bound CFTR Target Sequence 1 (SEQ ID NO: CFTR2). The cells were then collected for analysis and sorted using a fluorescence activated cell sorter.

**Target Sequence detected by signaling probe**

CFTR Target Sequence 1

5'- GTTCTTAAGGCACAGGAACTGGGAC -3' (SEQ ID NO: CFTR2)

**Signaling probe**

Supplied as 100μM stock

CFTR Signaling probe 1

5' - **Cy5** GCCAGTCCCAGTTCCTGTGCCTTAAGAACCTCGC **BHQ2** -3' (SEQ ID NO: CFTR3)

In addition, a similar probe using a Quasar® Dye (BioSearch) with spectral properties similar to Cy5 was used in certain experiments. In some experiments, 5-MedC and 2-amino dA mixmers were used rather than DNA probes. A non-targeting FAM labeled probe was also used as a loading control.

*Step* 5: *Isolation of positive cells*

The cells were dissociated and collected for analysis and sorting using a fluorescence activated cell sorter. Standard analytical methods were used to gate cells fluorescing above background and to isolate individual cells falling within the gate into bar-coded 96-well plates. The following gating hierarchy was used: coincidence gate → singlets gate → live gate → Sort gate in plot FAM vs. Cy5: 0.1 - 0.4 % of cells.

*Step 6: Additional cycles of steps 1-5 and/or 3-5*

Steps 1-5 and/or 3-5 were repeated to obtain a greater number of cells. Two rounds of steps 1-5 were performed, and for each of these rounds, two internal cycles of steps 3-5 were performed.

*Step 7: Estimation of growth rates for the populations of cells*

The plates were transferred to a Microlab Star (Hamilton Robotics). Cells were incubated for 9 days in 100 μl of 1:1 mix of fresh complete growth media and 2 to 3 day-conditioned growth media, supplemented with 100 units/ml penicillin and 0.1 mg/ml streptomycin. Then the cells were dispersed by trypsinization once or twice to minimize clumps and later transferred to new 96-well plates. Plates were imaged to determine confluency of wells (Genetix). Each plate was focused for reliable image acquisition across the plate. Reported confluencies of greater than 70% were not relied upon. Confluency measurements were obtained on consecutive days between days 1 and 10 post-dispersal and used to calculate growth rates.

*Step 8: Binning populations of cells according to growth rate estimates*

Cells were binned (independently grouped and plated as a cohort) according to growth rate less than two weeks following the dispersal step in step 7. Each of the three growth bins was separated into individual 96 well plates; some growth bins resulted in more than one 96 well plate. Bins were calculated by considering the spread of growth rates and bracketing a high percentage of the total number of populations of cells. Bins were calculated to capture 12-16 hour differences in growth rate.

Cells can have doubling times from less 1 day to more than 2 week. In order to process the most diverse clones that at the same time can be reasonably binned according to growth rate, it may be preferable to use 3-9 bins with a 0.25 to 0.7 day doubling time per bin. One skilled in the art will appreciate that the tightness of the bins and number of bins can be adjusted for the particular situation and that the tightness and number of bins can be further adjusted if cells are synchronized for their cell cycle.

*Step 9: Replica plating to speed parallel processing and provide stringent quality control*

The plates were incubated under standardized and fixed conditions (*i.e.*, Ham's F12-FBS media, 37°C/5%CO2) without antibiotics. The plates of cells were split to produce 4 sets of 96 well plates (3 sets for freezing, 1 set for assay and passage). Distinct and independent tissue culture reagents, incubators, personnel, and carbon dioxide sources were used for each of the sets of the plate. Quality control steps were taken to ensure the proper production and quality of all tissue culture reagents: each component added to each bottle of media prepared for use was added by one designated person in one designated hood with only that reagent in the hood while a second designated person monitored to avoid mistakes. Conditions for liquid handling were set to eliminate cross contamination across wells. Fresh tips were used for all steps or stringent tip washing protocols were used. Liquid handling conditions were set for accurate volume transfer, efficient cell manipulation, washing cycles, pipetting speeds and locations, number of pipetting cycles for cell dispersal, and relative position of tip to plate.

*Step 10: Freezing early passage stocks of populations of cells*

Three sets of plates were frozen at -70 to -80°C. Plates in the set were first allowed to attain confluencies of 70 to 100%. Medium was aspirated and 90%FBS and 10% DMSO was added. The plates were individually sealed with Parafilm, individually surrounded by 1 to 5cm of foam, and then placed into a -80°C freezer.

*Steep 11: Methods and conditions for initial transformative steps to produce viable, stable and functional (VSF) cell lines*

The remaining set of plates was maintained as described in step 9. All cell splitting was performed using automated liquid handling steps, including media removal, cell washing, trypsin addition and incubation, quenching and cell dispersal steps.

*Step 12: Normalization methods to correct any remaining variability of growth rates*

The consistency and standardization of cell and culture conditions for all populations of cells was controlled. Differences across plates due to slight differences in growth rates were controlled by normalization of cell numbers across plates and occurred every 8 passages after the rearray. Populations of cells that were outliers were detected and eliminated.

*Step 13: Characterization of population of cells*

The cells were maintained for 6 to 10 weeks post rearray in culture to allow for their *in vitro* evolution under these conditions. During this time, we observed size, morphology, tendency towards microconfluency, fragility, response to trypsinization and average circularity post-trypsinization, or other aspects of cell maintenance such as adherence to culture plate surfaces and resistance to blow-off upon fluid addition.

*Step 14: Assessment of potential functionality of populations of cells under VSF conditions*

Populations of cells were tested using functional criteria. Membrane potential dye kits (Molecular Devices, MDS) were used according to manufacturer's instructions.

Cells were tested at varying densities in 384-well plates (i.e., 12.5 x $10^3$ to 20 x $10^3$ cells/per well) and responses were analyzed. Time between cell plating and assay read was tested. Dye concentration was also tested. Dose response curves and Z' scores were both calculated as part of the assessment of potential functionality.

The following steps *(i.e.,* steps 15 -18) can also be conducted to select final and back-up viable, stable and functional cell lines.

*Step 15:*

The functional responses from experiments performed at low and higher passage numbers are compared to identify cells with the most consistent responses over defined periods of time (*e.g.*, 3 - 9 weeks). Other characteristics of the cells that change over time are also noted.

*Step 16:*

Populations of cells meeting functional and other criteria are further evaluated to determine those most amenable to production of viable, stable and functional cell lines. Selected populations of cells are expanded in larger tissue culture vessels and the characterization steps described above are continued or repeated under these conditions. At this point, additional standardization steps, such as different cell densities; time of plating, length of cell culture passage; cell culture dishes format and coating; fluidics optimization, including speed and shear force; time of passage; and washing steps, are introduced for consistent and reliable passages.

In addition, viability of cells at each passage is determined. Manual intervention is increased and cells are more closely observed and monitored. This information is used to help identify and select final cell lines that retain the desired properties. Final cell lines and back-up cell lines are selected that show appropriate adherence/stickiness, growth rate, and even plating (lack of microconfluency) when produced following this process and under these conditions.

*Step 17: Establishment of cell banks*

The low passage frozen stocks corresponding to the final cell line and back-up cell lines are thawed at 37°C, washed two times with Ham's F12-FBS and then incubated in Ham's F12-FBS. The cells are then expanded for a period of 2 to 4 weeks. Cell banks of clones for each final and back-up cell line are established, with 25 vials for each clonal cells being cryopreserved.

*Step 18:*

At least one vial from the cell bank is thawed and expanded in culture. The resulting cells are tested to determine if they meet the same characteristics for which they are originally selected.

**Example 13 Characterizing Stable Cell Lines for Native CFTR Function**

We used a high-throughput compatible fluorescence membrane potential assay to characterize native CFTR function in the produced stable CFTR-expressing cell lines.

CHO cell lines stably expressing CFTR were maintained under standard cell culture conditions in Ham's F12 medium supplemented with 10% fetal bovine serum and glutamine. On the day before assay, the cells were harvested from stock plates and plated into black clear-bottom 384 well assay plates. The assay plates were maintained in a 37°C cell culture incubator under 5% $CO_2$ for 22-24 hours. The media was then removed from the assay plates and blue membrane potential dye (Molecular Devices Inc.) diluted in loading buffer (137 mM NaCl, 5 mM KCl, 1.25 mM $CaCl_2$, 25 mM HEPES, 10 mM glucose) was added and allowed to incubate for 1 hour at 37°C. The assay plates were then loaded on a fluorescent plate reader (Hamamatsu FDSS) and a cocktail of forskolin and IBMX dissolved in compound buffer (137 mM sodium gluconate, 5 mM potassium gluconate, 1.25 mM $CaCl_2$, 25 mM HEPES, 10mM giucose) was added.

Representative data from the fluorescence membrane potential assay showed that the ion flux attributable to functional CFTR in stable CFTR-expressing CHO cell lines (cell line 1, M11, J5, E15, and 015) were all higher than control cells lacking CFTR as indicated by the assay response.

The ion flux attributable to functional CFTR in stable CFTR-expressing CHO cell lines (cell line 1, M11, J5, E15, and 015) were also all higher than transiently CFTR-transfected CHO cells. The transiently CFTR-transfected cells were generated by plating CHO cells at 5-16 million per 10cm tissue culture dish and incubating them for 18-20 hours before transfection. A transfection complex consisting of lipid transfection reagent and plasmids encoding CFTR was directly added to each dish. The cells were then incubated at 37°C in a $CO_2$ incubator for 6-12 hours. After incubation, the cells were lifted, plated into black clear-bottom 384 well assay plates, and assayed for function using the above-described fluorescence membrane potential assay.

For forskolin dose-response experiments, cells of the produced stable CFTR-expressing cell lines, plated at a density of 15,000 cells/well in a 384-well plate were challenged with increasing concentration of forskolin, a known CFTR agonist. The cellular response as a function of changes in cell fluorescence was monitored over time by a fluorescent plate reader (Hamamatsu FDSS). Data were then plotted as a function of forskolin concentration and analyzed using non-linear regression analysis using GraphPad Prism 5.0 software, resulting in an $EC_{50}$ of 256 nM. The produced CFTR-expressing cell line shows a $EC_{50}$ value of forskolin within the ranges of $EC_{50}$ if forskolin previously reported in other cell lines (between 250 and 500 nM) (Galietta *et al.,* Am J Physiol Cell Physiol. 281 (5): C1734-1742 (2001)), indicating the potency of the clone.

**Example 14 Determination of Z' Value for CFTR Cell-Based Assay**

Z' value for the produced stable CFTR-expressing cell line was calculated using a high-throughput compatible fluorescence membrane potential assay. The fluorescence membrane potential assay protocol was performed substantially according to the protocol in **Example 13.** Specifically for the Z' assay, 24 positive control wells in a 384-well assay plate (plated at a density of 15,000 cells/well) were challenged with a CFTR activating cocktail of forskolin and IBMX. An equal number of wells were challenged with vehicle alone and containing DMSO (in the absence of activators). Cell responses in the two conditions were monitored using a fluorescent plate reader (Hamamatsu FDSS). Mean and standard deviations in the two conditions were calculated and Z' was computed using the method disclosed in Zhang et al., J Biomol Screen, 4(2): 67-73, (1999). The Z' value of the produced stable CFTR-expressing cell line was determined to be higher than or equal to 0.82.

**Example 15 High-Throughput Screening and Identification of CFTR Modulators**

A high-throughput compatible fluorescence membrane potential assay is used to screen and identify CFTR modulator. On the day before assay, the cells are harvested from stock plates into growth media without antibiotics and plated into black clear-bottom 384 well assay plates. The assay plates are maintained in a 37°C cell culture incubator under 5% $CO_2$ for 19-24 hours. The media is then removed from the assay plates and blue membrane potential dye (Molecular Devices Inc.) diluted in load buffer (137 mM NaCl, 5 mM KCl, 1.25 mM $CaCl_2$, 25 mM HEPES, 10 mM glucose) is added and the cells are incubated for 1 hr at 37°C. Test compounds are solubilized in dimethylsulfoxide, diluted in assay buffer (137 mM sodium gluconate, 5 mM potassium gluconate, 1.25 mM $CaCl_2$, 25 mM HEPES, 10mM glucose) and then loaded into 384 well polypropylene micro-titer plates. The cell and compound plates are loaded into a fluorescent plate reader (Hamamatsu FDSS) and run for 3 minutes to identify test compound activity. The instrument will then add a forskolin solution at a concentration of 300 nM - 1 $\mu$M to the cells to allow either modulator or blocker activity of the previously added compounds to be observed. The activity of the compound is determined by measuring the change in fluorescence produced following the addition of the test compounds to the cells and/or following the subsequent agonist addition.

**Example 16 Characterizing Stable CFTR-Expressing Cell Lines for Native CFTR Function using Short-Circuit Current Measurements**

Ussing chamber experiments are performed 7-14 days after plating CFTR-expressing cells (primary or immortalized epithelial cells including but not limited to lung and intestinal) on culture inserts (Snapwell, Corning Life Sciences). Cells on culture inserts are rinsed, mounted in an Ussing type apparatus (EasyMount Chamber System, Physiologic instruments) and bathed with continuously gassed Ringer solution (5% $CO_2$ in $O_2$, pH 7.4) maintained at 37°C containing 120 mM NaCl, 25 mM $NaHCO_3$, 3.3 mM $KH_2PO_4$, 0.8 mM $K_2HPO_4$, 1.2 mM $CaCl_2$, 1.2 mM $MgCl_2$, and 10 mM glucose. The hemichambers are connected to a multichannel voltage and current clamp (VCC-MC8 Physiologic Instruments). Electrodes [agar bridged (4% in 1 M KCl) Ag-AgCl] are used and the inserts are voltage clamped to 0 mV. Transepithelial current, voltage and resistance are measured every 10 seconds for the duration of the experiment. Membranes with a resistance of < 200 m$\Omega$s are discarded.

**Example 17 Characterizing Stable CFTR-expressing Cell Lines for Native CFTR Function using Electrophysiological Assay**

While both manual and automated electrophysiology assays have been developed and both can be applied to assay this system, described below is the protocol for manual patch clamp experiments.

Cells are seeded at low densities and are used 2-4 days after plating. Borosilicate glass pipettes are fire-polished to obtain tip resistances of 2-4 mega $\Omega$. Currents are sampled and low pass filtered. The extracellular (bath) solution contains: 150 mM NaCl, 1 mM $CaCl_2$, 1 mM $MgCl_2$, 10 mM glucose, 10 mM mannitol, and 10 mM TES, pH 7.4. The pipette solution contains: 120 mM CsCl, 1 mM $MgCl_2$, 10 mM TEA-Cl, 0.5 mM EGTA, 1 mM Mg-ATP, and 10 mM HEPES (pH 7.3). Membrane conductances are monitored by alternating the membrane potential between -80 mV and -100 mV. Current-voltage relationships are generated by applying voltage pulses between -100 mV and +100 mV in 20-mV steps.

## Example 18 Generating a Stable NaV 1.7 Heterotrimer-Expressing Cell Line

### *Generating Expression Constructs*

Plasmid expression vectors that allowed streamlined cloning were generated based on pCMV-SCRIPT (Stratagene) and contained various necessary components for transcription and translation of a gene of interest, including: CMV and SV40 eukaryotic promoters; SV40 and HSV-TK polyadenylation sequences; multiple cloning sites; Kozak sequences; and Neomycin/Kanamycin resistance cassettes (or Ampicillin, Hygromycin, Puromycin, Zeocin resistance cassettes).

### *Generation of Cell Lines*

*Step 1: Transfection*

293T cells were cotransfected with three separate plasmids, one encoding a human NaV 1.7 α subunit (SEQ ID NO: NAV-1), one encoding a human NaV 1.7 β1 subunit (SEQ ID NO: NAV-2) and one encoding a human NaV 1.7 β2 subunit (SEQ ID NO: NAV-3), using standard techniques. (Examples of reagents that may be used to introduce nucleic acids into host cells include, but are not limited to, LIPOFECTAMINE™, LIPOFECTAMINET™ 2000, OLIGOFECTAMINE™, TFX™ reagents, FUGENE® 6, DOTAP/DOPE, Metafectine or FECTURIN™.)

Although drug selection is optional to produce the cells or cell lines of this invention, we included one drug resistance marker per plasmid. The sequences were under the control of the CMV promoter. An untranslated sequence encoding a NaV Target Sequence for detection by a signaling probe was also present along with the sequence encoding the drug resistance marker. The NaV Target Sequences utilized were NaV Target Sequence 1 (SEQ ID NO: NAV-4), NaV Target Sequence 2 (SEQ ID NO: NAV-5) and NaV Target Sequence 3 (SEQ ID NO: NAV-6). In this example, the NaV 1.7 α subunit gene-containing vector comprised NaV Target Sequence 1 (SEQ ID NO: NAV-4); the NaV 1.7 β1 subunit gene-containing vector comprised NaV Target Sequence 2 (SEQ ID NO: NAV-5); and the NaV 1.7 β2 subunit gene-containing vector comprised NaV Target Sequence 3 (SEQ ID NO: NAV-6).

*Step 2: Selection*

Transfected cells were grown for 2 days in DMEM-FBS media, followed by 10 days in antibiotic-containing DMEM-FBS media. During the antibiotic containing period, antibiotics were added to the media as follows: puromycin (0.1 μg/ml), hygromycin (100 μg/ml), and zeocin (200 μg/ml).

*Step 3: Cell passaging*

Following enrichment on antibiotic, cells were passaged 6-18 times in the absence of antibiotic selection to allow time for expression that was not stable over the selected period of time to subside.

*Step 4: Exposure of cells to fluorogenic probes*

Cells were harvested and transfected with signaling probes (SEQ ID NOS: NaV-7, NaV-8, NaV-9) using standard techniques. (Examples of reagents that may be used to introduce nucleic acids into host cells include, but are not limited to, LIPOFECTAMINE™, LIPOFECTAMINE™ 2000, OLIGOFECTAMINE™, TFX™ reagents, FUGENE® 6, DOTAP/DOPE, Metafectine or FECTURIN™.)

NaV Signaling Probe 1(SEQ ID NO: NAV-7) bound NaV Target Sequence 1 (SEQ ID NO: NAV-4); NaV Signaling Probe 2 (SEQ ID NO: NAV-8) bound NaV Target Sequence 2 (SEQ ID NO: NAV-5); and NaV Signaling Probe 3 (SEQ ID NO: NAV-9) bound NaV Target Sequence 3 (SEQ ID NO: NAV-6). The cells were then dissociated and collected for analysis and sorted using a fluorescence activated cell sorter.

**Target Sequences detected by signaling probes**
The following tag sequences were used for the NaV 1.7 subunit transgenes.

**NaV Target Sequence 1**
5'-GTTCTTAAGGCACAGGAACTGGGAC-3' (SEQ ID NO: NAV-4) (NaV 1.7 α subunit)
**NaV Target Sequence 2**
5'-GAAGTTAACCCTGTCGTTCTGCGAC-3' (SEQ ID NO: NAV-5) (NaV 1.7 β1 subunit )
**NaV Target Sequence 3**
5'-GTTCTATAGGGTCTGCTTGTCGCTC-3' (SEQ ID NO: NAV-6) (NaV 1.7 β2 subunit )

**Signaling probes**

Supplied as 100 μM stocks.

**NaV Signaling probe 1 -** This probe binds target sequence 1.
**5**' - **Cy5** GCCAGTCCCAGTTCCTGTGCCTTAAGAACCTCGC **BHQ3 quench -3**' (SEQ ID NO: NAV-7)
**NaV Signaling probe 2** - This probe binds target sequence 2.
**5'- Cy5.5** CGAGTCGCAGAACGACAGGGTTAACTTCCTCGC **BHQ3 quench -3**' (SEQ ID NO: NAV-8)
**NaV Signaling probe 3** - This probe binds target sequence 3.
**5'- Fam** CGAGAGCGACAAGCAGACCCTATAGAACCTCGC **BHQ1 quench** - **3'** (SEQ ID NO: NAV-9)

BHQ3 in NaV Signaling probes 1 and 2 can be replaced by BHQ2 or gold particle. BHQ1 in NaV Signaling probe 3 can be replaced by BHQ2, gold particle, or DABCYL.

In addition, a similar probe using a Quasar® Dye (BioSearch) with spectral properties similar to Cy5 was used in certain experiments. In some experiments, 5-MedC and 2-amino dA mixmer probes were used rather than DNA probes.

*Step 5: Isolation of positive cells*

Standard analytical methods were used to gate cells fluorescing above background and to isolate cells falling within the defined gate directly into 96-well plates. Flow cytometric cell sorting was operated such that a single cell was deposited per well. After selection, the cells were expanded in media lacking drug.
The following gating hierarchy was used:
coincidence gate → singlets gate → live gate → Sort gate in plot FAM vs. Cy5: 0.1 - 1.0% of live cells.

*Step 6: Additional cycles of steps 1-5 and/or 3-5*

Steps 1-5 and/or 3-5 were repeated to obtain a greater number of cells. At least four independent rounds of steps 1-5 were completed, and for each of these cycles, at least two internal cycles of steps 3-5 were performed for each independent round.

*Step 7*: *Estimation of growth rates for the populations of cells*

The plates were transferred to a Microlabstar automated liquid handler (Hamilton Robotics). Cells were incubated for 5-7 days in a 1:1 mix of fresh complete growth medium (DMEM/10% FBS) and 2-3 day conditioned growth medium, supplemented with 100 units/ml penicillin and 0.1mg/ml streptomycin. Then the cells were dispersed by trypsinization to minimize clumps and transferred to new 96-well plates. After the clones were dispersed, plates were imaged to determine confluency of wells (Genetix). Each plate was focused for reliable image acquisition across the plate. Reported confluencies of greater than 70% were not relied upon. Confluency measurements were obtained at days every 3 times over 9 days (i.e, between days 1 and 10 post-dispersal) and used to calculate growth rates.

*Step 8: Binning populations of cells according to growth rate estimates*

Cells were binned (independently grouped and plated as a cohort) according to growth rate between 10-11 days following the dispersal step in step 7. Bins were independently collected and plated on individual 96 well plates for downstream handling; some growth bins resulted in more than one 96-well plate. Bins were calculated by considering the spread of growth rates and bracketing a high percentage of the total number of populations of cells. Depending on the sort iteration described in Step 5, between 5 and 9 growth bins were used with a partition of 1- 4 days. Therefore, each bin corresponded to a growth rate or population doubling time between 8 and 14.4 hours depending on the iteration.

Cells can have doubling times from less 1 day to more than 2 weeks. In order to process the most diverse clones that at the same time can be reasonably binned according to growth rate, it is preferable to use 3-9 bins with a 0.25 to 0.7 day doubling time per bin. One skilled in the art will appreciate that the tightness of the bins and number of bins can be adjusted for the particular situation and that the tightness and number of bins can be further adjusted if cells are synchronized for their cell cycle.

*Step 9: Replica plating to speed parallel processing and provide stringent quality control*

The plates were incubated under standard and fixed conditions (humidified 37°C, 5%CO2) in antibiotics-free DMEM-10%FBS media. The plates of cells were split to produce 4 sets of target plates. These 4 sets of plates comprised all plates with all growth bins to ensure there were 4 replicates of the initial set. Up to 3 target plate sets were committed for cryopreservation (described in step 10), and the remaining set was scaled and further replica plated for passage and functional assay experiments. Distinct and independent tissue culture reagents, incubators, personnel, and carbon dioxide sources were used for downstream replica plates. Quality control steps were taken to ensure the proper production and quality of all tissue culture reagents: each component added to each bottle of media prepared for use was added by one designated person in one designated hood with only that reagent in the hood while a second designated person monitored to avoid mistakes. Conditions for liquid handling were set to eliminate cross contamination across wells. Fresh tips were used for all steps, or stringent tip washing protocols were used. Liquid handling conditions were set for accurate volume transfer, efficient cell manipulation, washing cycles, pipetting speeds and locations, number of pipetting cycles for cell dispersal, and relative position of tip to plate.

*Step 10: Freezing early passage stocks of populations of cells*

Three sets of plates were frozen at -70 to -80°C. Plates in each set were first allowed to attain confluencies of 70 to 80%. Medium was aspirated and 90%FBS and 5%-10% DMSO was added. The plates were sealed with Parafilm, individually surrounded by 1 to 5cm of foam, and then placed into a -80°C freezer.

*Step 11: Methods and conditions for initial transrormative steps to produce viable, stable and functional (VSF) cell lines*

The remaining set of plates was maintained as described in step 9. All cell splitting was performed using automated liquid handling steps, including media removal, cell washing, trypsin addition and incubation, quenching and cell dispersal steps. For some assay plating steps, cells were dissociated with cell dissociation buffer (*e.g.*, CDB, invitrogen or CellStripper, CellGro) rather than trypsin.

*Step 12: Normalization methods to correct any remaining variability of growth rates*

The consistency and standardization of cell and culture conditions for all populations of cells was controlled. Differences across plates due to slight differences in growth rates were controlled by periodic normalization of cell numbers across plates every 2 to 8 passages. Populations of cells that were outliers were detected and eliminated.

*Step 13: Characterization of population of cells*

The cells were maintained for 3 to 8 weeks to allow for their *in vitro* evolution under these conditions. During this time, we observed size, morphology, fragility, response to trypsinization or dissociation, roundness/average circularity post-dissociation, percentage viability, tendency towards microconfluency, or other aspects of cell maintenance such as adherence to culture plate surfaces.

*Step 14*: *Assessment of potential functionality of populations of cells under VSF conditions*

Populations of cells were tested using functional criteria. Membrane potential assay kits (Molecular Devices/MDS) were used according to manufacturer's instructions. Cells were tested at multiple different densities in 96- or 384-well plates and responses were analyzed. A variety of post-plating time points were used, *e.g.*, 12-48 hours post plating. Different densities of plating were also tested for assay response differences.

*Step 15:*

The functional responses from experiments performed at low and higher passage numbers were compared to identify cells with the most consistent responses over defined periods of time, ranging from 3 to 9 weeks. Other characteristics of the cells that changed over time were also noted.

*Step 16:*

Populations of cells meeting functional and other criteria were further evaluated to determine those most amenable to production of viable, stable and functional cell lines. Selected populations of cells were expanded in larger tissue culture vessels and the characterization steps described above were continued or repeated under these conditions. At this point, additional standardization steps, such as different plating cell densities; time of passage; culture dish size/format and coating); fluidics optimization; cell dissociation optimization (e.g., type, volume used, and length of time); and washing steps, were introduced for consistent and reliable passages. Temperature differences were also used for standardization (*i.e.*, 30°C vs 37°C).

In addition, viability of cells at each passage was determined. Manual intervention was increased and cells were more closely observed and monitored. This information was used to help identify and select final cell lines that retained the desired properties. Final cell lines and back-up cell lines were selected that showed consistent growth, appropriate adherence, and functional response.

*Step 17: Establishment of cell banks*

The low passage frozen plates described above corresponding to the final cell line and back-up cell lines were thawed at 37°C, washed two times with DMEM-10% FBS and incubated in humidified 37°C /5% CO2 conditions. The cells were then expanded for a period of 2-3 weeks. Cell banks for each final and back-up cell line consisting of 15-20 vials were established.

*Step 18:*

The following step can also be conducted to confirm that the cell lines are viable, stable, and functional. At least one vial from the cell bank is thawed and expanded in culture. The resulting cells are tested to determine if they meet the same characteristics for which they were originally selected.

**Example 19 Characterizing Relative Expression of Heterologous NaV 1.7 Subunits in Stable NaV 1.7-Expressing Cell Lines**

Quantitative RT-PCR (qRT-PCR) was used to determine the relative expression of the heterologous human NaV 1.7 α, β1, and β2 subunits in the produced stable NaV 1.7-expressing cell lines. Total RNA was purified from 1-3x$10^6$ mammalian cells using an RNA extraction kit (RNeasy Mini Kit, Qiagen). DNase treatment was done according to rigorous DNase treatment protocol (TURBO DNA-free Kit, Ambion). First strand cDNA synthesis was performed using a reverse transcriptase kit (SuperScript III, Invitrogen) in 20 μL reaction volume with 1 μg DNA-free total RNA and 250 ng Random Primers (Invitrogen). Samples without reverse transcriptase and sample without RNA were used as negative controls for this reaction. Synthesis was done in a thermal cycler (Mastercycler, Eppendorf) at the following conditions: 5 min at 25°C, 60 min at 50°C; reaction termination was conducted for 15 min at 70°C.

For analysis of gene expression, primers and probes for qRT-PCR (MGB TaqMan probes, Applied Biosystems) were designed to specifically anneal to the target sequences (SEQ ID NOS: NaV-4, NaV-5, NaV-6). For sample normalization, control (glyceraidehyde 3-phosphate dehydrogenase (GAPDH)) Pre-Developed Assay reagents (TaqMaN, Applied Biosystems) were used. Reactions, including negative controls and positive controls (plasmid DNA), were set up in triplicates with 40 ng of cDNA in 50 μL reaction volume. The relative amounts of each of the three NaV 1.7 subunits being expressed were determined. All three subunits were successfully expressed in the produced stable NaV 1.7-expressing cell line.

**Example 20 Characterizing Stable NaV 1.7-Expressing Cell Lines for Native NaV Function using Electrophysiological Assay**

Automated patch-clamp system was used to record sodium currents from the produced stable HEK293T cell lines expressing NaV 1.7 α, β1, and β2 subunits. The following illustrated protocol can also be used for QPatch, Sophion or Patchliner, Nanion systems. The extracellular Ringer's solution contained 140 mM NaCl, 4.7 mM KCl, 2.6 mM $MgCl_2$, 11 mM glucose and 5 mM HEPES, pH 7.4 at room temperature. The intracellular Ringer's solution contained 120 mM CsF, 20 mM Cs-EGTA, 1 mM $CaCl_2$, 1 mM $MgCl_2$, and 10 mM HEPES, pH 7.2. Experiments were conducted at room temperature.

Cells stably expressing NaV 1.7 α, β1, and β2 subunits were grown under standard culturing protocols as described in **Example 18.** Cells were harvested and kept in suspension with continuous stirring for up to 4 hours with no significant change in quality or ability to patch. Electrophysiological experiment (whole-cell) was performed using the standard patch plate. The patch-clamp hole (micro-etched in the chip) is approximately 1 μm in diameter and has a resistance of ~2 MΩ. The membrane potential was clamped to a holding potential of -100 mV.

Current-voltage relation and inactivation characteristics of voltage-gated human NaV 1.7 sodium channel stably expressed in HEK293T cells were characterized. Sodium currents were measured in response to 20 ms depolarization pulses from - 80 mV to +50 mV with a holding potential of -100 mV. The resulting current-voltage (I-V) relationship for peak sodium channel currents was characterized. The activation threshold was - 35 mV (midpoint of activation, Va = - 24.9 mV +/- 3.7 mV), and the maximal current amplitude was obtained at -10 mV. The inactivation graph for the sodium channel was plotted. The membrane potential was held at a holding potential of - 100 mV, subsequently shifted to conditioning potentials ranging from -110 mV to +10 mV for 1000 ms, and finally the current was measured upon a step to 0 mV. The resulting current amplitude indicates the fraction of sodium channels in the inactivated state. At potentials more negative than - 85 mV the channels were predominantly in the closed state, whereas at potentials above -50 mV they were predominantly in the inactivated state. The curve represents the Boltzmann fit from which the $V_{1/2}$ for steady-state inactivation was estimated to be - 74 mV. The current-voltage profile for the produced stable NaV 1.7-expressing cell lines is consistent with previously reported current-voltage profile (Va= - 28.0 mV $\pm$1.1 mV; $V_{1/2}$ = -71.3 mV $\pm$ 0.8 mV) (Sheets et al., J Physiol. 581 (Pt 3):1019-1031. (2007)).

**Example 21 Characterizing Stable NaV 1.7-Expressing Cell Lines for Native NaV Function using Membrane Potential Assay**

The produced stable cells expressing NaV 1.7 α, β1, and β2 subunits were maintained under standard cell culture conditions in Dulbecco's Modified Eagles medium supplemented with 10% fetal bovine serum, glutamine and HEPES. On the day before assay, the cells were harvested from stock plates using cell dissociation buffer, e.g., CDB (GIBCO) or cell-stripper (Mediatech), and plated at 10, 000 - 25,000 cells per well in 384 well plates in growth media. The assay plates were maintained in a 37°C cell culture incubator under 5%CO2 for 22-24 hours. The media were then removed from the assay plates and blue fluorescence membrane potential dye (Molecular Devices Inc.) diluted in load buffer (137 mM NaCl, 5 mM KCl, 1.25 mM $CaCl_2$, 25 mM HEPES, 10 mM glucose) was added. The cells were incubated with blue membrane potential dye for 1 hour at 37°C. The assay plates were then loaded onto the high-throughput fluorescent plate reader (Hamamastu FDSS). The fluorescent plate reader measures cell fluorescence in images taken of the cell plate once per second and displays the data as relative florescence units.

The assay response of stable NaV 1.7-expressing cells and control cells (*i.e.*, HEK293T parental cells) to addition of buffer and channel activators (*i.e.*, veratridine and scorpion venom (SV)) were measured. In a first addition step (*i.e.*, Addition 1), only buffer was added, with no test compounds added. If desired, test compounds can be added in this step. In a second addition step, veratridine and scorpion venom, which are sodium channels activators, were diluted in assay buffer to the desired concentration (*i.e.*, 25 μM veratridine and 5 - 25 μg/ml scorpion venom) and added into 384 well polypropylene microtiter plates. Once bound, veratridine and scorpion venom proteins modulate the activity of voltage-gated sodium channels through a combination of mechanisms, including an alteration of the activation and inactivation kinetics. The resulted activation of sodium channels in stable NaV 1.7-expressing cells changes cells membrane potential and the fluorescent signal increases. The above-described functional assay can also be used to characterize the relative potencies of test compounds at NaV 1.7 ion channels.

**Example 22 Characterizing Regulation of NaV 1.7 Alpha Subunit by Beta Subunits**

***Regulation of Alpha*** *Subunit Gene Expression by Beta Subunits*

Pools of HEK293T cells were engineered to express various ratios of α and β subunits by manipulating the molar ratios of independent plasmid DNAs or α and control plasmids (*e.g.*, α:β1:β2 = 1:1:1). After drug selection the subunits expression in six different cell pools were evaluated with qRT-PCR as described in **Example 19.** Comparative qRT-PCR indicated that α subunit expression in drug-selected cells detection was increased when all three human NaV 1.7 subunits (*i.e.*, α, β1, and β2) were co-transfected in compared to only α subunit and control plasmid transfected. The presence of the β subunit transcripts affects α subunit gene expression, demonstrating the importance of co-expressing all three NaV 1.7 subunits for a physiologically relevant functional assay.

*Regulation of Pharmacological Properties by Beta Subunits*

A membrane potential cell-based assay was used to measure the response to test compounds of the cells stably co-expressing all three NaV 1.7 subunits (*i.e.*, α, β1, and β2) and control cells stably expressing only a NaV 1.7 α subunit. Two compounds (*i.e.*, C18 and K21) were tested in the membrane potential assay performed substantially according to the protocol in **Example 21.** Specifically for this example, the test compounds were added in the first addition step.

C18 and K21 potentiated the response of clone C44 (expressing NaV 1.7 α, β1, and β2 subunits) and blocked the response of clone C60 (expressing NaV 1.7 α subunit only). The assay response of the two test compounds was normalized to the response of buffer alone for each of the two clones.

Table 2 Mammalian G proteins, their families and descriptions

| **Class** | **Family/*Subtype*** | **Protein # (UniProt)** | **Description** |
|---|---|---|---|
| **G-alpha** | **$G_s$** | | |
| | *Gs* | P04896 | Galpha-s-Bos taurus |
| | *Gs* | P16052 | Galpha-s-Cricetulus longicaudatus |
| | *Gs* | P63092 | Galpha-s-Homo sapiens-2 |
| | *Gs* | P63091 | Galpha-s-Canis familiaris |
| | *Gs* | P63093 | Galpha-s-Mesocricetus auratus |
| | *Gs* | P63094 | Galpha-s-Mus musculus-2 |
| | *Gs* | P63095 | Galpha-s-Rattus norvegicus-2 |
| | *Gs* | P29797 | Galpha-s-Sus scrofa |
| | *Gs* | O60726 | Galpha-s-Homo sapiens-4 |
| | *Gs* | O75632 | Galpha-s-Homo sapiens-5 |
| | *Gs* | O75633 | Galpha-s-Homo sapiens-6 |
| | *Gs* | Q14433 | Galpha-s-Homo sapiens-7 |
| | *Gs* | Q14455 | Galpha-s-Homo sapiens |
| | *Gs* | Q8R4A8 | Galpha-s-Cricetulus griseus |
| | *Gs* | Q9JJ33 | Galpha-s-Mus musculus |
| | *Gs* | Q9JLG1 | Galpha-s-Rattus norvegicus-1 |
| | *Gs* | Q5JWF2 | Galpha-s-Homo sapiens-3 |
| | *Golf* | P38405 | Galpha-olf-Homo sapiens-2 |
| | *Golf* | Q8CGK7 | Galpha-olf-Mus musculus |
| | *Golf* | P38406 | Galpha-olf-Rattus norvegicus |
| | *Golf* | Q86XU3 | Galpha-olf-Homo sapiens-1 |
| | **$G_{i/o}$** | | |
| | *Gi* | Q29047 | Galpha-i-Sus scrofa |
| | *Gí1* | P38401 | Galpha-i1-Cavia porcellus |
| | *Gi1* | P50146 | Galpha-i1-Gallus gallus |
| | *Gí1* | P63096 | Galpha-i1-Homo sapiens-1 |
| | *Gí1* | P63097 | Galpha-i1-Bos taurus |
| | *Gí1* | P10824 | Galpha-i1-Rattus norvegicus |
| | *Gí1* | O43383 | Galpha-i1-Homo sapiens-2 |
| | *Gi1* | Q61018 | Galpha-i1-Mus musculus |
| | *Gi2* | P38400 | Galpha-i2-Canis familiaris |
| | *Gi2* | P38402 | Galpha-i2-Cavia porcellus |
| | *Gi2* | P50147 | Galpha-i2-Gallus gallus |
| | *Gi2* | P04899 | Galpha-i2-Homo sapiens-2 |
| | *Gi2* | P08752 | Galpha-i2-Mus musculus-2 |
| | Gi2 | P04897 | Galpha-i2-Rattus norvegicus |
| | *Gi2* | Q7M3G8 | Galpha-i2-Sus scrofa |

(continued)

| Class | Family/*Subtype* | Protein # (UniProt) | Description |
|---|---|---|---|
| **G-alpha** | **$G_s$** | | |
| | *Gi2* | Q7M3G9 | Galpha-i2-Bos taurus-2 |
| | *Gi2* | Q7M3H0 | Galpha-i2-Bos taurus-1 |
| | *Gi2* | Q8JZT4 | Galpha-i2-Mus musculus-1 |
| | *Gi2* | Q96C71 | Galpha-i2-Homo sapiens-1 |
| | *Gi3* | P38403 | Galpha-i3-Cavia porcellus |
| | *Gi3* | Q60397 | Galpha-i3-Cricetulus griseus |
| | *Gi3* | P08754 | Galpha-i3-Homo sapiens |
| | *Gi3* | P08753 | Galpha-i3-Rattus norvegicus |
| | *Gi3* | Q9DC51 | Galpha-i3-Mus musculus |
| | *Go* | P59215 | Galpha-o-Rattus norvegicus |
| | *Go* | Q8N6I9 | Galpha-o-Homo sapiens |
| | *Go1* | P08239 | Galpha-o1-Bos taurus |
| | *Go1* | P59216 | Galpha-o1-Cricetulus longicaudatus |
| | *Go1* | P09471 | Galpha-o1-Homo sapiens |
| | *Go1* | P18872 | Galpha-o1-Mus musculus |
| | *Gz* | P19086 | Galpha-z-Homo sapiens-2 |
| | *Gz* | O70443 | Galpha-z-Mus musculus |
| | *Gz* | P19627 | Galpha-z-Rattus norvegicus |
| | *Gz* | Q8IY73 | Galpha-z-Homo sapiens-3 |
| | *Gz* | Q8N652 | Galpha-z-Homo sapiens-1 |
| | *Gz* | Q95LC0 | Galpha-z-Sus scrofa |
| | *Gt* | Q18162 | Galpha-t-Homo sapiens |
| | *Gt* | Q9D7B3 | Galpha-t-Mus musculus |
| | *Gt1* | P04695 | Galpha-t1-Bos taurus |
| | *Gt1* | Q28300 | Galpha-t1-Canis familiaris |
| | *Gt1* | P11488 | Galpha-t1-Homo sapiens |
| | *Gt1* | P20612 | Galpha-t1-Mus musculus |
| | *Gt2* | P04696 | Galpha-t2-Bos taurus |
| | *Gt2* | P19087 | Galpha-t2-Homo sapiens |
| | *Gt2* | P50149 | Galpha-t2-Mus musculus-2 |
| | *Gt2* | Q8BSY7 | Galpha-t2-Mus musculus-1 |
| | *Ggust* | P29348 | Galpha-gust-Rattus norvegicus |
| | **$G_{q/11}$** | | |
| | *Gq* | Q6NT27 | Galpha-q-Homo sapiens-2 |
| | *Gq* | Q28294 | Galpha-q-Canis familiaris |
| | *Gq* | P50148 | Galpha-q-Homo sapiens-1 |
| | *Gq* | P21279 | Galpha-q-Mus musculus |

(continued)

| Class | Family/*Subtype* | Protein # (UniProt) | Description |
|---|---|---|---|
| **G-alpha** | **$G_s$** | | |
| | *Gq* | P82471 | Galpha-q-Rattus norvegicus |
| | *G11* | Q71RI7 | Galpha-11-Gallus gallus |
| | *G11* | P38409 | Galpha-11-Bos taurus |
| | *G11* | P52206 | Galpha-11-Canis familiaris |
| | *G11* | P29992 | Galpha-11-Homo sapiens |
| | *G11* | P45645 | Galpha-11-Meleagris qallopavo |
| | *G11* | P21278 | Galpha-11-Mus musculus-2 |
| | *G11* | Q9JID2 | Galpha-11-Rattus norvegicus |
| | *G11* | Q8SPP3 | Galpha-11-Macaca mulatta |
| | *G11* | Q91X95 | Galpha-11-Mus musculus-1 |
| | *G14* | P38408 | Galpha-14-Bos taurus |
| | *G14* | 095837 | Galpha-14-Homo sapiens |
| | *G14* | P30677 | Galpha-14-Mus musculus-2 |
| | *G14* | Q6C3M7 | Galpha-14-Mus musculus-3 |
| | *G14* | Q8CBT5 | Galpha-14-Mus musculus-4 |
| | *G14* | Q8R2X9 | Galpha-14-Mus musculus-1 |
| | *G15* | P30678 | Galpha-15-Mus musculus |
| | *G15* | 088302 | Galpha-15-Rattus norvegicus |
| | *G16* | P30679 | Galpha-16-Homo sapiens |
| | **$G_{12/13}$** | | |
| | *G12* | Q03113 | Galpha-12-Homo sapiens |
| | *G12* | P27600 | Galpha-12-Mus musculus |
| | *G12* | Q63210 | Galpha-12-Rattus norvegicus |
| | *G13* | Q14344 | Galpha-13-Homo sapiens |
| | *G13* | P27601 | Galpha-13-Mus musculus-2 |
| | *G13* | Q8C5L2 | Galpha-13-Mus musculus-3 |
| | *G13* | Q9D034 | Galpha-13-Mus musculus-1 |
| **G-beta** | **$B_{1-5}$** | | |
| | *B1* | Q6TMK6 | Gbeta-1-Cricetulus griseus |
| | *B1* | P62871 | Gbeta-1-Bos taurus |
| | *B1* | P62872 | Gbeta-1-Canis familiaris |
| | *B1* | P62873 | Gbeta-1-Homo sapiens |
| | *B1* | P62874 | Gbeta-1-Mus musculus |
| | *B1* | P54311 | Gbeta-1-Rattus norvegicus-2 |
| | *B1* | Q9QX36 | Gbeta-1-Rattus norvegicus-1 |
| | *B2* | P11017 | Gbeta-2-Bos taurus |
| | *B2* | P62879 | Gbeta-2-Homo sapiens |

(continued)

| Class | Family/*Subtype* | Protein # (UniProt) | Description |
|---|---|---|---|
| **G-alpha** | **$G_s$** | | |
| | *B2* | P62880 | Gbeta-2-Mus musculus |
| | *B2* | P54313 | Gbeta-2-Rattus norvegicus-2 |
| | *B2* | Q9QX35 | Gbeta-2-Rattus norvegicus-1 |
| | *B3* | P79147 | Gbeta-3-Canis familiaris |
| | *B3* | P16520 | Gbeta-3-Homo sapiens-1 |
| | *B3* | Q61011 | Gbeta-3-Mus musculus |
| | *B3* | P52287 | Gbeta-3-Rattus norvegicus |
| | *B3* | Q96B71 | Gbeta-3-Homo sapiens-2 |
| | *B4* | Q9HAV0 | Gbeta-4-Homo sapiens |
| | *B4* | P29387 | Gbeta-4-Mus musculus |
| | *B4* | O35353 | Gbeta-4-Rattus norvegicus |
| | *B5* | O14775 | Gbeta-5-Homo sapiens-2 |
| | *B5* | P62881 | Gbeta-5-Mus musculus-2 |
| | *B5* | P62882 | Gbeta-5-Rattus norvegicus |
| | *B5* | Q60525 | Gbeta-5-Mesocricetus auratus |
| | *B5* | Q96F32 | Gbeta-5-Homo sapiens-1 |
| | *B5* | Q9CSQ0 | Gbeta-5-Mus musculus-3 |
| | *B5* | Q9CU21 | Gbeta-5-Mus musculus-1 |
| | **$B_{unclassified}$** | | |
| | *B Unclassified* | Q61621 | unclassified_Gbeta-Mus musculus-1 |
| | *B unclassified* | Q8BMQ1 | unclassified_Gbeta-Mus musculus-2 |
| | *B unclassified* | Q9UFT3 | unclassified_Gbeta-Homo sapiens |
| G-gamma | $\gamma_{1-12}$ | | |
| | *γ1* | Q8R1U6 | Ggamma-1-Mus musculus |
| | *γ2* | P59768 | Ggamma-2-Homo sapiens |
| | *γ2* | P63212 | Ggamma-2-Bos taurus |
| | *γ2* | P63213 | Ggamma-2-Mus musculus |
| | *γ2* | O35355 | Ggamma-2-Rattus norvegicus |
| | *γ3* | P63214 | Ggamma-3-Bos taurus |
| | *γ3* | P63215 | Ggamma-3-Homo sapiens |
| | *γ3* | P63216 | Ggamma-3-Mus musculus |
| | *γ3* | O35356 | Ggamma-3-Rattus norvegicus |
| | *γ4* | P50150 | Ggamma-4-Homo sapiens |
| | *γ4* | P50153 | Ggamma-4-Mus musculus |
| | *γ4* | O35357 | Ggamma-4-Rattus norvegicus |
| | *γ5* | P63217 | Ggamma-5-Bos taurus |
| | *γ5* | P63218 | Ggamma-5-Homo sapiens-2 |

(continued)

| Class | Family/*Subtype* | Protein # (UniProt) | Description |
|---|---|---|---|
| **G-alpha** | **$G_s$** | | |
| | *γ5* | Q80SZ7 | Ggamma-5-Mus musculus |
| | *γ5* | P63219 | Ggamma-5-Rattus norvegicus |
| | *γ5* | Q9Y3K8 | Ggamma-5-Homo sapiens-1 |
| | *γ7* | P30671 | Ggamma-7-Bos taurus |
| | *γ7* | O60262 | Ggamma-7-Homo sapiens |
| | *γ7* | Q61016 | Ggamma-7-Mus musculus |
| | *γ7* | P43425 | Ggamma-7-Rattus norvegicus |
| | *γ8* | Q9UK08 | Ggamma-8-Homo sapiens-2 |
| | *γ8* | P63078 | Gqamma-8-Mus musculus-2 |
| | *γ8* | P63077 | Ggamma-8-Rattus norvegicus |
| | *γ8* | P50154 | Ggamma-8-Bos taurus |
| | *γ8* | O14610 | Ggamma-8-Homo sapiens-1 |
| | *γ8* | Q61017 | Gqamma-8-Mus musculus-1 |
| | *γ10* | P50151 | Ggamma-10-Homo sapiens-2 |
| | *γ10* | O35358 | Ggamma-10-Rattus norvegicus |
| | *γ10* | Q96BN9 | Ggamma-10-Homo sapiens-1 |
| | *γ10* | Q9CXP8 | Gqamma-10-Mus musculus |
| | *γ11* | P61952 | Ggamma-11-Homo sapiens |
| | *γ11* | P61953 | Ggamma-11-Mus musculus |
| | *γ11* | P61954 | Ggamma-11-Rattus norvegicus |
| | *γ12* | Q28024 | Ggamma-12-Bos taurus |
| | *γ12* | Q9UBI6 | Ggamma-12-Homo sapiens |
| | *γ12* | Q9DAS9 | Ggamma-12-Mus musculus |
| | *γ12* | O35359 | Ggamma-12-Rattus norvegicus |
| | *γ13* | Q9P2W3 | Ggamma-13-Homo sapiens |
| | *γ13* | Q9JMF3 | Ggamma-13-Mus musculus |
| | *γt1* | P02698 | Ggamma-t1-Bos taurus |
| | *γt1* | P63211 | Ggamma-t1-Homo sapiens |
| | *γt1* | P63210 | Ggamma-t1-Canis familiaris |
| | *γt1* | Q61012 | Ggamma-t1-Mus musculus |
| | $\gamma_{unclassified}$ | | |
| | *γ unclassified* | Q7M3H1 | unclassified Ggamma-Bos indicus |

Table 3 Human orphan GPCRs including their gene symbols and NCBI gene ID numbers

| Family | Human Gene Symbol | Human Gene ID |
|---|---|---|
| Bombesin | BRS3 | 680 |

(continued)

| Family | Human Gene Symbol | Human Gene ID |
|---|---|---|
| Free fatty acid | GPR42P | 2866 |
| N-Formylpeptide family | FPRL2 | 2359 |
| Nicotinic acid | GPR81 | 27198 |
| Opsin-like | OPN3 | 23596 |
| OrphanA2 | GPR52 | 9293 |
| OrphanA2 | GPR21 | 2844 |
| OrphanA3 | GPR78 | 27201 |
| OrphanA3 | GPR26 | 2849 |
| OrphanA4 | GPR37 | 2861 |
| Orphan4 | GPR37L1 | 9283 |
| OrphanA6 | GPR63 | 81491 |
| OrphanA6 | GPR45 | 11250 |
| OrphanA7 | GPR83 | 10888 |
| OrphanA9 | GRCAe | 27239 |
| OrphanA9 | GPR153 | 387509 |
| OrphanA12 | P2RY5 | 10161 |
| OrphanA13 | P2RY10 | 27334 |
| OrphanA13 | GPR174 | 84636 |
| OrphanA14 | GPR142 | 350383 |
| OrphanA14 | GPR139 | 124274 |
| OrphanA15 | ADMR | 11318 |
| OrphanA15 | CMKOR1 | 57007 |
| OrphanLGR | LGR4 | 55366 |
| OrphanLGR | LGR5 | 8549 |
| OrphanLGR | LGR6 | 59352 |
| OrphanSREB | GPR85 | 54329 |
| OrphanSREB | GPR27 | 2850 |
| OrphanSREB | GPR173 | 54328 |
| Orphan (chemokine receptor-like) | CCRL2 | 9034 |
| Orphan (Mas-related) | MAS1 | 4142 |
| Orphan (Mas-related) | MAS1L | 116511 |
| Orphan (Mas-related) | MRGPRE | 116534 |
| Orphan (Mas-related) | MRGPRF | 116535 |
| Orphan (Mas-related) | MRGPRG | 386746 |
| Orphan (Mas-related) | MRGX3e | 117195 |
| Orphan (Mas-related) | MRGX4e | 117196 |
| Orphan (melatonin-like) | GPR50 | 9248 |
| Orphan (P2Y-like) | GPR87 | 53836 |

(continued)

| Family | Human Gene Symbol | Human Gene ID |
|---|---|---|
| Orphan (trace amine-like) | TRAR3f | 134860 |
| Orphan (trace amine-like) | TRAR4 | 319100 |
| Orphan (trace amine-like) | TRAR5 | 83551 |
| Orphan (trace amine-like) | PNRe | 9038 |
| Orphan (trace amine-like) | GPR57g | 9288 |
| Orphan (trace amine-like) | GPR58 | 9287 |
| Other orphan genes | EBI2 | 1880 |
| Other orphan genes | GPR160 | 26996 |
| Other orphan genes | GPRe | 11245 |
| Other orphan genes | GPR1 | 2825 |
| Other orphan genes | GPR101 | 83550 |
| Other orphan genes | GPR135 | 64582 |
| Other orphan genes | OPN5 | 221391 |
| Other orphan genes | GPR141 | 353345 |
| Other orphan genes | GPR146 | 115330 |
| Other orphan genes | GPR148 | 344561 |
| Other orphan genes | GPR149 | 344758 |
| Other orphan genes | GPR15 | 2838 |
| Other orphan genes | GPR150 | 285601 |
| Other orphan genes | GPR152 | 390212 |
| Other orphan genes | GPR161 | 23432 |
| Other orphan genes | GPR17 | 2840 |
| Other orphan genes | GPR171 | 29909 |
| Other orphan genes | GPR18 | 2841 |
| Other orphan genes | GPR19 | 2842 |
| Other orphan genes | GPR20 | 2843 |
| Other orphan genes | GPR22 | 2845 |
| Other orphan genes | GPR25 | 2848 |
| Other orphan genes | GPR31 | 2853 |
| Other orphan genes | GPR32 | 2854 |
| Other orphan genes | GPR33 | 2856 |
| Other orphan genes | GPR34 | 2857 |
| Other orphan genes | GPR55 | 9290 |
| Other orphan gene | GPR61 | 83873 |
| Other orphan genes | GPR62 | 118442 |
| Other orphan genes | GPR79h | 27200 |
| Other orphan genes | GPR82 | 27197 |
| Other orphan genes | GPR84 | 53831 |

(continued)

| Family | Human Gene Symbol | Human Gene ID |
|---|---|---|
| Other orphan genes | GPR88 | 54112 |
| Other orphan genes | GPR92 | 57121 |
| Other orphan genes | P2RY8 | 286530 |
| Other orphan genes | GPR151 | 134391 |
| LNB7TM | GPR64 | 10149 |
| LNB7TM | GPR56 | 9289 |
| LNB7TM | GPR115 | 221393 |
| LNB7TM | GPR114 | 221188 |
| LNB7TM:Brain specific angiogenesis inhibitor | BAI1 | 575 |
| LNB7TM:Brain specific angiogenesis inhibitor | BAI2 | 576 |
| LNB7TM:Brain specific angiogenesis inhibitor | BAI3 | 577 |
| LNB7TM:Proto-cadherin | CELSR1 | 9620 |
| LNB7TM:Proto-cadherin | CELSR2 | 1952 |
| LNB7TM:Proto-cadherin | CELSR3 | 1951 |
| LNB7TM:EGF, mucin-like receptor | EMR1 | 2015 |
| LNB7TM:EGF, mucin-like receptor | EMR2 | 30817 |
| LNB7TM | GPR97 | 222487 |
| LNB7TM | GPR110 | 266977 |
| LNB7TM | GPR111 | 222611 |
| LNB7TM | GPR112 | 139378 |
| LNB7TM | GPR113 | 165082 |
| LNB7TM | GPR116 | 221395 |
| LNB7TM | MASS1 | 84059 |
| LNB7TM | ELTD1 | 64123 |
| LNB7TM | GPR123 | 84435 |
| LNB7TM | GPR124 | 25960 |
| LNB7TM | GPR125 | 166647 |
| LNB7TM | GPR126 | 57211 |
| LNB7TM | GPR128 | 84873 |
| LNB7TM | GPR144 | 347088 |
| LNB7TM:EGF, mucin-like receptor | EMR3 | 84658 |
| LNB7TM:EGF, mucin-like receptor | EMR4b | 326342 |
| LNB7TM | CD97 | 976 |
| LNB7TM:Latrophilin substrate | LPHN2 | 23266 |
| LNB7TM:Latrophilin substrate | LPHN3 | 23284 |
| LNB7TM:Latrophilin substrate | LPHN1 | 22859 |
| Unclassified | GPR157 | 80045 |
| GABAB | GPR51 | 9568 |

(continued)

| Family | Human Gene Symbol | Human Gene ID |
|---|---|---|
| GABAB | GPR156 | 165829 |
| Calcium sensor | GPRC6A | 222545 |
| GPRC5 | GPRC5A | 9052 |
| GPRC5 | GPRC5B | 51704 |
| GPRC5 | GPRC5C | 55890 |
| GPRC5 | GPRC5D | 55507 |
| Unclassified | GPR158 | 57512 |
| Unclassified | GPR158L1 | 342663 |

Table 4 Human opioid receptors, their gene symbols, NCBI gene ID numbers and related synonyms

| Type | Subunit | Gene Symbol | Splice form | NCBI Gene ID | Synonyms |
|---|---|---|---|---|---|
| Opioid | Mu | OPRM1 | 1 | 4988 | KIAA0403, MOR, MOR1, MOR-1, Mu-type opioid receptor, OPRM |
| | | | 2 | | |
| | Delta | OPRD1 | 1 | 4985 | Delta-type opioid receptor, DOR-1, OPRD |
| | Kappa | OPRK1 | 1 | 4986 | Kappa-type opioid receptor, KOR, KOR-1, OPRK |
| | Sigma | OPRS1 | 1 | 10280 | AAG8, Aging-associated gene 8 protein, FLJ25585, hSigmaR1, MGC3851, SIG-1R, Sigma1R, Sigma1-receptor, Sigma 1-type opioid receptor, SIGMAR1, SR31747-binding protein, SRBP, SR-BP, SR-BP1 |
| | | | 2 | | |
| | | | 3 | | |
| | | | 4 | | |
| | | | 5 | | |
| Opioid Like Receptor | | OPRL1 | 1 | 4987 | Kappa-type 3 opioid receptor, KOR-3, MGC34578, Nociceptin receptor, NOCIR, OOR, ORL1, Orphanin FQ receptor |
| | | | 2 | | |
| opioid binding protein/cell adhesion molecule-like | | OPCML | 1 | 4978 | OBCAM, OPCM, Opioid-binding cell adhesion molecule, Opioid-binding protein/cell adhesion molecule precursor |
| opioid growth factor receptor | | OGFR | 1 | 11054 | 7-60, 7-60 protein, OGFr, Opioid growth factor receptor, Zeta-type opioid receptor |
| | | | 2 | | |
| opioid growth factor receptor-like 1 | | OGFRL1 | 1 | 79627 | dJ331H24.1, FLJ21079, MGC102783 |

Table 5 Human olfactory receptors, their gene symbols, and common names

| Name | Common Name |
|---|---|
| ORL1003 | OR2W1 |
| ORL1004 | OR10H1 |
| ORL1009 | CR1K1 |
| ORL1011 | sdolf |
| ORL1015 | OR3A3 |
| ORL1016 | OR1E1 |
| ORL1017 | LOC113744 |
| ORL1018 | OR1D2 |
| ORL1019 | OR2B2 |
| ORL1020 | sdolf |
| ORL1021 | LOC113117 |
| ORL1022 | OR1 F2 |
| ORL1023 | OR1 F1 |
| ORL1025 | LOC116408 |
| ORL1026 | LOC91013 |
| ORL1027 | LOC93312 |
| ORL1028 | OR7A17 |
| ORL1029 | OR7C2 |
| ORL1030 | OR12D3 |
| ORL1031 | OR5V1 |
| ORL1032 | OR2J2 |
| ORL1033 | OR2W1 |
| ORL1037 | OR2W1 |
| ORL1038 | LOC89905 |
| ORL1040 | OR2K2 |
| ORL1041 | OR3A2 |
| ORL1043 | OR11A1 |
| ORL1046 | OR2S2 |
| ORL1048 | JCG10 |
| ORL1049 | JCG4 |
| ORL1050 | PJCG1 |
| ORL1051 | JCG5 |
| ORL1052 | JCG5 |
| ORL1053 | JCG3 |
| ORL1054 | JCG1 |
| ORL1055 | JCG2 |
| ORL1063 | LOC120835 |

(continued)

| Name | Common Name |
|---|---|
| ORL1064 | LOC119206 |
| ORL1066 | LOC119205 |
| ORL1069 | LOC125962 |
| ORL1075 | LOC122751 |
| ORL1081 | LOC122745 |
| ORL1082 | OR10H4 |
| ORL1083 | OR1M1 |
| ORL1084 | LOC122744 |
| ORL1085 | OR1M1 |
| ORL1086 | OR7G1 |
| ORL1087 | LOC125961 |
| ORL1088 | LOC125960 |
| ORL1089 | OR7D4 |
| ORL1090 | LOC125901 |
| ORL1091 | LOC125801 |
| ORL1092 | LOC123492 |
| ORL1093 | LOC123491 |
| ORL1094 | LOC122743 |
| ORL1095 | LOC122741 |
| ORL1096 | OR4K14 |
| ORL1097 | LOC122737 |
| ORL1098 | LOC122736 |
| ORL154 | FAT11 |
| ORL165 | OLF1 |
| ORL166 | OLF3 |
| ORL167 | OLA-7501 |
| ORL19 | HGMP07E |
| ORL20 | HGMP07I |
| ORL203 | TPCR100 |
| ORL204 | TPCR106 |
| ORL205 | TPCR110 |
| ORL206 | TPCR120 |
| ORL207 | TPCR16 |
| ORL208 | TPCR24 |
| ORL209 | TPCR25 |
| ORL21 | HGMP07J |
| ORL210 | TPCR26 |
| ORL211 | TPCR27 |

(continued)

| Name | Common Name |
|---|---|
| ORL212 | TPCR85 |
| ORL213 | TPCR86 |
| ORL214 | TPCR92 |
| ORL229 | ht2 |
| ORL230 | htpcr2 |
| ORL231 | EST112838 |
| ORL249 | nq20a09.s1 |
| ORL253 | OR1-25 |
| ORL254 | OR1-26 |
| ORL255 | OR13-66 |
| ORL256 | OR16-35 |
| ORL257 | OR16-36 |
| ORL258 | OR16-37 |
| ORL259 | OR16-88 |
| ORL260 | OR16-89 |
| ORL261 | OR16-90 |
| ORL262 | OR17-130 |
| ORL263 | OR17-135 |
| ORL264 | OR17-136 |
| ORL265 | OR17-137 |
| ORL266 | OR17-15 |
| ORL267 | yq70e01.s1 |
| ORL268 | OR17-16 |
| ORL269 | OR19-18 |
| ORL270 | OR3-145 |
| ORL271 | OR5-40 |
| ORL272 | OR7-138 |
| ORL273 | OR7-139 |
| ORL274 | OR7-140 |
| ORL281 | OLFR42A |
| ORL282 | OLFR42B |
| ORL283 | OLFMF |
| ORL3001 | OR10K1/OR01.09.04/HGPCR1104 |
| ORL3002 | OR6Y1/OR01.12.02/HGPCR0041 |
| ORL3003 | OR2T4/OR01.04.03/HGPCR0269 |
| ORL3004 | OR10Z1/OR01.09.01/HGPCR1073 |
| ORL3005 | OR6N2/OR01.10.02/HGPCR1102 |
| ORL3006 | OR5BF1/OR01.01.01/HGPCR1048 |

(continued)

| Name | Common Name |
|---|---|
| ORL3007 | OR5AV1/OR01.01.02/HGPCR0911 |
| ORL3008 | OR5AT1/OR01.01.05/HGPCR0150 |
| ORL3009 | OR11L1/OR01.13.01/HGPCR0152 |
| ORL3010 | OR6K6/OR01.10.05/HGPCR1099 |
| ORL3011 | OR10T2/OR01.09.07/HGPCR0914 |
| ORL3012 | OR10R2/OR01.09.06/HGPCR0804 |
| ORL3013 | OR2T5/OR01.04.04/HGPCR0537 |
| ORL3014 | OR6P1/OR01.12.01/HGPCR0043 |
| ORL3015 | OR2L8/OR01.02.01/HGPCR0855 |
| ORL3016 | OR13G1/OR01.07.01/HGPCR0054 |
| ORL3017 | OR2L8/OR01.02.01/HGPCR0221 |
| ORL3018 | OR10J5/OR01.09.02/HGPCR0461 |
| ORL3019 | OR6N1/OR01.10.01/HGPCR0101 |
| ORL3020 | OR6F1/OR01.11.01/HGPCR0602 |
| ORL3023 | OR1OK2/OR01.09.05 |
| ORL3024 | OR6K2/OR01.10.03 |
| ORL3025 | OR5AX1/OR01.01.04 |
| ORL3026 | OR2C4/OR01.05.01 |
| ORL3027 | OR01.01.03/HGPCR0770 |
| ORL3028 | OR01.04.05/HGPCR1143 |
| ORL3029 | OR01.08.01/HGPCR1038 |
| ORL3030 | OR01.10.06/HGPCR0574 |
| ORL3031 | OR01.06.01/HGPCR0389 |
| ORL3032 | OR01.04.08/HGPCR0569 |
| ORL3033 | OR10J6/HGPCR0207 |
| ORL3034 | OR6K3/HGPCR0667 |
| ORL3037 | OR2L4P/HGPCR0871 |
| ORL3038 | OR2T6P/HGPCR0342 |
| ORL3039 | OR2L3 |
| ORL3040 | OR2T3 |
| ORL3041 | OR5AY1 |
| ORL3042 | OR2G2 |
| ORL3043 | OR2G3 |
| ORL3044 | OR01.04.02 |
| ORL3045 | OR01.03.02 |
| ORL3046 | OR01.04.01 |
| ORL3047 | OR01.04.09 |
| ORL3048 | OR01.04.06 |

(continued)

| Name | Common Name |
|---|---|
| ORL3049 | OR01.04.07 |
| ORL305 | dJ193B12.4 |
| ORL3050 | OR01.03.05 |
| ORL3051 | OR01.03.04 |
| ORL3052 | OR01.03.03 |
| ORL3053 | OR01.03.01 |
| ORL3054 | OR01.06.02 |
| ORL3055 | OR2T2P |
| ORL3056 | OR10T1P |
| ORL3057 | OR10R1P |
| ORL3058 | OR10R3P |
| ORL3059 | OR2W3P |
| ORL306 | AC002085 |
| ORL3060 | OR2AS1P |
| ORL3061 | OR2AK1P |
| ORL3062 | OR10X1P |
| ORL3063 | OR6K1P |
| ORL3064 | OR6K4P |
| ORL3065 | OR6K5P |
| ORL3066 | OR2AQ1P |
| ORL3067 | OR2L5P |
| ORL3068 | OR10AA1P |
| ORL3069 | OR10J2P |
| ORL307 | BC62940_2 |
| ORL3070 | OR10J3P |
| ORL3071 | OR2L7P |
| ORL3072 | OR2L9P |
| ORL3073 | OR2AJ1P |
| ORL3074 | OR2T8P |
| ORL3075 | OR6R1P |
| ORL3076 | OR2L6P |
| ORL3077 | OR2T7P |
| ORL3078 | OR7E26P |
| ORL3079 | OR11I1P |
| ORL308 | oh91h07.s1 |
| ORL3080 | OR10AE1P |
| ORL3081 | OR9H1P |
| ORL3082 | OR7E102/HGPCR0317 |

(continued)

| Name | Common Name |
|---|---|
| ORL3083 | OR7E89P |
| ORL3084 | OR7E90P |
| ORL3085 | OR7E91P |
| ORL3086 | OR7E62P |
| ORL3087 | OR7E46P |
| ORL3088 | OR7E107P |
| ORL3089 | OR6B2P |
| ORL309 | hsolf4 |
| ORL3090 | OR5S1P |
| ORL3091 | OR6B3P |
| ORL3092 | OR4G6P |
| ORL3093 | OR5H2/OR03.01.03 |
| ORL3094 | OR5H6/OR03.01.04 |
| ORL3095 | OR03.01.02 |
| ORL3096 | OR7E55P |
| ORL3097 | OR7E66P |
| ORL3098 | OR5H4P |
| ORL3099 | OR5H5P |
| ORL310 | AC003956 |
| ORL3100 | OR5H7P |
| ORL3101 | OR5H8P |
| ORL3102 | OR7E29P |
| ORL3103 | OR7E93P |
| ORL3104 | OR7E53P |
| ORL3105 | OR7E97P |
| ORL3106 | OR5BM1P |
| ORL3107 | OR5H3P |
| ORL3108 | OR5AC1P |
| ORL3109 | OR7E121P |
| ORL311 | R30385_1 |
| ORL3110 | OR7E122P |
| ORL3111 | OR7E127P |
| ORL3112 | OR7E129P |
| ORL3113 | OR5G1P |
| ORL3114 | OR7E131P |
| ORL3115 | OR7E132P |
| ORL3116 | OR7E100P |
| ORL3117 | OR5B5P |

(continued)

| Name | Common Name |
|---|---|
| ORL3118 | OR7E83P |
| ORL3119 | OR7E84P |
| ORL312 | F20722_1 |
| ORL3120 | OR7E85P |
| ORL3121 | OR7E86P |
| ORL3122 | OR7E43P |
| ORL3123 | OR7E94P |
| ORL3124 | OR7E99P |
| ORL3125 | OR7E103P |
| ORL3126 | OR4H11P |
| ORL3127 | OR8N1P |
| ORL3128 | OR7E35P |
| ORL3129 | OR5M14P |
| ORL313 | F20722_2 |
| ORL3130 | OR7E130P |
| ORL3131 | OR2Y1/OR05.02.01/HGPCR0495 |
| ORL3132 | OR2V3/OR05.01.01/HGPCR0932 |
| ORL3133 | OR2AI1P |
| ORL3134 | OR1X1P |
| ORL3135 | OR2V1P |
| ORL3136 | OR4H5P |
| ORL3137 | OR5U1/OR06.01.01/HGPCR0647 |
| ORL3138 | OR4F12/OR06.12.05/HGPCR0990 |
| ORL3139 | OR1F12/OR06.07.01/HGPCR0348 |
| ORL314 | F20569_1 |
| ORL3140 | OR4F14/OR06.12.03/HGPCR0266 |
| ORL3141 | OR4F16/OR06.12.02/HGPCR0404 |
| ORL3142 | OR2H2/OR06.03.02 |
| ORL3143 | OR4F15/OR06.12.04/HGPCR0055 |
| ORL3144 | OR4F10/OR06.12.02 |
| ORL3145 | OR2B8/HGPCR0702 |
| ORL3146 | OR2W6P/HGPCR0734 |
| ORL3147 | OR2I2 |
| ORL3148 | OR06.06.01 |
| ORL3149 | OR4F2P |
| ORL315 | ol62g08.s1 |
| ORL3150 | OR2P1P |
| ORL3151 | OR4F1P |

(continued)

| Name | Common Name |
|---|---|
| ORL3152 | OR7E22P |
| ORL3153 | OR2U2P |
| ORL3154 | OR2U1P |
| ORL3155 | OR2H5P |
| ORL3156 | OR2G1P |
| ORL3157 | OR2AD1P |
| ORL3158 | OR12D1P |
| ORL3159 | OR2W4P |
| ORL316 | on81f02.s1 |
| ORL3160 | OR2W2P |
| ORL3161 | OR2B7P |
| ORL3162 | OR4F13P |
| ORL3163 | OR2W7P |
| ORL3164 | OR5B7P |
| ORL3165 | OR2J1P |
| ORL3166 | OR2N1P |
| ORL3167 | OR2J4P |
| ORL3168 | OR2H4P |
| ORL3169 | OR2E1P |
| ORL317 | om42b11.s1 |
| ORL3170 | OR2B4P |
| ORL3171 | OR2AE1/OR07.02.01/HGPCR1138 |
| ORL3172 | OR6V1/OR07.04.01/HGPCR0240 |
| ORL3173 | OR9A2/OR07.04.02/HGPCR0322 |
| ORL3174 | OR9A4/OR07.04.03/HGPCR0175 |
| ORL3175 | OR2A6/OR07.01.05 |
| ORL3176 | OR2A16P/OR07.01.06 |
| ORL3177 | OR2A12P/OR07.01.04 |
| ORL3178 | OR07.01.03/HGPCR0491 |
| ORL3179 | OR2F2/OR07.03.02/HGPCR1049 |
| ORL3180 | OR4F5 |
| ORL3181 | OR2A7 |
| ORL3182 | OR4F4 |
| ORL3183 | OR2Q1P |
| ORL3184 | OR7E38P |
| ORL3185 | OR7E7P |
| ORL3186 | OR2R1P |
| ORL3187 | OR10AC1P |

(continued)

| Name | Common Name |
|---|---|
| ORL3188 | OR4G4P |
| ORL3189 | OR4F7P |
| ORL3190 | OR9P1P |
| ORL3191 | OR9A1P |
| ORL3192 | OR2A11P |
| ORL3193 | OR2A2P |
| ORL3194 | OR2A13P |
| ORL3195 | OR2A14P |
| ORL3196 | OR2A15P |
| ORL3197 | OR9A3P |
| ORL3198 | OR9N1P |
| ORL3199 | OR7E118P |
| ORAL32 | HTPCRX11 |
| ORL3200 | OR7E9P |
| ORL3201 | OR2A17P |
| ORL3202 | OR2A3P |
| ORL3203 | OR2A9P |
| ORL3204 | OR2V2 |
| ORL3205 | OR9L1P |
| ORL3206 | OR4D4P |
| ORL3207 | OR4K8P |
| ORL3208 | OR7E96P |
| ORL3209 | OR5B1P |
| ORL3210 | OR5D11P |
| ORL3211 | OR7E50P |
| ORL3212 | OR7E8F |
| ORL3213 | OR7E80P |
| ORL3214 | OR7E10P |
| ORL3215 | OR7E125P |
| ORL3216 | OR1L8/OR09.04.04/HGPCR0009 |
| ORL3217 | OR1K1/OR09.03.01/HGPCR0521 |
| ORL3218 | OR1L3/OR09.04.06/HGPCR0733 |
| ORL3219 | OR1L6/OR09.04.02/HGPCR0473 |
| ORL3220 | OR2AR1P/OR09.01.02 |
| ORL3221 | OR2K2/OR09.01.02/HGPCR0567 |
| ORL3222 | OR13C3/OR09.01.09/HGPCR0194 |
| ORL3223 | OR13C4/OR09.01.08/HGPCR0197 |
| ORL3224 | OR13C5/OR09.01.05/HGPCR1120 |

(continued)

| Name | Common Name |
|---|---|
| ORL3225 | OR13C8/OR09.01.10/HGPCR1124 |
| ORL3226 | OR13C9/OR09.01.07/HGPCR0557 |
| ORL3227 | OR5C2P/OR09.02.01/HGPCR0477 |
| ORL3228 | OR13C2/OR09.01.06 |
| ORL3229 | OR13F1/OR09.01.03 |
| ORL3231 | OR13J1/OR09.01.01 |
| ORL3232 | OR1J1/OR09.05.01 |
| ORL3233 | OR13C7/OR09.01.11 |
| ORL3234 | OR1B1/OR09.03.02 |
| ORL3235 | OR09.04.03/HGPCR0457 |
| ORL3236 | OR09.04.01/HGPOR0453 |
| ORL3237 | OR19.04.11/HGPCR0888 |
| ORL3238 | OR09.06.02/HGPCR0994 |
| ORL3239 | OR09.04.05/HGPCR0454 |
| ORL3240 | H38g587/HGPCR0254 |
| ORL3241 | OR1L1/HGPCR0036 |
| ORL3242 | OR13D1 |
| ORL3243 | OR1Q1 |
| CRL3244 | OR1L4 |
| ORL3245 | OR5C1 |
| ORL3246 | OR1 N2 |
| ORL3247 | OR09.01.04 |
| ORL3248 | CR13C1P |
| ORL3249 | OR13I1P |
| ORL3250 | OR7E108P |
| ORL3251 | OR7E109P |
| ORL3252 | OR1H1P |
| ORL3253 | OR7E112P |
| ORL3254 | OR7E113P |
| ORL3255 | OR7E114P |
| ORL3256 | OR13E1P |
| ORL3257 | OR7E31P |
| ORL3258 | OR7E116P |
| ORL3259 | OR2AN1P |
| ORL3260 | OR13D2P |
| ORL3261 | OR13C6P |
| ORL3262 | OR2S1P |
| ORL3263 | OR2AM1P |

(continued)

| Name | Common Name |
|---|---|
| ORL3264 | OR13D3P |
| ORL3265 | OR13A1/OR10.01.01/HGPCR0425 |
| ORL3266 | OR6D1P |
| ORL3267 | OR7E110P |
| ORL3268 | OR7E68P |
| ORL3269 | OR7E115P |
| ORL3270 | OR6L1P |
| ORL3271 | OR6L2P |
| ORL3272 | OR7M1P |
| ORL3273 | OR6D2P |
| ORL3274 | OR10G6P/OR11.48.06/HGPCR0037 |
| ORL3275 | OR10G6P/OR11.48.06/HGPCR1012 |
| ORL3276 | OR10G6P/OR11.48.06/HGPCR0129 |
| ORL3277 | OR9G4/OR11.24.01/HGPCR0829 |
| ORL3278 | OR9Q1/OR11.25.02/HGPCR0131 |
| ORL3279 | OR9G5/OR11.24.03/HGPCR0880 |
| ORL3280 | OR9G5/OR11.24.03/HGPCR1118 |
| ORL3281 | OR2AG1/OR11.21.01/HGPCR0485 |
| ORL3282 | OR52E1/OR11.15.06/HGPCR0671 |
| ORL3283 | OR56A1/OR11.01.05/HGPCR0795 |
| ORL3284 | OR5P3/OR11.29.01/HGPCR0765 |
| ORL3285 | OR52L1/OR11.20.02/HGPCR0068 |
| ORL3286 | OR52L2/OR11.20.03/HGPCR0494 |
| ORL3287 | OR52J3/OR11.15.01/HGPCR0299 |
| ORL3288 | OR10G4/OR11.48.04/HGPCR0039 |
| ORL3289 | OR8D4/OR11.38.01/HGPCR0688 |
| ORL3290 | OR10G7/OR11.48.02/HGPCR0908 |
| ORL3291 | OR51M1/OR11.08.01/HGPCR0071 |
| ORL3292 | OR4D5/OR11.50.01/HGPCR0445 |
| ORL3293 | OR52E4/OR11.15.04/HGPCR0154 |
| ORL3294 | OR52E5/OR11.15.05/HGPCR0390 |
| ORL3295 | OR5M10/OR11.40.02/HGPCR0424 |
| ORL3296 | OR5T2/OR11.35.01/HGPCR0149 |
| ORL3297 | OR52N4/OR11.17.02/HGPCR0530 |
| ORL3298 | OR56A6/OR11.01.04/HGPCR0472 |
| ORL3299 | OR51E1/OR11.06.01/HGPCR0376 |
| ORL33 | OR17-30 |
| ORL3300 | OR51A7/OR11.11.05/HGPCR0353 |

(continued)

| Name | Common Name |
|---|---|
| ORL3301 | OR5A1/OR11.26.02/HGPCR0335 |
| ORL3302 | OR5A2/OR11.26.03/HGPCR0784 |
| ORL3303 | OR5A2/OR11.26.03/HGPCR1128 |
| ORL3304 | OR9G4/OR11.24.01/HGPCR0259 |
| ORL3305 | OR51I2/OR11.09.01/HGPCR0925 |
| ORL3306 | OR4A4/OR11.49.09/HGPCR0670 |
| ORL3307 | OR5AS1/OR11.36.02/HGPCR0737 |
| ORL3308 | OR4A5/OR11.49.10/HGPCR0593 |
| ORL3309 | OR1S2/OR11.41.02/HGPCR0597 |
| ORL3310 | OR5B13/OR11.33.03/HGPCR0251 |
| ORL3311 | OR4P4/OR11.49.02/HGPCR0910 |
| ORL3312 | OR10V1/OR11.43.01/HGPCR0811 |
| ORL3313 | OR4C15/OR11.49.11/HGPCR0284 |
| ORL3314 | OR5M3/OR11.40.07/HGPCR0514 |
| ORL3315 | OR1S1/OR11.41.01/HGPCR1026 |
| ORL3316 | OR5M3/OR11.40.07/HGPCR1006 |
| ORL3317 | OR8D1/OR11.38.02/HGPCR0236 |
| ORL3318 | OR52M1P/OR11.19.02/HGPCR0352 |
| ORL3319 | OR10D4/OR11.48.08 |
| ORL3320 | OR56A4/OR11.01.06 |
| ORL3321 | OR8K1/OR11.39.05 |
| ORL3322 | OR5M8/OR11.40.05 |
| ORL3323 | OR4X1/OR11.49.07 |
| ORL3324 | OR52N2/OR11.17.03 |
| ORL3325 | OR51S1/OR11.03.01 |
| ORL3326 | OR52B4/OR11.13.04 |
| ORL3327 | OR5AK3/OR11.30.01 |
| ORL3328 | OR5F1/OR11.31.01 |
| ORL3329 | OR8J3/OR11.39.02 |
| ORL3330 | OR8K5/OR11.39.07 |
| ORL3331 | OR52A1/OR11.16.01 |
| ORL3332 | OR8A1/OR11.38.04 |
| ORL3333 | OR8B12/OR11.38.09 |
| ORL3334 | OR52E8/OR11.15.02 |
| ORL3335 | OR4C12/OR11.49.12 |
| ORL3336 | OR4C13/OR11.49.13 |
| ORL3337 | OR5G3/OR11.27.01 |
| ORL3338 | OR5T3/OR11.35.02 |

(continued)

| Name | Common Name |
|---|---|
| ORL3339 | OR1A2/OR17.02.02 |
| ORL334 | BC85395_1 |
| ORL3340 | OR5AU1/OR14.01.01 |
| ORL3342 | OR52H1/OR11.13.02 |
| ORL3343 | OR4F17/OR19.06.01 |
| ORL3344 | OR5R1P/OR11.39.04 |
| ORL3345 | OR11.18.02/HGPCR0026 |
| ORL3346 | OR11.18.02/HGPCR0823 |
| ORL3347 | OR11.19.02/HGPCR0586 |
| ORL3348 | OR11.14.01/HGPCR0333 |
| ORL3349 | OR11.14.01/HGPCR0496 |
| ORL335 | BC85395_2 |
| ORL3350 | OR11.11.04/HGPCR1031 |
| ORL3351 | OR11.11.06/HGPCR0748 |
| ORL3352 | OR11.39.01/HGPCR0854 |
| ORL3353 | OR11.23.01/HGPCR0440 |
| ORL3354 | OR11.01.02/HGPCR0359 |
| ORL3355 | OR11.09.02/HGPCR0924 |
| ORL3356 | OR11.24.03/HGPCR0930 |
| ORL3357 | OR11.24.02/HGPCR0660 |
| ORL3358 | OR11.42.03/HGPCR0186 |
| ORL3359 | OR11.42.03/HGPCR0217 |
| ORL336 | BC85395_3 |
| ORL3360 | OR11.32.03/HGPCR0098 |
| ORL3361 | OR11.30.02/HGPCR1093 |
| ORL3362 | OR11.40.08/HGPCR0420 |
| ORL3363 | OR11.50.04/HGPCR0601 |
| ORL3364 | OR11.49.01/HGPCR0224 |
| ORL3365 | OR11.43.03/HGPCR0612 |
| ORL3366 | OR11.28.01/HGPCR1039 |
| ORL3367 | OR9I1/OR11.25.01/HGPCR1015 |
| ORL3368 | OR6B1/OR11.47.01/HGPCR1052 |
| ORL3369 | OR6M1/OR11.45.01/HGPCR0584 |
| ORL337 | BC85395_4 |
| ORL3370 | OR51L1/OR11.11.02/HGPCR0603 |
| ORL3371 | OR51A2/OR11.11.07/HGPCR1139 |
| ORL3372 | OR52E2/OR11.15.07/HGPCR0212 |
| ORL3373 | OR5P2/OR11.29.02/HGPCR0943 |

(continued)

| Name | Common Name |
|---|---|
| ORL3374 | OR10S1/OR11.48.05/HGPCR0936 |
| ORL3375 | OR10S1/OR11.48.05/HGPCR0431 |
| ORL3376 | OR51H1/OR11.07.01/HGPCR0615 |
| ORL3377 | OR10G8/OR11.48.01/HGPCR0512 |
| ORL3378 | OR6T1/OR11.45.02/HGPCR0443 |
| ORL3379 | OR4B1/OR11.49.05/HGPCR0433 |
| ORL3380 | OR51Q1/OR11.11.01/HGPCR0755 |
| ORL3381 | OR52N1/OR11.17.04/HGPCR1061 |
| ORL3382 | OR10G9/OR11.48.03/HGPCR0527 |
| ORL3383 | OR4X2/OR11.49.06/HGPCR1087 |
| ORL3384 | OR5M9/OR11.40.06/HGPCR1096 |
| ORL3385 | OR8K3/OR11.39.06/HGPCR0872 |
| ORL3386 | OR52E6/OR11.15.03/HGPCR0682 |
| ORL3387 | OR2AG1/OR11.21.01/HGPCR0485 |
| ORL3388 | OR56B2/OR11.01.03/HGPCR0926 |
| ORL3389 | OR1M1/OR19.02.01/HGPCR0449 |
| ORL339 | op88e11.s1 |
| ORL3390 | OR51G2/OR11.11.03/HGPCR0356 |
| ORL3391 | OR51F2/OR11.10.01/HGPCR0619 |
| ORL3392 | OR5D16/OR11.32.06/HGPCR0679 |
| ORL3393 | OR10Q1/OR11.43.02/HGPCR0749 |
| ORL3394 | OR5D18/OR11.32.05/HGPCR0271 |
| ORL3395 | OR5D18/OR11.32.05/HGPCR0948 |
| ORL3396 | OR5L1/OR11.32.01/HGPCR0243 |
| ORL3397 | OR51E2/OR11.06.02/HGPCR0820 |
| ORL3398 | OR51D1/OR11.06.03/HGPCR0814 |
| ORL3399 | OR5AR1/OR11.37.01/HGPCR0758 |
| ORL34 | HTPCRH03 |
| ORL3400 | OR5M1/OR11.40.03/HGPCR0286 |
| ORL3401 | OR5AP2/OR11.34.01/HGPCR0288 |
| ORL3402 | OR5AP2/OR11.34.01/HGPCR0288 |
| ORL3403 | OR52B2/OR11.13.01/HGPCR0654 |
| ORL3404 | OR52K2/OR11.18.01/HGPCR0969 |
| ORL3405 | OR52K2/OR11.18.01/HGPCR0231 |
| ORL3406 | OR52B4/OR11.13.04/HGPCR0189 |
| ORL3407 | OR51I1/OR11.09.02/HGPCR0924 |
| ORL3408 | OR8H2/OR11.31.04/HGPCR0337 |
| ORL3409 | OR8I2/OR11.31.02/HGPCR0339 |

(continued)

| Name | Common Name |
|---|---|
| ORL341 | nc48c07.s1 |
| ORL3410 | OR8H3/OR11.31.05/HGPCR0336 |
| ORL3411 | OR4A15/OR11.49.08/HGPCR0941 |
| ORL3412 | OR4D9/OR11.50.03/HGPCR0746 |
| ORL3413 | OR5B16/OR11.33.01/HGPCR0056 |
| ORL3414 | OR10A6/OR11.42.02/HGPCR0645 |
| ORL3415 | OR5B17/OR11.33.02/HGPCR0070 |
| ORL3416 | OR8H1/OR11.31.03/HGPCR0893 |
| ORL3417 | OR52P1/OR11.20.01/HGPCR0565 |
| ORL3418 | OR51T1/OR11.05.01/HGPCR0812 |
| ORL3419 | OR52R1/OR11.19.01/HGPCR0624 |
| ORL342 | AI017815 |
| ORL3420 | OR56B4/OR11.01.01/HGPCR1134 |
| ORL3421 | OR4D6/OR11.50.02/HGPCR0460 |
| ORL3422 | OR8B8/OR11.38.10/HGPCR0539 |
| ORL3423 | OR8B4/OR11.38.06/HGPCR0179 |
| ORL3424 | OR52B6/OR11.13.03/HGPCR0782 |
| ORL3425 | OR4C6/OR11.49.14/HGPCR0046 |
| ORL3426 | OR5D14/OR11.32.04/HGPCR0685 |
| ORL3427 | OR6Q1/OR11.46.01/HGPCR1025 |
| ORL3428 | OR52I1/OR11.02.01/HGPCR0673 |
| ORL3429 | OR52I2/OR11.02.02/HGPCR0469 |
| ORL343 | AI023490 |
| ORL3430 | OR2D3/OR11.22.02/HGPCR0950 |
| ORL3431 | OR52W1P/OR11.12.01/HGPCR0130 |
| ORL3432 | OR2D2/OR11.22.01/HGPCR0954 |
| ORL3433 | OR5M11/OR11.40.01/HGPCR0253 |
| ORL3434 | OR8G3P/OR11.40.04/HGPCR0380 |
| ORL3435 | OR4C16/OR11.49.04/HGPCR0692 |
| ORL3436 | OR52N5/OR11.17.01/HGPCR0053 |
| ORL3438 | OR6X1/OR11.44.01 |
| ORL3440 | OR51C1P/HGPCR0065 |
| OL3441 | OR51J1P/HGPCR0768 |
| ORL3442 | OR51R1P/HGPCR0731 |
| ORL3443 | OR9I2P/HGPCR0326 |
| ORL3444 | OR51A4 |
| ORL3445 | OR51G1 |
| ORL3446 | OR5D13 |

(continued)

| Name | Common Name |
|---|---|
| ORL3447 | OR8J1 |
| ORL3449 | OR9G1 |
| ORL3450 | OR52K1 |
| ORL3451 | OR5B2 |
| ORL3452 | OR52D1 |
| ORL3453 | OR5AN1 |
| ORL3454 | OR5AK2 |
| ORL3455 | OR8B3 |
| ORL3456 | OR8B2 |
| ORL3457 | OR11.38.08 |
| ORL3458 | OR11.38.07 |
| ORL3459 | OR11.28.02 |
| ORL3460 | OR11.25.02 |
| ORL3461 | OR11.39.03 |
| ORL3462 | OR11.50.05 |
| ORL3463 | OR11.49.03 |
| ORL3464 | OR11.26.01 |
| ORL3465 | OR11.33.04 |
| ORL3466 | OR11.37.02 |
| ORL3467 | OR11.48.07 |
| ORL3468 | OR11.35.03 |
| ORL3469 | OR11.38.05 |
| ORL347 | hsORL-124 |
| ORL3470 | OR11.42.06 |
| ORL3471 | OR10D3P |
| ORL3472 | OR10N1P |
| ORL3473 | OR8F1P |
| ORL3474 | OR10D1P |
| ORL3476 | OR7E5P |
| ORL3477 | OR51B3P |
| ORL3478 | OR7E87P |
| ORL3479 | OR7E4P |
| ORL3480 | OR2AL1P |
| ORL3481 | OR6M2P |
| ORL3482 | OR5D2P |
| ORL3483 | OR4V1P |
| ORL3484 | OR8B10P |
| ORL3485 | OR4P1P |

(continued)

| Name | Common Name |
|---|---|
| ORL3486 | OR51N1P |
| ORL3487 | OR52J1P |
| ORL3488 | OR51P1P |
| ORL3489 | OR4C7P |
| ORL349 | hsORL-125 |
| ORL3490 | OR5P1P |
| ORL3491 | OR56A2P |
| ORL3492 | OR5E1P |
| ORL3493 | OR56A3P |
| ORL3494 | OR52X1P |
| ORL3495 | OR56A5P |
| ORL3496 | OR52E3P |
| ORL3497 | OR51A3P |
| ORL3498 | OR4C9P |
| ORL3499 | OR52J2P |
| ORL35 | HTPCRH06 |
| ORL350 | hsORL-126 |
| ORL3500 | OR4R1P |
| ORL3501 | OR4C10P |
| ORL3502 | OR51A5P |
| ORL3503 | OR5M2P |
| ORL3504 | OR10AB1P |
| ORL3505 | OR52S1P |
| ORL3506 | OR5M4P |
| ORL3507 | OR5M5P |
| ORL3508 | OR10G5P |
| ORL3509 | OR5M6P |
| ORL351 | hsORL-127 |
| ORL3510 | OR5M7P |
| ORL3511 | OR5T1P |
| ORL3512 | OR8I1P |
| ORL3513 | OR8K2P |
| ORL3514 | OR10D5P |
| ORL3515 | OR5BD1P |
| ORL3516 | OR5AL1P |
| ORL3517 | OR5AL2P |
| ORL3518 | OR10A2P |
| ORL3519 | OR8L1P |

(continued)

| Name | Common Name |
|---|---|
| ORL352 | hsORL-128 |
| ORL3520 | OR5BP1P |
| ORL3521 | OR8J2P |
| ORL3522 | OR52N3P |
| ORL3523 | OR4B2P |
| ORL3524 | OR51K1P |
| ORL3525 | OR52Q1P |
| ORL3526 | OR52E7P |
| ORL3527 | OR6A2P |
| ORL3528 | OR52U1P |
| ORL3529 | OR6M3P |
| ORL353 | hsORL-129 |
| ORL3530 | OR5D3P |
| ORL3531 | OR8B9P |
| ORL3532 | OR56B1P |
| ORL3533 | OR2AG2P |
| ORL3534 | OR52Y1P |
| ORL3535 | OR51A6P |
| ORL3535 | OR51F1P |
| ORL3537 | OR7E1P |
| ORL3538 | OR51H2P |
| ORL3539 | OR5BG1P |
| ORL354 | hsORL-130 |
| ORL3540 | OR5W1P |
| ORL3541 | OR5W2P |
| ORL3542 | OR51A8P |
| ORL3543 | OR5D15P |
| ORL3544 | OR9L2P |
| ORL3545 | OR5D17P |
| ORL3546 | OR9Q2P |
| ORL3547 | OR5W3P |
| ORL3548 | OR9I3P |
| ORL3549 | OR51A9P |
| ORL355 | hsORL-131 |
| ORL3550 | OR5BL1P |
| ORL3551 | OR9M1P |
| ORL3552 | OR52M2P |
| ORL3553 | OR52M3P |

(continued)

| Name | Common Name |
|---|---|
| ORL3554 | OR2AH1P |
| ORL3555 | OR56B3P |
| ORL3557 | OR5AM1P |
| ORL3558 | OR52B1P |
| ORL3559 | OR5M12P |
| ORL356 | hsORL-132 |
| ORL3560 | OR5AP1P |
| ORL3561 | OR5M13P |
| ORL3562 | OR52K3P |
| ORL3563 | OR52B3P |
| ORL3564 | OR5BB1P |
| ORL3565 | OR9G2P |
| ORL3566 | OR9G3P |
| ORL3567 | OR51A10P |
| ORL3568 | OR52P2P |
| ORL3569 | OR4A2P |
| ORL357 | hsORL-133 |
| ORL3570 | OR5AK1P |
| ORL3571 | OR5BQ1P |
| ORL3572 | OR4A3P |
| ORL3573 | OR4R2P |
| ORL3574 | OR7E117P |
| ORL3575 | OR5F2P |
| ORL3576 | OR5AQ1P |
| ORL3577 | OR5J1P |
| ORL3578 | OR5BE1P |
| ORL3579 | OR5BN1P |
| ORL358 | hsORL-134 |
| ORL3580 | OR8K4P |
| ORL3581 | OR7E11P |
| ORL3582 | OR7A3P |
| ORL3583 | OR7E3P |
| ORL3584 | OR4A6P |
| ORL3585 | OR4A7P |
| ORL3586 | OR8C1P |
| ORL3587 | OR4A8P |
| ORL3588 | OR7E15P |
| ORL3589 | OR4A9P |

(continued)

| Name | Common Name |
|---|---|
| ORL359 | hsORL-135 |
| ORL3590 | OR4A10P |
| ORL3591 | OR4A11P |
| ORL3592 | OR4A12P |
| ORL3593 | OR4A13P |
| ORL3594 | OR4A14P |
| ORL3595 | OR51C3P |
| ORL3596 | OR51B1P |
| ORL3597 | OR8B6P |
| ORL3598 | OR8B5P |
| ORL3599 | OR8B7P |
| ORL36 | HTPCRH07 |
| ORL360 | hsORL-136 |
| ORL3600 | OR10D6P |
| ORL3601 | OR8C3P |
| ORL3602 | OR4P3P |
| ORL3603 | OR8B1P |
| ORL3604 | OR4D7P |
| ORL3605 | OR4D8P |
| ORL3606 | OR2AT1P |
| ORL3607 | OR4D10P |
| ORL3608 | OR4C11P |
| ORL3609 | OR4D11P |
| ORL361 | hsORL-137 |
| ORL3610 | OR55C1P |
| ORL3611 | OR55B1P |
| ORL3612 | OR52V1P |
| ORL3613 | OR52T1P |
| ORL3614 | OR52H2P |
| ORL3615 | OR52B5P |
| ORL3616 | OR5BA1P |
| ORL3617 | OR5AZ1P |
| ORL3618 | OR5B14P |
| ORL3619 | OR5B15P |
| ORL362 | hsORL-138 |
| ORL3620 | OR51A11P |
| ORL3621 | OR8R1P |
| ORL3622 | OR5AN2P |

(continued)

| Name | Common Name |
|---|---|
| ORL3623 | OR5BR1P |
| ORL3624 | OR10W1P |
| ORL3625 | OR5B18P |
| ORL3626 | OR56A7P |
| ORL3627 | OR5BC1P |
| ORL3628 | OR10Q2P |
| ORL3629 | OR5B19P |
| ORL363 | hsORL-139 |
| ORL3630 | OR4A17P |
| ORL3631 | OR10V2P |
| ORL3632 | OR5AK4P |
| ORL3633 | OR10Y1P |
| ORL3634 | OR7E14P |
| ORL3635 | OR4R3P |
| ORL3636 | OR4A18P |
| ORL3637 | OR4A19P |
| ORL3638 | OR4A20P |
| ORL3639 | OR10V3P |
| ORL364 | hsORL-140 |
| ORL3640 | OR7E2P |
| ORL3641 | OR7E13P |
| ORL3642 | OR7E126P |
| ORL3643 | OR8Q1P |
| ORL3644 | OR7E128P |
| ORL3645 | OR5P4P |
| ORL3646 | OR5G4P |
| ORL3647 | OR4S2P |
| ORL3648 | OR5G5P |
| ORL3649 | OR8A2P |
| ORL365 | hsORL-141 |
| ORL3650 | OR7E12P |
| ORL3651 | OR4A1P |
| ORL3652 | OR4A21P |
| ORL3653 | OR4C1P |
| ORL3654 | OR4C14P |
| ORL3655 | OR10A7/OR12.03.01 |
| ORL3656 | OR9K2/OR12.02.01 |
| ORL3657 | OR10P1P/OR12.03.02/HGPCR0636 |

(continued)

| Name | Common Name |
|---|---|
| ORL3658 | OR10AD1P/OR12.01.01 |
| ORL3659 | OR9K1P/HGPCR0894 |
| ORL366 | hsORL-142 |
| ORL3660 | OR10P3P/HGPCR0351 |
| ORL3661 | OR12.01.01 |
| ORL3662 | OR12.04.02 |
| ORL3663 | OR12.04.01 |
| ORL3664 | OR7E95P |
| ORL3665 | OR5BK1P |
| ORL3666 | OR11M1P |
| ORL3667 | OR9R1P |
| ORL3668 | OR10P2P |
| ORL3669 | OR2A18P |
| ORL367 | hsORL-131 |
| ORL3670 | OR7A19P |
| ORL3671 | OR2AP1P |
| ORL3672 | OR6U1P |
| ORL3673 | OR10U1P |
| ORL3674 | OR11H2P |
| ORL3675 | OR7E101P |
| ORL3676 | OR7E104P |
| ORL3677 | OR7E111P |
| ORL3678 | OR7E37P |
| ORL3679 | OR7E33P |
| ORL368 | hsORL-144 |
| ORL3680 | OR5B10P |
| ORL3681 | OR4K2/OR14.07.07 |
| ORL3682 | OR4K3/OR14.07.06 |
| ORL3683 | OR6J2/OR14.02.01 |
| ORL3684 | OR4K5/OR14.07.09 |
| ORL3685 | OR4N5/OR14.06.02 |
| ORL3686 | OR11H4/OR14.03.01 |
| ORL3687 | OR11G2/OR14.03.03 |
| ORL3688 | OR4L1/OR14.07.01 |
| ORL3689 | OR4K13/OR14.07.04 |
| ORL369 | hsORL-145 |
| ORL3690 | OR4K15/OR14.07.08 |
| ORL3691 | OR4K17/OR14.07.02 |

(continued)

| Name | Common Name |
|---|---|
| ORL3692 | OR14.07.03/HGPCR0058 |
| ORL3693 | OR4N2/OR14.06.03/HGPCR0320 |
| ORL3694 | OR6S1/OR14.02.02/HGPCR0135 |
| ORL3695 | OR10G3/OR14.04.02/HGPCR0263 |
| ORL3697 | OR10G2/OR14.04.01/HGPCR0272 |
| ORL3698 | OR4E2/OR14.05.01/HGPCR0273 |
| ORL3699 | OR11H6/OR14.03.02/HGPCR0190 |
| ORL37 | HTPCRH02 |
| ORL370 | hsORL-146 |
| ORL3700 | OR4K14/OR14.07.05/HGPCR0588 |
| ORL3701 | OR4K1 |
| ORL3702 | OR6J1P |
| ORL3703 | OR6E1P |
| ORL3704 | OR4N1P |
| ORL3705 | OR4K4P |
| ORL3706 | OR4K6P |
| ORL3707 | OR7E105P |
| ORL3708 | OR7E106P |
| ORL3709 | OR11G1P |
| ORL371 | hsORL-147 |
| ORL3710 | OR11H5P |
| ORL3711 | OR4U1P |
| ORL3712 | OR4L2P |
| ORL3713 | OR4Q2P |
| ORL3714 | OR4K16P |
| ORL3715 | OR4T1P |
| ORL3716 | OR4H8P |
| ORL3717 | OR4E1P |
| ORL3718 | OR10G1P |
| ORL3719 | OR7K1P |
| ORL372 | hsORL-148 |
| ORL3720 | OR7A12P |
| ORL3721 | OR4N4/OR15.02.02/HGPCR1149 |
| ORL3722 | OR4N4/OR15.02.02/HGPCR0703 |
| ORL3723 | OR4M2/OR15.02.01/HGPCR0928 |
| ORL3725 | OR15.01.01 |
| ORL3726 | OR4Q1P |
| ORL3727 | OR11K1P |

(continued)

| Name | Common Name |
|---|---|
| ORL3728 | OR4N3P |
| ORL3729 | OR4H6P |
| ORL373 | hsORL-149 |
| ORL3730 | OR11H3P |
| ORL3731 | OR4H10P |
| ORL3732 | OR11J1P |
| ORL3733 | OR11J2P |
| ORL3734 | OR8B11P |
| ORL3735 | OR11I2P |
| ORL3736 | OR4C5P/OR16.03.03 |
| ORL3737 | OR4S1/OR16.03.01/HGPCR0474 |
| ORL3738 | OR4C3/OR16.03.02/HGPCR0976 |
| ORL3739 | OR4C2P |
| ORL374 | hsORL-150 |
| ORL3740 | OR4C4P |
| ORL3741 | OR4F11P |
| ORL3742 | OR4G5P |
| ORL3743 | OR2C2P |
| ORL3744 | OR4G1P |
| ORL3745 | OR4D2/OR17.06.02/HGPCR0095 |
| ORL3746 | OR3A2/OR17.01.04/HGPCR0766 |
| ORL3747 | OR1G1/OR17.05.01 |
| ORL3748 | OR17.06.01 |
| ORL3749 | OR1E3P |
| ORL375 | hsORL-151 |
| ORL3750 | OR1R1P |
| ORL3751 | OR4K7P |
| ORL3752 | OR1R2P |
| ORL3753 | OR1D3P |
| ORL3754 | OR1E9P |
| ORL3755 | OR1R3P |
| ORL3756 | OR4K9P |
| ORL3757 | OR4K10P |
| ORL3758 | OR5D12P |
| ORL3759 | OR5D5P |
| ORL376 | hsORL-152 |
| ORL3760 | OR10H4/OR19.05.05/HGPCR0324 |
| ORL3761 | OR10H5/OR19.05.02/HGPCR0167 |

(continued)

| Name | Common Name |
|---|---|
| ORL3762 | OR7G1/OR19.04.03/HGPCR0435 |
| ORL3763 | OR2Z1/OR19.01.01/HGPCR0216 |
| ORL3764 | OR2Z1/OR19.01.01/HGPCR0725 |
| ORL3765 | OR7G3/OR19.04.04/HGPCR0434 |
| ORL3766 | OR7D4P/OR19.04.05/HGPCR0977 |
| ORL3767 | OR7C1/OR19.04.08/HGPCR0887 |
| ORL3768 | OR4F19/OR19.06.01 |
| ORL3769 | OR19.04.01/HGPCR0980 |
| ORL377 | hsORL-153 |
| ORL3770 | OR7A2/HGPCR0709 |
| ORL3771 | OR7G2/HGPCR0436 |
| ORL3772 | OR4F18 |
| ORL3773 | OR7A10 |
| ORL3774 | OR19.04.02 |
| ORL3775 | OR19.04.07 |
| ORL3776 | OR5AH1P |
| ORL3777 | OR7D1P |
| ORL3778 | OR7E24P |
| ORL3779 | OR7E19P |
| ORL378 | hsORL-154 |
| ORL3780 | OR7D4P |
| ORL3781 | OR7E25P |
| ORL3782 | OR7E16P |
| ORL3783 | OR4F8P |
| ORL3784 | OR4F9P |
| ORL3785 | OR7E98P |
| ORL3786 | OR1AB1P |
| ORL3787 | OR7A18P |
| ORL3788 | OR7A1P |
| ORL3789 | OR10B1P |
| ORL379 | oq79g01.s1 |
| ORL3790 | OR7H1P |
| ORL3791 | OR7A11P |
| ORL3792 | OR7A15P |
| ORL3793 | OR7A8P |
| ORL3794 | OR7A14P |
| ORL3795 | OR4G3P |
| ORL3796 | OR4G7P |

(continued)

| Name | Common Name |
|---|---|
| ORL3797 | OR4G8P |
| ORL3798 | OR7E92P |
| ORL3799 | OR4K11P |
| ORL38 | HTPCRX01 |
| ORL380 | ah40c03.s1 |
| ORL3800 | OR4K12P |
| ORL3801 | OR7E23P |
| ORL3802 | OR11H1/OR22.01.01/HGPCR0191 |
| ORL3803 | OR13H1/OR0X.01.01/HGPCR0012 |
| ORL3804 | H38g522/HGPCR0369 |
| ORL3805 | OR2D1 |
| ORL3806 | OR1F11 |
| ORL3807 | OR2A19 |
| ORL3808 | OR7E120 |
| ORL3809 | OR2M1 |
| ORL381 | AA042813 |
| ORL3810 | OR5AC2 |
| ORL3811 | OR5B3 |
| ORL3812 | OR6C2 |
| ORL3813 | OR52A2 |
| ORL3814 | OR4Q3 |
| ORL3815 | OR6C1 |
| ORL3816 | OR2A20 |
| ORL3817 | OR2M2 |
| ORL3818 | OR2A21 |
| ORL3819 | OR6C3 |
| ORL382 | yd62d03.r1 |
| ORL3820 | OR1E7 |
| ORL3821 | HGPCR0003 |
| ORL3822 | HGPCR0004 |
| ORL3823 | HGPCR0005 |
| ORL3824 | HGPCR0013 |
| ORL3825 | HGPCR0014 |
| ORL3826 | HGPCR0016 |
| ORL3827 | HGPCR0017 |
| ORL3828 | HGPCR0019 |
| ORL3829 | HGPCR0042 |
| ORL383 | yq74a09.r1 |

(continued)

| Name | Common Name |
|---|---|
| ORL3830 | HGPCR0050 |
| ORL3831 | HGPCR0052 |
| ORL3832 | HGPCR0060 |
| ORL3833 | HGPCR0061 |
| ORL3834 | HGPCR0062 |
| ORL3835 | HGPCR0074 |
| ORL3836 | HGPCR0076 |
| ORL3837 | HGPCR0078 |
| ORL3838 | HGPCR0082 |
| ORL3839 | HGPCR0083 |
| ORL384 | za65c09.r1 |
| ORL3840 | HGPCR0086 |
| ORL3841 | HGPCR0087 |
| ORL3842 | HGPCR0094 |
| ORL3843 | HGPCR0097 |
| ORL3844 | HGPCR0100 |
| ORL3845 | HGPCR0111 |
| ORL3846 | HGPCR0112 |
| ORL3847 | HGPCR0113 |
| ORL3848 | HGPCR0122 |
| ORL3849 | HGPCR0124 |
| ORL385 | yh39c04.r1 |
| ORL3850 | HGPCR0125 |
| ORL3851 | HGPCR0127 |
| ORL3852 | HGPCR0128 |
| ORL3853 | HGPCR0143 |
| ORL3854 | HGPCR0146 |
| ORL3855 | HGPCR0153 |
| ORL3856 | HGPCR0164 |
| ORL3857 | HGPCR0168 |
| ORL3858 | HGPCR0174 |
| ORL3859 | HGPCR0178 |
| ORL386 | zs42g05.r1 |
| ORL3860 | HGPCR0185 |
| ORL3861 | HGPCR0188 |
| ORL3862 | HGPCR0192 |
| ORL3863 | HGPCR0199 |
| ORL3864 | HGPCR0211 |

(continued)

| Name | Common Name |
|---|---|
| ORL3865 | HGPCR0213 |
| ORL3866 | HGPCR0215 |
| ORL3867 | HGPCR0238 |
| ORL3868 | HGPCR0244 |
| ORL3869 | HGPCR0245 |
| ORL387 | zx51h08.s1 |
| ORL3870 | HGPCR0246 |
| ORL3871 | HGPCR0247 |
| ORL3872 | HGPCR0249 |
| ORL3873 | HGPCR0250 |
| ORL3874 | HGPCR0256 |
| ORL3875 | HGPCR0260 |
| ORL3876 | HGPCR0264 |
| ORL3877 | HGPCR0265 |
| ORL3878 | HGPCR0268 |
| ORL3879 | HGPCR0274 |
| ORL388 | yd40h07.r1 |
| ORL3880 | HGPCR0276 |
| ORL3881 | HGPCR0278 |
| ORL3882 | HGPCR0283 |
| ORL3883 | HGPCR0285 |
| ORL3884 | HGPCR0287 |
| ORL3885 | HGPCR0291 |
| ORL3886 | HGPCR0309 |
| ORL3887 | HGPCR0313 |
| ORL3888 | HGPCR0319 |
| ORL3889 | HGPCR0321 |
| ORL389 | yp84e02.r1 |
| ORL3890 | HGPCR0325 |
| ORL3891 | HGPCR0327 |
| ORL3892 | HGPCR0329 |
| ORL3893 | HGPCR0330 |
| ORL3894 | HGPCR0340 |
| ORL3895 | HGPCR0347 |
| ORL3896 | HGPCR0355 |
| ORL3897 | HGPCR0358 |
| ORL3898 | HGPCR0367 |
| ORL3899 | HGPCR0370 |

(continued)

| Name | Common Name |
|---|---|
| ORL39 | HTPCRX02 |
| ORL390 | yr79d08.r1 |
| ORL3900 | HGPCR0378 |
| ORL3901 | HGPCR0392 |
| ORL3902 | HGPCR0393 |
| ORL3903 | HGPCR0398 |
| ORL3904 | HGPCR0400 |
| ORL3905 | HGPCR0401 |
| ORL3906 | HGPCR0409 |
| ORL3907 | HGPCR0417 |
| ORL3908 | HGPCR0422 |
| ORL3909 | HGPCR0428 |
| ORL391 | yr79e08.r1 |
| ORL3910 | HGPCR0439 |
| ORL3911 | HGPCR0442 |
| ORL3912 | HGPCR0448 |
| ORL3913 | HGPCR0451 |
| ORL3914 | HGPCR0455 |
| ORL3915 | HGPCR0456 |
| ORL3916 | HGPCR0459 |
| ORL3917 | HGPCR0464 |
| ORL3918 | HGPCR0465 |
| ORL3919 | HGPCR0467 |
| ORL392 | yh39c04.s1 |
| ORL3920 | HGPCR0468 |
| ORL3921 | HGPCR0479 |
| ORL3922 | HGPCR0481 |
| ORL3923 | HGPCR0483 |
| ORL3924 | HGPCR0486 |
| ORL3925 | HGPCR0487 |
| ORL3926 | HGPCR0489 |
| ORL3927 | HGPCR0501 |
| ORL3928 | HGPCR0507 |
| ORL3929 | HGPCR0508 |
| ORL393 | zb47d11.s1 |
| ORL3930 | HGPCR0510 |
| ORL3931 | HGPCR0513 |
| ORL3932 | HGPCR0516 |

(continued)

| Name | Common Name |
|---|---|
| ORL3933 | HGPCR0519 |
| ORL3934 | HGPCR0531 |
| ORL3935 | HGPCR0534 |
| ORL3936 | HGPCR0053 |
| ORL3937 | HGPCR0543 |
| ORL3938 | HGPCR0546 |
| ORL3939 | HGPCR0566 |
| ORL394 | af94a05.s1 |
| ORL3940 | HGPCR0570 |
| ORL3941 | HGPCR0571 |
| ORL3942 | HGPCR0578 |
| ORL3943 | HGPCR0581 |
| ORL3944 | HGPCR0585 |
| ORL3945 | HGPCR0586 |
| ORL3946 | HGPCR0589 |
| ORL3947 | HGPCR0590 |
| ORL3948 | HGPCR0591 |
| ORL3949 | HGPCR0599 |
| ORL395 | OR5-85 |
| ORL3950 | HGPCR0611 |
| ORL3951 | HGPCR0618 |
| ORL3952 | HGPCR0620 |
| ORL3953 | HGPCR0621 |
| ORL3954 | HGPCR0625 |
| ORL3955 | HGPCR0630 |
| ORL3956 | HGPCR0632 |
| ORL3957 | HGPCR0637 |
| ORL3958 | HGPCR0640 |
| ORL3959 | HGPCR0643 |
| ORL396 | OR7-141 |
| ORL3960 | HGPCR0650 |
| ORL3961 | HGPCR0653 |
| ORL3962 | HGPCR0655 |
| ORL3963 | HGPCR0656 |
| ORL3964 | HGPCR0665 |
| ORL3965 | HGPCR0668 |
| ORL3966 | HGPCR0672 |
| ORL3967 | HGPCR0674 |

(continued)

| Name | Common Name |
|---|---|
| ORL3968 | HGPCR0676 |
| ORL3969 | HGPCR0680 |
| ORL397 | OR17-228 |
| ORL3970 | HGPCR0700 |
| ORL3971 | HGPCR0703 |
| ORL3972 | HGPCR0705 |
| ORL3973 | HGPCR0706 |
| ORL3974 | HGPCR0710 |
| ORL3975 | HGPCR0713 |
| ORL3976 | HGPCR0719 |
| ORL3977 | HGPCR0720 |
| ORL3978 | HGPCR0725 |
| ORL3979 | HGPCR0726 |
| ORL3980 | HGPCR0727 |
| ORL3981 | HGPCR0728 |
| ORL3982 | HGPCR0732 |
| ORL3983 | HGPCR0747 |
| ORL3984 | HGPCR0762 |
| ORL3985 | HGPCR0764 |
| ORL3986 | HGPCR0769 |
| ORL3987 | HGPCR0775 |
| ORL3988 | HGPCR0778 |
| ORL3989 | HGPCR0781 |
| ORL3990 | HGPCR0792 |
| ORL3991 | HGPCR0796 |
| ORL3992 | HGPCR0799 |
| ORL3993 | HGPCR0805 |
| ORL3994 | HGPCR0806 |
| ORL3995 | HGPCR0809 |
| ORL3996 | HGPCR0813 |
| ORL3997 | HGPCR0816 |
| ORL3998 | HGPCR0832 |
| ORL3999 | HGPCR0836 |
| ORL40 | HTPCRX03 |
| ORL400 | af90d06.s1 |
| ORL4000 | HGPCR0837 |
| ORL4001 | HGPCR0844 |
| ORL4002 | HGPCR0845 |

(continued)

| Name | Common Name |
|---|---|
| ORL4003 | HGPCR0848 |
| ORL4004 | HGPCR0858 |
| ORL4005 | HGPCR0860 |
| ORL4006 | HGPCR0866 |
| ORL4007 | HGPCR0868 |
| ORL4008 | HGPCR0876 |
| ORL4009 | HGPCR0877 |
| ORL401 | yq19f08.s1 |
| ORL4010 | HGPCR0878 |
| ORL4011 | HGPCR0879 |
| ORL4012 | HGPCR0885 |
| ORL4013 | HGPCR0895 |
| ORL4014 | HGPCR0897 |
| ORL4015 | HGPCR0900 |
| ORL4016 | HGPCR0902 |
| ORL4017 | HGPCR0905 |
| ORL4018 | HGPCR0907 |
| ORL4019 | HGPCR0909 |
| ORL4020 | HGPCR0915 |
| ORL4021 | HGPCR0944 |
| ORL4022 | HGPCR0957 |
| ORL4023 | HGPCR0958 |
| ORL4024 | HGPCR0961 |
| ORL4025 | HGPCR0962 |
| GRL4026 | HGPCR0965 |
| ORL4027 | HGPCR0967 |
| ORL4028 | HGPCR0975 |
| ORL4029 | HGPCR0986 |
| ORL4030 | HGPCR0989 |
| ORL4031 | HGPCR0993 |
| ORL4032 | HGPCR0995 |
| ORL4033 | HGPCR0997 |
| ORL4034 | HGPCR0999 |
| ORL4035 | HGPCR1000 |
| ORL4036 | HGPCR1016 |
| ORL4037 | HGPCR1020 |
| ORL4038 | HGPCR1021 |
| ORL4039 | HGPCR1022 |

(continued)

| Name | Common Name |
|---|---|
| ORL4040 | HGPCR1023 |
| ORL4041 | HGPCR1028 |
| ORL4042 | HGPCR1034 |
| ORL4043 | HGPCR1041 |
| ORL4044 | HGPCR1043 |
| ORL4045 | HGPCR1050 |
| ORL4046 | HGPCR1059 |
| ORL4047 | HGPCR1072 |
| ORL4048 | HGPCR1075 |
| ORL4049 | HGPCR1077 |
| ORL4050 | HGPCR1082 |
| ORL4051 | HGPCR1083 |
| ORL4052 | HGPCR1091 |
| ORL4053 | HGPCR1092 |
| ORL4054 | HGPCR1095 |
| ORL4055 | HGPCR1097 |
| ORL4056 | HGPCR1105 |
| ORL4057 | HGPCR1106 |
| ORL4058 | HGPCR1109 |
| ORL4059 | HGPCR1113 |
| ORL4060 | HGPCR1122 |
| ORL4061 | HGPCR1125 |
| ORL4062 | HGPCR1126 |
| ORL4063 | HGPCR1128 |
| ORL4064 | HGPCR1131 |
| ORL4065 | HGPCR1136 |
| ORL4066 | HGPCR1137 |
| ORL4067 | HGPCR1144 |
| ORL4068 | HGPCR1147 |
| ORL4069 | HGPCR1154 |
| ORL4070 | HGPCR1158 |
| ORL4071 | OR7E88P |
| ORL4072 | OR2AF1P |
| ORL4073 | OR13K1P |
| ORL4074 | OR1AA1P |
| ORL4075 | OR7L1P |
| ORL4076 | OR2AF2P |
| ORL4077 | OR3B1P |

(continued)

| Name | Common Name |
|---|---|
| ORL4078 | |
| ORL4079 | OR5BH1P |
| ORL4080 | OR2W5P |
| ORL4081 | OR51C2P |
| ORL4082 | OR5BJ1P |
| ORL4083 | OR2C5P |
| ORL4084 | OR5B12P |
| ORL4085 | OR7E39P |
| ORL4086 | OR7E27P |
| ORL4087 | OR5D10P |
| ORL4088 | OR2I3P |
| ORL4089 | OR7E119P |
| ORL4090 | OR7E47P |
| ORL4091 | OR7E42P |
| ORL4092 | OR2M3P |
| ORL4093 | OR7E57P |
| ORL4094 | OR7E34P |
| ORL4095 | OR7E56P |
| ORL4096 | OR7E21P |
| ORL4097 | OR7E45P |
| ORL4098 | OR7E77P |
| ORL4099 | OR7E81P |
| ORL41 | HTPCRX06 |
| CRL4100 | OR7E44P |
| ORL4101 | OR2I5P |
| ORL4102 | OR7E59P |
| ORL4103 | OR7E28P |
| ORL4104 | OR7E54P |
| ORL4105 | OR7E48P |
| ORL4106 | OR51E3P |
| ORL4107 | OR7E40P |
| ORL4108 | OR7E52P |
| ORL4109 | OR2I7P |
| ORL4110 | OR7E30P |
| ORL4111 | OR2I8P |
| ORL4112 | OR52A3P |
| ORL4113 | OR2I9P |
| ORL4114 | OR7E20P |

(continued)

| Name | Common Name |
|---|---|
| ORL4115 | OR2A22P |
| ORL4116 | OR5BH2P |
| ORL4117 | OR1E8P |
| ORL4118 | OR4W1P |
| ORL4119 | OR7E124P |
| ORL4120 | OR10J4P |
| ORL4121 | OR7E123P |
| ORL4122 | OR7E36P |
| ORL4123 | OR4G2P |
| ORL4124 | OR06.03.02 |
| ORL4125 | HGPCR0405 |
| ORL4126 | OR09.01.09/HGPCR019 |
| ORL4127 | OR09.01.08/HGPCR019 |
| ORL4128 | OR13E2/HGPCR0369 |
| ORL4129 | OR93 |
| ORL42 | HTPCRX09 |
| ORL420 | dJ88J8.1 |
| ORL423 | OR2C1 |
| ORL43 | HTPCRX10 |
| ORL430 | olfr89 |
| ORL44 | HTPCRX11 |
| ORL45 | HTPCRX12 |
| ORL4501 | HsOR1.4.9 |
| ORL4502 | HsOR1.5.1 |
| ORL4503 | HsOR1.1.1P |
| ORL4504 | HsOR1.1.2P |
| ORL4505 | HsOR1.1.3 |
| ORL4506 | HsOR1.2.1P |
| ORL4507 | HsOR1.3.1P |
| ORL4508 | HsOR1.3.2P |
| ORL4509 | HsOR1.4.3P |
| ORL4510 | HsOR1.4.11P |
| ORL4511 | HsOR1.4.14P |
| ORL4512 | HsOR1.4.15P |
| ORL4513 | HsOR1.4.19P |
| ORL4514 | HsOR1.4.20P |
| ORL4515 | HsOR1.4.21P |
| ORL4516 | HsOR1.4.22P |

(continued)

| Name | Common Name |
|---|---|
| ORL4517 | HsOR1.4.23P |
| ORL4518 | HsOR1.4.24P |
| ORL4519 | HsOR1.4.25P |
| ORL4520 | HsOR1.4.28P |
| ORL4521 | HsOR1.5.2P |
| ORL4522 | HsOR1.5.11P |
| ORL4523 | HsOR1.5.16 |
| ORL4524 | HsOR1.5.17 |
| ORL4525 | HsOR1.5.19 |
| ORL4526 | HsOR1.5.20P |
| ORL4527 | HsOR1.5.22P |
| ORL4528 | HsOR1.5.23 |
| ORL4529 | HsOR1.5.24 |
| ORL4530 | HsOR1.5.25 |
| ORL4531 | HsOR1.5.26P |
| ORL4532 | HsOR1.5.28P |
| ORL4533 | HsOR1.5.27 |
| ORL4534 | HsOR1.5.29 |
| ORL4535 | HsOR1.5.30 |
| ORL4536 | HsOR1.5.38 |
| ORL4537 | HsOR1.5.42 |
| ORL4538 | HsOR1.5.44 |
| ORL4539 | HsOR1.5.48 |
| ORL4540 | HsOR1.5.50 |
| ORL4541 | HsOR2.1.1P |
| ORL4542 | HsOR2.1.2P |
| ORL4543 | HsOR2.1.3P |
| ORL4544 | HsOR2.2.1P |
| ORL4545 | HsOR2.3.1P |
| ORL4546 | HsOR2.3.2P |
| ORL4547 | HsOR2.3.3P |
| ORL4548 | HsOR2.4.1 |
| ORL4549 | HsOR2.4.2 |
| ORL4550 | HsOR2.4.3P |
| ORL4551 | HsOR3.1.1P |
| ORL4552 | HsOR3.2.1P |
| ORL4553 | HsOR3.2.2P |
| ORL4554 | HsOR3.2.3P |

(continued)

| Name | Common Name |
|---|---|
| ORL4555 | HsOR3.2.4P |
| ORL4556 | HsOR3.3.1P |
| ORL4557 | HsOR3.3.2 |
| ORL4558 | HsOR3.3.4 |
| ORL4559 | HsOR3.3.5 |
| ORL4560 | HsOR3.3.6 |
| ORL4561 | HsOR3.3.3P |
| ORL4562 | HsOR3.3.7P |
| ORL4563 | HsOR3.3.8P |
| ORL4565 | HsOR3.3.9P |
| ORL4566 | HsOR3.3.10P |
| ORL4567 | HsOR3.3.13P |
| ORL4568 | HsOR3.3.14 |
| ORL4569 | HsOR3.3.15 |
| ORL4570 | HsOR3.3.16 |
| ORL4571 | HsOR3.4.1P |
| ORL4572 | HsOR3.5.1P |
| ORL4573 | HsOR3.5.2P |
| ORL4574 | HsOR3.5.3P |
| ORL4575 | HsOR3.5.4P |
| ORL4576 | HsOR3.5.5P |
| ORL4577 | HsOR3.6.1P |
| ORL4578 | HsOR3.6.2P |
| ORL4579 | HsOR4.1.1P |
| ORL4580 | HsOR4.1.2P |
| ORL4581 | HsOR4.1.3P |
| ORL4582 | HsOR4.2.1P |
| ORL4583 | HsOR4.2.2P |
| ORL4584 | HsOR4.2.3P |
| ORL4585 | HsOR4.2.4P |
| ORL4586 | HsOR4.2.5P |
| ORL4587 | HsOR4.3.1P |
| ORL4588 | HsOR4.4.1P |
| ORL4589 | HsOR5.1.1P |
| ORL459 | OLFR17-30 |
| ORL4590 | HsOR5.2.1P |
| ORL4591 | HsOR5.3.1P |
| ORL4592 | HsOR5.4.1P |

(continued)

| Name | Common Name |
|---|---|
| ORL4593 | HsOR5.4.3 |
| ORL4594 | HsOR6.1.1P |
| ORL4597 | HsOR6.2.2P |
| ORL4598 | HsOR6.2.4P |
| ORL4599 | HsOR6.2.5P |
| ORL46 | HTPCRX13 |
| ORL4600 | HsOR6.2.6P |
| ORL4601 | HsOR6.2.7P |
| ORL4602 | HsOR6.2.9P |
| ORL4603 | HsOR6.3.1P |
| ORL4604 | HsOR6.3.3P |
| ORL4605 | HsOR6.3.5P |
| ORL4606 | HsOR6.3.7P |
| ORL4607 | HsOR6.3.9P |
| ORL4608 | HsOR6.3.10P |
| ORL4609 | HsOR6.3.11P |
| ORL4610 | HsOR6.3.12P |
| ORL4611 | HsOR6.3.13P |
| ORL4612 | HsOR6.3.14P |
| ORL4613 | HsOR6.3.15P |
| ORL4614 | HsOR6.3.20P |
| ORL4615 | HsOR6.3.24P |
| ORL4616 | HsOR6.3.25P |
| ORL4617 | HsOR6.5.1P |
| ORL4618 | HsOR7.1.1P |
| ORL4619 | HsOR7.2.1P |
| ORL4620 | HsOR7.2.2P |
| ORL4621 | HsOR7.2.3P |
| ORL4622 | HsOR7.3.1P |
| ORL4623 | HsOR7.3.2P |
| ORL4624 | HsOR7.5.1P |
| ORL4625 | HsOR7.6.3P |
| ORL4626 | HsOR7.6.4P |
| ORL4627 | HsOR7.6.5P |
| ORL4628 | HsOR7.6.8P |
| ORL4629 | HsOR7.6.10 |
| ORL4630 | HsOR7.6.11 |
| ORL4631 | HsOR7.6.13 |

(continued)

| Name | Common Name |
|---|---|
| ORL4632 | HsOR7.6.14P |
| ORL4633 | HsOR7.6.16P |
| ORL4634 | HsOR7.6.17P |
| ORL4635 | HsOR7.6.18P |
| ORL4636 | HsOR7.6.20P |
| ORL4637 | HsOR7.6.21 |
| ORL4638 | HsOR7.6.22P |
| ORL4639 | HsOR8.2.1P |
| ORL4640 | HsOR8.3.1P |
| ORL4641 | HsOR8.4.1P |
| ORL4642 | HsOR8.4.2P |
| ORL4643 | HsOR8.4.3P |
| ORL4644 | HsOR8.5.1P |
| ORL4645 | HsOR8.5.2P |
| ORL4646 | HsOR8.5.3P |
| ORL4647 | HsOR9.1.1P |
| ORL4648 | HsOR9.1.4P |
| ORL4649 | HsOR9.1.5P |
| ORL4650 | HsOR9.1.6P |
| ORL4651 | HsOR9.1.7P |
| ORL4652 | HsOR9.2.1P |
| ORL4653 | HsOR9.2.2P |
| ORL4654 | HsOR9.3.1P |
| ORL4655 | HsOR9.3.2P |
| ORL4656 | HsOR9.4.5P |
| ORL4657 | HsOR9.4.9P |
| ORL4658 | HsOR9.4.10P |
| ORL4659 | HsOR9.4.12P |
| ORL4660 | HsOR9.6.7P |
| ORL4661 | HsOR10.1.1P |
| ORL4662 | HsOR10.1.2P |
| ORL4663 | HsOR10.1.3P |
| ORL4664 | HsOR10.2.1P |
| ORL4665 | HsOR11.8.13 |
| ORL4666 | HsOR11.9.7 |
| ORL4667 | HsOR11.10.8 |
| ORL4668 | HsOR11.2.1P |
| ORL4669 | HsOR11.2.2P |

(continued)

| Name | Common Name |
|---|---|
| ORL4670 | HsOR11.3.1P |
| ORL4671 | HsOR11.3.2 |
| ORL4672 | HsOR11.3.3P |
| ORL4673 | HsOR11.3.4P |
| ORL4674 | HsOR11.3.5P |
| ORL4675 | HsOR11.3.7P |
| ORL4676 | HsOR11.3.9P |
| ORL4677 | HsOR11.3.15P |
| ORL4678 | HsOR11.3.18 |
| ORL4679 | HsOR11.3.19P |
| ORL4680 | HsOR11.3.20P |
| ORL4681 | HsOR11.3.21P |
| ORL4682 | HsOR11.3.22 |
| ORL4683 | HsOR11.3.23P |
| ORL4684 | HsOR11.3.26P |
| ORL4685 | HsOR11.3.29P |
| ORL4686 | HsOR11.3.31P |
| ORL4687 | HsOR11.3.32P |
| ORL4688 | HsOR11.3.36P |
| ORL4689 | HsOR11.3.39P |
| ORL4690 | HsOR11.3.41P |
| ORL4691 | HsOR11.3.42P |
| ORL4692 | HsOR11.3.45P |
| ORL4693 | HsOR11.3.46P |
| ORL4694 | HsOR11.3.47P |
| ORL4695 | HsOR11.3.48P |
| ORL4696 | HsOR11.3.49P |
| ORL4697 | HsOR11.3.50 |
| ORL4698 | HsOR11.3.52P |
| ORL4699 | HsOR11.3.53P |
| ORL47 | HTPCRX14 |
| ORL4700 | HsOR11.3.54 |
| ORL4701 | HsOR11.3.56P |
| ORL4702 | HsOR11.3.57 |
| ORL4703 | HsOR11.3.58P |
| ORL4704 | HsOR11.3.59 |
| ORL4705 | HsOR11.3.60 |
| ORL4706 | HsOR11.3.61 |

(continued)

| Name | Common Name |
|---|---|
| ORL4707 | HsOR11.3.62P |
| ORL4708 | HsOR11.3.64P |
| ORL4709 | HsOR11.3.67P |
| ORL4710 | HsOR11.3.69P |
| ORL4711 | HsOR11.3.71P |
| ORL4712 | HsOR11.3.72P |
| ORL4713 | HsOR11.3.73P |
| ORL4714 | HsOR11.3.74 |
| ORL4715 | HsOR11.3.75P |
| ORL4716 | HsOR11.3.76P |
| ORL4717 | HsOR11.3.78 |
| ORL4718 | HsOR11.3.79 |
| ORL4719 | HsOR11.3.82P |
| ORL4720 | HsOR11.3.86P |
| ORL4721 | HsOR11.3.89P |
| ORL4722 | HsOR11.3.91 |
| ORL4723 | HsOR11.3.92 |
| ORL4724 | HsOR11.3.95P |
| ORL4725 | HsOR11.3.97P |
| ORL4726 | HsOR11.3.99P |
| ORL4727 | HsOR11.3.100P |
| ORL4728 | HsOR11.4.1 |
| ORL4729 | HsOR11.5.1P |
| ORL4730 | HsOR11.5.2P |
| ORL4731 | HsOR11.5.3P |
| ORL4732 | HsOR11.5.6P |
| ORL4733 | HsOR11.6.1P |
| ORL4734 | HsOR11.7.1P |
| ORL4735 | HsOR11.8.2P |
| ORL4736 | HsOR11.8.7P |
| ORL4737 | HsOR11.8.8P |
| ORL4738 | HsOR11.8.10P |
| ORL4739 | HsOR11.8.11P |
| ORL4740 | HsOR11.8.12P |
| ORL4741 | HsOR11.8.14P |
| ORL4742 | HsOR11.8.15P |
| ORL4743 | NsOR11.8.16P |
| ORL4744 | HsOR11.8.17P |

(continued)

| Name | Common Name |
|---|---|
| ORL4745 | HsOR11.8.18P |
| ORL4746 | HsOR11.8.19P |
| ORL4747 | HsOR11.8.20P |
| ORL4748 | HsOR11.8.21P |
| ORL4749 | HsOR11.9.1P |
| ORL4750 | HsOR11.9.2P |
| ORL4751 | HsOR11.9.3P |
| ORL4752 | HsOR11.9.6P |
| ORL4753 | HsOR11.9.8P |
| ORL4754 | HsOR11.10.1P |
| ORL4755 | HsOR11.10.3P |
| ORL4756 | HsOR11.10.4P |
| ORL4757 | HsOR11.10.5P |
| ORL4758 | HsOR11.10.7P |
| ORL4759 | HsOR11.10.9P |
| ORL4760 | HsOR11.11.1P |
| ORL4761 | HsOR11.11.2P |
| ORL4762 | HsOR11.11.6P |
| ORL4763 | HsOR11.11.7P |
| ORL4764 | HsOR11.11.8P |
| ORL4765 | HsOR11.11.10P |
| ORL4766 | HsOR11.11.11P |
| ORL4767 | HsOR11.11.12P |
| ORL4768 | HsOR11.11.13P |
| ORL4769 | HsOR11.11.14P |
| ORL4770 | HsOR11.11.21P |
| ORL4771 | HsOR11.11.22P |
| ORL4772 | HsOR11.11.23P |
| ORL4773 | HsOR11.11.24P |
| ORL4774 | HsOR11.11.26P |
| ORL4775 | HsOR11.11.32P |
| ORL4776 | HsOR11.11.33P |
| ORL4777 | HsOR11.11.36P |
| ORL4778 | HsOR11.11.38P |
| CRL4779 | HsOR11.11.40P |
| ORL4780 | HsOR11.11.43P |
| ORL4781 | HsOR11.11.44P |
| ORL4782 | HsOR11.11.50P |

(continued)

| Name | Common Name |
|---|---|
| ORL4783 | HsOR11.11.52P |
| ORL4784 | HsOR11.11.53P |
| ORL4785 | HsOR11.11.58P |
| ORL4786 | HsOR11.11.60P |
| ORL4787 | HsOR11.11.64P |
| ORL4788 | HsOR11.11.65P |
| ORL4789 | HsOR11.11.66P |
| ORL4790 | HsOR11.11.68P |
| ORL4791 | HsOR11.11.71P |
| ORL4792 | HsOR11.11.73P |
| ORL4793 | HsOR11.11.74P |
| ORL4794 | HsOR11.11.75P |
| ORL4795 | HsOR11.11.80P |
| ORL4796 | HsOR11.11.81P |
| ORL4797 | HsOR11.11.82P |
| ORL4798 | HsOR11.11.83P |
| ORL4799 | HsOR11.11.86P |
| ORL48 | HTPCRX15 |
| ORL4800 | HsOR11.11.88P |
| ORL4801 | HsOR11.11.90P |
| ORL4802 | HsOR11.11.91P |
| ORL4803 | HsOR11.11.92P |
| ORL4804 | HsOR11.11.93P |
| ORL4805 | HsOR11.11.94P |
| ORL4806 | HsOR11.11.97P |
| ORL4807 | HsOR11.11.98P |
| ORL4808 | HsOR11.12.2P |
| ORL4809 | HsOR11.12.4P |
| ORL481 | HOR5'Beta3 |
| ORL4810 | HsOR11.12.6P |
| ORL4811 | HsOR11.12.8 |
| ORL4812 | HsOR11.12.13P |
| ORL4813 | HsOR11.12.14P |
| ORL4814 | HsOR11.12.15P |
| ORL4815 | HsOR11.12.16P |
| ORL4816 | HsOR11.12.18P |
| ORL4817 | HsOR11.12.19P |
| ORL4818 | HsOR11.12.23 |

(continued)

| Name | Common Name |
|---|---|
| ORL4819 | HsOR11.13.1P |
| ORL482 | HOR5 |
| ORL4820 | HsOR11.13.2P |
| ORL4821 | HsOR11.13.9P |
| ORL4822 | HsOR11.13.11 |
| ORL4823 | HsOR11.13.12P |
| ORL4824 | HsOR11.13.14P |
| ORL4825 | HsOR11.13.15P |
| ORL4826 | HsOR11.14.1P |
| ORL4827 | HsOR11.14.2P |
| ORL4828 | HsOR11.14.3P |
| ORL4829 | HsOR11.15.1P |
| ORL483 | OR2D2 |
| ORL4830 | HsOR11.15.2P |
| ORL4831 | HsOR11.15.3P |
| ORL4832 | HsOR11.15.4P |
| ORL4833 | HsOR11.16.1P |
| ORL4834 | HsOR11.16.2 |
| ORL4835 | HsOR11.16.3P |
| ORL4836 | HsOR11.17.1P |
| ORL4837 | HsOR11.17.2P |
| ORL4838 | HsOR11.18.3P |
| ORL4839 | HsOR11.18.4P |
| ORL484 | OR10A1 |
| ORL4840 | HsOR11.18.10P |
| ORL4841 | HsOR11.18.15P |
| ORL4842 | HsOR11.18.17P |
| ORL4843 | HsOR11.18.18P |
| ORL4844 | HsOR11.18.20P |
| ORL4845 | HsOR11.18.21P |
| ORL4846 | HsOR11.18.22 |
| ORL4847 | HsOR11.18.23P |
| ORL4848 | HsOR11.18.24P |
| ORL4849 | HsOR11.18.28P |
| ORL485 | OR5F1 |
| ORL4850 | HsOR11.18.29P |
| ORL4851 | HsOR11.18.30P |
| ORL4852 | HsOR11.18.31P |

(continued)

| Name | Common Name |
|---|---|
| ORL4853 | HsOR11.18.32P |
| ORL4854 | HsOR11.18.33 |
| ORL4855 | HsOR11.18.34 |
| ORL4856 | HsOR11.18.37P |
| ORL4857 | HsOR11.18.38P |
| ORL4858 | HsOR11.18.39P |
| ORL4859 | HsOR11.18.43P |
| ORL486 | OR5D4 |
| ORL4860 | HsOR12.1.1P |
| ORL4861 | HsOR12.1.2P |
| ORL4862 | HsOR12.1.3P |
| ORL4862 | HsOR12.2.1P |
| ORL4863 | HsOR12.3.2P |
| ORL4864 | HsOR12.3.3P |
| ORL4865 | HsOR12.3.4P |
| ORL4866 | HsOR12.3.5P |
| ORL4867 | HsOR12.3.7P |
| ORL4868 | HsOR12.3.8P |
| ORL4869 | HsOR12.4.1P |
| ORL487 | OR5D3 |
| ORL4870 | HsOR12.5.1P |
| ORL4871 | HsOR12.5.3P |
| ORL4872 | HsOR12.5.4P |
| ORL4873 | HsOR12.5.6 |
| ORL4874 | HsOR12.5.7P |
| ORL4875 | HsOR12.5.8P |
| ORL4876 | HsOR12.5.9 |
| ORL4877 | HsOR12.5.10P |
| ORL4878 | HsOR12.5.11 |
| ORL4879 | HsOR12.5.12 |
| ORL4880 | HsOR12.5.13P |
| ORL4881 | HsOR12.5.14 |
| ORL4882 | HsOR12.5.15P |
| ORL4883 | HsOR12.5.16 |
| ORL4884 | HsOR12.5.17 |
| ORL4885 | HsOR12.5.18 |
| ORL4886 | HsOR12.5.19 |
| ORL4887 | HsOR12.5.20 |

(continued)

| Name | Common Name |
|---|---|
| ORL4888 | HsOR12.5.21 |
| ORL4889 | HsOR12.5.22P |
| ORL4890 | HsOR12.5.25P |
| ORL4891 | HsOR13.1.1P |
| ORL4892 | HsOR13.1.2P |
| ORL4893 | HsOR13.1.3P |
| ORL4894 | HsOR13.3.1P |
| ORL4895 | HsOR13.3.2P |
| ORL4896 | HsOR13.4.1P |
| ORL4897 | HsOR13.4.2P |
| ORL4898 | HsOR14.1.1 |
| ORL4899 | HsOR14.1.2P |
| ORL49 | HTPCRX16 |
| ORL4900 | HsOR14.1.3 |
| ORL4901 | HsOR14.1.4P |
| ORL4902 | HsOR14.1.6P |
| ORL4903 | HsOR14.1.8P |
| ORL4904 | HsOR14.1.9P |
| ORL4905 | HsOR14.1.11P |
| ORL4906 | HsOR14.1.14P |
| ORL4907 | HsOR14.1.16P |
| ORL4908 | HsOR14.1.19P |
| ORL4909 | HsOR14.1.21P |
| ORL491 | hsORL491 |
| ORL4910 | HsOR14.1.24P |
| ORL4911 | HsOR14.1.26P |
| ORL4912 | HsOR14.1.28P |
| ORL4913 | HsOR14.2.3P |
| ORL4914 | HsOR14.2.6P |
| ORL4915 | HsOR14.3.2P |
| ORL4916 | HsOR14.4.1P |
| ORL4917 | HsOR14.5.1P |
| ORL4918 | HsOR14.5.3P |
| ORL4919 | HsOR15.2.6 |
| ORL492 | hsORL492 |
| ORL4920 | HsOR15.1.1P |
| ORL4921 | HsOR15.1.2P |
| ORL4922 | HsOR15.1.3P |

(continued)

| Name | Common Name |
|---|---|
| ORL4923 | HsOR15.1.4P |
| ORL4924 | HsOR15.1.5P |
| ORL4925 | HsOR15.1.6P |
| ORL4926 | HsOR15.1.7P |
| ORL4927 | HsOR15.1.10P |
| ORL4928 | HsOR15.2.4P |
| ORL4929 | HsOR15.2.5P |
| ORL493 | hsORL493 |
| ORL4930 | HsOR15.2.7P |
| ORL4931 | HsOR15.2.8P |
| ORL4932 | HsOR16.1.2P |
| ORL4933 | HsOR17.1.3P |
| ORL4934 | HsOR17.1.5P |
| ORL4935 | HsOR17.1.8P |
| ORL4936 | HsOR17.1.9P |
| ORL4937 | HsOR17.1.13 |
| ORL4938 | HsOR18.1.1P |
| ORL4939 | HsOR19.1.1P |
| ORL494 | hsORL494 |
| ORL4940 | HsOR19.1.2P |
| ORL4941 | HsOR19.1.4P |
| ORL4942 | HsOR19.2.2P |
| ORL4943 | HsOR19.2.6P |
| ORL4944 | HsOR19.2.10P |
| ORL4945 | HsOR19.2.12P |
| ORL4946 | HsOR19.2.13P |
| ORL4947 | HsOR19.2.15P |
| ORL4948 | HsOR19.2.17P |
| ORL4949 | HsOR19.3.4P |
| ORL495 | hsORL495 |
| ORL4950 | HsOR19.3.7P |
| ORL4951 | HsOR19.3.9P |
| ORL4952 | HsOR19.3.10P |
| ORL4953 | HsOR19.3.13P |
| ORL4954 | HsOR19.4.6P |
| ORL4955 | HsOR19.5.1P |
| ORL4956 | HsOR21.1.1P |
| ORL4957 | HsOR21.1.2P |

(continued)

| Name | Common Name |
|---|---|
| ORL4958 | HsOR21.2.1P |
| ORL4959 | HsORX.1.1P |
| ORL496 | hsORL496 |
| ORL4960 | HsORX.1.2P |
| ORL4961 | HsORX.1.3P |
| ORL4962 | HsORX.1.4P |
| ORL4963 | HsORX.1.6P |
| ORL4964 | HsORX.2.1P |
| ORL4965 | HsOR17.1.1 |
| ORL4966 | HsOR14.1.5 |
| ORL497 | hsORL497 |
| ORL498 | hsORL498 |
| ORL499 | hsORL499 |
| ORL50 | HTPCRX17 |
| ORL500 | hsORL500 |
| ORL501 | hsORL501 |
| ORL502 | hsORL502 |
| ORL504 | NCI_CGAP_Ut7 |
| ORL505 | |
| ORL506 | NP_058638.1 |
| ORL507 | hsORL507 |
| ORL508 | hsORL508 |
| ORL509 | NCI_CGAP_Co14 |
| ORL51 | HTPCRX19 |
| ORL510 | HPFH6OR |
| ORL511 | OR1D5 |
| ORL512 | OR1A1 |
| ORL513 | OR6A1 |
| ORL520 | OR3A1 |
| ORL521 | OR1D2 |
| ORL522 | Soares_NFL_T_GBC_S1 |
| ORL523 | OR12D2 |
| ORL524 | OR11A1 |
| ORL525 | OR10H1 |
| ORL526 | OR10C1 |
| ORL527 | OR10H3 |
| ORL528 | OR10H2 |
| ORL536 | hf30a07.x1 |

(continued)

| Name | Common Name |
|---|---|
| ORL589 | OR17-2 |
| ORL590 | OR17-228 |
| ORL591 | OR17-4 |
| ORL592 | OR17-23 |
| ORL593 | OR17-24 |
| ORL594 | OR17-40 |
| ORL671 | 6M1-3*02 |
| ORL672 | 6M1-7P*01 |
| ORL673 | 6M1-16*03 |
| ORL674 | 6M1-16*02 |
| ORL675 | 6M1-16*01 |
| ORL676 | 6M1-15*03 |
| ORL677 | 6M1-15*02 |
| ORL678 | 6M1-15*01 |
| ORL68 | OR17-23 |
| ORL680 | 6M1-10*02 |
| ORL681 | 6M1-10*01 |
| ORL682 | 6M1-6*03 |
| ORL683 | 6M1-6*02 |
| ORL684 | 6M1-6*01 |
| ORL685 | 6M1-02P*02 |
| ORL686 | 6M1-4P*04 |
| ORL687 | 6M1-4P*05 |
| ORL688 | 6M1-4P*03 |
| ORL689 | 6M1-4P*02 |
| ORL69 | OR17-24 |
| ORL690 | 6M1-4P*01 |
| ORL691 | 6M1-3*04 |
| ORL692 | 6M1-3*01 |
| ORL693 | 6M1-1*02 |
| ORL694 | 6M1-1*01 |
| ORL697 | 6M1-7P*02 |
| ORL70 | OR17-32 |
| ORL71 | OR17-82 |
| ORL72 | OR17-93 |
| ORL729 | 6M1-18*02 |
| ORL73 | OR17-207 |
| ORL732 | OR2A4 |

(continued)

| Name | Common Name |
|---|---|
| ORL735 | OR6A1 |
| ORL736 | OR5I1 |
| ORL737 | OR1D4 |
| ORL738 | OR1E2 |
| ORL739 | OR1E1 |
| ORL74 | OR17-201 |
| ORL740 | OR1A2 |
| ORL741 | OR1A1 |
| ORL742 | LOC82475 |
| ORL743 | OR12D2 |
| ORL75 | OR17-209 |
| ORL76 | OR17-210 |
| ORL77 | OR17-219 |
| ORL78 | OR17-2 |
| ORL79 | OR17-4 |
| ORL830 | LOC83361 |
| ORL869 | |
| ORL870 | hB2 |
| ORL871 | hP2 |
| ORL872 | hP4 |
| ORL873 | hP3 |
| ORL874 | hI7 |
| ORL875 | hT3 |
| ORL925 | OR51B2 |
| ORL929 | OR7A17 |
| ORL931 | OR10H2 |
| ORL932 | OR10H3 |
| ORL933 | OR1I1 |
| ORL934 | OR2B3 |
| ORL935 | OR2J3 |
| ORL936 | OR2J2 |
| ORL937 | OR7C1 |
| ORL938 | OR7A10 |
| ORL939 | OR2F2 |
| ORL940 | OR6B1 |
| ORL941 | OR4F3 |
| ORL942 | OR2A4 |
| ORL943 | OLFR89 |

(continued)

| Name | Common Name |
|---|---|
| ORL944 | OR2H2 |
| ORL946 | OR52A1 |
| ORL947 | |
| ORL948 | |
| ORL949 | DJ25J61 |
| ORL950 | OR17-1 |
| ORL993 | |
| ORL994 | |
| ORL995 | OR5U1 |
| ORL996 | OR5V1 |
| ORL997 | OR12D3 |
| ORL998 | OLFR |
| ORL999 | |

Table 6 Canine olfactory receptors, their gene names

| Name | Name | Name | Name |
|---|---|---|---|
| CfOLF1 | cOR1J6 | cOR52A13 | cOR6K5P |
| CfOLF2 | cOR1K2 | cOR52A14 | cOR6K7P |
| CfOLF3 | cOR1L5 | cOR52A15 | cOR6K8 |
| CfOLF4 | cOR1L8 | cOR52A16P | cOR6K9 |
| TPCR62 | cOR1L9 | cOR52A17 | cOR6M4 |
| TPCR63 | cOR1M1P | cOR52A6 | cOR6M5 |
| TPCR64 | cOR1M2 | cOR52A7 | cOR6M6 |
| TPCR71 | cOR1P1P | cOR52A8 | cOR6M7 |
| TPCR72 | cOR1P2 | cOR52A9 | cOR6M8 |
| TPCR79 | cOR1R4 | cOR52AA1P | cOR6n |
| DTMT | cOR1S3P | cOR52AB1 | cOR6N1 |
| DOPCRH01 | cOR1X2 | cOR52AB2 | cOR6P1 |
| DOPCRH02 | cOR2A13P | cOR52AB3 | cOR6Q2 |
| DOPCRH07 | cOR2A29 | cOR52AB4 | cOR6T2 |
| DOPCRX01 | cOR2A30 | cOR52AC1 | cOR6U3 |
| DOPCRX04 | cOR2A31 | cOR52AD1 | cOR6V2 |
| DOPCRX07 | cOR2A32 | cOR52AE1 | cOR6W1 |
| DOPCRX09 | cOR2A33 | cOR52B10P | cOR6Y3 |
| DOPCRX16 | cOR2A34P | cOR52B2 | cOR6Z1 |
| DTPCRH02 | cOR2A35 | cOR52B6 | cOR6Z2 |
| DTPCRH09 | cOR2A36 | cOR52B7 | cOR6Z3 |

(continued)

| Name | Name | Name | Name |
|---|---|---|---|
| OR4A16/HGPCR0945 | cOR2A37 | cOR52B8 | cOR7A21 |
| cOR7C50P | cOR2A38 | cOR52B9P | cOR7A22P |
| cOR7C49P | cOR2A39 | cOR52D1P | cOR7A23 |
| cOR7H8P | cOR2A40 | cOR52D2 | cOR7A24P |
| cOR13C22P | cOR2A7 | cOR52D3 | cOR7A25P |
| cOR5BW2P | cOR2AG1 | cOR52D4P | cOR7A26 |
| cOR13C20P | cOR2AG4P | cOR52E10P | cOR7A27 |
| cOR5AN4P | cOR2AG5P | cOR52E11P | cOR7A28 |
| cOR10Q4P | cOR2AG6 | cOR52E12 | cOR7C10P |
| cOR1302P | cOR2AG7 | cOR52E13 | cOR7C11 |
| cOR5L3P | cOR2AG8 | cOR52E14 | cOR7C12P |
| cOR10J17P | cOR2AG9 | cOR52E15P | cOR7C13 |
| cOR10J15P | cOR2AI2 | cOR52E16P | cOR7C14P |
| cOR2AG5P | cOR2AK3 | cOR52E17 | cOR7C15P |
| cOR1D9P | cOR2AT5P | cOR52E18 | cOR7C16 |
| cOR2AG4P | cOR2AT6 | cOR52E19P | cOR7C17 |
| cOR13N1P | cOR2AT7 | cOR52E2 | cOR7C18P |
| cOR5B22P | cOR2AT8P | cOR52E20P | cOR7C19 |
| cOR7C52 | cOR2AV1 | cOR52E4 | cOR7C20 |
| cOR4Z5 | cOR2AV2 | cOR52E8 | cOR7C21 |
| cOR7D10 | cOR2AV3 | cOR52E9 | cOR7C22 |
| cOR1E12 | cOR2AX1P | cOR52H1 | cOR7C23P |
| cOR4X6 | cOR2AX2 | cOR52H10P | cOR7C24 |
| cOR7G14 | cOR2AZ1 | cOR52H11 | cOR7C25 |
| cOR7H9 | cOR2B10P | cOR52H2P | cOR7C26 |
| cOR5F3 | cOR2B2P | cOR52H3P | cOR7C27 |
| cOR9I5 | cOR2B7P | cOR52H4 | cOR7C28 |
| cOR4Z4 | cOR2B9 | cOR52H5 | cOR7C29P |
| cOR5M22 | cOR2BA1P | cOR52H6 | cOR7C3 |
| cOR9S20 | cOR2C1 | cOR52H7 | cOR7C30 |
| cOR2M12 | cOR2C6 | cOR52H8 | cOR7C31 |
| cOR2L19 | cOR2D10P | cOR52H9 | cOR7C32 |
| cOR7C46 | cOR2D2 | cOR52I2 | cOR7C33P |
| cOR4H14 | cOR2D4 | cOR52J5 | cOR7C34 |
| cOR13C21 | cOR2D5P | cOR52J6P | cOR7C35 |
| cOR7C45 | cOR2D6 | cOR52J7 | cOR7C36 |
| cOR7C44 | cOR2D7P | cOR52J8 | cOR7C37 |
| cOR5D23 | cOR2D8 | cOR52J9P | cOR7C38 |

(continued)

| Name | Name | Name | Name |
|---|---|---|---|
| cOR4K23 | cOR2D9 | cOR52K4 | cOR7C39 |
| cOR8C6 | cOR2G4 | cOR52K5 | cOR7C4 |
| cOR5L7 | cOR2G5 | cOR52K6 | cOR7C40 |
| cOR2A40 | cOR2H8 | cOR52L3 | cOR7C41 |
| cOR11M3 | cOR2H9P | cOR52M1P | cOR7C42 |
| cOR7H7 | cOR2K2 | cOR52M5 | cOR7C43 |
| cOR7C43 | cOR2L15P | cOR52M6P | cOR7C44 |
| cOR3A13 | cOR2L16 | cOR52N10 | cOR7C45 |
| cOR10J21 | cOR2L17 | cOR52N11 | cOR7C46 |
| cOR3A12 | cOR2L18 | cOR52N12P | cOR7C47 |
| cOR8S16 | cOR2L19 | cOR52N2P | cOR7C48 |
| cOR8J6 | cOR2M10 | cOR52N6P | cOR7C49P |
| cOR7C40 | cOR2M11 | cOR52N7P | cOR7C50P |
| cOR2A36 | cOR2M12 | cOR52N8 | cOR7C51 |
| cOR7C39 | cOR2M8 | cOR52N9 | cOR7C52 |
| cOR7H6 | cOR2M9P | cOR52P1P | cOR7C53 |
| cOR12F3 | cOR2Q1P | cOR52P2P | cOR7C5P |
| cOR7H4 | cOR2S3P | cOR52P3 | cOR7C6 |
| cOR2AY1 | cOR2T1 | cOR52R2 | cOR7C7 |
| cOR2AG6 | cOR2T13 | cOR52R3P | cOR7C8 |
| cOR2A31 | cOR2T14P | cOR52S2 | cOR7C9P |
| cOR7C14 | cOR2T15 | cOR52S3 | cOR7D10 |
| cOR10J16 | cOR2T16P | cOR52S4P | cOR7D4P |
| cOR7H2 | cOR2T17 | cOR52S5 | cOR7D5 |
| cOR7C13 | cOR2T18P | cOR52U2 | cOR7D7 |
| cOR10A9 | cOR2T19 | cOR52U3P | cOR7D8 |
| cOR2D6 | cOR2T20 | cOR52V2 | cOR7D9P |
| cOR7A21 | cOR2T21 | cOR52W2 | cOR7E152 |
| cOR10J13 | cOR2T22 | cOR52X2 | cOR7E153 |
| cOR1D7 | cOR2T23 | cOR52X3 | cOR7E154 |
| cOR1L9 | cOR2T24 | cOR52Z2 | cOR7G10 |
| cOR4Z1 | cOR2T25 | cOR52Z3 | cOR7G11 |
| cOR7C4 | cOR2T26 | cOR52Z4 | cOR7G12 |
| cOR13D4 | cOR2V4 | cOR52Z5 | cOR7G13 |
| cOR7C3 | cOR2W10 | cOR55B3 | cOR7G14 |
| cOR7G4 | cOR2W11 | cOR55D1 | cOR7G4 |
| CfOLF4 | cOR2W12 | cOR56A10 | cOR7G5 |
| CfOLF3 | cOR2W13P | cOR56A11 | cOR7G6 |

(continued)

| Name | Name | Name | Name |
|---|---|---|---|
| CfOLF2 | cOR2W14 | cOR56A12 | cOR7G7 |
| CfOLF1 | cOR2W15 | cOR56A13P | cOR7G8 |
| cOR10A10 | cOR2W16P | cOR56A14 | cOR7G9 |
| cOR10A11P | cOR2W9 | cOR56A15 | cOR7H2 |
| cOR10A12P | cOR2Y2 | cOR56A16 | cOR7H3P |
| cOR10A13 | cOR2Z2 | cOR56A17 | cOR7H4 |
| cOR10A14 | cOR2Z3 | cOR56A18 | cOR7H5P |
| cOR10A3 | cOR2Z4 | cOR56A19P | cOR7H6 |
| cOR10A4P | cOR3A10 | cOR56A20 | cOR7H7 |
| cOR10A5 | cOR3A11 | cOR56A21P | cOR7H8P |
| cOR10A4P | cOR3A12 | cOR56A22 | cOR7H9 |
| cOR10A8P | cOR3A13 | cOR56A23 | cOR7P1 |
| cOR10A5 | cOR3A9 | cOR56A24 | cOR7R1 |
| cOR10A9 | cOR3n | cOR56A4 | cOR8A1P |
| cOR10A8P | cOR4A26 | cOR56A6 | cOR8B14 |
| cOR10A9 | cOR4A27 | cOR56A8 | cOR8B15 |
| cOR10AB2 | cOR4A28 | cOR56A9 | cOR8B16 |
| cOR10AD1 | cOR4A29 | cOR56B10P | cOR8B17 |
| cOR10AD2 | cOR4A30 | cOR56B11 | cOR8B18 |
| cOR10AD3 | cOR4A31P | cOR56B12P | cOR8B19 |
| cOR10AG2P | cOR4A32P | cOR56B2 | cOR8B1P |
| cOR10AH1P | cOR4A33P | cOR56B5 | cOR8B20 |
| cOR10AI1 | cOR4A34 | cOR56B6 | cOR8B21 |
| cOR10AJ1P | cOR4A35 | cOR56B7 | cOR8B3 |
| cOR10B1P | cOR4A36 | cOR56B8P | cOR8B8 |
| cOR10D1P | cOR4A37P | cOR56B9P | cOR8C4 |
| cOR10D4P | cOR4A38 | cOR5A2 | cOR8C5 |
| cOR10D5P | cOR4A39 | cOR5A3 | cOR8C6 |
| cOR10D7 | cOR4A4P | cOR5A4P | cOR8D2P |
| cOR10D8 | cOR4B1 | cOR5AC3 | cOR8D4 |
| cOR10D9 | cOR4B3P | cOR5AK6 | cOR8D5 |
| cOR10G11 | cOR4B4 | cOR5AK7 | cOR8D6 |
| cOR10G12 | cOR4C11P | cOR5AL1P | cOR8F2 |
| cOR10G13P | cOR4C18 | cOR5AL3 | cOR8F3 |
| cOR10G11 | cOR4C19 | cOR5AN2P | cOR8F4 |
| cOR10G7 | cOR4C1P | cOR5AN3 | cOR8G8P |
| cOR10H10 | cOR4C20P | cOR5AN4P | cOR8G9P |
| cOR10G12 | cOR4C21 | cOR5AP3 | cOR8H4 |

(continued)

| Name | Name | Name | Name |
|---|---|---|---|
| cOR10G13P | cOR4C22P | cOR5AP4P | cOR8I3P |
| cOR10G7 | cOR4C23P | cOR5AR1P | cOR8J4 |
| cOR10H10 | cOR4C24 | cOR5B22P | cOR8J5 |
| cOR10H11P | cOR4C25P | cOR5B23 | cOR8J6 |
| cOR10H12P | cOR4C26 | cOR5B24 | cOR8J7 |
| cOR10H13 | cOR4C27 | cOR5B25 | cOR8K1 |
| cOR10H14P | cOR4C28 | cOR5B26 | cOR8K6P |
| cOR10H6P | cOR4C29 | cOR5B27P | cOR8S10 |
| cOR10H7 | cOR4C3 | cOR5B28 | cOR8S11 |
| cOR10H8 | cOR4C30 | cOR5B29 | cOR8S12 |
| cOR10H9 | cOR4C31 | cOR5B30P | cOR8S13 |
| cOR10J10P | cOR4C32 | cOR5B31 | cOR8S14 |
| cOR10J11P | cOR4C33P | cOR5B32 | cOR8S15 |
| cOR10J12 | cOR4C34 | cOR5BA2 | cOR8S16 |
| cOR10J13 | cOR4C35 | cOR5BC2 | cOR8S17 |
| cOR10J14 | cOR4C36 | cOR5BC3 | cOR8S18P |
| cOR10J15P | cOR4C37 | cOR5BG2 | cOR8S19P |
| cOR10J16 | cOR4C38 | cOR5BH3 | cOR8S20 |
| cOR10J17P | cOR4C39P | cOR5BU2 | cOR8S2P |
| cOR10J18P | cOR4C40 | cOR5BV1P | cOR8S3P |
| cOR10J19 | cOR4C41P | cOR5BW1P | cOR8S4 |
| cOR10J20 | cOR4C42 | cOR5BW2P | cOR8S5 |
| cOR10J21 | cOR4C43 | cOR5C1G | cOR8S6P |
| cOR10J22 | cOR4C44 | cOR5D14 | cOR8S7 |
| cOR10J23 | cOR4D11P | cOR5D19 | cOR8S8 |
| cOR10J7P | cOR4D13 | cOR5D20 | cOR8S9 |
| cOR10K2 | cOR4D14P | cOR5D21 | cOR8T2 |
| cOR10K3 | cOR4D15 | cOR5D22 | cOR8T3P |
| cOR10K4 | cOR4D2P | cOR5D23 | cOR8T4 |
| cOR10n | cOR4D5 | cOR5E1P | cOR8T5 |
| cOR10N1P | cOR4E1P | cOR5F3 | cOR8U2 |
| cOR10P4P | cOR4E3P | cOR5G1P | cOR8U3 |
| cOR10Q1 | cOR4F22 | cOR5G3P | cOR8U4P |
| cOR10Q4P | cOR4F23P | cOR5G7P | cOR8U5 |
| cOR10Q3 | cOR4F24P | cOR5G8P | cOR8U6 |
| cOR10Q5 | cOR4F25 | cOR5G9 | cOR8U7 |
| cOR10R4 | cOR4F26P | cOR5H10 | cOR8V10 |
| cOR10R5 | cOR4F27P | cOR5H11 | cOR8V11 |

(continued)

| Name | Name | Name | Name |
|---|---|---|---|
| cOR10R6P | cOR4G10 | cOR5H12 | cOR8V2 |
| cOR10R7 | cOR4G7P | cOR5H13P | cOR8V3 |
| cOR10S2P | cOR4G8 | cOR5H9 | cOR8V4 |
| cOR10T3 | cOR4G9 | cOR5I1 | cOR8V5 |
| cOR10T4P | cOR4H13 | cOR5I2 | cOR8V6 |
| cOR10V4P | cOR4H14 | cOR5J1P | cOR8V7P |
| cOR10V5 | cOR4K15P | cOR5J3 | cOR8V8P |
| cOR10V6 | cOR4K18 | cOR5J4 | cOR8V9 |
| cOR10X2 | cOR4K19P | cOR5K5 | cOR9A7 |
| cOR10Z1 | cOR4K20 | cOR5K6 | cOR9A8 |
| cOR11G10 | cOR4K21P | cOR5K7 | cOR9G1 |
| cOR11G11 | cOR4K22 | cOR5L1P | cOR9G4 |
| cOR11G1P | cOR4K23 | cOR5L3P | cOR9G7 |
| cOR11G3P | cOR4K24 | cOR5L4P | cOR9G8P |
| cOR11G4 | cOR4K6P | cOR5L5 | cOR9I2P |
| cOR11G5P | cOR4L1 | cOR5L6P | cOR9I4P |
| cOR11G6 | cOR4L3P | cOR5L7 | cOR9I5 |
| cOR11G7 | cOR4L4 | cOR5M12P | cOR9K3 |
| cOR11G8 | cOR4M3P | cOR5M13P | cOR9K4 |
| cOR11G9P | cOR4M3P | cOR5M17P | cOR9K5P |
| cOR11H10 | cOR4N5 | cOR5M16 | cOR9K6 |
| cOR11H11P | cOR4N6 | cOR5M18P | cOR9Q3 |
| cOR11H7P | cOR4P10 | cOR5M19P | cOR9R2 |
| cOR11H8 | cOR4P5P | cOR5M20 | cOR9R3P |
| cOR11H9 | cOR4P6 | cOR5M21 | cOR9R4 |
| cOR11I3 | cOR4P7 | cOR5M22 | cOR9S10 |
| cOR11J3 | cOR4P8 | cOR5M8 | cOR9S11 |
| cOR11J4 | cOR4P9 | cOR5P4P | cOR9S12 |
| cOR11K3 | cOR4Q4 | cOR5P5 | cOR9S13 |
| cOR11K4 | cOR4Q5 | cOR5P6P | cOR9S14 |
| cOR11L2 | cOR4Q6 | cOR5R2 | cOR9S15 |
| cOR11M2 | cOR4Q7 | cOR5T4 | cOR9S16 |
| cOR11M3 | cOR4S3 | cOR5T5 | cOR9S17 |
| cOR11S1 | cOR4S4 | cOR5T6 | cOR9S18 |
| cOR11S2 | cOR4S5 | cOR5T7 | cOR9S19 |
| cOR12E1 | cOR4S6 | cOR5W4 | cOR9S1P |
| cOR12E2 | cOR4S7P | cOR5W5 | cOR9S2 |
| cOR12E3 | cOR4T2P | cOR5W6 | cOR9S20 |

(continued)

| Name | Name | Name | Name |
|---|---|---|---|
| cOR12E4P | cOR4X3 | cOR5W7 | cOR9S21P |
| cOR12E5 | cOR4X4 | cOR5W8 | cOR9S22P |
| cOR12E7P | cOR4X5P | cOR6A2P | cOR9S23 |
| cOR12E8 | cOR4X6 | cOR6AA1P | cOR9S3P |
| cOR12F1 | cOR4Y1 | cOR6AB1P | cOR9S4 |
| cOR12F2P | cOR4Y2 | cOR6B4 | cOR9S5P |
| cOR12G1 | cOR4Y3P | cOR6B5P | cOR9S6 |
| cOR12H1P | cOR4Y4 | cOR6B6 | cOR9S7P |
| cOR12J1 | cOR4Y5 | cOR6B7 | cOR9S8P |
| cOR13C10 | cOR4Z1 | cOR6B8 | cOR9S9P |
| cOR13C11 | cOR4Z2 | cOR6C10P | |
| cOR13C12 | cOR4Z3 | cOR6C11 | |
| cOR13C13P | cOR4Z4 | cOR6C12 | |
| cOR13C14 | cOR4Z5 | cOR6C13P | |
| cOR13C15 | cOR51A14P | cOR6C14 | |
| cOR13C16 | cOR51A15P | cOR6C15 | |
| cOR13C17 | cOR51A16 | cOR6C16P | |
| cOR13C18 | cOR51A17 | cOR6C17 | |
| cOR13C19 | cOR51A18 | cOR6C18P | |
| cOR13C20P | cOR51A19 | cOR6C19P | |
| cOR13C21 | cOR51A20P | cOR6C20P | |
| cOR13C22P | cOR51A21 | cOR6C21 | |
| cOR13C23 | cOR51AA1 | cOR6C22 | |
| cOR13C9 | cOR51B10 | cOR6C23 | |
| cOR13D1 | cOR51B4 | cOR6C25 | |
| cOR13D4 | cOR51B7 | cOR6C27 | |
| cOR13D5 | cOR51B8P | cOR6C26 | |
| cOR13D6P | cOR51B9 | cOR6C28 | |
| cOR13D7P | cOR51C4 | cOR6C29 | |
| cOR13E3 | cOR51C5 | cOR6C30 | |
| cOR13F2P | cOR51C6P | cOR6C31 | |
| cOR13F3 | cOR51C7P | cOR6C32P | |
| cOR13F4 | cOR51D2P | cOR6C33 | |
| cOR13G1 | cOR51E2P | cOR6C34P | |
| cOR13L1 | cOR51E4 | cOR6C35 | |
| cOR13L2 | cOR51F2P | cOR6C36 | |
| cOR13M1 | cOR51F2P | cOR6C37 | |
| cOR13M2P | cOR51G2 | cOR6C38 | |

(continued)

| Name | Name | Name | Name |
|---|---|---|---|
| cOR13M3 | cOR51G4 | cOR6C39P | |
| cOR13M4 | cOR51H3 | cOR6C4 | |
| cOR13N1P | cOR51H4 | cOR6C40P | |
| cOR13N2 | cOR51H5 | cOR6C41P | |
| cOR13N3P | cOR51I1P | cOR6C42P | |
| cOR13N4 | cOR51I2 | cOR6C43 | |
| cOR13N5 | cOR51I3 | cOR6C44P | |
| cOR13P1 | cOR51J3 | cOR6C45P | |
| cOR13P2P | cOR51K1P | cOR6C46 | |
| cOR13P3 | cOR51I4P | cOR6C47P | |
| cOR13P4 | cOR51K2 | cOR6C48P | |
| cOR13P5 | cOR51L2 | cOR6C49P | |
| cOR13Q1P | cOR51L2 | cOR6C50P | |
| cOR13Q2P | cOR51M1 | cOR6C51 | |
| cOR13Q3 | cOR51P3 | cOR6C52P | |
| cOR13R1 | cOR51Q1P | cOR6C53P | |
| cOR13R2 | cOR51Q2P | cOR6C54P | |
| cOR13S1P | cOR51Q3 | cOR6C55 | |
| cOR1A3P | cOR51R2 | cOR6C56P | |
| cOR1AB2 | cOR51T2 | cOR6C57P | |
| cOR1AB3 | cOR51V2 | cOR6C58P | |
| cOR1AD1 | cOR51V3 | cOR6C59P | |
| cOR1AE1 | cOR51V4 | cOR6C5P | |
| cOR1AF1 | cOR51V5P | cOR6C6 | |
| cOR1AG1P | cOR51V5P | cOR6C60 | |
| cOR1D10 | cOR51V6 | cOR6C61P | |
| cOR1D11P | cOR51V6 | cOR6C62 | |
| cOR1D12 | cOR51V7 | cOR6C63 | |
| cOR1D7 | cOR51W1 | cOR6C7 | |
| cOR1D8 | cOR51X1 | cOR6C8 | |
| cOR1D9P | cOR51X2 | cOR6C9 | |
| cOR1E10 | cOR51X3P | cOR6D3P | |
| cOR1E11 | cOR51X4 | cOR6D4 | |
| cOR1E12 | cOR51Z1P | cOR6D5 | |
| cOR1F14P | cOR52A10 | cOR6D6P | |
| cOR1F15 | cOR52A11 | cOR6D7P | |
| cOR1I2 | cOR52A12 | cOR6K2P | |

Table 7 Mosquito olfactory receptors, gene symbols

| Gene Symbol | Gene Symbol |
|---|---|
| GPRor53 | GPRor18 |
| GPRor54 | GPRor19 |
| GPRor55 | GPRor20 |
| GPRor56 | GPRor21 |
| GPRor57 | GPRor22 |
| GPRor58 | GPRor23 |
| GPRor59 | GPRor24 |
| GPRor60 | GPRor25 |
| GPRor61 | GPRor26 |
| GPRor62 | GPRor27 |
| GPRor63 | GPRor28 |
| GPRor64 | GPRor29 |
| GPRor65 | GPRor30 |
| GPRor66 | GPRor31 |
| GPRor67 | GPRor32 |
| GPRor68 | GPRor33 |
| GPRor69 | GPRor34 |
| GPRor70 | GPRor35 |
| GPRor71 | GPRor36 |
| GPRor72 | GPRor37 |
| GPRor73 | GPRor38 |
| GPRor74 | GPRor39 |
| GPRor75 | GPRor40 |
| GPRor76 | GPRor41 |
| GPRor77 | GPRor42 |
| GPRor78 | GPRor43 |
| GPRor79 | GPRor44 |
| GPRor12 | GPRor45 |
| GPRor1 | GPRor46 |
| GPRor2 | GPRor47 |
| GPRor3 | GPRor48 |
| GPRor4 | GPRor49 |
| GPRor5 | GPRor50 |
| GPRor6 | GPRor51 |
| GPRor7 | GPRor52 |
| GPRor8 | |
| GPRor9 | |

(continued)

| Gene Symbol | Gene Symbol |
|---|---|
| GPRor10 | |
| GPRor11 | |
| GPRor13 | |
| GPRor14 | |
| GPRor15 | |
| GPRor16 | |
| GPRor17 | |

Table 8 Other heteromultimeric receptors, gene name, NCBI gene ID numbers and related synonyms

| Type | Subunit | Gene | NCBI Gene ID | Synonyms |
|---|---|---|---|---|
| GABAA | gamma-aminobutyric acid (GABA) A receptor, alpha 1 | *GABRA 1* | 2554 | ECA4, EJM, GABA(A) receptor, GABA(A) receptor subunit alpha-1, Gamma-aminobutyric-acid receptor alpha-1 subunit precursor, Gamma-aminobutyric-acid receptor subunit alpha-1 precursor |
| | gamma-aminobutyric acid (GABA) A receptor, alpha 2 | *GABRA 2* | 2555 | GABA(A) receptor, GABA(A) receptor subunit alpha-2, Gamma-aminobutyric-acid receptor alpha-2 subunit precursor, Gamma-aminobutyric-acid receptor subunit alpha-2 precursor |
| | gamma-aminobutyric acid (GABA) A receptor, alpha 3 | *GABRA 3* | 2556 | GABA(A) receptor, GABA(A) receptor subunit alpha-3, Gamma-aminobutyric-acid receptor alpha-3 subunit precursor, Gamma-aminobutyric-acid receptor subunit alpha-3 precursor |
| | gamma-aminobutyric acid (GABA) A receptor, alpha 4 | *GABRA 4* | 2557 | GABA(A) receptor, GABA(A) receptor subunit alpha-4, Gamma-aminobutyric-acid receptor alpha-4 subunit precursor, Gamma-aminobutyric-acid receptor subunit alpha-4 precursor |
| | gamma-aminobutyric acid (GABA) A receptor, alpha 5 | *GABRA 5* | 2558 | GABA(A) receptor, GABA(A) receptor subunit alpha-5, Gamma-aminobutyric-acid receptor alpha-5 subunit precursor, Gamma-aminobutyric-acid receptor subunit alpha-5 precursor |

(continued)

| Type | Subunit | Gene | NCBI Gene ID | Synonyms |
|---|---|---|---|---|
| | gamma-aminobutyric acid (GABA) A receptor, alpha 6 | *GABRA 6* | 2559 | GABA(A) receptor, GABA(A) receptor subunit alpha-6, Gamma-aminobutyric-acid receptor alpha-6 subunit precursor, Gamma-aminobutyric-acid receptor subunit alpha-6 precursor, MGC116903, MGC116904 |
| | gamma-aminobutyric acid (GABA) A receptor, beta 1 | *GABRB 1* | 2560 | GABA(A) receptor, GABA(A) receptor subunit beta-1, Gamma-aminobutyric-acid receptor beta-1 subunit precursor, Gamma-aminobutyric-acid receptor subunit beta-1 precursor |
| | gamma-aminobutyric acid (GABA) A receptor, beta2 | *GABRB 2* | 2561 | GABA(A) receptor, GABA(A) receptor subunit beta-2, Gamma-aminobutyric-acid receptor beta-2 subunit precursor, Gamma-aminobutyric-acid receptor subunit beta-2 precursor, MGC119386, MGC119388, MGC119389 |
| | gamma-aminobutyric acid (GABA) A receptor, beta3 | *GABRB 3* | 2562 | GABA(A) receptor, GABA(A) receptor subunit beta-3, Gamma-aminobutyric-acid receptor beta-3 subunit precursor, Gamma-aminobutyric-acid receptor subunit beta-3 precursor |
| | gamma-aminobutyric acid (GABA) A receptor, gamma1 | *GABRG 1* | 2565 | DKFZp686H2042, GABA(A) receptor, GABA(A) receptor subunit gamma-1, Gamma-aminobutyric-acid receptor gamma-1 subunit precursor, Gamma-aminobutyric-acid receptor subunit gamma-1 precursor, MGC33838 |
| | gamma-aminobutyric acid (GABA) A receptor, gamma2 | *GABRG 2* | 2566 | CAE2, ECA2, GABA(A) receptor, GABA(A) receptor subunit gamma-2, Gamma-aminobutyric-acid receptor gamma-2 subunit precursor, Gamma-aminobutyric-acid receptor subunit gamma-2 precursor, GEFSP3 |

(continued)

| Type | Subunit | Gene | NCBI Gene ID | Synonyms |
|---|---|---|---|---|
| | gamma-aminobutyric acid (GABA) A receptor, gamma3 | *GABRG 3* | 2567 | GABA(A) receptor, GABA(A) receptor subunit gamma-3, Gamma-aminobutyric-acid receptor gamma-3 subunit precursor, Gamma-aminobutyric-acid receptor subunit gamma-3 precursor |
| | gamma-aminobutyric acid (GABA) A receptor, delta | *GABRD* | 2563 | GABA(A) receptor, GABA(A) receptor subunit delta, Gamma-aminobutyric-acid receptor delta subunit precursor, Gamma-aminobutyric-acid receptor subunit delta precursor |
| | gamma-aminobutyric acid (GABA) A receptor, epsilon | *GABRE* | 2564 | GABA(A) receptor, GABA(A) receptor subunit epsilon, Gamma-aminobutyric-acid receptor epsilon subunit precursor, Gamma-aminobutyric-acid receptor subunit epsilon precursor |
| | gamma-aminobutyric acid (GABA) A receptor, pi | *GABRP* | 2568 | GABA(A) receptor, GABA(A) receptor subunit pi, Gamma-aminobutyric-acid receptor pi subunit precursor, Gamma-aminobutyric-acid receptor subunit pi precursor, MGC126386, MGC126387 |
| | gamma-aminobutyric acid (GABA) Areceptor, theta | *GABRQ* | 5587 9 | GABA(A) receptor, GABA(A) receptor subunit theta, Gamma-aminobutyric-acid receptor subunit theta precursor, Gamma-aminobutyric-acid receptor theta subunit precursor, MGC129629, MGC129630, THETA |
| GABAC | gamma-aminobutyric acid (GABA) receptor, rho 1 | *GABRR 1* | 2569 | GABA(A) receptor, GABA(A) receptor subunit rho-1, Gamma-aminobutyric-acid receptor rho-1 subunit precursor, Gamma-aminobutyric-acid receptor subunit rho-1 precursor H |

(continued)

| Type | Subunit | Gene | NCBI Gene ID | Synonyms |
|---|---|---|---|---|
| | gamma-aminobutyric acid (GABA) receptor, rho 2 | *GABRR 2* | 2570 | GABA(A) receptor, GABA(A) receptor subunit rho-2, Gamma-aminobutyric-acid receptor rho-2 subunit precursor, Gamma-aminobutyric-acid receptor subunit rho-2 precursor |
| | gamma-aminobutyric acid (GABA) receptor, rho 3 | *GABRR 3* | 2009 59 | gamma-aminobutyric acid (GABA) receptor, rho 3 |
| | | | | |
| nAChR | cholinergic receptor, nicotinic, alpha 1 (muscle) | *CHRNA 1* | 1134 | Acetylcholine receptor protein, alpha subunit precursor, Acetylcholine receptor subunit alpha precursor, ACHRA, ACHRD, CHNRA, CHRNA, CMS2A, FCCMS, SCCMS |
| | cholinergic receptor, nicotinic, alpha 1 (muscle) | | 1134 | Acetylcholine receptor protein, alpha subunit precursor, Acetylcholine receptor subunit alpha precursor, ACHRA ACHRD, CHNRA, CHRNA, CMS2A, FCCMS, SCCMS |
| | cholinergic receptor, nicotinic, alpha 2 (neuronal) | *CHRNA 2* | 1135 | Neuronal acetylcholine receptor protein, alpha-2 subunit precursor, Neuronal acetylcholine receptor subunit alpha-2 precursor |
| | cholinergic receptor, nicotinic, alpha 3 | *CHRNA 3* | 1136 | LNCR2, MGC104879, NACHRA3, Neuronal acetylcholine receptor protein, alpha-3 subunit precursor, Neuronal acetylcholine receptor subunit alpha-3 precursor, PAOD2 |
| | cholinergic receptor, nicotinic, alpha 4 | *CHRNA 4* | 1137 | BFNC, EBN, EBN1, FLJ95812, NACHR, NACHRA4, NACRA4, Neuronal acetylcholine receptor protein, alpha-4 subunit precursor, Neuronal acetylcholine receptor subunit alpha-4 precursor |
| | cholinergic receptor, nicotinic, alpha 5 | *CHRNA 5* | 1138 | NACHRA5, Neuronal acetylcholine receptor protein, alpha-5 subunit precursor, Neuronal acetylcholine receptor subunit alpha-5 precursor |

(continued)

| Type | Subunit | Gene | NCBI Gene ID | Synonyms |
|---|---|---|---|---|
| | cholinergic receptor, nicotinic, alpha 6 | *CHRNA 6* | 8973 | Neuronal acetylcholine receptor protein, alpha-6 subunit precursor, Neuronal acetylcholine receptor subunit alpha-6 precursor |
| | cholinergic receptor, nicotinic, alpha 7 | *CHRNA 7* | 1139 | CHRNA7-2, NACHRA7, Neuronal acetylcholine receptor protein, alpha-7 subunit precursor, Neuronal acetylcholine receptor subunit alpha-7 precursor |
| | cholinergic receptor, nicotinic, alpha | *CHRNA 9* | 5558 4 | HSA243342, MGC142109, MGC142135, NACHRA9, NACHR alpha 9, Neuronal acetylcholine receptor protein, alpha-9 subunit precursor, Neuronal acetylcholine receptor subunit alpha-9 precursor, Nicotinic acetylcholine receptor subunit alpha 9 |
| | cholinergic receptor, nicotinic, alpha 10 | *CHRNA 10* | 5705 3 | NACHRA10, NACHR alpha 10, Neuronal acetylcholine receptor protein, alpha-10 subunit precursor, Neuronal acetylcholine receptor subunit alpha-10 precursor, Nicotinic acetylcholine receptor subunit alpha 10 |
| | cholinergic receptor, nicotinic, beta 1 (muscle) | *CHRNB 1* | 1140 | Acetylcholine receptor protein, beta subunit precursor, Acetylcholine receptor subunit beta precursor, ACHRB, CHRNB, CMS1D, CMS2A, SCCMS |
| | cholinergic receptor, nicotinic, beta 2 (neuronal) | *CHRNB 2* | 1141 | EFNL3, nAChRB2, Neuronal acetylcholine receptor protein, beta-2 subunit precursor, Neuronal acetylcholine receptor subunits beta-2 precursor |
| | cholinergic receptor, nicotinic, beta 3 | *CHRNB 3* | 1142 | acetylcholine receptor protein, beta-3 subunit precursor, Neuronal acetylcholine receptor, subunit beta-3 precursor |

(continued)

| Type | Subunit | Gene | NCBI Gene ID | Synonyms |
|---|---|---|---|---|
| | cholinergic receptor, nicotinic, beta 4 | *CHRNB 4* | 1143 | Neuronal acetylcholine receptor protein, beta-4 subunit precursor, Neuronal acetylcholine receptor subunit beta-4 precursor |
| | cholinergic receptor, nicotinic, gamma | *CHRNG* | 1146 | Acetylcholine receptor protein, gamma subunit precursor, Acetylcholine receptor subunit gamma precursor, ACHRG, MGC133376 |
| | cholinergic receptor, nicotinic, delta | *CNRND* | 1144 | receptor protein, delta subunit precursor, Acetylcholine receptor subunit delta precursor, ACHRD, CMS2A, FCCMS, SCCMS |
| | cholinergic receptor, nicotinic, epsilon | *CHRNE* | 1145 | Acetylcholine receptor protein, epsilon subunit precursor, Acetylcholine receptor subunit epsilon precursor, ACHRE, CMS1D, CMS1E, CMS2A, FCCMS, SCCMS |
| | | | | |
| 5-HT3 | 5-hydroxytryptamine (serotonin) receptor 3A | *HTR3A* | 3359 | 5-HT-3, 5-HT3A, 5HT3R, 5-HT3R, 5-hydroxytryptamine 3 receptor precursor, HTR3, Serotonin-gated ion channel receptor |
| | 5-hydroxytryptamine (serotonin) receptor 3B | *HTR3B* | 9177 | 5-HT3B |
| | 5-hydroxytryptamine (serotonin) receptor 3C | *HTR3C* | 170572 | none |
| | 5-hydroxytryptamine (serotonin) receptor 3D | *HTR3D* | 200909 | MGC119636, MGC119637 |
| | 5-hyd roxytrypta mine (serotonin) receptor 3E | *HTR3E* | 285242 | 5-HT3c1, MGC120035, MGC120036, MGC120037 |
| | | | | |

(continued)

| Type | Subunit | Gene | NCBI Gene ID | Synonyms |
|---|---|---|---|---|
| Glycine (GlyR) | glycine receptor, alpha 1 | *GLRA1* | 2741 | glycine receptor, alpha 1 (startle disease/hyperekplexia, stiff man syndrome), Glycine receptor 48 kDa subunit, Glycine receptor alpha-1 chain precursor, Glycine receptor strychnine-binding subunit, Glycine receptor subunit alpha-1 precursor, MGC138878, MGC138879, STHE, Strychnine-binding subunit |
| | glycine receptor, alpha 2 | *GLRA2* | 2742 | Glycine receptor alpha-2 chain precursor, Glycine receptor subunit alpha-2 precursor |
| | glycine receptor, alpha 3 | *GLRA3* | 8001 | Glycine receptor alpha-3 chain precursor, Glycine receptor subunit alpha-3 precursor |
| | glycine receptor, alpha 4 | *GLRA4* | 441509 | none |
| | glycine receptor, beta | *GLRB* | 2743 | Glycine receptor 58 kDa subunit, Glycine receptor beta chain precursor, Glycine receptor subunit beta precursor |
| | | | | |
| Glutamate receptors: | glutamate receptor, ionotropic, AMPA 1 | *GRIA1* | 2890 | AMPA-selective glutamate receptor 1, GLUH1, GLUR1, GluR-1, GLURA, GluR-A, GluR-K1, Glutamate receptor 1 precursor, Glutamate receptor ionotropic, AMPA 1, HBGR1, MGC133252 |
| | glutamate receptor, ionotropic, AMPA 2 | *GRIA2* | 2891 | AMPA-selective glutamate receptor 2, GLUR2, GluR-2, GLURB, GluRB, GluR-K2, Glutamate receptor 2 precursor, Glutamate receptor ionotropic, AMPA 2, HBGR2 |
| | glutamate receptor, ionotrophic, AMPA 3 | *GRIA3* | 2892 | AMPA-selective glutamate receptor 3, GLUR3, GluR-3, GLURC, gluR-C, GluR-C, GLUR-C, GluR-K3, GLUR-K3, Glutamate receptor 3 precursor, Glutamate receptor ionotropic, AMPA 3, MRX94 |

(continued)

| Type | Subunit | Gene | NCBI Gene ID | Synonyms |
|---|---|---|---|---|
| | glutamate receptor, ionotrophic, AMPA 4 | *GRIA4* | 2893 | AMPA-selective glutamate receptor 4, GluR4, GLUR4, GluR-4, GLUR4C, GLURD, GluR-D, Glutamate receptor 4 precursor, Glutamate receptor ionotropic, AMPA 4 |
| | glutamate receptor, ionotropic, kainate 1 | *GRIK1* | 2897 | EAA3, EEA3, Excitatory amino acid receptor 3, GLR5, GluR5, GLUR5, GluR-5, Glutamate receptor, ionotropic kainate 1 precursor, Glutamate receptor 5 |
| | glutamate receptor, ionotropic, kainate 2 | *GRIK2* | 2898 | EAA4, Excitatory amino acid receptor 4, GLR6, GLUK6, GluR6, GLUR6, GluR-6, Glutamate receptor, ionotropic kainate 2 precursor, Glutamate receptor 6, MGC74427, MRT6 |
| | glutamate receptor, ionotropic, kainate 3 | *GRIK3* | 2899 | EAA5, Excitatory amino acid receptor 5, GLR7, GluR7, GLUR7, GluR-7, GluR7a, Glutamate receptor, ionotropic kainate 3 precursor, Glutamate receptor 7 |
| | glutamate receptor, ionotropic, kainate 4 | *GRIK4* | 2900 | EAA1, Excitatory amino acid receptor 1, Glutamate receptor, ionotropic kainate 4 precursor, Glutamate receptor KA-1, GRIK, KA1 |
| | glutamate receptor, ionotropic, kainate 5 | *GRIK5* | 2901 | EAA2, Excitatory amino acid receptor 2, Glutamate receptor, ionotropic kainate 5 precursor, Glutamate receptor KA-2, GRIK2, KA2 |
| | glutamate receptor, ionotropic, N-methyl D-aspartate 1 | *GRIN1* | 2902 | NMDA1, NMDAR1, N-methyl-D-aspartate receptor subunit NR1, NR1 |
| | glutamate receptor, ionotropic, N-methyl D-aspartate-like 1A | *GRINL1 A* | 81488 | none |
| | glutamate receptor, ionotropic, N-methyl D-aspartate-like 1B | *GRINL1 B* | 84534 | GLURR2 |
| | glutamate receptor, ionotropic, N-methyl D-aspartate 2A | *GRIN2 A* | 2903 | hNR2A, NMDAR2A, N-methyl D-aspartate receptor subtype 2A, NR2A |

(continued)

| Type | Subunit | Gene | NCBI Gene ID | Synonyms |
|---|---|---|---|---|
| | glutamate receptor, ionotropic, N-methyl D-aspartate 2B | *GRIN2 B* | 2904 | hNR3, MGC142178, MGC142180, NMDAR2B, N-methyl D-aspartate receptor subtype 2B, N-methyl-D-aspartate receptor subunit 3, NR2B, NR3 |
| | glutamate receptor, ionotropic, N-methyl D-aspartate 2C | *GRIN2 C* | 2905 | NMDAR2C, N-methyl D-aspartate receptor subtype 2C, NR2C |
| | glutamate receptor, ionotropic, N-methyl D-aspartate 2D | *GRIN2 D* | 2906 | EB11, NMDAR2D, N-methyl D-aspartate receptor subtype 2D, NR2D |
| | glutamate receptor, ionotropic, N-methyl-D-aspartate 3A | *GRIN3 A* | 116443 | FLJ45414, KIAA1973, NMDAR-L, N-methyl-D-aspartate receptor subtype NR3A, NR3A |
| | GluN3B | *GRIN3 B* | | |
| | | | | |
| ATP-gated channels: | purinergic receptor P2X, ligand-gated ion channel, 1 | *P2RX1* | 5023 | ATP receptor, P2X1, P2X purinoceptor 1, Purinergic receptor |
| | purinergic receptor P2X, ligand-gated ion channel, 2 | *P2RX2* | 22953 | ATP receptor, MGC129601, P2X2, P2X purinoceptor 2, Purinergic receptor |
| | purinergic receptor P2X, ligand-gated ion channel, 3 | *P2RX3* | 5024 | ATP receptor, MGC129956, P2X3, P2X purinoceptor 3, Purinergic receptor |
| | purinergic receptor P2X, ligand-gated ion channel, 4 | *P2RX4* | 5025 | ATP receptor, P2X4, P2X4R, P2X purinoceptor 4, Purinergic receptor |
| | purinergic receptor P2X, ligand-gated ion channel, 5 | *P2RX5* | 5026 | ATP receptor, MGC47755, P2X5, P2X5R, P2X purinoceptor 5, Purinergic receptor |
| | purinergic receptor P2X, ligand-gated ion channel, 6 | *P2RX6* | 9127 | ATP receptor, MGC129625, P2RXL1, P2X6, P2XM, P2X purinoceptor 6, Purinergic receptor, Purinergic receptor P2X-like 1, purinergic receptor P2X-like 1, orphan receptor |
| | purinergic receptor P2X, ligand-gated ion channel, 7 | *P2RX7* | 5027 | ATP receptor, MGC20089, P2X7, P2X purinoceptor 7, P2Z receptor, Purinergic receptor |
| | | | | |

(continued)

| Type | Subunit | Gene | NCBI Gene ID | Synonyms |
|---|---|---|---|---|
| ENaC /DEG family | sodium channel, nonvoltage-gated 1 alpha | *SCNN1A* | 6337 | Alpha ENaC, Alpha NaCH, Amiloride-sensitive sodium channel alpha-subunit, Amiloride-sensitive sodium channel subunit alpha, ENaCa, ENaCalpha, Epithelial Na(+) channel subunit alpha, Epithelial Na+ channel alpha subunit, FLJ21883, Nonvoltage-gated sodium channel 1 alpha subunit, Nonvoltage-gated sodium channel 1 subunit alpha, SCNEA, SCNN1 |
| | sodium channel, nonvoltage-gated 1, beta | *SCNN1B* | 6338 | Amiloride-sensitive sodium channel beta-subunit, Amiloride-sensitive sodium channel subunit beta, Beta ENaC, Beta NaCH, ENaCb, ENaCB, ENaCbeta, Epithelial Na(+) channel subunit beta, Epithelial Na+ channel beta subunit, Nonvoltage-gated sodium channel 1 beta subunit, Nonvoltage-gated sodium channel 1 subunit beta, SCNEB, sodium channel, nonvoltage-gated 1, beta (Liddle syndrome) |
| | sodium channel, nonvoltage-gated 1, gamma | *SCNN1G* | 6340 | Amiloride-sensitive sodium channel gamma-subunit, Amiloride-sensitive sodium channel subunit gamma, ENaCg, ENaCgamma, Epithelial Na(+) channel subunit gamma, Epithelial Na+ channel gamma subunit, Gamma ENaC, Gamma NaCH, Nonvoltage-gated sodium channel 1 gamma subunit, Nonvoltage-gated sodium channel 1 subunit gamma, PHA1, SCNEG |

(continued)

| Type | Subunit | Gene | NCBI Gene ID | Synonyms |
|---|---|---|---|---|
| | sodium channel, nonvoltage-gated 1, delta | *SCNN1D* | 6339 | Amiloride-sensitive sodium channel delta-subunit, Amiloride-sensitive sodium channel subunit delta, Delta ENaC, Delta NaCH, dNaCh, DNACH, ENaCd, ENaCdelta, Epithelial N(+) channel subunit delta, Epithelial Na+ channel delta subunit, MGC149710, MGC149711, Nonvoltage-gated sodium channel 1 delta subunit, Nonvoltage-gated sodium channel 1 subunit delta, SCNED |
| | amiloride-sensitive cation channel 1, neuronal | ACCN2 | 41 | ion channel 1, Amiloride-sensitive cation channel 2, neuronal, ASIC, ASIC1, ASIC1A, BNaC2, BNAC2, Brain sodium channel 2, hBNaC2 |
| | amiloride-sensitive cation channel 2, neuronal | ACCN1 | 40 | ACCN, Acid-sensing ion channel 2, Amiloride-sensitive brain sodium channel, Amiloride-sensitive cation channel 1, neuronal, amiloride-sensitive cation channel 1, neuronal (degenerin), Amiloride-sensitive cation channel neuronal 1, ASIC2, ASIC2a, BNaC1, BNAC1, BNC1, Brain sodium channel 1, hBNaC1, Mammalian degenerin homolog, MDEG |
| | amiloride-sensitive cation channel 3 | ACCN3 | 9311 | Acid-sensing ion channel 3, Amiloride-sensitive cation channel 3, ASIC3, DRASIC, hASIC3, hTNaC1, SLNAC1, Testis sodium channel 1, TNaC1, TNAC1 |
| | amiloride-sensitive cation channel 4, pituitary | ACCN4 | 55515 | ion channel 4, Amiloride-sensitive cation channel 4, Amiloride-sensitive cation channel 4, pituitary, ASIC4, BNAC4, MGC17248, MGC24860 |
| | amiloride-sensitive cation channel 5, intestinal | ACCN5 | 518022 | HINAC, INAC |
| | | | | |

(continued)

| Type | Subunit | Gene | NCBI Gene ID | Synonyms |
|---|---|---|---|---|
| TRP family | transient receptor potential cation channel, subfamily A, member 1 | TRPA1 | 8989 | ANKTM1, Ankyrin-like with transmembrane domains protein 1, Transformation sensitive-protein p120, Transient receptor potential cation channel subfamily A member 1 |
| | transient receptor potential cation channel, subfamily C, member 1 | TRPC1 | 7220 | HTRP-1, MGC133334, MGC133335, Short transient receptor potential channel 1, TRP1, TRP-1 protein, TrpC1 |
| | transient receptor potential cation channel subfamily C, member 2 (pseudogene) | TRPC2 | 7221 | transient receptor potential cation channel, subfamily C, member 2, transient receptor potential channel 2 |
| | transient receptor potential cation channel, subfamily C, member 3 | TRPC3 | 7222 | Htrp3, Htrp-3, Short transient receptor potential channel 3, TRP3, TrpC3 |
| | transient receptor potential cation channel, subfamily, C, member 4 | TRPC4 | 7223 | hTrp4, HTRP4, hTrp-4, MGC119570, MGC119571, MGC119572, MGC119573, Short transient receptor potential channel 4, TRP4, TrpC4, trp-related protein 4, Trp-related protein 4 |
| | transient receptor potential cation channel, subfamily C, member 4 associated protein | TRPC4 AP | 26133 | C20orf188, dJ756N5.2, DKFZp586C1223, DKFZP727M231, Protein TRUSS, Short transient receptor potential channel 4-associated protein, TAP1 protein, TNF-receptor ubiquitous scaffolding/signaling protein, Trp4-associated protein, Trpc4-associated protein, TRRP4AP, TRUSS, TRUSS protein |
| | transient receptor potential cation channel, subfamily C, member 5 | TRPC5 | 7224 | Harp5, Htrp-5, Short transient receptor potential channel 5, TRP5, TrpC5 |
| | transient receptor potential cation channel, subfamily C, member 6 | TRPC6 | 7225 | FLJ11098, FLJ14863, FSGS2, Short transient receptor potential channel 6, TRP6, TrpC6 |

(continued)

| Type | Subunit | Gene | NCBI Gene ID | Synonyms |
|---|---|---|---|---|
| | transient receptor potential cation channel, subfamily C, member 6 pseudogene | TRPC6 P | 644218 | LOC644218, similar to transient receptor potential cation channel, subfamily C, member 6, TRPC6L |
| | transient receptor potential cation channel, subfamily C, member 7 | TRPC7 | 5711 3 | Short transient receptor potential channel 7, TRP7, TRP7 protein, TrpC7 |
| | transient receptor potential cation channel, subfamily M, member 1 | TRPM1 | 4308 | LTRPC1, MLSN, MLSN1 |
| | transient receptor potential cation channel, subfamily M, member 2 | TRPM2 | 7226 | EREG1, Estrogen-responsive element-associated gene 1 protein, KNP3, Long transient receptor potential channel 2, LTrpC2, LTRPC2, LTrpC-2, MGC133383, NUDT9H, NUDT9L1, Transient receptor potential cation channel subfamily M member 2, Transient receptor potential channel 7, TrpC7, TRPC7 |
| | transient receptor potential cation channel, subfamily M, member 3 | TRPM3 | 80036 | GON-2, KIAA1616, Long transient receptor potential channel 3, LTrpC3, LTRPC3, Melastatin-2, MLSN2, Transient receptor potential cation channel subfamily M member 3 |
| | transient receptor potential cation channel, subfamily M, member 4 | TRPM4 | 54795 | Calcium-activated non-selective cation channel 1, FLJ20041, hTRPM4, Long transient receptor potential channel 4, LTRPC4, Melastatin-4, Transient receptor potential cation channel subfamily M member 4, TRPM4B |
| | transient receptor potential cation channel, subfamily M, member 5 | TRPM5 | 29850 | LTRPC5, MTR1 |

(continued)

| Type | Subunit | Gene | NCBI Gene ID | Synonyms |
|---|---|---|---|---|
| | transient receptor potential cation channel, subfamily M, member 6 | TRPM6 | 140803 | CHAK2, Channel kinase 2, FLJ22628, HMGX, HOMG, HOMG1, HSH, Melastatin-related TRP cation channel 6, Transient receptor potential cation channel subfamily M member 6 |
| | transient receptor potential cation channel, subfamily M, member 7 | TRPM7 | 54822 | CHAK, CHAK1, Channel-kinase 1, FLJ20117, FLJ25718, Long transient receptor potential channel 7, LTrpC7, LTRPC7, Transient receptor potential cation channel subfamily M member 7, TRP-PLIK |
| | transient receptor potential cation channel, subfamily M, member 8 | TRPM8 | 79054 | Long transient receptor potential channel 6, LTrpC6, LTRPC6, MGC2849, Transient receptor potential cation channel subfamily M member 8, Transient receptor potential-p8, TRPP8, Trp-p8 |
| | trichorhinophalangeal syndrome I | TRPS1 | 7227 | GC79, LGCR. MGC134928, Tricho-rhino-phalangeal syndrome type I protein, Zinc finger protein GC79, Zinc finger transcription factor Trps1 |
| | tRNA phosphotransferase 1 | TRPT1 | 83707 | MGC11134, tRNA 2'-phosphotransferase 1 |
| | transient receptor potential cation channel, subfamily V, member 1 | TRPV1 | 7442 | Capsaicin receptor, DKFZp434K0220, osm-9-like TRP channel 1, OTRPC1, Transient receptor potential cation channel subfamily V member 1, TrpV1, Vanilloid receptor 1, VR1 |
| | transient receptor potential cation channel, subfamily V, member 2 | TRPV2 | 51393 | MGC12549, osm-9-like TRP channel 2, OTRPC2, Transient channel 2, OTRPC2, Transient receptor potential cation channel subfamily V member 2, TrpV2, Vanilloid receptor-like protein 1, VRL, VRL1, VRL-1 |
| | transient receptor potential cation channel, subfamily V, member 3 | TRPV3 | 162514 | Transient receptor potential cation channel subfamily V member 3, TrpV3, Vanilloid receptor-like 3, VRL3, VRL-3 |

(continued)

| Type | Subunit | Gene | NCBI Gene ID | Synonyms |
|---|---|---|---|---|
| | transient receptor potential cation channel, subfamily V, member 4 | TRPV4 | 59341 | osm-9-like TRP channel 4, OTRPC4, Transient receptor potential cation channel subfamily V member 4, Transient receptor potential protein 12, TRP12, TrpV4, Vanilloid receptor-like channel 2, Vanilloid receptor-like protein 2, Vanilloid receptor-related osmotically-activated channel, VRL2, VRL-2, VROAC, VR-OAC |
| | transient receptor potential cation channel, subfamily V, member 5 | TRPV5 | 56302 | Calcium transport protein 2, CaT2, CAT2, ECaC, ECaC1, ECAC1, Epithelial calcium channel 1, osm-9-like TRP channel 3, OTRPC3, Transient receptor potential cation channel subfamily V member 5, TrpV5 |
| | transient receptor potential cation channel, subfamily V, member 6 | TRPV6 | 55503 | ABP/ZF, Calcium transport protein 1, CaT1, CAT1, CATL, CaT-L, CaT-like, ECaC2, ECAC2, Epithelial calcium channel 2, HSA277909, LP6728, Transient receptor potential cation channel subfamily V member 6, TrpV6, ZFAB |
| | | | | |
| CNG family | cyclic nucleotide gated channel alpha 1 | CNGA1 | 1259 | cGMP-gated cation channel alpha 1, CNCG, CNCG1, CNG1, CNG-1, CNG channel alpha 1, Cyclic nucleotide-gated cation channel 1, Cyclic-nucleotide-gated cation channel 1, Cyclic nucleotide gated channel, photoreceptor, Cyclic nucleotide-gated channel, photoreceptor, Cyclic nucleotide gated channel alpha 1, Cyclic nucleotide-gated channel alpha 1, RCNC1, RCNCa, RCNCalpha, Rod photoreceptor cGMP-gated channel alpha subunit, Rod photoreceptor cGMP-gated channel subunit alpha |

(continued)

| Type | Subunit | Gene | NCBI Gene ID | Synonyms |
|---|---|---|---|---|
| | cyclic nucleotide gated channel alpha 2 | CNGA2 | 1260 | CNCA, CNCA1, CNCG2, CNG2, CNG-2, CNG channel 2, Cyclic nucleotide-gated cation channel 2, Cyclic nucleotide-gated olfactory channel, FLJ46312, OCNC1, OCNCa, OCNCalpha, OCNCALPHA |
| | cyclic nucleotide gated channel alpha 3 | CNGA3 | 1261 | ACHM2, CCNC1, CCNCa, CCNCalpha, CNCG3, CNG3, CNG-3, CNG channel alpha 3, Cone photoreceptor cGMP-gated channel alpha subunit, Cone photoreceptor cGMP-gated channel subunit alpha, Cyclic nucleotide-gated cation channel alpha 3, Cyclic nucleotide-gated channel alpha 3 |
| | cyclic nucleotide gated channel alpha 4 | CNGA4 | 338753, 1262 | CNCA2, CNG5, CNGB2, MGC126168, MGC126169, OCNC2, OCNCb, OCNCBETA |
| | cyclic nucleotide gated channel beta 1 | CNGB1 | 1258 | CNCG2, CNCG3L, CNCG4, CNG4, CNG-4, CNGB1B, CNG channel 4, Cyclic nucleotide-gated cation channel 4, Cyclic nucleotide-gated cation channel modulatory subunit, GAR1, GARP, RCNC2, RCNCb, RCNCbeta |
| | cyclic nucleotide gated channel beta 3 | CNGB3 | 54714 | ACHM1, ACHM3, CNG channel beta 3, Cone photoreceptor cGMP-gated channel beta subunit, Cone photoreceptor cGMP-gated channel subunit beta, Cyclic nucleotide-gated cation channel beta 3, Cyclic nucleotide-gated cation channel modulatory subunit, Cyclic nucleotide-gated channel beta 3, RMCH, RMCH1 |
| | | | | |

(continued)

| Type | Subunit | Gene | NCBI Gene ID | Synonyms |
|---|---|---|---|---|
| HCN family | hyperpolarization activated cyclic nucleotide-gated potassium channel 1 | HCN1 | 348980 | BCNG1, BCNG-1, Brain cyclic nucleotide gated channel 1, Brain cyclic nucleotide-gated channel 1, HAC-2, Potassium/sodium hyperpolarization-activated cyclic nucleotide-gated channel 1 |
| | hyperpolarization activated cyclic nucleotide-gated potassium channel 2 | HCN2 | 610 | BCNG2, BCNG-2, Brain cyclic nucleotide gated channel 2, Brain cyclic nucleotide-gated channel 2, HAC-1, Potassium/sodium hyperpolarization-activated cyclic nucleotide-gated channel 2 |
| | hyperpolarization activated cyclic nucleotide-gated potassium channel 3 | HCN3 | 5765 7 | KIAA1535, MGC131493, Potassium/sodium hyperpolarization-activated cyclic nucleotide-gated channel 3 |
| | hyperpolarization activated cyclic nucleotide-gated potassium channel 4 | HCN4 | 10021 | Potassium/sodium hyperpolarization-activated cyclic nucleotide-gated channel 4 |
| | | | | |
| KCN family | potassium voltage-gated channel, shaker-related subfamily, member 1 (episodic ataxia with myokymia) | KCNA1 | 3736 | AEMK, EA1, HBK1, HUK1, HUKI, Kv1.1, KV1.1, MBK1, MGC126782, MGC138385, MK1, Potassium voltage-gated channel subfamily A member 1, RBK1, Voltage-gated potassium channel subunit Kv1.1 |
| | potassium voltage-gated channel, shaker-related subfamily, member 2 | KCNA2 | 3737 | HBK5, HK4, HUKIV, Kv1.2, KV1.2, MGC50217, MK2, NGK1, Potassium voltage-gated channel subfamily A member 2, RBK2, Voltage-gated potassium channel subunit Kv1.2 |
| | potassium voltage-gated channel, shaker-related subfamily, member 3 | KCNA3 | 3738 | HGK5, HLK3, HPCN3, HuKIII, HUKIII, Kv1.3, KV1.3, MK3, PCN3, Potassium voltage-gated channel subfamily A member 3, Voltage-gated potassium channel subunit Kv1.3 |

(continued)

| Type | Subunit | Gene | NCBI Gene ID | Synonyms |
|---|---|---|---|---|
| | potassium voltage-gated channel, shaker-related subfamily, member 4 | KCNA4 | 3739 | HBK4, HK1, HPCN2, HUKII, KCNA4L, KCNA8, Kv1.4, KV1.4, PCN2, Potassium voltage-gated channel subfamily A member 4, Voltage-gated potassium channel subunit Kv1.4 |
| | potassium voltage-gated channel, shaker-related subfamily, member 5 | KCNA5 | 3741 | ATFB7, HCK1, HK2, HPCN1, Kv1.5, KV1.5, MGC117058, MGC117059, PCN1, Potassium voltage-gated channel subfamily A member 5, Voltage-gated potassium channel subunit Kv1.5 |
| | potassium voltage-gated channel, shaker-related subfamily, member 6 | KCNA6 | 3742 | FLJ25134, HBK2, Kv1.6, KV1.6, Potassium voltage-gated channel subfamily A member 6, Voltage-gated potassium channel subunit Kv1.6 |
| | potassium voltage-gated channel, shaker-related subfamily, member 7 | KCNA7 | 3743 | HAK6, Kv1.7, KV1.7 |
| | potassium voltage-gated channel, shaker-related subfamily, member 10 | KCNA1 0 | 3744 | Kcn1, Kv1.8 |
| | potassium voltage-gated channel, shaker-related subfamily, beta member 1 | KCNA6 1 | 7881 | AKR6A3, hKvb3, hKvBeta3, K(+) channel beta-1 subunit, K(+) channel subunit beta-1, KCNA1B, Kvb1.3, Kv-beta-1, KV-BETA-1, Voltage-gated potassium channel beta-1 subunit, Voltage-gated potassium channel subunit beta-1 |
| | potassium voltage-gated channel, shaker-related subfamily, beta member 2 | KCNAB 2 | 8514 | AKR6A5, HKvbeta2, HKvbeta2.1, HKvbeta2.2, K(+) channel beta-2 subunit, K(+) channel subunit beta-2, KCNA2B, KCNK2, Kv-beta-2, KV-BETA-2, MGC117289, Voltage-gated potassium channel beta-2 subunit, Voltage-gated potassium channel subunit beta-2 |

(continued)

| Type | Subunit | Gene | NCBI Gene ID | Synonyms |
|---|---|---|---|---|
| | potassium voltage-gated channel, shaker-related subfamily, beta member 3 | KCNAB 3 | 9196 | AKR6A9, K(+) channel beta-3 subunit, K(+) channel subunit beta-3, KCNA3.1 B, KCNA3B, Kv-beta-3, KV-BETA-3, MGC116886, Voltage-gated potassium channel beta-3 subunit, Voltage-gated potassium channel subunit beta-3 |
| | potassium voltage-gated channel, Shab-related subfamily, member 1 | KCNB1 | 3745 | DRK1, h-DRK1, Kv2.1, KV2.1, Potassium voltage-gated channel subfamily B member 1, Voltage-gated potassium channel subunit Kv2.1 |
| | potassium voltage-gated channel, Shab-related subfamily, member 2 | KCNB2 | 9312 | Kv2.2, Potassium voltage-gated channel subfamily B member 2, Voltage-gated potassium channel subunit Kv2.2 |
| | potassium voltage-gated channel, Shaw-related subfamily, member 1 | KCNC1 | 3746 | FLJ41162, FLJ42249, FLJ43491, Kv3.1, KV3.1, Kv4, KV4, MGC129855, NGK2, Potassium voltage-gated channel subfamily C member 1, Voltage-gated potassium channel subunit Kv3.1 |
| | potassium voltage-gated channel, Shaw-related subfamily, member 2 | KCNC2 | 3747 | Kv3.2, KV3.2, MGC138196 |
| | potassium voltage-gated channel, Shaw-related subfamily, member 3 | KCNC3 | 3748 | KSHIIID, Kv3.3, KV3.3, Potassium voltage-gated channel subfamily C member 3, SCA13, Voltage-gated potassium channel subunit Kv3.3 |
| | potassium voltage-gated channel, Shaw-related subfamily, member 4 | KCNC4 | 3749 | HKSHIIIC, KSHIIIC, Kv3.4, KV3.4, MGC126818, Potassium voltage-gated channel subfamily C member 4, Voltage-gated potassium channel subunit Kv3.4 |
| | potassium voltage-gated channel, Shal-related subfamily, member 1 | KCND1 | 3750 | Kv4.1, Potassium voltage-gated channel subfamily D member 1, Voltage-gated potassium channel subunit Kv4.1 |

(continued)

| Type | Subunit | Gene | NCBI Gene ID | Synonyms |
|---|---|---|---|---|
| | potassium voltage-gated channel, Shal-related subfamily, member 2 | KCND2 | 3751 | KIAA1044, Kv4.2, KV4.2, MGC119702, MGC119703, Potassium voltage-gated channel subfamily D member 2, RK5, Voltage-gated potassium channel subunit Kv4.2 |
| | potassium voltage-gated channel, Shal-related subfamily, member 3 | KCND3 | 3752 | KCND3L, KCND3S, KSHIVB, Kv4.3, v4.3, MGC142035, MGC142037, Potassium voltage-gated channel subfamily D member 3, Voltage-gated potassium channel subunit Kv4.3 |
| | potassium voltage-gated channel, Isk-related family, member 1 | KCNE1 | 3753 | Delayed rectifier potassium channel subunit IsK, FLJ18426, FLJ38123, FLJ94103, IKs producing slow voltage-gated potassium channel beta subunit Mink, IKs producing slow voltage-gated potassium channel subunit beta Mink, ISK, JLNS, JLNS2, LQT2/5, LQT5, MGC33114, Minimal potassium channel, minK, MinK, Potassium voltage-gated channel subfamily E member 1 |
| | KCNE1-like | KCNE1 L | 23630 | AMMECR2 protein, AMME syndrome candidate gene 2 protein, Potassium voltage-gated channel subfamily E member 1-lika protein |
| | potassium voltage-gated channel, Isk-related family, member 2 | KCNE2 | 9992 | LQT5, LQT6, MGC138292, Minimum potassium ion channel-related peptide 1, MinK-related peptide 1, MiRP1, MIRP1, Potassium channel beta subunit MiRP1, Potassium channel subunit beta MiRP1, Potassium voltage-gated channel subfamily E member 2 |

(continued)

| Type | Subunit | Gene | NCBI Gene ID | Synonyms |
|---|---|---|---|---|
| | potassium voltage-gated channel, Isk-related family, member 3 | KCNE3 | 10008 | DKFZp781H21101, HOKPP, MGC102685, MGC129924, Minimum potassium ion channel-related peptide 2, MinK-related peptide 2, MiRP2, Potassium channel beta subunit MiRP2, Potassium channel subunit beta MiRP2, Potassium voltage-gated channel subfamily E member 3 |
| | potassium voltage-gated channel, Isk-reflated family, member 4 | KCNE4 | 23704 | MGC20353, Minimum potassium ion channel-related peptide 3, MinK-related peptide 3, MiRP3, MIRP3, Potassium channel beta subunit MiRP3, Potassium channel subunit beta MiRP3, Potassium voltage-gated channel subfamily E member 4 |
| | potassium voltage-gated channel, subfamily F, member 1 | KCNF1 | 3754 | IK8, KCNF, kH1, Kv5.1, KV5.1, MGC33316, Potassium voltage-gated channel subfamily F member 1, Voltage-gated potassium channel subunit Kv5.1 |
| | potassium voltage-gated channel, subfamily G, member 1 | KCNG1 | 3755 | K13, KCNG, kH2, Kv6.1, KV6.1, MGC12878, Potassium voltage-gated channel subfamily G member 1, Voltage-gated potassium channel subunit Kv6.1 |
| | potassium voltage-gated channel, subfamily G, member 2 | KCNG2 | 26251 | Cardiac potassium channel subunit, KCNF2, Kv6.2, KV6.2, Potassium voltage-gated channel subfamily G member 2, Voltage-gated potassium channel subunit Kv6.2 |
| | potassium voltage-gated channel, subfamily G, member 3 | KCNG3 | 170850 | Kv10.1, KV10.1, Kv6.3, KV6.3, Potassium voltage-gated channel subfamily G member 3, Voltage-gated potassium channel subunit Kv6.3 |

(continued)

| Type | Subunit | Gene | NCBI Gene ID | Synonyms |
|---|---|---|---|---|
| | potassium voltage-gated channel, subfamily G, member 4 | KCNG4 | 93107 | KCNG3, Kv6.3, KV6.3, Kv6.4, KV6.4, MGC129609, MGC4558, Potassium voltage-gated channel subfamily G member 4, Voltage-gated potassium channel subunit Kv6.4 |
| | potassium voltage-gated channel, subfamily H (eag-related), member 1 | KCNH1 | 3756 | eag, EAG, eag1, EAG1, Ether-a-go-go potassium channel 1, h-eag, hEAG1, Kv10.1, MGC142269, Potassium voltage-gated channel subfamily H member 1, Voltage-gated potassium channel subunit Kv10.1 |
| | potassium voltage-gated channel, subfamily H (eag-related), member 2 | KCNH2 | 3757 | eag homolog, Eag-related protein 1, ERG, erg1, Erg1, ERG1, Ether-a-go-go related gene potassium channel 1, Ether-a-go-go-related gene potassium channel 1, Ether-a-go-go related protein 1, Ether-a-go-go-related protein 1, HERG, H-ERG, HERG1, Kv11.1, LQT2, Potassium voltage-gated channel subfamily H member 2, SQT1, Voltage-gated potassium channel, subunit Kv11.1 |
| | potassium voltage-gated channel, subfamily H (eag-related), member 3 | KCNH3 | 23416 | BEC1, Brain-specific eag-like channel 1, elk2, ELK2, ELK channel 2, Ether-a-go-go-like potassium channel 2, KIAA1282, Kv12.2, Potassium voltage-gated channel subfamily H member 3, Voltage-gated potassium channel subunit Kv12.2 |
| | potassium voltage-gated channel, subfamily H (eag-related), member 4 | KCNH4 | 23415 | BEC2, Brain-specific eag-like channel 2, elk1, ELK1, ELK channel 1, Ether-a-go-go-like potassium channel 1, Kv12.3, Potassium voltage-gated channel subfamily H member 4, Voltage-gated potassium channel subunit Kv12.3 |

(continued)

| Type | Subunit | Gene | NCBI Gene ID | Synonyms |
|---|---|---|---|---|
| | potassium voltage-gated channel, subfamily H (eag-related), member 5 | KCNH5 | 27133 | eag2, Eag2, EAG2, Ether-a-go-go potassium channel 2, hEAG2, H-EAG2, Kv10.2, Potassium voltage-gated channel subfamily H member 5, Voltage-gated potassium channel subunit Kv10.2 |
| | potassium voltage-gated channel, subfamily H (eag-related), member 6 | KCNH6 | 81033 | Eag-related protein 2, erg2, ERG2, Ether-a-go-go-related gene potassium channel 2, Ether-a-go-go-related protein 2, HERG2, Kv11.2, Potassium voltage-gated channel subfamily H member 6, Voltage-gated potassium channel subunit Kv11.2 |
| | potassium voltage-gated channel, subfamily H (eag-related), member 7 | KCNH7 | 90134 | Eag-related protein 3, erg3, ERG3, Ether-a-go-go-related gene potassium channel 3, Ether-a-go-go-related protein 3, HERG3, HERG-3, Kv11.3, MGC45986, Potassium voltage-gated channel subfamily H member 7, Voltage-gated potassium channel subunit Kv11.3 |
| | potassium voltage-gated channel, subfamily H (eag-related), member 8 | KCNH8 | 131096 | ELK, ELK1, elk3, ELK3, ELK channel 3, Ether-a-go-go-like potassium channel 3, hElk1, Kv12.1, Potassium voltage-gated channel subfamily H member 8, Voyage-gated potassium channel subunit Kv12.1 |
| | Kv channel interacting protein 1 | KCNIP1 | 30820 | A-type potassium channel modulatory protein 1, KChIP1, KCHIP1, Kv channel-interacting protein 1, MGC95, Potassium channel-interacting protein 1, VABP, Vesicle APC-binding protein |

(continued)

| Type | Subunit | Gene | NCBI Gene ID | Synonyms |
|---|---|---|---|---|
| | Kv channel interacting protein 2 | KCNIP2 | 30819 | A-type potassium channel modulatory protein 2, Cardiac voltage gated potassium channel modulatory subunit, Cardiac voltage-gated potassium channel modulatory subunit, DKFZp566L1246, KChIP2, KCHIP2, Kv channel-interacting protein 2, MGC17241, Potassium channel-interacting protein 2 |
| | Kv channel interacting protein 3, calsenilin | KCNIP3 | 30818 | A-type potassium channel modulatory protein 3, calsenilin, Calsenilin, CSEN, DREAM, DRE-antagonist modulator, KChIP3, KCHIP3, Kv channel-interacting protein 3, MGC18289 |
| | Kv channel interacting protein 4 | KCNIP4 | 80333 | A-type potassium channel modulatory protein 4, CALP, Calsenilin-like protein, KChIP4, KCHIP4, Kv channel-interacting protein 4, MGC44947, Potassium channel-interacting protein 4 |
| | potassium inwardly-rectifying channel, subfamily J, member 1 | KCNJ1 | 3758 | ATP-regulated potassium channel ROM-K, ATP-sensitive inward rectifier potassium channel 1, Kir1.1, KIR1.1, Potassium channel, inwardly rectifying subfamily J member 1, ROMK, ROMK1 |
| | potassium inwardly-rectifying channel, subfamily J, member 2 | KCNJ2 | 3759 | Cardiac inward rectifier potassium channel, HHBIRK1, HHIRK1, HIRK1, Inward rectifier K(+) channel Kir2.1, Inward rectifier potassium channel 2, IRK1, Kir2.1, KIR2.1, LQT7, Potassium channel, inwardly rectifying subfamily J member 2, SQT3 |
| | Potassium inwardly-rectifying channel, subfamily J, member 3 | KCNJ3 | 3760 | GIRK1, G protein-activated inward rectifier potassium channel 1, Inward rectifier K(+) channel Kir3.1, KGA, Kir3.1, KIR3.1, Potassium channel, inwardly rectifying subfamily J member 3 |

(continued)

| Type | Subunit | Gene | NCBI Gene ID | Synonyms |
|---|---|---|---|---|
| | potassium inwardly-rectifying channel, subfamily J, member 4 | KCNJ4 | 3761 | Hippocampal inward rectifier, HIR, hIRK2, HIRK2, HRK1, inward rectifier K(+) channel Kir2.3, Inward rectifier potassium channel 4, IRK3, Kir2.3, MGC142066, MGC142068, Potassium channel, inwardly rectifying subfamily J member 4 |
| | potassium inwardly-rectifying channel, subfamily J, member 5 | KCNJ5 | 3762 | Cardiac inward rectifier, CIR, GIRK4, G protein-activated inward rectifier potassium channel 4, Heart KATP channel, Inward rectifier K(+) channel Kir3.4, KATP1, KATP-1, Kir3.4, KIR3.4, Potassium channel, inwardly rectifying subfamily J member 5 |
| | potassium inwardly-rectifying channel, subfamily J, member 6 | KCNJ6 | 3763 | BIR1, GIRK2, G protein-activated inward rectifier potassium channel, 2, hiGIRK2, Inward rectifier K(+) channel Kir3.2, KATP2, KATP-2, KCNJ7, Kir3.2, KIR3.2, MGC126596, Potassium channel, inwardly rectifying subfamily J member 6 |
| | potassium inwardly-rectifying channel, subfamily J, member 8 | KCNJ8 | 3764 | ATP-sensitive inward rectifier potassium channel 8, Inwardly rectifier K(+) channel Kir6.1, Kir6.1, KIR6.1, Potassium channel, inwardly rectifying subfamily J member 8, uKATP-1 |
| | potassium inwardly-rectifying channel, subfamily J, member 9 | KCNJ9 | 3765 | GIRK3, G protein-activated inward rectifier potassium channel 3, Inwardly rectifier K(+) channel Kir3.3, Kir3.3, KIR3.3, Potassium channel, inwardly rectifying subfamily J member 9 |

(continued)

| Type | Subunit | Gene | NCBI Gene ID | Synonyms |
|---|---|---|---|---|
| | potassium inwardly-rectifying channel, subfamily J, member 10 | KCNJ1 0 | 3766 | ATP-dependent inwardly rectifying potassium channel Kir4.1, ATP-sensitive inward rectifier potassium channel 10, BIRK-10, Inward rectifier K(+) channel Kir1.2, KCNJ13-PEN, Kir1.2, KIR1.2, Kir4.1, KIR4.1, Potassium channel, inwardly rectifying subfamily J member 10 |
| | potassium inwardly-rectifying channel, subfamily J, member 11 | KCNJ1 1 | 3767 | ATP-sensitive inward rectifier potassium channel 11, BIR, HHF2, IKATP, Inward rectifier K(+) channel Kir6.2, Kir6.2, KIR6.2, MGC133230, PHHI, Potassium channel, inwardly rectifying subfamily J member 11, TNDM3 |
| | potassium inwardly-rectifying channel, subfamily J, member 12 | KCNJ1 2 | 3768 | ATP-sensitive inward rectifier potassium channel 12, FLJ14167, hIRK, hIRK1, hkir2.2x, Inward rectifier K(+) channel Kir2.2, Inward rectifying K(+) channel negative regulator Kir2.2v, IRK2, kcnj12x, KCNJN1, Kir2.2, Kir2.2v, Potassium channel, inwardly rectifying subfamily J member 12 |
| | potassium inwardly-rectifying channel, subfamily J, member 13 | KCNJ1 3 | 3769 | Inward rectifier K(+) channel Kir7.1, Inward rectifier potassium channel 13, Kir1.4, KIR1.4, Kir7.1, KIR7.1, MGC33328, Potassium channel, inwardly rectifying subfamily J member 13, SVD |
| | potassium inwardly-rectifying channel, subfamily J, member 14 | KCNJ 1 4 | 3770 | ATP-sensitive inward rectifier potassium channel 14, Inward rectifier K(+) channel Kir2.4, IRK4, Kir2.4, KIR2.4, MGC46111, Potassium channel, inwardly rectifying subfamily J member 14 |
| | potassium inwardly-rectifying channel, subfamily J, member 15 | KCNJ1 5 | 3772 | ATP-sensitive inward rectifier potassium channel 15, Inward rectifier K(+) channel Kir4.2, IRKK, KCNJ14, Kir1.3, KIR1.3, Kir4.2, KIR4.2, MGC13584, Potassium channel, inwardly rectifying subfamily J member 15 |

(continued)

| Type | Subunit | Gene | NCBI Gene ID | Synonyms |
|---|---|---|---|---|
| | potassium inwardly-rectifying channel, subfamily J, member 16 | KCNJ1 6 | 3773 | BIR9, Inward rectifier K(+) channel Kir5.1, Inward rectifier potassium channel 16, Kir5.1, KIR5.1, MGC33717, Potassium channel, inwardly rectifying subfamily J member 16 |
| | potassium channel, subfamily K, member 1 | KCNK1 | 3775 | DPK, HOHO, HOHO1, Inward rectifying potassium channel protein TWIK-1, K2p1.1, KCNO1, Potassium channel KCNO1, Potassium channel subfamily K member 1, TWIK1, TWIK-1 |
| | potassium channel, subfamily K, member 2 | KCNK2 | 3776 | hTREK-1c, hTREK-1e, K2p2.1, MGC126742, MGC126744, Outward rectifying potassium channel protein TREK-1, Potassium channel subfamily K member 2, TPKC1, TREK, TREK1, TREK-1, TREK-1 K(+) channel subunit, Two-pore domain potassium channel TREK-1, Two-pore potassium channel TPKC1 |
| | potassium channel, subfamily K, member 3 | KCNK3 | 3777 | Acid-sensitive potassium channel protein TASK-1, K2p3.1, OAT1, Potassium channel subfamily K member 3, TASK, TASK1, TASK-1, TBAK1, TWIK-related acid-sensitive K(+) channel 1, Two pore potassium channel KT3.1 |
| | potassium channel, subfamily K, member 4 | KCNK4 | 50801 | K2p4.1, Potassium channel subfamily K member 4, TRAAK, TRAAK1, TWIK-related arachidonic acid-stimulated potassium channel protein, Two pore K(+) channel KT4.1 |
| | potassium channel, subfamily K, member 5 | KCNK5 | 8645 | Acid-sensitive potassium channel protein TASK-2, FLJ11035, K2p5.1, Potassium channel subfamily K member 5, TASK2, TASK-2, TWIK-related acid-sensitive K(+) channel 2 |

(continued)

| Type | Subunit | Gene | NCBI Gene ID | Synonyms |
|---|---|---|---|---|
| | potassium channel, subfamily K, member 6 | KCNK6 | 9424 | FLJ12282, Inward rectifying potassium channel protein TWIK-2, K2p6.1, KCNK8, Potassium channel subfamily K member 6, TOSS, TWIK2, TWIK-2, TWIK-originated similarity sequence |
| | potassium channel, subfamily K, member 7 | KCNK7 | 10089 | K2p7.1, MGC118782, MGC118784, Potassium channel subfamily K member 7, PRO1716, TWIK3 |
| | potassium channel, subfamily K, member 9 | KCNK9 | 51305 | Acid-sensitive potassium channel protein TASK-3, K2p9.1, KT3.2, MGC138268, MGC138270, Potassium channel subfamily K member 9, TASK3, TASK-3, TWiK-related acid-sensitive K(+) channel 3, Two pore potassium channel KT3.2 |
| | potassium channel, subfamily K, member 10 | KCNK10 | 54207 | K2p10.1, Outward rectifying potassium channel protein TREK-2, Potassium channel subfamily K member 10, TREK2, TREK-2, TREK-2 K(+) channel subunit |
| | potassium channel, subfamily K, member 12 | KCNK12 | 56660 | Potassium channel subfamily K member 12, Tandem pore domain halothane-inhibited potassium channel 2, THIK2, THIK-2 |
| | potassium channel, subfamily K, member 13 | KCNK13 | 56659 | K2p13.1, Potassium channel subfamily K member 13, Tandem pore domain halothane-inhibited potassium channel 1, THIK1, THIK-1 |
| | potassium channel, subfamily K, member 15 | KCNK15 | 60598 | Acid-sensitive potassium channel protein TASK-5, dJ781B1.1, K2p15.1, KCNK11, KCNK14, KIAA0237, KT3.3, Potassium channel subfamily K member 15, TASK5, TASK-5, TWIK-related acid-sensitive K(+) channel 5, Two pore potassium channel KT3.3 |

(continued)

| Type | Subunit | Gene | NCBI Gene ID | Synonyms |
|---|---|---|---|---|
| | potassium channel, subfamily K, member 16 | KCNK16 | 83795 | 2P domain potassium channel Talk-1, K2p16.1, MGC133123, Potassium channel subfamily K member 16, TALK1, TALK-1, TWIK-related alkaline pH-activated K(+) channel 1 |
| | potassium channel, subfamily K, member 17 | KCNK17 | 89822 | 2P domain potassium channel Talk-2, K2p17.1, Potassium channel subfamily K member 17, TALK2, TALK-2, TASK4, TASK-4, TWIK-related acid-sensitive K(+) channel 4, TWIK-related alkaline pH-activated K(+) channel 2, UNQ5816/PRO19634 |
| | potassium channel, subfamily K, member 18 | KCNK18 | 338567 | K2p18.1, TRESK, TRESK2, TRESK-2, TRIK |
| | potassium large conductance calcium-activated channel, subfamily M, alpha member 1 | KCNMA1 | 3778 | BKCA alpha, BK channel, BKTM, Calcium-activated potassium channel, subfamily M, alpha subunit 1, Calcium-activated potassium channel, subfamily M subunit alpha 1, Calcium-activated potassium channel alpha subunit 1, Calcium-activated potassium channel subunit alpha 1, DKFZp686K1437, hSlo, K(VCA)alpha, KCa1.1, KCNMA, MaxiK, Maxi K channel, MGC71881, mSLO1, SAKCA, SLO, Slo1, SLO1, Slo-alpha, SLO-ALPHA, Slo homolog, Slowpoke homolog |

(continued)

| Type | Subunit | Gene | NCBI Gene ID | Synonyms |
|---|---|---|---|---|
| | potassium large conductance calcium-activated channel, subfamily M, beta member 1 | KCNMB1 | 3779 | BKbeta, BKbeta1, BK channel beta subunit 1, BK channel subunit beta 1, Calcium-activated potassium channel, subfamily M, beta subunit 1, Calcium-activated potassium channel, subfamily M subunit beta 1, Calcium-activated potassium channel beta-subunit, Calcium-activated potassium channel beta subunit 1, Calcium-activated potassium channel subunit beta, Calcium-activated potassium channel subunit beta 1, Charybdotoxin receptor beta subunit 1, Charybdotoxin receptor subunit beta 1, Hbeta1, hslo-beta, K(VCA)beta, K(VCA)beta 1, Maxi K channel beta subunit 1, Maxi K channel subunit beta 1, Slo-beta, SLO-BETA, Slo-beta 1 |
| | potassium large conductance calcium-activated channel, subfamily M, beta member 2 | KCNMB2 | 10242 | BKbeta2, BK channel beta subunit 2, BK channel subunit beta 2, Calcium-activated potassium channel, subfamily M, beta subunit 2, Calcium-activated potassium channel, subfamily M subunit beta 2, Calcium-activated potassium channel beta subunit 2, Calcium-activated potassium channel subunit beta 2, Charybdotoxin receptor beta subunit 2, Charybdotoxin receptor subunit beta 2, Hbeta2, Hbeta3, K(VCA)beta 2, Maxi K channel beta subunit 2, Maxi K channel subunit beta 2, Slo-beta 2 |

(continued)

| Type | Subunit | Gene | NCBI Gene ID | Synonyms |
|---|---|---|---|---|
| | potassium large conductance calcium-activated channel, subfamily M beta member 3 | KCNMB3 | 27094 | BKbeta3, BK channel beta subunit 3, BK channel subunit beta 3, Calcium-activated potassium channel, subfamily M, beta subunit 3, Calcium-activated potassium channel, subfamily M subunit beta 3, Calcium-activated potassium channel beta subunit 3, Calcium-activated potassium channel subunit beta 3, Charybdotoxin receptor beta subunit 3, Charybdotoxin receptor subunit beta 3, Hbeta3, K(VCA)beta 3, KCNMB2, KCNMBL, Maxi K channel beta subunit 3, Maxi K channel subunit beta 3, Slo-beta 3 |
| | potassium large conductance calcium-activated channel, subfamily M, beta member 3-like | KCNMB3L | 27093 | KCNMB2L, KCNMB3L1, KCNMBLP |
| | potassium large conductance calcium-activated channel, subfamily M, beta member 4 | KCNMB4 | 27345 | BKbeta4, BK channel beta subunit 4, BK channel subunit beta 4, Calcium-activated potassium channel, subfamily M, beta subunit 4, Calcium-activated potassium channel, subfamily M subunit beta 4, Calcium-activated potassium channel beta subunit 4, Calcium-activated potassium channel subunit beta 4, Charybdotoxin receptor beta subunit 4, Charybdotoxin receptor subunit beta 4, Hbeta4, K(VCA)beta 4, Maxi K channel beta subunit 4, Maxi K channel subunit beta 4, Slo-beta 4 |
| | potassium intermediate/small conductance calcium-activated channel, subfamily N, member 1 | KCNN1 | 3780 | hSK1, KCa2.1, SK, SK1, SKCA1, Small conductance calcium-activated potassium channel protein 1 |

(continued)

| Type | Subunit | Gene | NCBI Gene ID | Synonyms |
|---|---|---|---|---|
| | potassium intermediate/small conductance calcium-activated channel, subfamily N, member 2 | KCNN2 | 3781 | hSK2, KCa2.2, SK2, SKCA2, Small conductance calcium-activated potassium channel protein 2 |
| | potassium intermediate/small conductance calcium-activated channels, subfamily N, member 3 | KCNN3 | 3782 | hSK3, K3, KCa2.3, SK3, SKCa3, SKCA3, Small conductance calcium-activated potassium channel protein 3 |
| | potassium intermediate/small conductance calcium-activated channel, subfamily N, member 4 | KCNN4 | 3783 | hIKCa1, hKCa4, hSK4, IK1, IKCa1, IKCA1, Intermediate conductance calcium-activated potassium channel protein 4, KCa3.1, KCa4, KCA4, Putative Gardos channel, SK4 |
| | potassium voltage-gated channel, KQT-like subfamily, member 1 | KCNQ1 | 3784 | ATFB1, FLJ26167, IKs producing slow voltage-gated potassium channel alpha subunit KvLQT1, IKs producing slow voltage-gated potassium channel subunit alpha KvLQT1, JLNS1, KCNA8, KCNA9, KQT-like 1, Kv1.9, Kv7.1, KVLQT1, LQT, LQT1, Potassium voltage-gated channel subfamily KQT member 1, RWS, SQT2, Voltage-gated potassium channel subunit Kv7.1, WRS |
| | KCNQ1 downstream neighbor | KCNQ1DN | 55539 | Beckwith-Wiedemann region transcript protein, BWRT, HSA404617, KCNQ1 downstream neighbor protein |
| | KCNQ1 overlapping transcript 1 (non-protein coding) | KCNQ1OT1 | 10984 | FLJ41078, KCNQ10T1, KCNQ1 overlapping transcript 1, KvDMR1, KvLQT1-AS, LIT1, long QT intronic transcript 1, NCRNA00012 |

(continued)

| Type | Subunit | Gene | NCBI Gene ID | Synonyms |
|---|---|---|---|---|
| | potassium voltage-gated channel, KQT-like subfamily, member 2 | KCNQ2 | 3785 | BFNC, EBN, EBN1, ENB1, HNSPC, KCNA11, KQT-like 2, Kv7.2, KV7.2, KVEBN1, Neuroblastoma-specific potassium channel alpha subunit KvLQT2, Neuroblastoma-specific potassium channel subunit alpha KvLQT2, Potassium voltage-gated channel subfamily KQT member 2, Voltage-gated potassium channel subunit Kv7.2 |
| | potassium voltage-gated channel, KQT-like subfamily, member 3 | KCNQ3 | 3786 | BFNC2, EBN2, KQT-like 3, Kv7.3, KV7.3, Potassium channel alpha subunit KvLQT3, Potassium channel subunit alpha KvLQT3, Potassium voltage-gated channel subfamily KQT member 3, Voltage-gated potassium channel subunit Kv7.3 |
| | potassium voltage-gated channel, KQT-like subfamily, member 4 | KCNQ4 | 9132 | DFNA2, KQT-like 4, Kv7.4, KV7.4, Potassium channel alpha subunit KvLQT4, Potassium channel subunit alpha KvLQT4, Potassium voltage-gated channel subfamily KQT member 4, Voltage-gated potassium channel subunit Kv7.4 |
| | potassium voltage-gated channel, KQT-like subfamily, member 5 | KCNQ5 | 5647 9 | KQT-like 5, Kv7.5, Potassium channel alpha subunit KvLQT5, Potassium channel subunit alpha KvLQT5, Potassium voltage-gated channel subfamily KQT member 5, Voltage-gated potassium channel subunit Kv7.5 |
| | potassium channel regulator | KCNRG | 283518 | None |
| | potassium voltage-gated channel, delayed-rectifier, subfamily S, member 1 | KCNS1 | 3787 | Delayed-rectifier K(+) channel alpha subunit 1, Kv9.1, Potassium voltage-gated channel subfamily S member 1, Voltage-gated potassium channel subunit Kv9.1 |

(continued)

| Type | Subunit | Gene | NCBI Gene ID | Synonyms |
|---|---|---|---|---|
| | potassium voltage-gated channel, delayed-rectifier, subfamily S, member 2 | KCNS2 | 3788 | Delayed-rectifier K(+) channel alpha subunit 2, KIAA1144, Kv9.2, Potassium voltage-gated channel subfamily, S member 2, Voltage-gated potassium channel subunit Kv9.2 |
| | potassium voltage-gated channel, delayed-rectifier, subfamily S, member 3 | KCNS3 | 3790 | Delayed-rectifier K(+) channel alpha subunit 3, Kv9.3, KV9.3, MGC9481, Potassium voltage-gated channel subfamily S member 3, Voltage-gated potassium channel subunit Kv9.3 |
| | potassium channel, subfamily T, member 1 | KCNT1 | 57582 | bA100C15.2, FLJ41282, KCa4.1, KIAA1422, Potassium channel subfamily T member 1, SLACK |
| | potassium channel, subfamily T, member 2 | KCNT2 | 343350 | KCa4.2, MGC119610, MGC119611, MGC119612, MGC119613, SLICK, SLO2.1 |
| | Potassium channel, subfamily U, member 1 | KCNU1 | 157855 | KCa5.1, Kcnma3, KCNMA3, KCNMC1, Slo3, SLO3 |
| | potassium channel, subfamily V, member 1 | KCNV1 | 27012 | HNKA, KCNB3, KV2.3, Kv8.1, KV8.1 |
| | potassium channel, subfamily V, member 2 | KCNV2 | 169522 | KV11.1, Kv8.2, MGC120515, Potassium voltage-gated channel subfamily V member 2, RCD3B, Voltage-gated potassium channel subunit Kv8.2 |
| | | | | |
| Recep tor tyrosi ne kinase s | Fibroblast growth factor receptor 1 | FGFR1 | 2260 | Basic fibroblast growth factor receptor 1 precursor, BFGFR, bFGF-R, CD331, CD331 antigen, CEK, c-fgr, C-FGR, FGFBR, FGFR-1, fibroblast growth factor receptor 1 (fms-related tyrosine kinase 2, Pfeiffer syndrome), FLG, FLJ99988, FLT2, Fms-like tyrosine kinase 2, H2, H3, H4, H5, HBGFR, KAL2, N-SAM |

(continued)

| Type | Subunit | Gene | NCBI Gene ID | Synonyms |
|---|---|---|---|---|
| | Fibroblast growth factor receptor 2 | FGFR2 | 2263 | BEK, BFR-1, CD332, CD332 antigen, CEK3, CFD1, ECT1, FGFR-2, Fibroblast growth factor receptor 2 precursor, FLJ98662, JWS, Keratinocyte growth factor receptor 2, KGFR, KSAM, K-SAM, TK14, TK25 |
| | Fibroblast growth factor receptor 3 | FGFR3 | 2261 | ACH, CD333, CD333 antigen, CEK2, FGFR-3, fibroblast growth factor receptor 3 (achondroplasia, thanatophoric dwarfism), Fibroblast growth factor receptor 3 precursor, HSFGFR3EX, JTK4 |
| | Fibroblast growth factor receptor 4 | FGFR4 | 2264 | CD334, CD334 antigen, FGFR-4, Fibroblast growth factor receptor 4 precursor, JTK2, MGC20292, TKF |
| | Fibroblast growth factor receptor 6 | FGFR6 | 2265 | None |
| | platelet derived growth factor receptor A | PDGFR A | 5156 | Alpha platelet-derived growth factor receptor precursor, CD140a, CD140A, CD140a antigen, MGC74795, PDGFR2, PDGF-R-alpha, Rhe-PDGFRA |
| | platelet derived growth factor receptor B | PDGFR B | 5159 | Beta platelet-derived growth factor receptor precursor, CD140b, CD140B. CD140b antigen, JTK12, PDGFR, PDGFR1, PDGF-R-beta |
| | epidermal growth factor receptor | EGFR | 1956 | Epidermal growth factor receptor precursor, ERBB, ERBB1, HER1, mENA, PIG61, Receptor tyrosine-protein kinase ErbB-1 |
| | v-erb-b2 erythroblastic leukemia viral oncogene homolog 2 | ERBB2 | 2064 | CD340 antigen, C-erbB-2, c-erb B2, HER2, HER-2, HER-2/neu, MLN 19, NEU, NEU proto-oncogene, NGL, p185erbB2, Receptor tyrosine-protein kinase erbB-2 precursor, TKR1, Tyrosine kinase-type cell surface receptor HER2 |

(continued)

| Type | Subunit | Gene | NCBI Gene ID | Synonyms |
|---|---|---|---|---|
| | v-erb-b2 erythroblastic leukemia viral oncogene homolog 3 | ERBB3 | 2065 | c-erbB3, c-erbB-3, ErbB-3, erbB3-S, HER3, LCCS2, MDA-BF-1, MGC88033, p180-ErbB3, p45-sErbB3, p85-sErbB3, Receptor tyrosine-protein kinase erbB-3 precursor, Tyrosine kinase-type cell surface receptor HER3 |
| | v-erb-b2 erythroblastic leukemia viral oncogene homolog 4 | ERBB4 | 266 | HER4, MGC138404, p180erbB4, Receptor tyrosine-protein kinase erbB-4 precursor, Tyrosine kinase-type cell surface receptor HER4 |
| | | | | |
| Nuclear steroid receptors | estrogen receptor 1 | ESR1 | 2099 | DKFZp686N23123, ER, Era, ER-alpha, ESR, ESRA, Estradiol receptor, Estrogen receptor, major ORF, NR3A1 |
| | estrogen receptor 2 | ESR2 | 2100 | Erb, ER-beta, ER-BETA, ESRB, ESR-BETA, ESTRB, Estrogen receptor beta, NR3A2 |
| | Thyroid hormone receptor-α | THRA | 7067 | AR7, c-erbA-1, c-ERBA-1, C-erbA-alpha, c-ERBA-ALPHA-2, EAR7, EAR-7, EAR-7.1, EAR-7.1/EAR-7.2, EAR-7.2, ERBA, ERBA1, ERBA-ALPHA, ERB-T-1, MGC000261, MGC43240, NR1A1, THRA1, THRA2, THRA3, Thyroid hormone receptor alpha, TR-ALPHA-1 |
| | Thyroid hormone receptor-β | THRB | 7068 | ERBA2, ERBA-BETA, GRTH, MGC126109, MGC126110, NR1A2, PRTH, THR1, THRB1, THRB2, Thyroid hormone receptor beta-1, Thyroid hormone receptor beta-2 |
| | Retinoic acid receptor-α | RARA | 5914 | NR1 B1, RAR |
| | Retinoic acid receptor-β | RARB | 5915 | HAP, HBV-activated protein, NR1B2, RAR-beta, RAR-epsilon, Retinoic acid receptor beta, RRB2 |

(continued)

| Type | Subunit | Gene | NCBI Gene ID | Synonyms |
|---|---|---|---|---|
| | Retinoic acid receptor-γ | RARG | 5916 | RARC, RAR-gamma-1, RAR-gamma-2, Retinoic acid receptor gamma-1, Retinoic acid receptor gamma-2 |
| | Peroxisome proliferator-activated receptor-α | PPARA | 5465 | hPPAR, MGC2237, MGC2452, NR1C1, peroxisome proliferative activated receptor, alpha, Peroxisome proliferator-activated receptor alpha, PPAR, PPAR-alpha |
| | Peroxisome proliferator-activated receptor-β/δ | PPARD | 5467 | FAAR, MGC3931, NR1C2, NUC1, NUCI, NUCII, Nuclear hormone receptor 1, peroxisome proliferative activated receptor, delta, Peroxisome proliferator-activated receptor delta, PPARB, PPAR-beta, PPAR-delta |
| | Peroxisome proliferator-activated receptor-γ | PPARG | 5468 | HUMPPARG, NR1C3, peroxisome proliferative activated receptor, gamma, Peroxisome proliferator-activated receptor gamma, PPARG1, PPARG2, PPARgamma, PPAR-gamma |
| | Rev-ErbAα | NR1D1 | 9572 | EAR1, ear-1, hRev, HREV, Orphan nuclear receptor NR1D1, Rev-ErbAalpha, Rev-erbA-alpha, THRA1; THRAL, V-erbA-related protein EAR-1 |
| | Rev-ErbAβ | NR1D2 | 9975 | EAR-1 r, EAR-1 R, Hs.37288, HZF2, Orphan nuclear hormone receptor BD73, Orphan nuclear receptor NR1D2, Rev-erb-beta, RVR |
| | RAR-related orphan receptor-α | RORA | 6095 | MGC119326, MGC119329, NR1F1, Nuclear receptor ROR-alpha, Nuclear receptor RZR-alpha, Retinoid-related orphan receptor-alpha, ROR1, ROR2, ROR3, RZRA, RZR-ALPHA |
| | RAR-related orphan receptors-β | RORB | 6096 | bA133M9.1, NR1F2, Nuclear receptor ROR-beta, Nuclear receptor RZR-beta, Retinoid-related orphan receptor-beta, ROR-BETA, RZRB, RZR-BETA |

(continued)

| Type | Subunit | Gene | NCBI Gene ID | Synonyms |
|---|---|---|---|---|
| | RAR-related orphan receptor-γ | RORC | 6097 | NR1F3, Nuclear receptor ROR-gamma, Nuclear receptor RZR-gamma, Retinoid-related orphan receptor-gamma, RORG, RZRG, RZR-GAMMA, TOR |
| | Liver X receptor-α | NR1H3 | 10062 | Liver X receptor alpha, LXRA, LXR-a, Nuclear orphan receptor LXR-alpha, Oxysterols receptor LXR-alpha, RLD-1 |
| | Liver X receptor-β | NR1 H2 | 7376 | Liver X receptor beta, LXRB, LXR-b, NEAR, NER-I, Nuclear orphan receptor LXR-beta, Nuclear receptor NER, Oxysterols receptor LXR-beta, RIP15, Ubiquitously-expressed nuclear receptor, UNR |
| | Farnesoid X receptor | NR1 H4 | 9971 | BAR, Bile acid receptor, Farnesoid X-activated receptor, Farnesol receptor HRR-1, FXR, HRR1, HRR-1, MGC163445, Retinoid X receptor-interacting protein 14, RIP14, RXR-interacting protein 14 |
| | Vitamin D receptor | VDR | 7421 | 1,25-dihydroxyvitamin D3 receptor, NR1I1, Vitamin D3 receptor |
| | Pregnane X receptor | NR112 | 8856 | BXR, ONR1, Orphan nuclear receptor PAR1, Orphan nuclear receptor PXR, PAR, PAR1, PAR2, PARq, Pregnane X receptor, PRR, PXR, SAR, Steroid and xenobiotic receptor, SXR |

(continued)

| Type | Subunit | Gene | NCBI Gene ID | Synonyms |
|---|---|---|---|---|
| | Constitutive androstane receptor | NR113 | 9970 | CAR, CAR1, CAR-BETA, CAR-SV1, CAR-SV10, CAR-SV11, CAR-SV12, CAR-SV13, CAR-SV14, CAR-SV15, CAR-SV17, CAR-SV18, CAR-SV19, CAR-SV20, CAR-SV21, CAR-SV4, CAR-SV6, CAR-SV7, CAR-SV8, CAR-SV9, Constitutive activator of retinoid response, Constitutive active response, Constitutive androstane receptor, MB67, MGC150433, MGC97144, MGC97209, Orphan nuclear receptor MB67, Orphan nuclear receptor NR1I3 |
| | | | | |
| TGFbeta superfamily receptors | bone morphogenic protein receptor 1A | BMPR1A | 657 | Activin receptor-like kinase 3, ACVRLK3, ALK3, ALK-3, Bone morphogenetic protein receptor type IA precursor, CD292, CD292 antigen, Serine/threonine-protein kinase receptor R5, SKR5 |
| | bone morphogenic protein receptor 1B | BMPR1B | 658 | ALK6, ALK-6, Bone morphogenetic protein receptor type IB precursor, CDw293, CDw293 antigen |
| | bone morphogenic protein receptor 2A | BMPR2 | 659 | BMPR3, BMPR-II, BMP type II receptor, BMR2, Bone morphogenetic protein receptor type-2 precursor, Bone morphogenetic protein receptor type II, BRK-3, FLJ41585, FLJ76945, PPH1, T-ALK |
| | Activin receptor 2A | ACVR2A | 92 | Activin receptor type 2A precursor, Activin receptor type-2A precursor, Activin receptor type IIA, ACTRII, ACTRIIA, ACTR-IIA, ACVR2 |
| | Activin receptor 1B | ACVR1B | 91 | Activin receptor-like kinase 4, Activin receptor type 1 B precursor, ActRIB, ACTRIB, ACTR-IB, ACVRLK4, ALK4, ALK-4, Serine/threonine-protein kinase receptor R2, SKR2 |

(continued)

| Type | Subunit | Gene | NCBI Gene ID | Synonyms |
|---|---|---|---|---|
| | Activin receptor 2B | ACVR2B | 93 | receptor type 2B precursor, Activin receptor type-2B precursor, Activin receptor type IIB, ACTRIIB, ActR-IIB, ACTR-IIB, MGC116908 |
| | Activin receptor 1C | ACVR1C | 130399 | Activin receptor-like kinase 7, Activin receptor type 1C precursor, ACTR-IC, ACVRLK7, ALK7, ALK-7 |
| | transforming growth factor beta receptor 1 | TGFBRI | 7046 | AAT5, Activin receptor-like kinase 5, ACVRLK4, ALK5, ALK-5, LDS1A, LDS2A, Serine/threonine-protein kinase receptor R4, SKR4, TbetaR-I, TGF-beta receptor type-1 precursor, TGF-beta receptor type I, TGF-beta type I receptor, TGFR-1, transforming growth factor, beta receptor I (activin A receptor type II-like kinase, 53kDa), Transforming growth factor-beta receptor type I |
| | transforming growth factor beta receptor 2 | TGFBRII | 7048 | AAT3, FAA3, HNPCC6, LDS1B, LDS2B, MFS2, RIIC, TAAD2, TbetaR-II, TGF-beta receptor type-2 precursor, TGF-beta receptor type II, TGFbeta-RII, TGF-beta type II receptor, TGFR-2, Transforming growth factor-beta receptor type II |
| | transforming growth factor beta receptor 3 | TGFBRIII | 7049 | betaglycan, Betaglycan, BGCAN, TGF-beta receptor type III precursor, TGFR-3, transforming growth factor, beta receptor III (betaglycan, 300kDa), Transforming growth factor beta receptor III |
| | | | | |
| T- cell receptors | T- cell receptors | http://www.bioinf.org.uk/abs/ | | |
| B-cell receptors | B-cell receptors | http://www.bioinf.org.uk/abs/ | | |

Table 9 GABA subunits from various species.

| Receptor subunit | Gene name | Spicies |
|---|---|---|
| **GABAA:** | | |
| gamma-aminobutyric acid (GABA) A receptor, alpha 1 | GABRA1 | Homo sapiens |
| gamma-aminobutyric acid (GABA) A receptor, alpha 1 | Gabra1 | Mus musculus |
| gamma-aminobutyric acid (GABA) A receptor, alpha 1 | gabra1 | Danio rerio |
| gamma-aminobutyric acid (GABA) A receptor, alpha 1 | GABRA1 | Pan troglodytes |
| gamma-aminobutyric acid (GABA) A receptor, aipha 1 | GABRA1 (variant 1) | Bos taurus |
| gamma-aminobutyric acid (GABA) A receptor, alpha 1 | GABRA1 (variant 2) | Bos taurus |
| gamma-aminobutyric acid (GABA) A receptor, alpha 1 | GABRA1 | Gallus gallus |
| gamma-aminobutyric acid (GABA) A receptor, alpha 1 | GABRA1 | Canis familiaris |
| gamma-aminobutyric acid (GABA) A receptor, alpha 1 | Gabra1 | Rattus norvegicus |
| | | |
| gamma-aminobutyric acid (GABA) A receptor, alpha 2 | GABRA2 | Homo sapiens |
| gamma-aminobutyric acid (GABA) A receptor, alpha 2 | Gabra2 | Mus musculus |
| gamma-aminobutyric acid (GABA) A receptor, alpha 2 | LOC100150704 | Danio rerio |
| gamma-aminobutyric acid (GABA) A receptor, alpha 2 | GABRA2 | Pan troglodytes |
| gamma-aminobutyric acid (GABA) A receptor, alpha 2 | GABRA2 | Bos taurus |
| gamma-aminobutyric acid (GABA) A receptor, alpha 2 | GABRA2 | Gallus gallus |
| gamma-aminobutyric acid (GABA) A receptor, alpha 2 | GABRA2 | Canis familiaris |
| gamma-aminobutyric acid (GABA) A receptor, alpha 2 | LOC289606 | Rattus norvegicus |
| | | |
| gamma-aminobutyric acid (GABA) A receptor, alpha 3 | GABRA3 | Homo sapiens |
| gamma-aminobutyric acid (GABA) A receptor, alpha 3 | Gabra3 | Mus musculus |
| gamma-aminobutyric acid (GABA) A receptor, alpha 3 | Grd | Drosophila melanogaster |
| gamma-aminobutyric acid (GABA) A receptor, alpha 3 | GABRA3 | Bos taurus |
| gamma-aminobutyric acid (GABA) A receptor, alpha 3 | GABRA3 | Gallus gallus |
| gamma-aminobutyric acid (GABA) A receptor, alpha 3 | GABRA3 | Canis familiaris |
| gamma-aminobutyric acid (GABA) A receptor, alpha 3 | Gabra3 | Rattus norvegicus |
| | | |
| gamma-aminobutyric acid (GABA) A receptor, alpha 4 | GABRA4 | Homo sapiens |
| gamma-aminobutyric acid (GABA) A receptor, alpha 4 | Gabra4 | Mus musculus |
| gamma-aminobutyric acid (GABA) A receptor, alpha 4 | zgc:110204 | Danio rerio |
| gamma-aminobutyric acid (GABA) A receptor, alpha 4 | GABRA4 | Pan troglodytes |
| gamma-aminobutyric acid (GABA) A receptor, alpha 4 | GABRA4 | Bos taurus |
| gamma-aminobutyric acid (GABA) A receptor, alpha 4 | GABRA4 | Gallus gallus |
| gamma-aminobutyric acid (GABA) A receptor, alpha 4 | GABRA4 | Canis familiaris |
| gamma-aminobutyric acid (GABA) A receptor, alpha 4 | Gabra4 | Rattus norvegicus |
| | | |

(continued)

| Receptor subunit | Gene name | Spicies |
|---|---|---|
| **GABAA:** | | |
| gamma-aminobutyric acid (GABA) A receptor, alpha 5 | GABRA5 | Homo sapiens |
| gamma-aminobutyric acid (GABA) A receptor, alpha 5 | Gabra5 | Mus musculus |
| gamma-aminobutyric acid (GABA) A receptor, aipha 5 | CG8916 | Drosophila melanogaster |
| gamma-aminobutyric acid (GABA) A receptor, alpha 5 | Igc-37 | Caenorhabditis elegans |
| gamma-aminobutyric acid (GABA) A receptor, alpha 5 | LOC799124 | Danio rerio |
| gamma-aminobutyric acid (GABA) A receptor, alpha 5 | GABRA5 | Bos taurus |
| gamma-aminobutyric acid (GABA) A receptor, alpha 5 | GABRA5 | Gallus gallus |
| gamma-aminobutyric acid (GABA) A receptor, alpha 5 | GABRA5 | Canis familiaris |
| gamma-aminobutyric acid (GABA) A receptor, alpha 5 | Gabra5 | Rattus norvegicus |
| | | |
| gamma-aminobutyric acid (GABA) A receptor, alpha 6 | GABRA6 | Homo sapiens |
| gamma-aminobutyric acid (GABA) A receptor, alpha 6 | Gabra6 | Mus musculus |
| gamma-aminobutyric acid (GABA) A receptor, alpha 6 | Rdl | Drosophila melanogaster |
| gamma-aminobutyric acid (GABA) A receptor, alpha 6 | Igc-38 | Caenorhabditis elegans |
| gamma-aminobutyric acid (GABA) A receptor, alpha 6 | gabra6a | Danio rerio |
| gamma-aminobutyric acid (GABA) A receptor, aipha 6 | gabra6b | Danio rerio |
| gamma-aminobutyric acid (GABA) A receptor, alpha 6 | GABRA6 | Pan troglodytes |
| gamma-aminobutyric acid (GABA) A receptor, alpha 6 | GABRA6 | Bos taurus |
| gamma-aminobutyric acid (GABA) A receptor, alpha 6 | GABRA6 | Gallus gallus |
| gamma-aminobutyric acid (GABA) A receptor, alpha 6 | GABRA6 | Canis familiaris |
| gamma-aminobutyric acid (GABA) A receptor, alpha 6 | Gabra6 | Rattus norvegicus |
| | | |
| gamma-aminobutyric acid (GABA) A receptor, beta 1 | GABRB1 | Homo sapiens |
| gamma-aminobutyric acid (GABA) A receptor, beta 1 | Gabrb1 | Mus musculus |
| gamma-aminobutyric acid (GABA) A receptor, beta 1 | GABRB1 | Pan troglodytes |
| gamma-aminobutyric acid (GABA) A receptor, beta 1 | GABRB1 | Bos taurus |
| gamma-aminobutyric acid (GABA) A receptor, beta 1 | GABRB1 | Gallus gallus |
| gamma-aminobutyric acid (GABA) A receptor, beta 1 | Gabrb1 | Rattus norveqicus |
| | | |
| gamma-aminobutyric acid (GABA) A receptor, beta 2 | GABRB2 | Homo sapiens |
| gamma-aminobutyric acid (GABA) A receptor, beta 2 | Gabrb2 | Mus musculus |
| gamma-aminobutyric acid (GABA) A receptor, beta 2 | gabrb2 | Danio rerio |
| gamma-aminobutyric acid (GABA) A receptor, beta 2 | GABRB2 | Pan troglodytes |
| gamma-aminobutyric acid (GABA) A receptor, beta 2 | GABRB2 | Bos taurus |
| gamma-aminobutyric acid (GABA) A receptor, beta 2 | GABRB2 | Gallus gallus |
| gamma-aminobutyric acid (GABA) A receptor, beta 2 | GABRB2 | Canis familiaris |
| gamma-aminobutyric acid (GABA) A receptor, beta 2 | Gabrb2 | Rattus norvegicus |

(continued)

| Receptor subunit | Gene name | Spicies |
|---|---|---|
| **GABAA:** | | |
| gamma-aminobutyric acid (GABA) A receptor, beta 3 | GABRB3 | Homo sapiens |
| gamma-aminobutyric acid (GABA) A receptor, beta 3 | Gabrb3 | Mus musculus |
| gamma-aminobutyric acid (GABA) A receptor, beta 3 | Lcch3 | Drosophila melanogaster |
| gamma-aminobutyric acid (GABA) A receptor, beta 3 | gab-1 | Caenorhabditis elegans |
| gamma-aminobutyric acid (GABA) A receptor, beta 3 | LOC566922 | Danio rerio |
| gamma-aminobutyric acid (GABA) A receptor, beta 3 | GABRB3 | Pan troglodytes |
| gamma-aminobutyric acid (GABA) A receptor, beta 3 | GABRB3 | Gallus gallus |
| gamma-aminobutyric acid (GABA) A receptor, beta 3 | GABRB3 | Canis familiaris |
| gamma-aminobutyric acid (GABA) A receptor, beta 3 | Gabrb3 | Rattus norvegicus |
| | | |
| gamma-aminobutyric acid (GABA) A receptor, gamma 1 | GABRG1 | Homo sapiens |
| gamma-aminobutyric acid (GABA) A receptor, gamma 1 | Gabrg1 | Mus musculus |
| gamma-aminobutyric acid (GABA) A receptor, gamma 1 | LOC556202 | Danio rerio |
| gamma-aminobutyric acid (GABA) A receptor, gamma 1 | GABRG1 | Pan troglodytes |
| gamma-aminobutyric acid (GABA) A receptor, gamma 1 | GABRG1 | Bos taurus |
| gamma-aminobutyric acid (GABA) A receptor, gamma 1 | GABRG1 | Gallus gallus |
| gamma-aminobutyric acid (GABA) A receptor, gamma 1 | GABRG1 | Canis familiaris |
| gamma-aminobutyric acid (GABA) A receptor, gamma 1 | Gabrg1 | Rattus norvegicus |
| | | |
| gamma-aminobutyric acid (GABA) A receptor, gamma 2 | GABRG2 | Homo sapiens |
| gamma-aminobutyric acid (GABA) A receptor, gamma 2 | Gabrg2 | Mus musculus |
| gamma-aminobutyric acid (GABA) A receptor, gamma 2 | LOC553402 | Danio rerio |
| gamma-aminobutyric acid (GABA) A receptor, gamma 2 | GABRG2 | Bos taurus |
| gamma-aminobutyric acid (GABA) A receptor, gamma 2 | GABRG2 | Gallus gallus |
| gamma-aminobutyric acid (GABA) A receptor, gamma 2 | GABRG2 | Canis familiaris |
| gamma-aminobutyric acid (GABA) A receptor, gamma 2 | Gabrg2 | Rattus norvegicus |
| | | |
| gamma-aminobutyric acid (GABA) A receptor, gamma 3 | GABRG3 | Homo sapiens |
| gamma-aminobutyric acid (GABA) A receptor, gamma 3 | Gabrq3 | Mus musculus |
| gamma-aminobutyric acid (GABA) A receptor, gamma 3 | LOC567057 | Danio rerio |
| gamma-aminobutyric acid (GABA) A receptor, gamma 3 | GABRG3 | Gallus gallus |
| gamma-aminobutyric acid (GABA) A receptor, gamma 3 | Gabrg3 | Rattus norvegicus |
| | | |
| gamma-aminobutyric acid (GABA) A receptor, delta | GABRD | Homo sapiens |
| gamma-aminobutyric acid (GABA) A receptor, delta | Gabrd | Mus musculus |
| gamma-aminobutyric acid (GABA) A receptor, delta | DKEYP-87A12.2 | Danio rerio |
| gamma-aminobutyric acid (GABA) A receptor, delta | GABRD | Pan troglodytes |

(continued)

| Receptor subunit | Gene name | Spicies |
|---|---|---|
| **GABAA:** | | |
| gamma-aminobutyric acid (GABA) A receptor, delta | GABRD | Bos taurus |
| gamma-aminobutyric acid (GABA) A receptor, delta | GABRD | Gallus gallus |
| gamma-aminobutyric acid (GABA) A receptor, delta | GABRD | Canis familiaris |
| gamma-aminobutyric acid (GABA) A receptor, delta | Gabrd | Rattus norvegicus |
| | | |
| gamma-aminobutyric acid (GABA) A receptor, epsilon | GABRE | Homo sapiens |
| gamma-aminobutyric acid (GABA) A receptor, epsilon | Gabre | Mus musculus |
| gamma-aminobutyric acid (GABA) A receptor, epsilon | GABRE | Pan troglodytes |
| gamma-aminobutyric acid (GABA) A receptor, epsilon | GABRE | Bos taurus |
| gamma-aminobutyric acid (GABA) A receptor, epsilon | GABRE | Canis familiaris |
| gamma-aminobutyric acid (GABA) A receptor, epsilon | Gabre | Rattus norvegicus |
| | | |
| gamma-aminobutyric acid (GABA) A receptor, pi | GABRP | Homo sapiens |
| gamma-aminobutvric acid (GABA) A receptor, pi | Gabrp | Mus musculus |
| gamma-aminobutyric acid (GABA) A receptor, pi | GABRP | Pan troglodytes |
| gamma-aminobutyric acid (GABA) A receptor, pi | GABRP | Bos taurus |
| gamma-aminobutyric acid (GABA) A receptor, pi | GABRP | Gallus gallus |
| gamma-aminobutyric acid (GABA) A receptor, pi | GABRP | Canis familiaris |
| gamma-aminobutyric acid (GABA) A receptor, pi | Gabrp | Rattus norvegicus |
| | | |
| gamma-aminobutyric acid (GABA) A receptor, theta | GABRQ | Homo sapiens |
| gamma-aminobutyric acid (GABA) A receptor, theta | Gabrq | Mus musculus |
| gamma-aminobutyric acid (GABA) A receptor, theta | GABRQ | Pan troglodytes |
| gamma-aminobutyric acid (GABA) A receptor, theta | GABRQ | Bos taurus |
| gamma-aminobutyric acid (GABA) A receptor, theta | GABRQ | Canis familiaris |
| gamma-aminobutyric acid (GABA) A receptor, theta | Gabrq | Rattus norvegicus |
| | | |
| **GABAB:** | | |
| gamma-aminobutyric acid (GABA) B receptor, 1 | GABBR1 | Homo sapiens |
| gamma-aminobutyric acid (GABA) B receptor, 1 | Gabbr1 | Mus musculus |
| gamma-aminobutyric acid (GABA) B receptor, 1 | GABA-B-R1 | Drosophila melanogaster |
| gamma-aminobutyric acid (GABA) B receptor, 1 | Y41G9A.4 | Caenorhabditis elegans |
| gamma-aminobutyric acid (GABA) B receptor, 1 | gabbr1 | Danio rerio |
| gamma-aminobutyric acid (GABA) B receptor, 1 | GABBFc1 | Pan troglodytes |
| gamma-aminobutyric acid (GABA) B receptor, 1 | GABBR1 | Bos taurus |
| gamma-aminobutyric acid (GABA) B receptor, 1 | GABBR1 | Canis familiaris |
| gamma-aminobutyric acid (GABA) B receptors, 1 | Gabbr1 | Rattus norvegicus |

(continued)

| | | |
|---|---|---|
| **GABAB:** | | |
| | | |
| gamma-aminobutyric acid (GABA) B receptor, 2 | GABBR2 | Homo sapiens |
| gamma-aminobutyric acid (GABA) B receptor, 2 | Gabbr2 | Mus musculus |
| gamma-aminobutyric acid (GABA) B receptor, 2 | GABA-B-R2 | Drosophila melanogaster |
| gamma-aminobutyric acid (GABA) B receptor, 2 | si:dkey-190l1.2 | Danio rerio |
| gamma-aminobutyric acid (GABA) B receptor, 2 | GABBR2 | Pan troglodytes |
| gamma-aminobutyric acid (GABA) B receptor, 2 | GABBR2 | Bos taurus |
| gamma-aminobutyric acid (GABA) B receptor, 2 | GABBR2 | Gallus gallus |
| gamma-aminobutyric acid (GABA) B receptor, 2 | GABBR2 | Canis familiaris |
| gamma-aminobutyric acid (GABA) B receptor, 2 | Gabbr2 | Rattus norvegicus |
| | | |
| **GABAC:** | | |
| gamma-aminobutyric acid (GABA) A receptor, rho1 | GABRR1 | Homo sapiens |
| gamma-aminobutyric acid (GABA) A receptor, rho1 | Gabrr1 | Mus musculus |
| gamma-aminobutyric acid (GABA) A receptor, rho1 | gabrr1 | Danio rerio |
| gamma-aminobutyric acid (GABA) A receptor, rho1 | GABRR1 | Pan troglodytes |
| gamma-aminobutyric acid (GABA) A receptor, rho1 | GABRR1 | Bos taurus |
| gamma-aminobutyric acid (GABA) A receptor, rho1 | GABRR1 | Gallus gallus |
| gamma-aminobutyric acid (GABA) A receptor, rho1 | GABRR1 | Canis familiaris |
| gamma-aminobutyric acid (GABA) A receptor, rho1 | Gabrr1 | Rattus norvegicus |
| | | |
| gamma-aminobutyric acid (GABA) A receptor, rho2 | GABRR2 | Homo sapiens |
| gamma-aminobutyric acid (GABA) A receptor, rho2 | Gabrr2 | Mus musculus |
| gamma-aminobutyric acid (GABA) A receptor, rho2 | si:dkey-181i3.1 | Danio rerio |
| gamma-aminobutyric acid (GABA) A receptor, rho2 | GABRR2 | Pan troglodytes |
| gamma-aminobutyric acid (GABA) A receptor, rho2 | GABRR2 | Gallus gallus |
| gamma-aminobutyric acid (GABA) A receptor, rho2 | GABRR2 | Canis familiaris |
| gamma-aminobutyric acid (GABA) A receptor, rho2 | Gabrr2 | Rattus norvegicus |
| | | |
| gamma-aminobutyric acid (GABA) A receptor, rho3 | GABRR3 | Homo sapiens |
| gamma-aminobutyric acid (GABA) A receptor, rho3 | Gabrr3 | Mus musculus |
| gamma-aminobutyric acid (GABA) A receptor, rho3 | zgc: 194845 | Danio rerio |
| gamma-aminobutyric acid (GABA) A receptor, rho3 | GABRR3 | Pan troglodytes |
| gamma-aminobutyric acid (GABA) A receptor, rho3 | GABRR3 | Bos taurus |
| gamma-aminobutyric acid (GABA) A receptor, rho3 | GABRR3 | Gallus gallus |
| gamma-aminobutyric acid (GABA) A receptor, rho3 | Gabrr3 | Rattus norvegicus |

Table 10. Human bitter receptors

| Code | Receptor |
|---|---|
| F1 | hTAS2R1 |
| F5 | hTAS2R3 |
| F25 | hTAS2R4 |
| F11 | hTAS2R5 |
| F4 | hTAS2R7 |
| F2 | hTAS2R8 |
| F24 | hTAS2R9 |
| F16 | hTAS2R10 |
| F3 | hTAS2R13 |
| F15 | hTAS2R14 |
| F14 | hTAS2R16 |
| F7 | hTAS2R38 |
| F23 | hTAS2R39 |
| F19 | hTAS2R40 |
| F18 | hTAS2R41 |
| F6 | hTAS2R43 |
| F12 | hTAS2R44 |
| F8 | hTAS2R45 |
| F9 | hTAS2R46 |
| F22 | hTAS2R47 |
| F17 | hTAS2R48 |
| F21 | hTAS2R49 |
| F10 | hTAS2R50 |
| F13 | hTAS2R55 |
| F20 | hTAS2R60 |

Preferred G proteins in making bitter receptor cell lines

| |
|---|
| Mouse $G\alpha 15$ |
| Human GNA15 |

Table 11 Sweet and Umami receptors

| Type | Subunit | Gene Symbol | Splice form | NCBI Gene ID | Synonyms |
|---|---|---|---|---|---|
| Umami Taste | T1R1 | T1R1 | 1<br>2<br>3<br>4 | 80835 | TAS1R1, TR1, GPR70 |
| Sweet Taste | T1R2 | T1R2 | 1 | 80834 | TAS1R2, TR2, GPR71 |

(continued)

| Type | Subunit | Gene Symbol | Splice form | NCBI Gene ID | Synonyms |
|---|---|---|---|---|---|
| Umami/Sweet Taste | T1R3 | T1R3 | 1 | 83756 | TAS1R3 |

Table 12 Cystic Fibrosis Transmembrane-conductance Regulator

| Class | Protein # (UniProt) | Description |
|---|---|---|
| | | *Homo sapiens* cystic fibrosis transmembrane conductance regulator (CFTR) |
| | | *Homo sapiens* cystic fibrosis transmembrane conductance regulator (CFTR) mutant (F508) |

Table 13. Guanylyl Cyclases

| Class | Family/ *Subtype* | Protein # (UniProt) | Description |
|---|---|---|---|
| **Guanylyl cyclases** | | | Guanylate cyclase-A/ natriuretic peptide receptor A |
| | | | Guanylate cyclase-B/ natriuretic peptide receptor B |
| | | | Guanylate cyclase-C |
| | | | Guanylate cyclase-D |
| | | | Guanylate cyclase-E |
| | | | Guanylate cyclase-F |
| | | | Guanylate cyclase-G |
| Related receptor lacking guanylyl cyclase domain | | | Natriuretic peptide receptor C (NPR3) |

SEQUENCE TABLE

Human $GABA_A$ receptor alpha 1 subunit cDNA (SEQ ID NO: GABA1)

ATGAGGAAAAGTCCAGGTCTGTCTGACTGTCTTTGGGCCTGGATCCTC
CTTCTGAGCACACTGACTGGAAGAAGCTATGGACAGCCGTCATTACAA
GATGAACTTAAAGACAATACCACTGTCTTCACCAGGATTTTGGACAGA
CTCCTAGATGGTTATGACAATCGCCTGAGACCAGGATTGGGAGAGCG
TGTAACCGAAGTGAAGACTGATATCTTCGTCACCAGTTTCGGACCCGT
TTCAGACCATGATATGGAATATACAATAGATGTATTTTTCCGTCAAAGC
TGGAAGGATGAAAGGTTAAAATTTAAAGGACCTATGACAGTCCTCCGG
TTAAATAACCTAATGGCAAGTAAAATCTGGACTCCGGACACATTTTTCC
ACAATGGAAAGAAGTCAGTGGCCCACAACATGACCATGCCCAACAAA
CTCCTGCGGATCACAGAGGATGGCACCTTGCTGTACACCATGAGGCT
GACAGTGAGAGCTGAATGTCCGATGCATTTGGAGGACTTCCCTATGG
ATGCCCATGCTTGCCCACTAAAATTTGGAAGTTATGCTTATACAAGAG
CAGAAGTTGTTTATGAATGGACCAGAGAGCCAGCACGCTCAGTGGTT
GTAGCAGAAGATGGATCACGTCTAAACCAGTATGACCTTCTTGGACAA
ACAGTAGACTCTGGAATTGTCCAGTCAAGTACAGGAGAATATGTTGTT
ATGACCACTCATTTCCACTTGAAGAGAAAGATTGGCTACTTTGTTATTC
AAACATACCTGCCATGCATAATGACAGTGATTCTCTCACAAGTCTCCTT
CTGGCTCAACAGAGAGTCTGTACCAGCAAGAACTGTCTTTGGAGTAAC
AACTGTGCTCACCATGACAACATTGAGCATCAGTGCCAGAAACTCCCT
CCCTAAGGTGGCTTATGCAACAGCTATGGATTGGTTTATTGCCGTGTG
CTATGCCTTTGTGTTCTCAGCTCTGATTGAGTTTGCCACAGTAAACTAT
TTCACTAAGAGAGGTTATGCATGGGATGGCAAAAGTGTGGTTCCAGAA
AAGCCAAAGAAAGTAAAGGATCCTCTTATTAAGAAAAACAACACTTAC
GCTCCAACAGCAACCAGCTACACCCCTAATTTGGCCAGGGGCGACCC
GGGCTTAGCCACCATTGCTAAAAGTGCAACCATAGAACCTAAAGAGGT
CAAGCCCGAAACAAAACCACCAGAACCCAAGAAAACCTTTAACAGTGT
CAGCAAAATTGACCGACTGTCAAGAATAGCCTTCCCGCTGCTATTTGG
AATCTTTAACTTAGTCTACTGGGCTACGTATTTAAACAGAGAGCCTCAG
CTAAAAGCCCCCACACCACATCAATAG

Human $GABA_A$ receptor alpha 2 subunit cDNA (SEQ ID NO: GABA2)

ATGAAGACAAAATTGAACATCTACAACATGCAGTTCCTGCTTTTTGTTT
TCTTGGTGTGGGACCCTGCCAGGTTGGTGCTGGCTAACATCCAAGAA
GATGAGGCTAAAAATAACATTACCATCTTTACGAGAATTCTTGACAGAC
TTCTGGATGGTTACGATAATCGGCTTAGACCAGGACTGGGAGACAGT
ATTACTGAAGTCTTCACTAACATCTACGTGACCAGTTTTGGCCCTGTCT
CAGATACAGATATGGAATATACAATTGATGTTTTCTTTCGACAAAAATG
GAAAGATGAACGTTTAAAATTTAAAGGTCCTATGAATATCCTTCGACTA
AACAATTTAATGGCTAGCAAAATCTGGACTCCAGATACCTTTTTTCACA
ATGGGAAAAAATCAGTAGCTCATAATATGACAATGCCAAATAAGTTGCT
TCGAATTCAGGATGATGGGACTCTGCTGTATACCATGAGGCTTACAGT
TCAAGCTGAATGCCCAATGCACTTGGAGGATTTCCCAATGGATGCTCA
TTCATGTCCTCTGAAATTTGGCAGCTATGCATATACAACTTCAGAGGTC
ACTTATATTTGGACTTACAATGCATCTGATTCAGTACAGGTTGCTCCTG
ATGGCTCTAGGTTAAATCAATATGACCTGCTGGGCCAATCAATCGGAA

AGGAGACAATTAAATCCAGTACAGGTGAATATACTGTAATGACAGCTC
ATTTCCACCTGAAAAGAAAAATTGGGTATTTTGTGATTCAAACCTATCT
GCCTTGCATCATGACTGTCATTCTCTCCCAAGTTTCATTCTGGCTTAAC
AGAGAATCTGTGCCTGCAAGAACTGTGTTTGGAGTAACAACTGTCCTA
ACAATGACAACTCTAAGCATCAGTGCTCGGAATTCTCTCCCCAAAGTG
GCTTATGCAACTGCCATGGACTGGTTTATTGCTGTTTGTTATGCATTTG
TGTTCTCTGCCCTAATTGAATTTGCAACTGTTAATTACTTCACCAAAAG
AGGATGGACTTGGGATGGGAAGAGTGTAGTAAATGACAAGAAAAAAG
AAAAGGCTTCCGTTATGATACAGAACAACGCTTATGCAGTGGCTGTTG
CCAATTATGCCCCGAATCTTTCAAAAGATCCAGTTCTCTCCACCATCTC
CAAGAGTGCAACCACGCCAGAACCCAACAAGAAGCCAGAAAACAAGC
CAGCTGAAGCAAAGAAAACTTTCAACAGTGTTAGCAAAATTGACAGAA
TGTCCAGAATAGTTTTTCCAGTTTTGTTTGGTACCTTTAATTTAGTTTAC
TGGGCTACATATTTAAACAGAGAACCTGTATTAGGGGTCAGTCCTTGA

Human GABA$_A$ receptor alpha 3 subunit cDNA (SEQ ID NO: GABA3)

ATGATAATCACACAAACAAGTCACTGTTACATGACCAGCCTTGGGATT
CTTTTCCTGATTAATATTCTCCCTGGAACCACTGGTCAAGGGGAATCA
AGACGACAAGAACCCGGGGACTTTGTGAAGCAGGACATTGGCGGGCT
GTCTCCTAAGCATGCCCCAGATATTCCTGATGACAGCACTGACAACAT
CACTATCTTCACCAGAATCTTGGATCGTCTTCTGGACGGCTATGACAA
CCGGCTGCGACCTGGGCTTGGAGATGCAGTGACTGAAGTGAAGACTG
ACATCTACGTGACCAGTTTTGGCCCTGTGTCAGACACTGACATGGAGT
ACACTATTGATGTATTTTTTCGGCAGACATGGCATGATGAAAGACTGA
AATTTGATGGCCCCATGAAGATCCTTCCACTGAACAATCTCCTGGCTA
GTAAGATCTGGACACCGGACACCTTCTTCCACAATGGCAAGAAATCAG
TGGCTCATAACATGACCACGCCCAACAAGCTGCTCAGATTGGTGGAC
AACGGAACCCTCCTCTATACAATGAGGTTAACAATTCATGCTGAGTGT
CCCATGCATTTGGAAGATTTTCCCATGGATGTGCATGCCTGCCCACTG
AAGTTTGGAAGCTATGCCTATACAACAGCTGAAGTGGTTTATTCTTGG
ACTCTCGGAAAGAACAAATCCGTGGAAGTGGCACAGGATGGTTCTCG
CTTGAACCAGTATGACCTTTTGGGCCATGTTGTTGGGACAGAGATAAT
CCGGTCTAGTACAGGAGAATATGTCGTCATGACAACCCACTTCCATCT
CAAGCGAAAAATTGGCTACTTTGTGATCCAGACCTACTTGCCATGTAT
CATGACTGTCATTCTGTCACAAGTGTCGTTCTGGCTCAACAGAGAGTC
TGTTCCTGCCCGTACAGTCTTTGGTGTCACCACTGTGCTTACCATGAC
CACCTTGAGTATCAGTGCCAGAAATTCCTTACCTAAAGTGGCATATGC
GACGGCCATGGACTGGTTCATAGCCGTCTGTTATGCCTTTGTATTTTC
TGCACTGATTGAATTTGCCACTGTCAACTATTTCACCAAGCGGAGTTG
GGCTTGGGAAGGCAAGAAGGTGCCAGAGGCCCTGGAGATGAAGAAG
AAAACACCAGCAGCCCCAGCAAAGAAAACCAGCACTACCTTCAACATC
GTGGGGACCACCTATCCCATCAACCTGGCCAAGGACACTGAATTTTC
CACCATCTCCAAGGGCGCTGCTCCCAGTGCCTCCTCAACCCCAACAA
TCATTGCTTCACCCAAGGCCACCTACGTGCAGGACAGCCCGACTGAG
ACCAAGACCTACAACAGTGTCAGCAAGGTTGACAAAATTTCCCGCATC
ATCTTTCCTGTGCTCTTTGCCATATTCAATCTGGTCTATTGGGCCACAT
ATGTCAACCGGGAGTCAGCTATCAAGGGCATGATCCGCAAACAGTAG

Human GABA$_A$ receptor alpha 5 subunit cDNA (SEQ ID NO: GABA4)

ATGGACAATGGAATGTTCTCTGGTTTTATCATGATCAAAAACCTCCTTC
TCTTTTGTATTTCCATGAACTTATCCAGTCACTTTGGCTTTTCACAGAT
GCCAACCAGTTCAGTGAAAGATGAGACCAATGACAACATCACGATATT
TACCAGGATCTTGGATGGGCTCTTGGATGGCTACGACAACAGACTTC
GGCCCGGGCTGGGAGAGCGCATCACTCAGGTGAGGACCGACATCTA
CGTCACCAGCTTCGGCCCGGTGTCCGACACGGAAATGGAGTACACCA
TAGACGTGTTTTTCCGACAAAGCTGGAAAGATGAAAGGCTTCGGTTTA
AGGGGCCCATGCAGCGCCTCCCTCTCAACAACCTCCTTGCCAGCAAG
ATCTGGACCCCAGACACGTTCTTCCACAACGGGAAGAAGTCCATCGC
TCACAACATGACCACGCCCAACAAGCTGCTGCGGCTGGAGGACGACG
GCACCCTGCTCTACACCATGCGCTTGACCATCTCTGCAGAGTGCCCC
ATGCAGCTTGAGGACTTCCCGATGGATGCGCACGCTTGCCCTCTGAA
ATTTGGCAGCTATGCGTACCCTAATTCTGAAGTCGTCTACGTCTGGAC
CAACGGCTCCACCAAGTCGGTGGTGGTGGCGGAAGATGGCTCCAGA
CTGAACCAGTACCACCTGATGGGGCAGACGGTGGGCACTGAGAACAT
CAGCACCAGCACAGGCGAATACACAATCATGACAGCTCACTTCCACCT
GAAAAGGAAGATTGGCTACTTTGTCATCCAGACCTACCTTCCCTGCAT
AATGACCGTGATCTTATCACAGGTGTCCTTTTGGCTGAACCGGGAATC
AGTCCCAGCCAGGACAGTTTTTGGGGTCACCACGGTGCTGACCATGA
CGACCCTCAGCATCAGCGCCAGGAACTCTCTGCCCAAAGTGGCCTAC
GCCACCGCCATGGACTGGTTCATAGCCGTGTGCTATGCCTTCGTCTT
CTCGGCGCTGATAGAGTTTGCCACGGTCAATTACTTTACCAAGAGAGG
CTGGGCCTGGGATGGCAAAAAAGCCTTGGAAGCAGCCAAGATCAAGA
AAAAGCGTGAAGTCATACTAAATAAGTCAACAAACGCTTTTACAACTG
GGAAGATGTCTCACCCCCCAAACATTCCGAAGGAACAGACCCCAGCA
GGGACGTCGAATACAACCTCAGTCTCAGTAAAACCCTCTGAAGAGAA
GACTTCTGAAAGCAAAAAGACTTACAACAGTATCAGCAAAATTGACAA
AATGTCCCGAATCGTATTCCCAGTCTTGTTCGGCACTTTCAACTTAGTT
TACTGGGCAACGTATTTGAATAGGGAGCCGGTGATAAAAGGAGCCGC
CTCTCCAAAATAA

Human $GABA_A$ receptor beta 3 variant 1 subunit cDNA (SEQ ID NO: GABA5)

ATGTGGGGCCTTGCGGGAGGAAGGCTTTTCGGCATCTTCTCGGCCCC
GGTGCTGGTGGCTGTGGTGTGCTGCGCCCAGAGTGTGAACGATCCC
GGGAACATGTCCTTTGTGAAGGAGACGGTGGACAAGCTGTTGAAAGG
CTACGACATTCGCCTAAGACCCGACTTCGGGGGTCCCCCGGTCTGCG
TGGGGATGAACATCGACATCGCCAGCATCGACATGGTTTCCGAAGTC
AACATGGATTATACCTTAACCATGTATTTTCAACAATATTGGAGAGATA
AAAGGCTCGCCTATTCTGGGATCCCTCTCAACCTCACGCTTGACAATC
GAGTGGCTGACCAGCTATGGGTGCCCGACACATATTTCTTAAATGACA
AAAAGTCATTTGTGCATGGAGTGACAGTGAAAAACCGCATGATCCGTC
TTCACCCTGATGGGACAGTGCTGTATGGGCTCAGAATCACCACGACA
GCAGCATGCATGATGGACCTCAGGAGATACCCCCTGGACGAGCAGAA
CTGCACTCTGGAAATTGAAAGCTATGGCTACACCACGGATGACATTGA
GTTTTACTGGCGAGGCGGGGACAAGGCTGTTACCGGAGTGGAAAGG
ATTGAGCTCCCGCAGTTCTCCATCGTGGAGCACCGTCTGGTCTCGAG

GAATGTTGTCTTCGCCACAGGTGCCTATCCTCGACTGTCACTGAGCTT
TCGGTTGAAGAGGAACATTGGATACTTCATTCTTCAGACTTATATGCC
CTCTATACTGATAACGATTCTGTCGTGGGTGTCCTTCTGGATCAATTAT
GATGCATCTGCTGCTAGAGTTGCCCTCGGGATCACAACTGTGCTGAC
AATGACAACCATCAACACCCACCTTCGGGAGACCTTGCCCAAAATCCC
CTATGTCAAAGCCATTGACATGTACCTTATGGGCTGCTTCGTCTTTGT
GTTCCTGGCCCTTCTGGAGTATGCCTTTGTCAACTACATTTTCTTTGGA
AGAGGCCCTCAAAGGCAGAAGAAGCTTGCAGAAAAGACAGCCAAGGC
AAAGAATGACCGTTCAAAGAGCGAAAGCAACCGGGTGGATGCTCATG
GAAATATTCTGTTGACATCGCTGGAAGTTCACAATGAAATGAATGAGG
TCTCAGGCGGCATTGGCGATACCAGGAATTCAGCAATATCCTTTGACA
ACTCAGGAATCCAGTACAGGAAACAGAGCATGCCTCGAGAAGGGCAT
GGGCGATTCCTGGGGGACAGAAGCCTCCCGCACAAGAAGACCCATCT
ACGGAGGAGGTCTTCACAGCTCAAAATTAAAATACCTGATCTAACCGA
TGTGAATGCCATAGACAGATGGTCCAGGATCGTGTTTCCATTCACTTT
TTCTCTTTTCAACTTAGTTTACTGGCTGTACTATGTTAACTGA

Human $GABA_A$ receptor gamma 2 transcript variant 1 (short) subunit cDNA (SEQ ID NO: GABA6)

ATGAGTTCGCCAAATATATGGAGCACAGGAAGCTCAGTCTACTCGACT
CCTGTATTTTCACAGAAAATGACGGTGTGGATTCTGCTCCTGCTGTCG
CTCTACCCTGGCTTCACTAGCCAGAAATCTGATGATGACTATGAAGAT
TATGCTTCTAACAAAACATGGGTCTTGACTCCAAAAGTTCCTGAGGGT
GATGTCACTGTCATCTTAAACAACCTGCTGGAAGGATATGACAATAAA
CTTCGGCCTGATATAGGAGTGAAGCCAACGTTAATTCACACAGACATG
TATGTGAATAGCATTGGTCCAGTGAACGCTATCAATATGGAATACACT
ATTGATATATTTTTTGCGCAAACGTGGTATGACAGACGTTTGAAATTTA
ACAGCACCATTAAAGTCCTCCGATTGAACAGCAACATGGTGGGGAAAA
TCTGGATTCCAGACACTTTCTTCAGAAATTCCAAAAAAGCTGATGCACA
CTGGATCACCACCCCCAACAGGATGCTGAGAATTTGGAATGATGGTC
GAGTGCTCTACACCCTAAGGTTGACAATTGATGCTGAGTGCCAATTAC
AATTGCACAACTTTCCAATGGATGAACACTCCTGCCCCTTGGAGTTCT
CAAGTTATGGCTATCCACGTGAAGAAATTGTTTATCAATGGAAGCGAA
GTTCTGTTGAAGTGGGCGACACAAGATCCTGGAGGCTTTATCAATTCT
CATTTGTTGGTCTAAGAAATACCACCGAAGTAGTGAAGACAACTTCCG
GAGATTATGTGGTCATGTCTGTCTACTTTGATCTGAGCAGAAGAATGG
GATACTTTACCATCCAGACCTATATCCCCTGCACACTCATTGTCGTCCT
ATCCTGGGTGTCTTTCTGGATCAATAAGGATGCTGTTCCAGCCAGAAC
ATCTTTAGGTATCACCACTGTCCTGACAATGACCACCCTCAGCACCAT
TGCCCGGAAATCGCTCCCCAAGGTCTCCTATGTCACAGCGATGGATC
TCTTTGTATCTGTTTGTTTCATCTTTGTCTTCTCTGCTCTGGTGGAGTA
TGGCACCTTGCATTATTTTGTCAGCAACCGGAAACCAAGCAAGGACAA
AGATAAAAAGAAGAAAAACCCTGCCCCTACCATTGATATCCGCCCAAG
ATCAGCAACCATTCAAATGAATAATGCTACACACCTTCAAGAGAGAGA
TGAAGAGTACGGCTATGAGTGTCTGGACGGCAAGGACTGTGCCAGTT
TTTTCTGCTGTTTTGAAGATTGTCGAACAGGAGCTTGGAGACATGGGA
GGATACATATCCGCATTGCCAAAATGGACTCCTATGCTCGGATCTTCT

TCCCCACTGCCTTCTGCCTGTTTAATCTGGTCTATTGGGTCTCCTACC
TCTACCTGTGA

GABA Target 1 (SEQ ID NO: GABA7)
5'-GTTCTTAAGGCACAGGAACTGGGAC-3'

GABA Target 2 (SEQ ID NO: GABA8)
5'-GAAGTTAACCCTGTCGTTCTGCGAC-3'

GABA Target 3 (SEQ ID NO: GABA9)
5'-GTTCTATAGGGTCTGCTTGTCGCTC-3'

GABA Signal Probe 1 (SEQ ID NO: GABA10)
5' - **Cy5** GCCAGTCCCAGTTCCTGTGCCTTAAGAACCTCGC **BHQ3 quench** -3'

GABA Signal Probe 2 (SEQ ID NO: GABA11)
5'- **Cy5**.**5** GCGAGTCGCAGAACGACAGGGTTAACTTCCTCGC **BHQ3 quench** -3'

GABA Signal Probe 3 (SEQ ID NO: GABA12)

5'- **Fam** GCGAGAGCGACAAGCAGACCCTATAGAACCTCGC **BHQ1 quench** -3"

**SEQ ID NO: GCC1**

5'-GTTCTTAAGGCACAGGAACTGGGAC-3'

**SEQ ID NO: GCC 2**

5' - Cy5 GCCAGTCCCAGTTCCTGTGCCTTAAGAACCTCGC BHQ2 -3'

**SEQ ID NO: GCC 3 (GUCY2C (guanylate cyclase 2C) nucleotide sequence)**

ATGAAGACGTTGCTGTTGGACTTGGCTTTGTGGTCACTGCTCTTCCAG
CCCGGGTGGCTGTCCTTTAGTTCCCAGGTGAGTCAGAACTGCCACAA

TGGCAGCTATGAAATCAGCGTCCTGATGATGGGCAACTCAGCCTTTG
CAGAGCCCCTGAAAAACTTGGAAGATGCGGTGAATGAGGGGCTGGAA
ATAGTGAGAGGACGTCTGCAAAATGCTGGCCTAAATGTGACTGTGAAC
GCTACTTTCATGTATTCGGATGGTCTGATTCATAACTCAGGCGACTGC
CGGAGTAGCACCTGTGAAGGCCTCGACCTACTCAGGAAAATTTCAAAT
GCACAACGGATGGGCTGTGTCCTCATAGGGCCCTCATGTACATACTC
CACCTTCCAGATGTACCTTGACACAGAATTGAGCTACCCCATGATCTC
AGCTGGAAGTTTTGGATTGTCATGTGACTATAAAGAAACCTTAACCAG
GCTGATGTCTCCAGCTAGAAAGTTGATGTACTTCTTGGTTAACTTTTGG
AAAACCAACGATCTGCCCTTCAAAACTTATTCCTGGAGCACTTCGTAT
GTTTACAAGAATGGTACAGAAACTGAGGACTGTTTCTGGTACCTTAAT
GCTCTGGAGGCTAGCGTTTCCTATTTCTCCCACGAACTCGGCTTTAAG
GTGGTGTTAAGACAAGATAAGGAGTTTCAGGATATCTTAATGGACCAC
AACAGGAAAAGCAATGTGATTATTATGTGTGGTGGTCCAGAGTTCCTC
TACAAGCTGAAGGGTGACCGAGCAGTGGCTGAAGACATTGTCATTATT
CTAGTGGATCTTTTCAATGACCAGTACTTGGAGGACAATGTCACAGCC
CCTGACTATATGAAAAATGTCCTTGTTCTGACGCTGTCTCCTGGGAAT
TCCCTTCTAAATAGCTCTTTCTCCAGGAATCTATCACCAACAAAACGAG
ACTTTGCTCTTGCCTATTTGAATGGAATCCTGCTCTTTGGACATATGCT
GAAGATATTTCTTGAAAATGGAGAAAATATTACCACCCCCAAATTTGCT
CATGCTTTCAGGAATCTCACTTTTGAAGGGTATGACGGTCCAGTGACC
TTGGATGACTGGGGGGATGTTGACAGTACCATGGTGCTTCTGTATACC
TCTGTGGACACCAAGAAATACAAGGTTCTTTTGACCTATGATACCCAC
GTAAATAAGACCTATCCTGTGGATATGAGCCCCACATTCACTTGGAAG
AACTCTAAACTTCCTAATGATATTACAGGCCGGGGCCCTCAGATCCTG
ATGATTGCAGTCTTCACCCTCACTGGAGCTGTGGTGCTGCTCCTGCTC
GTCGCTCTCCTGATGCTCAGAAAATATAGAAAAGATTATGAACTTCGT
CAGAAAAAATGGTCCCACATTCCTCCTGAAAATATCTTTCCTCTGGAG
ACCAATGAGACCAATCATGTTAGCCTCAAGATCGATGATGACAAAAGA
CGAGATACAATCCAGAGACTACGACAGTGCAAATACGACAAAAAGCG
AGTGATTCTCAAAGATCTCAAGCACAATGATGGTAATTTCACTGAAAAA
CAGAAGATAGAATTGAACAAGTTGCTTCAGATTGACTATTACAACCTGA

CCAAGTTCTACGGCACAGTGAAACTTGATACCATGATCTTCGGGGTGA
TAGAATACTGTGAGAGAGGATCCCTCCGGGAAGTTTTAAATGACACAA
TTTCCTACCCTGATGGCACATTCATGGATTGGGAGTTTAAGATCTCTG
TCTTGTATGACATTGCTAAGGGAATGTCATATCTGCACTCCAGTAAGA
CAGAAGTCCATGGTCGTCTGAAATCTACCAACTGCGTAGTGGACAGTA
GAATGGTGGTGAAGATCACTGATTTTGGCTGCAATTCCATTTTACCTC
CAAAAAAGGACCTGTGGACAGCTCCAGAGCACCTCCGCCAAGCCAAC
ATCTCTCAGAAAGGAGATGTGTACAGCTATGGGATCATCGCACAGGA
GATCATTCTGCGGAAAGAAACCTTCTACACTTTGAGCTGTCGGGACCG
GAATGAGAAGATTTTCAGAGTGGAAAATTCCAATGGAATGAAACCCTT
CCGCCCAGATTTATTCTTGGAAACAGCAGAGGAAAAAGAGCTAGAAGT
GTACCTACTTGTAAAAAACTGTTGGGAGGAAGATCCAGAAAAGAGACC
AGATTTCAAAAAAATTGAGACTACACTTGCCAAGATATTTGGACTTTTT
CATGACCAAAAAAATGAAAGCTATATGGATACCTTGATCCGACGTCTA
CAGCTATATTCTCGAAACCTGGAACATCTGGTAGAGGAAAGGACACAG
CTGTACAAGGCAGAGAGGGACAGGGCTGACAGACTTAACTTTATGTT
GCTTCCAAGGCTAGTGGTAAAGTCTCTGAAGGAGAAAGGCTTTGTGG
AGCCGGAACTATATGAGGAAGTTACAATCTACTTCAGTGACATTGTAG
GTTTCACTACTATCTGCAAATACAGCACCCCCATGGAAGTGGTGGACA
TGCTTAATGACATCTATAAGAGTTTTGACCACATTGTTGATCATCATGA
TGTCTACAAGGTGGAAACCATCGGTGATGCGTACATGGTGGCTAGTG
GTTTGCCTAAGAGAAATGGCAATCGGCATGCAATAGACATTGCCAAGA
TGGCCTTGGAAATCCTCAGCTTCATGGGGACCTTTGAGCTGGAGCAT
CTTCCTGGCCTCCCAATATGGATTCGCATTGGAGTTCACTCTGGTCCC
TGTGCTGCTGGAGTTGTGGGAATCAAGATGCCTCGTTATTGTCTATTT
GGAGATACGGTCAACACAGCCTCTAGGATGGAATCCACTGGCCTCCC
TTTGAGAATTCACGTGAGTGGCTCCACCATAGCCATCCTGAAGAGAAC
TGAGTGCCAGTTCCTTTATGAAGTGAGAGGAGAAACATACTTAAAGGG
AAGAGGAAATGAGACTACCTACTGGCTGACTGGGATGAAGGACCAGA
AATTCAACCTGCCAACCCCTCCTACTGTGGAGAATCAACAGCGTTTGC
AAGCAGAATTTTCAGACATGATTGCCAACTCTTTACAGAAAAGACAGG
CAGCAGGGATAAGAAGCCAAAAACCCAGACGGGTAGCCAGCTATAAA

AAAGGCACTCTGGAATACTTGCAGCTGAATACCACAGACAAGGAGAG
CACCTATTTTTAA

**[0340]** Homo sapiens (H. s.) cystic fibrosis transmembrane conductance regulator (CFTR) nucleotide sequence (SEQ ID NO: CFTR1):

atgcagaggtcgcctctggaaaaggccagcgttgtctccaaactttttttcagctggaccagaccaatttt
gaggaaaggatacagacagcgcctggaattgtcagacatataccaaatcccttctgttgattctgctgac
aatctatctgaaaaattggaaagagaatgggatagagagctggcttcaaagaaaaatcctaaactcatt
aatgcccttcggcgatgtttttctggagatttatgttctatggaatctttttatatttaggggaagtcaccaaag
cagtacagcctctcttactgggaagaatcatagcttcctatgacccggataacaaggaggaacgctcta
tcgcgatttatctaggcataggcttatgccttctctttattgtgaggacactgctcctacacccagccatttttg
gccttcatcacattggaatgcagatgagaatagctatgtttagtttgatttataagaagactttaaagctgtc
aagccgtgttctagataaaataagtattggacaacttgttagtctcctttccaacaacctgaacaaatttgat
gaaggacttgcattggcacatttcgtgtggatcgctcctttgcaagtggcactcctcatggggctaatctgg
gagttgttacaggcgtctgccttctgtggacttggtttcctgatagtccttgccctttttcaggctgggctaggg
agaatgatgatgaagtacagagatcagagagctgggaagatcagtgaaagacttgtgattacctcag
aaatgattgaaaatatccaatctgttaaggcatactgctgggaagaagcaatggaaaaaatgattgaa
aacttaagacaaacagaactgaaactgactcggaaggcagcctatgtgagatacttcaatagctcagc
cttcttcttctcagggttctttgtggtgtttttatctgtgcttccctatgcactaatcaaaggaatcatcctccgga
aaatattcaccaccatctcattctgcattgttctgcgcatggcggtcactcggcaatttccctgggctgtaca
aacatggtatgactctcttggagcaataaacaaaatacaggatttcttacaaaagcaagaatataagac
attggaatataacttaacgactacagaagtagtgatggagaatgtaacagccttctgggaggagggatt
tggggaattatttgagaaagcaaaacaaaacaataacaatagaaaaacttctaatggtgatgacagcc
tcttcttcagtaatttctcacttcttggtactcctgtcctgaaagatattaatttcaagatagaaagaggacagt
tgttggcggttgctggatccactggagcaggcaagacttcacttctaatggtgattatgggagaactgga
gccttcagagggtaaaattaagcacagtggaagaatttcattctgttctcagttttcctggattatgcctggc
accattaaagaaaatatcatctttggtgtttcctatgatgaatatagatacagaagcgtcatcaaagcatg
ccaactagaagaggacatctccaagtttgcagagaaagacaatatagttcttggagaaggtggaatca
cactgagtggaggtcaacgagcaagaatttctttagcaagagcagtatacaaagatgctgatttgtattta
ttagactctccttttggatacctagatgttttaacagaaaaagaaatatttgaaagctgtgtctgtaaactgat
ggctaacaaaactaggattttggtcacttctaaaatggaacatttaaagaaagctgacaaaatattaatttt
gcatgaaggtagcagctatttttatgggacattttcagaactccaaaatctacagccagactttagctcaa
aactcatgggatgtgattctttcgaccaatttagtgcagaaagaagaaattcaatcctaactgagacctta
caccgtttctcattagaaggagatgctcctgtctcctggacagaaacaaaaaaacaatcttttaaacaga
ctggagagtttggggaaaaaaggaagaattctattctcaatccaatcaactctatacgaaaattttccatt
gtgcaaaagactcccttacaaatgaatggcatcgaagaggattctgatgagcctttagagagaaggct
gtccttagtaccagattctgagcagggagaggcgatactgcctcgcatcagcgtgatcagcactggccc
cacgcttcaggcacgaaggaggcagtctgtcctgaacctgatgacacactcagttaaccaaggtcaga
acattcaccgaaagacaacagcatccacacgaaaagtgtcactggcccctcaggcaaacttgactga
actggatatatattcaagaaggttatctcaagaaactggcttggaaataagtgaagaaattaacgaaga
agacttaaaggagtgcttttttgatgatatggagagcataccagcagtgactacatggaacacatacctt
cgatatattactgtccacaagagcttaattttgtgctaatttggtgcttagtaatttttctggcagaggtggctg
cttctttggttgtgctgtggctccttggaaacactcctcttcaagacaaagggaatagtactcatagtagaa
ataacagctatgcagtgattatcaccagcaccagttcgtattatgtgttttacatttacgtgggagtagccga
cactttgcttgctatgggattcttcagaggtctaccactggtgcatactctaatcacagtgtcgaaaattttac
accacaaaatgttacattctgttcttcaagcacctatgtcaaccctcaacacgttgaaagcaggtgggatt
cttaatagattctccaaagatatagcaattttggatgaccttctgcctcttaccatatttgacttcatccagttgt

tattaattgtgattggagctatagcagttgtcgcagttttacaaccctacatctttgttgcaacagtgccagtg
atagtggcttttattatgttgagagcatatttcctccaaacctcacagcaactcaaacaactggaatctgaa
ggcaggagtccaattttcactcatcttgttacaagcttaaaaggactatggacacttcgtgccttcggacg
gcagccttactttgaaactctgttccacaaagctctgaatttacatactgccaactggttcttgtacctgtcaa
cactgcgctggttccaaatgagaatagaaatgatttttgtcatcttcttcattgctgttaccttcatttccatttta
acaacaggagaaggagaaggaagagttggtattatcctgactttagccatgaatatcatgagtacattg
cagtgggctgtaaactccagcatagatgtggatagcttgatgcgatctgtgagccgagtctttaagttcatt
gacatgccaacagaaggtaaacctaccaagtcaaccaaaccatacaagaatggccaactctcgaaa
gttatgattattgagaattcacacgtgaagaaagatgacatctggccctcagggggccaaatgactgtc
aaagatctcacagcaaaatacacagaaggtggaaatgccatattagagaacatttccttctcaataagt
cctggccagagggtgggcctcttgggaagaactggatcagggaagagtactttgttatcagctttttgag
actactgaacactgaaggagaaatccagatcgatggtgtgtcttgggattcaataactttgcaacagtgg
aggaaagcctttggagtgataccacagaaagtatttatttttctggaacatttagaaaaaacttggatccc
tatgaacagtggagtgatcaagaaatatggaaagttgcagatgaggttgggctcagatctgtgatagaa
cagtttcctgggaagcttgactttgtccttgtggatgggggctgtgtcctaagccatggccacaagcagttg
atgtgcttggctagatctgttctcagtaaggcgaagatcttgctgcttgatgaacccagtgctcatttggatc
cagtaacataccaaataattagaagaactctaaaacaagcatttgctgattgcacagtaattctctgtga
acacaggatagaagcaatgctggaatgccaacaatttttggtcatagaagagaacaaagtgcggcag
tacgattccatccagaaactgctgaacgagaggagcctcttccggcaagccatcagcccctccgacag
ggtgaagctctttccccaccggaactcaagcaagtgcaagtctaagccccagattgctgctctgaaaga
ggagacagaagaagaggtgcaagatacaaggctttga

CFTR Target Sequence 1 (SEQ ID NO: CFTR2):
5'- GTTCTTAAGGCACAGGAACTGGGAC -3'

CFTR Signaling probe 1 (SEQ ID NO: CFTR3):
5' - **Cy5** GCCAGTCCCAGTTCCTGTGCCTTAAGAACCTCGC **BHQ2** -3'
H.s. SCN9A (SEQ ID NO: NAV-1)

atggcaatgttgcctcccccaggacctcagagctttgtccatttcacaaaacagtctcttgccctcattgaa
caacgcattgctgaaagaaaatcaaaggaacccaaagaagaaaagaaagatgatgatgaagaag
ccccaaagccaagcagtgacttggaagctggcaaacaactgcccttcatctatggggacattcctccc
ggcatggtgtcagagcccctggaggacttggacccctactatgcagacaaaaagactttcatagtattg
aacaaagggaaaacaatcttccgtttcaatgccacacctgctttatatatgctttctcctttcagtcctctaag
aagaatatctattaagattttagtacactccttattcagcatgctcatcatgtgcactattctgacaaactgca
tatttatgaccatgaataacccgccggactggaccaaaaatgtcgagtacacttttactggaatatatactt
ttgaatcacttgtaaaaatccttgcaagaggcttctgtgtaggagaattcacttttcttcgtgacccgtggaa
ctggctggattttgtcgtcattgtttttgcgtatttaacagaatttgtaaacctaggcaatgtttcagctcttcga
actttcagagtattgagagctttgaaaactatttctgtaatcccaggcctgaagacaattgtaggggctttg
atccagtcagtgaagaagctttctgatgtcatgatcctgactgtgttctgtctgagtgtgtttgcactaattgg
actacagctgttcatgggaaacctgaagcataaatgttttcgaaattcacttgaaaataatgaaacattag
aaagcataatgaataccctagagagtgaagaagactttagaaaatatttttattacttggaaggatccaa
agatgctctcctttgtggtttcagcacagattcaggtcagtgtccagaggggtacacctgtgtgaaaattgg
cagaaaccctgattatggctacacgagctttgacactttcagctgggccttcttagccttgtttaggctaatg

acccaagattactgggaaaacctttaccaacagacgctgcgtgctgctggcaaaacctacatgatcttct
ttgtcgtagtgattttcctgggctccttttatctaataaacttgatcctggctgtggttgccatggcatatgaaga
acagaaccaggcaaacattgaagaagctaaacagaaagaattagaatttcaacagatgttagaccgt
cttaaaaaagagcaagaagaagctgaggcaattgcagcggcagcggctgaatatacaagtattagg
agaagcagaattatgggcctctcagagagttcttctgaaacatccaaactgagctctaaaagtgctaaa
gaaagaagaaacagaagaaagaaaaagaatcaaaagaagctctccagtggagaggaaaaggg
agatgctgagaaattgtcgaaatcagaatcagaggacagcatcagaagaaaaagtttccaccttggtg
tcgaagggcataggcgagcacatgaaaagaggttgtctacccccaatcagtcaccactcagcattcgt
ggctccttgttttctgcaaggcgaagcagcagaacaagtctttttagtttcaaaggcagaggaagagata
taggatctgagactgaatttgccgatgatgagcacagcatttttggagacaatgagagcagaaggggct
cactgtttgtgccccacagaccccaggagcgacgcagcagtaacatcagccaagccagtaggtcccc
accaatgctgccggtgaacgggaaaatgcacagtgctgtggactgcaacggtgtggtctccctggttga
tggacgctcagccctcatgctccccaatggacagcttctgccagagggcacgaccaatcaaatacaca
agaaaaggcgttgtagttcctatctcctttcagaggatatgctgaatgatcccaacctcagacagagagc
aatgagtagagcaagcatattaacaaacactgtggaagaacttgaagagtccagacaaaaatgtcca
ccttggtggtacagatttgcacacaaattcttgatctggaattgctctccatattggataaaattcaaaagt
gtatctattttattgtaatggatccttttgtagatcttgcaattaccatttgcatagttttaaacacattatttatggc
tatggaacaccacccaatgactgaggaattcaaaaatgtacttgctataggaaatttggtctttactggaa
tctttgcagctgaaatggtattaaaactgattgccatggatccatatgagtatttccaagtaggctggaatat
ttttgacagccttattgtgactttaagtttagtggagctctttctagcagatgtggaaggattgtcagttctgcg
atcattcagactgctccgagtcttcaagttggcaaaatcctggccaacattgaacatgctgattaagatca
ttggtaactcagtaggggctctaggtaacctcaccttagtgttggccatcatcgtcttcatttttgctgtggtcg
gcatgcagctctttggtaagagctacaaagaatgtgtctgcaagatcaatgatgactgtacgctcccacg
gtggcacatgaacgacttcttccactccttcctgattgtgttccgcgtgctgtgtggagagtggatagagac
catgtgggactgtatggaggtcgctggtcaagctatgtgccttattgtttacatgatggtcatggtcattgga
aacctggtggtcctaaacctatttctggccttattattgagctcatttagttcagacaatcttacagcaattga
agaagaccctgatgcaaacaacctccagattgcagtgactagaattaaaaagggaataaattatgtga
aacaaaccttacgtgaatttattctaaaagcattttccaaaaagccaaagatttccagggagataagac
aagcagaagatctgaatactaagaaggaaaactatatttctaaccatacacttgctgaaatgagcaaa
ggtcacaatttcctcaaggaaaaagataaaatcagtggttttggaagcagcgtggacaaacacttgatg
gaagacagtgatggtcaatcatttattcacaatcccagcctcacagtgacagtgccaattgcacctggg
gaatccgatttggaaaatatgaatgctgaggaacttagcagtgattcggatagtgaatacagcaaagtg
agattaaaccggtcaagctcctcagagtgcagcacagttgataaccctttgcctggagaaggagaaga
agcagaggctgaacctatgaattccgatgagccagaggcctgtttcacagatggttgtgtacggaggttc
tcatgctgccaagttaacatagagtcagggaaaggaaaaatctggtggaacatcaggaaaacctgct
acaagattgttgaacacagttggtttgaaagcttcattgtcctcatgatcctgctcagcagtggtgccctgg
cttttgaagatatttatattgaaaggaaaaagaccattaagattatcctggagtatgcagacaagatcttca
cttacatcttcattctggaaatgcttctaaaatggatagcatatggttataaaacatatttcaccaatgcctgg
tgttggctggatttcctaattgttgatgtttctttggttactttagtggcaaacactcttggctactcagatcttggc
cccattaaatcccttcggacactgagagctttaagacctctaagagccttatctagatttgaaggaatgag
ggtcgttgtgaatgcactcataggagcaattccttccatcatgaatgtgctacttgtgtgtcttatattctggct
gatattcagcatcatgggagtaaatttgtttgctggcaagttctatgagtgtattaacaccacagatgggtc
acggtttcctgcaagtcaagttccaaatcgttccgaatgttttgcccttatgaatgttagtcaaaatgtgcgat
ggaaaaacctgaaagtgaactttgataatgtcggacttggttacctatctctgcttcaagttgcaacttttaa
gggatggacgattattatgtatgcagcagtggattctgttaatgtagacaagcagcccaaatatgaatata
gcctctacatgtatatttattttgtcgtctttatcatctttgggtcattcttcactttgaacttgttcattggtgtcatca
tagataatttcaaccaacagaaaaagaagcttggaggtcaagacatctttatgacagaagaacagaa
gaaatactataatgcaatgaaaaagctggggtccaagaagccacaaaagccaattcctcgaccagg

gaacaaaatccaaggatgtatatttgacctagtgacaaatcaagcctttgatattagtatcatggttcttatc
tgtctcaacatggtaaccatgatggtagaaaaggagggtcaaagtcaacatatgactgaagttttatattg
gataaatgtggtttttataatccttttcactggagaatgtgtgctaaaactgatctccctcagacactactactt
cactgtaggatggaatatttttgattttgtggttgtgattatctccattgtaggtatgtttctagctgatttgattga
aacgtattttgtgtcccctaccctgttccgagtgatccgtcttgccaggattggccgaatcctacgtctagtc
aaaggagcaaaggggatccgcacgctgctctttgctttgatgatgtcccttcctgcgttgtttaacatcggc
ctcctgctcttcctggtcatgttcatctacgccatctttggaatgtccaactttgcctatgttaaaaaggaagat
ggaattaatgacatgttcaattttgagacctttggcaacagtatgatttgcctgttccaaattacaacctctgc
tggctgggatggattgctagcacctattcttaacagtaagccacccgactgtgacccaaaaaaagttcat
cctggaagttcagttgaaggagactgtggtaacccatctgttggaatattctactttgttagttatatcatcat
atccttcctggttgtggtgaacatgtacattgcagtcatactggagaattttagtgttgccactgaagaaagt
actgaacctctgagtgaggatgactttgagatgttctatgaggtttgggagaagtttgatcccgatgcgac
ccagtttatagagttctctaaactctctgattttgcagctgccctggatcctcctcttctcatagcaaaaccca
acaaagtccagctcattgccatggatctgcccatggttagtggtgaccggatccattgtcttgacatcttatt
tgcttttacaaagcgtgttttgggtgagagtggggagatggattctcttcgttcacagatggaagaaaggtt
catgtctgcaaatccttccaaagtgtcctatgaacccatcacaaccacactaaaacggaaacaagagg
atgtgtctgctactgtcattcagcgtgcttatagacgttaccgcttaaggcaaaatgtcaaaaatatatcaa
gtatatacataaaagatggagacagagatgatgatttactcaataaaaaagatatggcttttgataatgtt
aatgagaactcaagtccagaaaaaacagatgccacttcatccaccacctctccaccttcatatgatagt
gtaacaaagccagacaaagagaaatatgaacaagacagaacagaaaaggaagacaaagggaa
agacagcaaggaaagcaaaaaatag

H.s. SCN1B (SEQ ID NO: NAV-2):

Atggggaggctgctggccttagtggtcggcgcggcactggtgtcctcagcctgcgggggctgcgtgga
ggtggactcggagaccgaggccgtgtatgggatgaccttcaaaattctttgcatctcctgcaagcgccgc
agcgagaccaacgctgagaccttcaccgagtggaccttccgccagaagggcactgaggagtttgtca
agatcctgcgctatgagaatgaggtgttgcagctggaggaggatgagcgcttcgagggccgcgtggtg
tggaatggcagccggggcaccaaagacctgcaggatctgtctatcttcatcaccaatgtcacctacaac
cactcgggcgactacgagtgccacgtctaccgcctgctcttcttcgaaaactacgagcacaacaccag
cgtcgtcaagaagatccacattgaggtagtggacaaagccaacagagacatggcatccatcgtgtctg
agatcatgatgtatgtgctcattgtggtgttgaccatatggctcgtggcagagatgatttactgctacaaga
agatcgctgccgccacggagactgctgcacaggagaatgcctcggaatacctggccatcacctctga
aagcaaagagaactgcacgggcgtccaggtggccgaatag

H.s. SCN2B (SEQ ID NO: NAV-3):

Atgcacagagatgcctggctacctcgccctgccttcagcctcacggggctcagtctctttttctctttggtgc
caccaggacggagcatggaggtcacagtacctgccaccctcaacgtcctcaatggctctgacgcccg
cctgccctgcaccttcaactcctgctacacagtgaaccacaaacagttctccctgaactggacttaccag
gagtgcaacaactgctctgaggagatgttcctccagttccgcatgaagatcattaacctgaagctggag
cggtttcaagaccgcgtggagttctcagggaaccccagcaagtacgatgtgtcggtgatgctgagaaa
cgtgcagccggaggatgaggggatttacaactgctacatcatgaacccccctgaccgccaccgtggc
catggcaagatccatctgcaggtcctcatggaagagcccccctgagcgggactccacggtggccgtgat
tgtgggtgcctccgtcgggggcttcctggctgtggtcatcttggtgctgatggtggtcaagtgtgtgaggag
aaaaaaagagcagaagctgagcacagatgacctgaagaccgaggaggagggcaagacggacg
gtgaaggcaacccggatgatggcgccaagtag

NaV Target sequence 1 (SEQ ID NO: NAV-4)
5'-GTTCTTAAGGCACAGGAACTGGGAC-3'

NaV Target sequence 2 (SEQ ID NO: NAV-5)
5'-GAAGTTAACCCTGTCGTTCTGCGAC-3'

NaV Target sequence 3 (SEQ ID NO: NAV-6)
5'-GTTCTATAGGGTCTGCTTGTCGCTC-3'

NaV Signaling probe 1 (binds target 1) (SEQ ID NO: NAV-7)
**5' - Cy5** GCCAGTCCCAGTTCCTGTGCCTTAAGAACCTCGC **BHQ2 quench -3'**

NaV Signaling probe 2 - (binds target 2) (SEQ ID NO: NAV-8)
**5'- Cy5.5** GCGAGTCGCAGAACGACAGGGTTAACTTCCTCGC **BHQ2 quench -3'**

NaV Signaling probe 3 - (binds target 3) (SEQ ID NO: NAV-9)
5'- **Fam** GCGAGAGCGACAAGCAGACCCTATAGAACCTCGC **BHQ1 quench -3'**

Furthermore, the present invention relates to the following items:

1. A cell that expresses a heterodimeric protein of interest from an introduced nucleic acid encoding at least one of the subunits of the heterodimeric protein of interest, said cell being characterized in that it produces the heterodimeric protein of interest in a form suitable for use in a functional assay, wherein said protein of interest does not comprise a protein tag, or said protein is produced in that form consistently and reproducibly such that the cell has a Z' factor of at least 0.4 in the functional assay, or said cell is cultured in the absence of selective pressure, or any combinations thereof.

2. A cell that expresses a heterodimeric protein of interest, wherein the cell is engineered to activate transcription of an endogenous nucleic acid encoding at least one of the subunits of the heterodimeric protein of interest, said cell being characterized in that it produces the heterodimeric protein of interest in a form suitable for use in a functional assay, wherein said protein of interest does not comprise a protein tag, or said protein is produced in that form consistently and reproducibly such that the cell has a Z' factor of at least 0.4 in the functional assay, or said cell is cultured in the absence of selective pressure, or any combinations thereof.

3. A cell that expresses a heterodimeric protein of interest from an introduced nucleic acid encoding at least one of the subunits of the heterodimeric protein of interest, said cell being characterized in that it produces the protein of interest in a form that is or is capable of becoming biologically active, wherein the cell is cultured in the absence of selective pressure.

4. A cell that expresses a heterodimeric protein of interest wherein the cell is engineered to activate transcription of an endogenous nucleic acid encoding at least one of the subunits of the heterodimeric protein of interest, said cell being characterized in that it produces the protein of interest in a form that is or is capable of becoming biologically active, wherein the cell is cultured in the absence of selective pressure.

5. The cell according to any one of items 1-4, wherein the nucleic acid encoding the second subunit of the heterodimeric protein of interest is endogenous.

6. The cell according to items 2 or 4, wherein the nucleic acid encoding the second subunit of the heterodimeric protein of interest is introduced.

7. The cell according to any one of items 1-6, wherein the protein of interest does not comprise a protein tag.

8. The cell according to any one of items 1-7, wherein the heterodimeric protein of interest is selected from the group consisting of: an ion channel, a G protein coupled receptor (GPCR), tyrosine receptor kinase, cytokine receptor, nuclear steroid hormone receptor and immunological receptor.

9. The cell according to item 8, wherein the heterodimeric protein of interest is selected from the group consisting of: a sweet taste receptor and an umami taste receptor.

10. The cell according to any one of items 1-8, wherein the heterodimeric protein of interest has no known ligand.

11. The cell according to any one of items 1-10, wherein the heterodimeric protein of interest is not expressed in a cell of the same type.

12. The cell according to any one of items 1-11, wherein the cell is a mammalian cell.

13. The cell according to any one of items 1-12, wherein the cell is further characterized in that it has an additional desired property selected from the group consisting of: a signal to noise ratio greater than 1, being stable over time, growth without selective pressure without losing expression, physiological EC50 values, and physiological IC50 values.

14. The cell according to any one of items 1, 2 and 5-13, wherein the heterodimeric protein of interest is produced in a form consistently and reproducibly for a period of time selected from: at least one week, at least two weeks, at least three weeks, at least one month, at least two months, at least three months at least four months, at least five months, at least six months, at least seven months, at least eight months, and at least nine months.

15. The cell according to any one of items 1, 2 and 5-13, wherein the functional assay is selected from the group consisting of: a cell-based assay, a fluorescent cell-based assay, a high throughput screening assay, a reporter cell-based assay, a G protein mediated cell-based assay, and a calcium flux cell-based assay.

16. The cell according to any one of items 1,2, and 5-13, wherein the cell is suitable for utilization in a cell based high throughput screening.

17. The cell according to any one of items 3-13, wherein the selective pressure is an antibiotic.

18. The cell according to any one of items 3-13 and 17, wherein the cell expresses the heterodimeric protein in the absence of selective pressure for at least 15 days, 30 days, 45 days, 60 days, 75 days, 100 days, 120 days, or 150 days.

19. A cell that expresses a heteromultimeric protein of interest wherein said heteromultimeric protein comprises at least 3 subunits, wherein at least one subunit of the heteromultimeric protein interest is encoded by an introduced nucleic acid, said cell being characterized in that it produces the heteromultimeric protein of interest in a form suitable for use in a functional assay, wherein said protein of interest does not comprise a protein tag, or said protein produced in that form consistently and reproducibly such that the cell has a Z' factor of at least 0.4 in the functional assay, or said cell is cultured in the absence of selective pressure, or any combinations thereof.

20. A cell that expresses a heteromultimeric protein of interest wherein said heteromultimeric protein comprises at least 3 subunits, wherein the cell is engineered to activate transcription of an endogenous nucleic acid encoding at least one of the subunits of the heteromultimeric protein of interest, said cell being characterized in that it produces the heteromultimeric protein of interest in a form suitable for use in a functional assay, wherein said protein of interest does not comprise a protein tag, or said protein produced in that form consistently and reproducibly such that the cell has a Z' factor of at least 0.4 in the functional assay, or said cell is cultured in the absence of selective pressure, or any combinations thereof.

21. A cell that expresses a heteromultimeric protein of interest wherein said heteromultimeric protein comprises at least 3 subunits, wherein at least one subunit of the heteromultimeric protein interest is encoded by an introduced nucleic acid, said cell being characterized in that it produces the protein of interest in a form that is or is capable of becoming biologically active.

22. A cell that expresses a heteromultimeric protein of interest wherein said heteromultimeric protein comprises at least 3 subunits, wherein the cell is engineered to activate transcription of an endogenous nucleic acid encoding at least one of the subunits of the heteromultimeric protein of interest, said cell being characterized in that it produces the protein of interest in a form that is or is capable of becoming biologically active.

23. The cell according to any one of items 19-22, wherein the nucleic acid encoding at least one of the subunits of the heteromultimeric protein of interest is endogenous.

24. The cell according to item 20 or 22, wherein the nucleic acid encoding at least one of the subunits of the heteromultimeric protein of interest is introduced.

25. The cell according to any one of items 19-24, wherein the protein of interest does not comprise a protein tag.

26. The cell according to any one of items 19-25, wherein the heteromultimeric protein of interest is selected from the group consisting of: an ion channel, a G protein coupled receptor (GPCR), tyrosine receptor kinase, cytokine receptor, nuclear steroid hormone receptor and immunological receptor.

27. The cell according to item 26, wherein the heteromultimeric protein of interest is selected from the group consisting of: GABA, ENaC and NaV.

28. The cell according to any one of items 19-26, wherein the heteromultimeric protein of interest has no known ligand.

29. The cell according to any one of items 19-28, wherein the heteromultimeric protein of interest is not expressed in a cell of the same type.

30. The cell according to any one of items 19-29, wherein the cell is a mammalian cell.

31. The cell according to any one of items 19-30, wherein the cell is further characterized in that it has an additional desired property selected from the group consisting of: a signal to noise ratio greater than 1, being stable over time, growth without selective pressure without losing expression, physiological EC50 values, and physiological IC50 values.

32. The cell according to any one of items 19, 20 and 23-31, wherein the heteromultimeric protein of interest is produced in a form consistently and reproducibly for a period of time selected from: at least one week, at least two weeks, at least three weeks, at least one month, at least two months, at least three months at least four months, at least five months, at least six months, at least seven months, at least eight months, and at least nine months.

33. The cell according to any one of items 19, 20 and 23-31, wherein the functional assay is selected from the group consisting of: a cell-based assay, a fluorescent cell-based assay, a high throughput screening assay, a reporter cell-based assay, a G protein mediated cell-based assay, and a calcium flux cell-based assay.

34. The cell according to any one of items 19, 20, and 23-31, wherein the cell is suitable for utilization in a cell based high throughput screening.

35. The cell according to any one of items 21-31, wherein said cells are cultured in the absence of selective pressure.

36. The cell according to item 35, wherein the selective pressure is an antibiotic.

37. The cell according to item 35 or 36, wherein the cell expresses the heteromultimeric protein in the absence of selective pressure for at least 15 days, 30 days, 45 days, 60 days, 75 days, 100 days, 120 days, or 150 days.

38. A cell that expresses two or more proteins of interest from an introduced nucleic acid encoding at least one of the proteins of interest, said cell being characterized in that it produces the proteins of interest in a form suitable for use in a functional assay, wherein said proteins of interest do not comprise a protein tag, or said proteins are produced in that form consistently and reproducibly such that the cell has a Z' factor of at least 0.4 in the functional assay, or said cell is cultured in the absence of selective pressure, or any combinations thereof.

39. A cell that expresses two or more proteins of interest, wherein the cell is engineered to activate transcription of an endogenous nucleic acid encoding at least one of the proteins of interest, said cell being characterized in that it produces the proteins of interest in a form suitable for use in a functional assay, wherein said proteins of interest do not comprise a protein tag, or said proteins are produced in that form consistently and reproducibly such that the cell has a Z' factor of at least 0.4 in the functional assay, or said cell is cultured in the absence of selective pressure, or any combinations thereof.

40. A cell that expresses two or more proteins of interest from an introduced nucleic acid encoding at least one of the proteins of interest, said cell being characterized in that it produces the proteins of interest in a form that is or is capable of becoming biologically active.

41. A cell that expresses two or more proteins of interest, wherein the cell is engineered to activate transcription of an endogenous nucleic acid encoding at least one of the proteins of interest, said cell being characterized in that it produces the proteins of interest in a form that is or is capable of becoming biologically active.

42. The cell according to any one of items 38-41, wherein at least one protein of interest is a dimeric protein.

43. The cell according to item 42, wherein the dimeric protein of interest is a homodimeric protein.

44. The cell according to item 42, wherein the dimeric protein of interest is a heterodimeric protein.

45. The cell according to any one of items 38-41, wherein at least one protein of interest is a multimeric protein.

46. The cell according to item 45, wherein the multimeric protein of interest is a homomultimeric protein.

47. The cell according to item 45, wherein the multimeric protein of interest is a heteromultimeric protein.

48. The cell according to any one of items 38-47, wherein one of the proteins is encoded by an endogenous nucleic acid.

49. The cell according to item 39 or 41, wherein one of the proteins is encoded by an introduced nucleic acid.

50. The cell according to any one of items 38-49, wherein the proteins of interest do not comprise a protein tag.

51. The cell according to any one of items 38-49, wherein one of the proteins of interest is selected from the group consisting of: an ion channel, a G protein coupled receptor (GPCR), tyrosine receptor kinase, cytokine receptor, nuclear steroid hormone receptor and immunological receptor.

52. The cell according to any one of items 38-51, wherein one of the proteins of interest has no known ligand.

53. The cell according to any one of items 38-52, wherein one of the proteins of interest is not expressed in a cell of the same type.

54. The cell according to any one of items 38-53, wherein the cell is a mammalian cell.

55. The cell according to any one of items 38-54, wherein the cell is further characterized in that it has an additional desired property selected from the group consisting of: a signal to noise ratio greater than 1, being stable over time, growth without selective pressure without losing expression, physiological EC50 values, and physiological IC50 values.

56. The cell according to any one of items 38, 39 and 42-55, wherein the two or more proteins of interest are produced in a form consistently and reproducibly for a period of time selected from: at least one week, at least two weeks, at least three weeks, at least one month, at least two months, at least three months at least four months, at least five months, at least six months, at least seven months, at least eight months, and at least nine months.

57. The cell according to any one of items 38, 39 and 42-55, wherein the functional assay is selected from the group consisting of: a cell-based assay, a fluorescent cell-based assay, a high throughput screening assay, a reporter cell-based assay, a G protein mediated cell-based assay, and a calcium flux cell-based assay.

58. The cell according to any one of items 38, 39, and 42-55, wherein the cell is suitable for utilization in a cell based high throughput screening.

59. The cell according to any one of items 40-55, wherein said cells are cultured in the absence of selective pressure.

60. The cell according to item 59, wherein the selective pressure is an antibiotic.

61. The cell according to item 59 or 60, wherein the cell expresses the two or more proteins in the absence of selective pressure for at least 15 days, 30 days, 45 days, 60 days, 75 days, 100 days, 120 days, or 150 days.

62. A cell that expresses at least one RNA of interest, wherein said RNA of interest is encoded by an introduced nucleic acid, said cell being characterized in that it produces the at least one RNA of interest in a form suitable for use in a functional assay, wherein said RNA of interest do not comprise a tag, or said RNA is produced in that form consistently and reproducibly such that the cell has a Z' factor of at least 0.4 in the functional assay, or said cell is cultured in the absence of selective pressure, or any combinations thereof.

63. A cell that expresses at least one RNA of interest, wherein the cell is engineered to activate transcription of an endogenous nucleic acid encoding the at least one RNA of interest, said cell being characterized in that it produces the at least one RNA of interest in a form suitable for use in a functional assay, wherein said RNA of interest do not comprise a tag, or said RNA is produced in that form consistently and reproducibly such that the cell has a Z' factor of at least 0.4 in the functional assay or said cell is cultured in the absence of selective pressure, or any combinations thereof.

64. A cell according to item 62 or 63, wherein the cell expresses at least two RNAs of interest.

65. A cell according to item 62 or 63, wherein the cell expresses at least three RNAs of interest.

66. The cell according to any one of items 64-65, wherein the cell further expresses a RNA encoded by an introduced nucleic acid.

67. The cell according to any one of items 62-66, wherein the RNA of interest is selected from the group consisting of: a RNA encoding an ion channel, a RNA encoding a G protein coupled receptor (GPCR), a RNA encoding a tyrosine receptor kinase, a RNA encodinga cytokine receptor, a RNA encoding a nuclear steroid hormone receptor and a RNA encoding an immunological receptor.

68. The cell according to any one of items 62-67, wherein the RNA of interest is not expressed in a cell of the same type.

69. The cell according to any one of items 62-68, wherein the cell is a mammalian cell.

70. The cell according to any one of items 62-69, wherein the cell is further characterized in that it has an additional desired property selected from the group consisting of: a signal to noise ratio greater than 1, being stable over time, growth without selective pressure without losing expression, physiological EC50 values, and physiological IC50 values.

71. The cell according to any one of items 62-70, wherein the RNA of interest is produced in a form consistently and reproducibly for a period of time selected from: at least one week, at least two weeks, at least three weeks, at least one month, at least two months, at least three months at least four months, at least five months, at least six months, at least seven months, at least eight months, and at least nine months.

72. The cell according to any one of items 62-71, wherein the functional assay is selected from the group consisting of: a cell-based assay, a fluorescent cell-based assay, a high throughput screening assay, a reporter cell-based assay, a G protein mediated cell-based assay, and a calcium flux cell-based assay.

73. The cell according to any one of items 61-72, wherein the cell is suitable for utilization in a cell based high throughput screening.

74. A cell line produced from the cell of any one of items 1-73.

75. A method for producing a cell that expresses a protein of interest, wherein the cell has at least one desired property that is consistent over time, comprising the steps of:

a) providing a plurality of cells that express mRNA encoding the protein of interest;
b) dispersing cells individually into individual culture vessels, thereby providing a plurality of separate cell cultures
c) culturing the cells under a set of desired culture conditions using automated cell culture methods characterized in that the conditions are substantially identical for each of the separate cell cultures, during which culturing the number of cells per separate cell culture is normalized from culture to culture, and wherein the separate cultures are passaged on the same schedule;
d) assaying the separate cell cultures for at least one desired characteristic of the protein of interest at least twice; and
e) identifying a separate cell culture that has the desired characteristic in both assays; wherein said cell is a cell that.

76. The method of item 75, wherein the plurality of cells in step a) are cultured for some period of time prior to the dispersing in step b).

77. The method of item 75, wherein the individual culture vessels are selected from the group consisting of: individual wells of a multiwell plate and vials.

78. The method of item 75, further comprising the step of determining the growth rate of a plurality of the separate cell cultures and grouping the separate cell cultures by their growth rates into groups such that the difference between the fastest and slowest growth rates in any group is no more than 1, 2, 3, 4, or 5 days between steps b) and c).

79. The method of item 75 or item 78, further comprising the step of preparing a stored stock of one or more of the separate cultures.

80. The method of item 75, further comprising the step of one or more replicate sets of the separate cell cultures and culturing the one or more replicate sets separately from the source separate cell cultures.

81. The method of item 75, wherein the assaying in step d) is a functional assay for the protein.

82. The method of item 75, wherein the at least one characteristic that has remained constant in step e) is protein function.

83. The method of item 75, wherein the culturing in step c) is in a robotic cell culture apparatus.

84. The method of item 83, wherein the robotic cell culture apparatus comprises a multi-channel robotic pipettor.

85. The method of item 84, wherein the multi-channel robotic pipettor comprises at least 96 channels.

86. The method of item 84, wherein the robotic cell culture apparatus further comprises a cherry-picking arm.

87. The method of item 75, wherein the automated methods are include one or more of: media removal, media replacement, cell washing, reagent addition, removal of cells, cell dispersal, and cell passaging.

88. The method of item 75, wherein the plurality of separate cell cultures is at least 50 cultures.

89. The method of item 75, wherein the plurality of separate cell cultures is at least 100 cultures.

90. The method of item 75, wherein the plurality of separate cell cultures is at least 500 cultures.

91. The method of item 75, wherein the plurality of separate cell cultures is at least 1000 cultures.

92. The method of item 78, wherein the growth rate is determined by a method selected from the group consisting of: measuring ATP, measuring cell confluency, light scattering, optical density measurement.

93. The method of item 78, wherein the difference between the fastest and slowest growth rates in a group is no more than 1, 2, 3, 4, or 5 hours.

94. The method of item 75, wherein the culturing in step c) is for at least 2 days.

95. The method of item 78, wherein the growth rates of the plurality of separate cell cultures are determined by dispersing the cells and measuring cell confluency.

96. The method of item 95, wherein the cells in each separate cell culture are dispersed prior to measuring cell confluency.

97. The method of item 95 or 96, wherein the dispersing step comprises adding trypsin to the well and to eliminate clumps.

98. The method of item 95, wherein the cell confluency of the plurality of separate cell cultures is measured using an automated microplate reader.

99. The method of item 95, wherein at least two confluency measurements are made before growth rate is calculated.

100. The method of item 98, wherein the cell confluency is measured by an automated plate reader and the confluency values are used with a software program that calculates growth rate.

101. The method of item 75, wherein the separate cell cultures in step d) are characterization for a desired trait selected from one or more of: fragility, morphology, adherence to a solid surface; lack of adherence to a solid surface and protein function.

102. The method of item 75, wherein the cells are eukaryotic cells.

103. The method of item 75, wherein the eukaryotic cells are mammalian cells.

104. The method of item 75, wherein the mammalian cell line is selected from the group consisting of: NS0 cells, CHO cells, COS cells, HEK-293 cells, HUVECs, 3T3 cells and HeLa cells.

105. The method of item 75, wherein the protein of interest is a human protein.

106. The method of item 75, wherein the protein of interest is a heteromultimer

107. The method of item 75, wherein the protein of interest is a G protein coupled receptor.

108. The method of item 75, wherein the protein has no known ligand.

109. The method of item 75, further comprising after the identifying step, the steps of:

a) expanding a stored aliquot of the cell culture identified in step e) under desired culture conditions;
b) determining if the expanded cell culture of a) has the desired characteristic.

110. A matched panel of clonal cell lines, wherein the clonal cell lines are of the same cell type, and wherein each cell line in the panel expresses a protein of interest, and wherein the clonal cell lines in the panel are matched to share the same physiological property to allow parallel processing.

111. The matched panel of clonal cell lines of item 110, wherein the physiological property is growth rate.

112. The matched panel of clonal cell lines of item 110, wherein the physiological property is adherence to a tissue culture surface.

113. The matched panel of clonal cell lines of item 110, wherein the physiological property is Z' factor.

114. The matched panel of clonal cell lines of item 110, wherein the physiological property is expression level of RNA encoding the protein of interest.

115. The matched panel of clonal cell lines of item 110, wherein the physiological property is expression level of the protein of interest.

116. The matched panel of clonal cell lines of item 111, wherein the growth rates of the clonal cell lines in the panel are within 1, 2, 3, 4, or 5 hours of each other.

117. The matched panel of clonal cell lines of item 110, wherein the culture conditions are the same for all clonal cell lines in the panel.

118. The matched panel of clonal cell lines of item 110, wherein the clonal cell line is a eukaryotic cell line.

119. The matched panel of clonal cell lines of item 110, wherein the eukaryotic cell line is a mammalian cell line.

120. The matched panel of clonal cell lines of item 110, wherein the cell line cells are selected from the group consisting of: primary cells and immortalized cells.

121. The matched panel of clonal cell lines of item 110, wherein the cell line cells are prokaryotic or eukaryotic.

122. The matched panel of clonal cell lines of item 121, wherein the cell line cells are eukaryotic and are selected from the group consisting of: fungal cells, insect cells, mammalian cells, yeast cells, algae, crustacean cells, arthropod cells , avian cells, reptilian cells, amphibian cells and plant cells.

123. The matched panel of clonal cell lines of item 122, wherein the cell line cells are mammalian and are selected from the group consisting of: human, non-human primate, bovine, porcine, feline, rat, marsupial, murine, canine, ovine, caprine, rabbit, guinea pig hamster.

124. The matched panel of clonal cell lines of item 110, wherein the cells in the cell line are engineered to express the protein of interest.

125. The matched panel of clonal cell lines of item 110, wherein the cells in the cell line express the protein of interest from an introduced nucleic acid encoding the protein or, in the case of a multimeric protein, encoding a subunit of the protein.

126. The matched panel of clonal cell lines of item 110, wherein the cells express the protein of interest from an endogenous nucleic acid and wherein the cell is engineered to activate transcription of the endogenous protein or, in the case of a multimeric protein, activates transcription of a Subunit of the protein.

127. The matched panel of clonal cell lines of item 110, wherein the panel comprises at least four clonal cell lines.

128. The matched panel of clonal cell lines of item 110, wherein the panel comprises at least six clonal cell lines.

129. The matched panel of clonal cell lines of item 110, wherein the panel comprises at least twenty five clonal cell lines.

130. The matched panel of clonal cell lines of item 110, wherein two or more of the clonal cell lines in the panel express the same protein of interest.

131. The matched panel of clonal cell lines of item 110, wherein two or more of the clonal cell lines in the panel express a different protein of interest.

132. The matched panel of clonal cell lines of item 110, wherein the cell lines in the panel express different forms of a protein of interest, wherein the forms are selected from the group consisting of: isoforms, amino acid sequence variants, splice variants, truncated forms, fusion proteins, chimeras, or combinations thereof.

133. The matched panel of clonal cell lines of item 110, wherein the cell lines in the panel express different proteins in a group of proteins of interest, wherein the groups of proteins of interest are selected from the group consisting of: proteins in the same signaling pathway, expression library of similar proteins, monoclonal antibody heavy chain library, monoclonal antibody light chain library and SNPs.

134. The matched panel of clonal cell lines of item 110, wherein the protein of interest is a single chain protein.

135. The matched panel of clonal cell lines of item 110, wherein the single chain protein is a G protein coupled receptor.

136. The matched panel of clonal cell lines of item 135, wherein the G protein coupled receptor is a taste receptor.

137. The matched panel of clonal cell lines of item 136, wherein the taste receptor is selected from the group consisting of: a bitter taste receptor, a sweet taste receptor, a salt taste receptor and a umami taste receptor.

138. The matched panel of clonal cell lines of item 110, wherein the protein is a multimeric protein.

139. The matched panel of clonal cell lines of item 110, wherein the protein is a heterodimer or a heteromultimer.

140. The matched panel of clonal cell lines of item 110, wherein the protein is selected from the group consisting of: an ion channel, an ion channel, a G protein coupled receptor (GPCR), tyrosine receptor kinase, cytokine receptor, nuclear steroid hormone receptor and immunological receptor.

141. The matched panel of clonal cell lines of item 140, wherein the protein is Epithelial sodium Channel (ENaC).

142. The matched panel of clonal cell lines of item 140, wherein the ENaC comprises an alpha subunit, a beta subunit and a gamma subunit.

143. The matched panel of clonal cell lines of item 110, wherein the cell lines in the panel express different ENaC isoforms.

144. The matched panel of clonal cell lines of item 110, wherein the cell lines in the panel comprise different proteolyzed isoforms of ENaC.

145. The matched panel of clonal cell lines of any one of items 141-143, wherein the ENaC is human ENaC.

146. The matched panel of clonal cell lines of item 140, wherein the protein is voltage gated sodium channel (NaV).

147. The matched panel of clonal cell lines of item 146, wherein the NaV comprises an alpha subunit and two beta subunits.

148. The matched panel of clonal cell lines of item 40, wherein the NaV is human NaV.

149. The matched panel of clonal cell lines of item 110, wherein the protein is selected from the group consisting of: gamma-aminobutyric acid A receptor ($GABA_A$ receptor), gamma-aminobutyric acid B receptor ($GABA_B$ receptor) and gamma-aminobutyric acid C receptor ($GABA_C$ receptor).

150. The matched panel of clonal cell lines of item 110, wherein the protein is $GABA_A$ receptor.

151. The matched panel of clonal cell lines of item 150, wherein the $GABA_A$ receptor comprises two alpha subunits, two beta subunits and a gamma or delta subunit.

152. The matched panel of clonal cell lines of item 110, wherein the clonal cell lines in the panel were produced simultaneously, or within no more than 4 weeks of each other.

153. A cell that expresses a monomeric protein of interest from an introduced nucleic acid encoding said monomeric protein of interest, characterized in that it produces the protein of interest in a form that is or is capable of becoming biologically active, wherein the cell is cultured in the absence of selective pressure and wherein the expression of the protein does not vary by more than 5% over 3 months.

154. The cell according to item 153, wherein the expression of the protein does not vary by more than 5% over 6 months

155. The cell according to item 153 or 154, wherein the protein of interest has no known ligand.

156. A method for identifying a modulator of a protein of interest comprising the steps of:

a) contacting a cell according to any one of items 1 to 74 with a test compound; and

b) detecting a change in the activity of the protein of interest in the cell contacted with the test compound compared to the activity of the protein in a cell not contacted by the test compound;
wherein a compound that produces a difference in the activity in the presence compared to in the absence is a modulator of the protein of interest.

157. A modulator identified by the method of item 156.

## Claims

1. A matched panel of clonal cell lines, wherein the clonal cell lines are of the same cell type, and wherein each cell line in the panel expresses an RNA or a protein of interest, and wherein the clonal cell lines in the panel are matched to share the same physiological property to allow parallel processing.

2. The matched panel of clonal cell lines of claim 1, wherein the physiological property is:

   a) growth rate;
   b) adherence to a tissue culture surface;
   c) a Z' factor in a functional assay;
   d) expression level of RNA encoding a protein of interest;
   e) expression level of the RNA of interest; or
   f) expression level of the protein of interest.

3. The matched panel of clonal cell lines of claim 2, wherein the physiological property is growth rate and the growth rates of the clonal cell lines in the panel are within 1, 2, 3, 4, 5, or 10 hours of each other.

4. The matched panel of clonal cell lines of any one of claims 1-3, wherein the culture conditions are the same for all clonal cell lines in the panel.

5. The matched panel of clonal cell lines of any one of claims 1-4, wherein the clonal cell line is:

   a) a eukaryotic cell line; or
   b) a prokaryotic cell line.

6. The matched panel of clonal cell lines of any one of claims 1-5, wherein the clonal cell line is a mammalian cell line.

7. The matched panel of clonal cell lines of any one of claims 1-6, wherein the clonal cell line is:

   a) a cell line of primary cells; or
   b) a cell line of immortalized cells.

**8.** The matched panel of clonal cell lines of any one of claims 1-7, wherein the cells in the cell line are engineered to express the protein of interest.

**9.** The matched panel of clonal cell lines of claim 8, wherein:

a) the cells in the cell line express the protein of interest from an introduced nucleic acid encoding the protein; or
b) the protein of interest is a multimeric protein and the cells in the cell line express at least one subunit of the multimeric protein from at least one introduced nucleic acid encoding the at least one subunit.

**10.** The matched panel of clonal cell lines of claim 8, wherein:

a) the cells express the protein of interest from an endogenous nucleic acid and wherein the cell is engineered to activate transcription of the endogenous protein; or
b) the protein of interest is a multimeric protein and the cells in the cell line express at least one subunit of the multimeric protein from an endogenous nucleic acid and wherein the cell is engineered to activate transcription of the at least one subunit.

**11.** The matched panel of clonal cell lines of any one of claims 1-10, wherein the panel comprises:

a) at least four clonal cell lines;
b) at least six clonal cell lines; or
c) at least twenty-five clonal cell lines.

**12.** The matched panel of clonal cell lines of claim any one of claims 1-11, wherein two or more of the clonal cell lines in the panel express the same protein of interest.

**13.** The matched panel of clonal cell lines of any one of claims 1-12, wherein two or more of the clonal cell lines in the panel express a different protein of interest.

**14.** The matched panel of clonal cell lines of any one of claims 1-13, wherein the cell lines in the panel express different forms of a protein of interest, wherein the forms are selected from the group consisting of: isoforms, amino acid sequence variants, splice variants, truncated forms, fusion proteins, chimeras, enzymatically modified forms of a protein of interest, or combinations thereof.

**15.** The matched panel of clonal cell lines of any one of claims 1-14, wherein the cell lines in the panel express different proteins in a group of proteins of interest, wherein the groups of proteins of interest are selected from the group consisting of: proteins in the same signaling pathway, expression library of similar proteins, monoclonal antibody heavy chain library, monoclonal antibody light chain library and SNPs.

**16.** The matched panel of clonal cell lines of any one of claims 1-15, wherein the protein of interest is a single chain protein.

**17.** The matched panel of clonal cell lines of claim 16, wherein the single chain protein is a G protein coupled receptor.

**18.** The matched panel of clonal cell lines of claim 17, wherein the G protein coupled receptor is a taste receptor.

**19.** The matched panel of clonal cell lines of 18, wherein the taste receptor is selected from the group consisting of: a bitter taste receptor, a sweet taste receptor, and a umami taste receptor.

**20.** The matched panel of clonal cell lines of any one of claims 1-15, wherein the protein is a multimeric protein.

**21.** The matched panel of clonal cell lines of claim 20, wherein the protein is a heterodimer or a heteromultimer.

**22.** The matched panel of clonal cell lines of claim 20, wherein the protein is selected from the group consisting of: an ion channel, a G protein coupled receptor (GPCR), tyrosine receptor kinase, cytokine receptor, nuclear steroid hormone receptor and immunological receptor.

**23.** The matched panel of clonal cell lines of claim 22, wherein the protein is:

a) Epithelial sodium Channel (ENaC); or
b) voltage gated sodium channel (NaV).

**24.** The matched panel of clonal cell lines of claim 20, wherein the protein is selected from the group consisting of: gamma-aminobutyric acid A receptor (GABA $_A$ receptor), gamma-aminobutyric acid B receptor (GABA $_B$ receptor) and gamma-aminobutyric acid C receptor (GABA $_C$ receptor).

**25.** The matched panel of clonal cell lines of claim any one of claims 1-24, wherein the clonal cell lines in the panel were produced simultaneously, or within no more than 4 weeks of each other.

Europäisches Patentamt
European Patent Office
Office européen des brevets

# EUROPEAN SEARCH REPORT

Application Number
EP 15 18 0871

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YULI WANG ET AL: "Broadening cell selection criteria with micropallet arrays of adherent cells", CYTOMETRY PART A, vol. 71A, no. 10, 1 October 2007 (2007-10-01), pages 866-874, XP055087020, ISSN: 1552-4922, DOI: 10.1002/cyto.a.20424 * abstract * * page 872 - page 874 * * figures 1, 7, 8 * ----- | 1-25 | INV. C12N15/85 C12N15/67 C07K14/435 |
| X | SMALES C M ET AL: "Comparative proteomic analysis of GS-NSO murine myeloma cell lines with varying recombinant monoclonal antibody production rate", BIOTECHNOLOGY AND BIOENGINEERING, WILEY & SONS, HOBOKEN, NJ, US, vol. 88, no. 4, 20 November 2004 (2004-11-20), pages 474-488, XP002617633, ISSN: 0006-3592, DOI: 10.1002/BIT.20272 [retrieved on 2004-09-30] * abstract * * page 478 * * table 1 * ----- | 1-25 | TECHNICAL FIELDS SEARCHED (IPC) C12N C07K |
| X | STEFAN GRIMM: "The art and design of genetic screens: mammalian culture cells", NATURE REVIEWS GENETICS, vol. 5, no. 3, 1 March 2004 (2004-03-01), pages 179-189, XP055242819, GB ISSN: 1471-0056, DOI: 10.1038/nrg1291 | 1 | |
| A | * abstract * * figure 3 * ----- -/-- | 2-25 | |

The present search report has been drawn up for all claims

1

| Place of search | Date of completion of the search | Examiner |
|---|---|---|
| Munich | 20 January 2016 | Brero, Alessandro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 18 0871

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SEONG-HOON KO ET AL: "Modulation of Nav1.5 by [beta]1 and [beta]3-subunit co-expression in mammalian cells", PFLÜGERS ARCHIV - EUROPEAN JOURNAL OF PHYSIOLOGY, SPRINGER, BERLIN, DE, vol. 449, no. 4, 1 January 2005 (2005-01-01), pages 403-412, XP019343973, ISSN: 1432-2013, DOI: 10.1007/S00424-004-1348-4 * the whole document * ----- | 17-23 | |
| A | IVERSEN PHILIP W ET AL: "A comparison of assay performance measures in screening assays: signal window, Z' factor, and assay variability ratio", JOURNAL OF BIOMOLECULAR SCREENING, SAGE; LIEBERT, US, vol. 11, no. 3, 1 April 2006 (2006-04-01), pages 247-252, XP009110624, ISSN: 1087-0571 * the whole document * ----- | 2 | |
| | | | TECHNICAL FIELDS SEARCHED (IPC) |

1

The present search report has been drawn up for all claims

| Place of search | Date of completion of the search | Examiner |
|---|---|---|
| Munich | 20 January 2016 | Brero, Alessandro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 0931936 W
- US 6692965 B
- WO 2005079462 A

### Non-patent literature cited in the description


- Current Protocols in Molecular Biology. John Wiley & Sons, 1989, 6.3.1-6.3.6
- **ALTSHUL et al.** *J. Mol. Biol.,* 1990, vol. 215, 403-410
- **NEEDLEMAN et al.** *J. Mol. Biol.,* 1970, vol. 48, 444-453
- **MEYERS et al.** *Comput. Appl. Biosci.,* 1988, vol. 4, 11-17
- **E. MEYERS ; W. MILLER.** *CABIOS,* 1989, vol. 4, 11-17
- **PEARSON.** *Methods Enzymol.,* 1990, vol. 183, 63-98
- **PEARSON.** *Methods Mol. Biol.,* 2000, vol. 132, 185-219
- **PEARSON.** *Methods Mo!. Biol.,* 1994, vol. 243, 307-31
- **GONNET et al.** *Science,* 1992, vol. 256, 1443-45
- **ROBINSON et al.** *Biochemistry,* 1997, vol. 36 (37), 11169-11178
- **DEFFIE et al.** *Cancer Res.,* 1988, vol. 48 (13), 3595-3602
- **THIEBAUT et al.** *J Histochem Cytochem.,* 1990, vol. 38 (5), 685-690
- **ROEPE et al.** *Biochemistry,* 1993, vol. 32 (41), 11042-11056
- **SIMON et al.** *Proc Natl Acad Sci USA.,* 1994, vol. 91 (3), 1128-1132
- **SCHINDLER et al.** *Biochemistry,* 1996, vol. 35 (9), 2811-2817
- **ALTAN et al.** *J Exp Med.,* 1998, vol. 187 (10), 1583-1598
- **GILL et al.** *Cell,* 1992, vol. 71 (1), 23-32
- **ABRAHAM et al.** *Proc Natl Acad Sci U S A.,* 1993, vol. 90 (1), 312-316
- **ALTAN et al.** *Proc Natl Acad Sci USA.,* 1999, vol. 96 (8), 4432-4437
- **ZHANG JH ; CHUNG TD ; OLDENBURG KR.** A Simple Statistical Parameter for Use in Evaluation and Validation of High Throughput Screening Assays. *J. Biomol. Screen.,* 1999, vol. 4 (2), 67-73
- **FORTE et al.** *Endocr.,* 1999, vol. 140 (4), 1800-1806
- **ZHANG et al.** *J Biomol Screen,* 1999, vol. 4 (2), 67-73
- **SHEETS et al.** *J Physiol.,* 2007, vol. 581, 1019-1031